(19) Europäisches Patentamt
European Patent Office
Office européen des brevets

(11) **EP 4 471 025 A1**

(12) **EUROPEAN PATENT APPLICATION**
published in accordance with Art. 153(4) EPC

(43) Date of publication:
**04.12.2024 Bulletin 2024/49**

(21) Application number: **22923540.3**

(22) Date of filing: **15.12.2022**

(51) International Patent Classification (IPC):
***C07D 405/14*** *(2006.01)* ***C07D 405/12*** *(2006.01)*
***A61K 31/44*** *(2006.01)* ***A61P 35/00*** *(2006.01)*

(52) Cooperative Patent Classification (CPC):
**A61K 31/44; A61K 31/4433; A61K 31/4439; A61K 31/4545; A61K 31/536; A61K 31/5377; A61K 31/55; A61P 29/00; A61P 31/00; A61P 35/00; A61P 37/06; C07D 211/18; C07D 319/20; C07D 405/12; C07D 405/14;** (Cont.)

(86) International application number:
**PCT/CN2022/139254**

(87) International publication number:
**WO 2023/142754 (03.08.2023 Gazette 2023/31)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **27.01.2022 CN 202210105673**

(71) Applicant: **Jiangsu Tasly Diyi Pharmaceutical Co., Ltd.**
**Huai'an, Jiangsu 223003 (CN)**

(72) Inventors:
- **HAN, Kailin**
  **Huai'an, Jiangsu 223003 (CN)**
- **MA, Xiaohui**
  **Huai'an, Jiangsu 223003 (CN)**
- **LI, Jiawen**
  **Huai'an, Jiangsu 223003 (CN)**
- **WAN, Zhifu**
  **Huai'an, Jiangsu 223003 (CN)**
- **WANG, Xiaotao**
  **Huai'an, Jiangsu 223003 (CN)**
- **DONG, Liming**
  **Huai'an, Jiangsu 223003 (CN)**
- **TANG, Hai**
  **Huai'an, Jiangsu 223003 (CN)**
- **ZHOU, Shuiping**
  **Huai'an, Jiangsu 223003 (CN)**
- **CAI, Jinyong**
  **Huai'an, Jiangsu 223003 (CN)**

(74) Representative: **Gulde & Partner**
**Patent- und Rechtsanwaltskanzlei mbB**
**Wallstraße 58/59**
**10179 Berlin (DE)**

(54) **EZH1/2 INHIBITOR, PREPARATION THEREOF, AND USE THEREOF IN ANTI-TUMOR THERAPY**

(57) The present invention relates to an EZH1/2 inhibitor, a preparation thereof, and a use thereof in anti-tumor therapy, specifically comprising a compound represented by formula I or formula II, or a deuterated compound, or a stereoisomer, or a pharmaceutically acceptable salt thereof, and further comprising a pharmaceutical composition of the compound, and a use of the foregoing as an EZH1/2 inhibitor in the preparation of medication for treating related diseases.

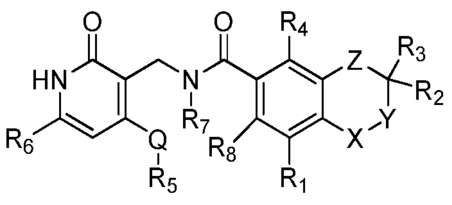

Formula I

or

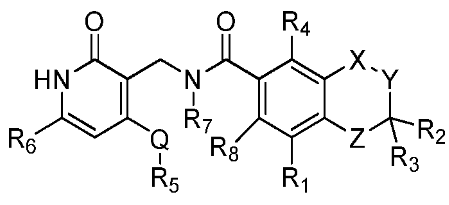

Formula II



EP 4 471 025 A1

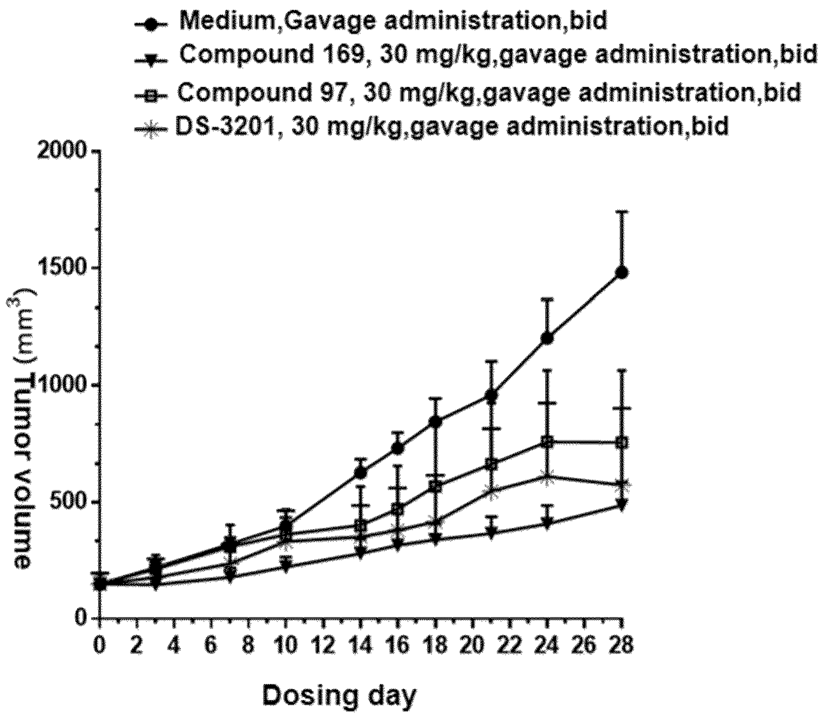


Figure 4

(52) Cooperative Patent Classification (CPC): (Cont.)
**C07D 407/04; C07D 407/12; C07D 413/12; C07D 413/14; C07D 491/048; C07D 491/056; C07D 491/107; C07D 498/04;** Y02P 20/55

## Description

## TECHNICAL FIELD

The present invention is related to the medicinal field, in particular, is related to a compound having EZH1/2 inhibitory activity, or a deuterated compound thereof, or a stereoisomer thereof, pharmaceutically acceptable salt, and its use thereof in the preparation of a medicament for used in the treatment of relevant diseases.

## BACKGROUND ART

Cancer treatment methods currently mainly include radiation therapy, surgery therapy, and drug therapy. Drug therapy targeting lesions has become the main means of clinical tumor treatment today. However, due to the rapid emergence of drug resistance in tumor cells, people are basically helpless about tumor metastasis and recurrence at this stage.

Lysine methyltransferase can methylate histones and non-histones, and its abnormal expression is closely related to the occurrence of various tumors. It has become a hot spot in the field of epigenetics for more than ten years. Targeting lysine methyltransferases to reverse abnormal histone or non-histone methylation levels is regarded as another new approach for cancer treatment. PRC2 (polycomb repressive complex 2) is a multi-subunit protein complex composed of EZH1 (Enhancer of zeste homologue 1, KMT6B) or EZH2 (Enhancer of zeste homologue 2, KMT6A), SUZ12 (Suppressor of zeste 12), EED (Embyronic ectoderm development), used to catalyze H3K27 trimethylation. The PCR2 complex methylates the nucleosomal protein H3K9 and lysine 27 through the SET domain of EZH2, and then triggers the aggregation of the PCR1 complex at specific gene sites to silence target genes (CDKN1C, CDH1, RUNX3, etc.), promote cell proliferation. Studies have shown that EZH2 overexpression or SET region mutation (Y641F, Y641N, A687V, A677G point mutation) can all lead to abnormal increase of H3K27me3, promote the growth and development of various types of tumors, such as breast cancer, prostate cancer, leukemia and the like.

However, the activity of EZH1/2 inhibitors in the prior art is still not good enough, and a compound with better activity is urgently needed.

## SUMMARY OF THE INVENTION

The purpose of the present invention is to provide a new small molecule inhibitor targeting EZH1/2.

In the first embodiment of the present invention, the present invention provides a compound represented by Formula I or Formula II, or a deuterated compound thereof, or a stereoisomer thereof, or a pharmaceutically acceptable salt thereof

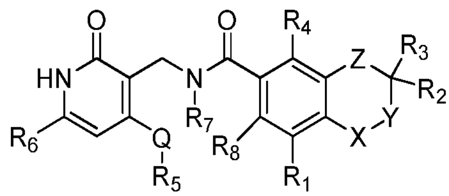

Formula I

or

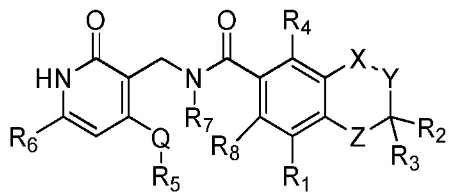

Formula II

wherein,

X is selected from a group consisting of single bond, O, $NR^{X1}$ or $CHR^{X2}$,
Y is selected from a group consisting of O, S, $NR^{Y1}$ or $CHR^{Y2}$;
Z is selected from a group consisting of C=O, O, S, $NR^{Z1}$ or $CHR^{Z2}$;
Q is selected from a group consisting of single bond, O or S;
when X is single bond, Y and Z are not both O;
$R^{X1}$ is selected from a group consisting of the following groups: hydrogen, -$C_{1\sim6}$alkyl, halo-$C_{1\sim6}$alkyl, -$C_{0\sim2}$alkylene-(3~10 membered cycloalkyl), -$C_{0\sim2}$alkylene-(3~10 membered heterocycloalkyl), -$C_{0\sim2}$alkylene-(5~10 membered aryl ring), -$C_{0\sim2}$alkylene-(5~10 membered heteroaryl ring);
$R^{X2}$ is selected from a group consisting of the following groups: hydrogen, halogen, cyano, hydroxyl, -$C_{1\sim6}$alkyl, halo-$C_{1\sim6}$alkyl, -O($C_{1\sim6}$alkyl), -O(halo-$C_{1\sim6}$alkyl), -$NH_2$, -NH($C_{1\sim6}$alkyl), -N($C_{1\sim6}$alkyl) ($C_{1\sim6}$alkyl), -$C_{0\sim2}$alkylene-(3~10 membered cycloalkyl), -$C_{0\sim2}$alkylene-(3~10 membered heterocycloalkyl), -$C_{0\sim2}$alkylene-(5~10 membered aryl ring), -$C_{0\sim2}$alkylene-(5~10 membered heteroaryl ring);

$R^{Y1}$ is selected from a group consisting of the following groups: hydrogen, $-C_{1\sim6}$alkyl, halo-$C_{1\sim6}$alkyl, $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring);

$R^{Y2}$ is selected from a group consisting of the following groups: hydrogen, halogen, cyano, hydroxyl, $-C_{1\sim6}$alkyl, halo-$C_{1\sim6}$alkyl, $-O(C_{1\sim6}$alkyl), -O(halo-$C_{1\sim6}$alkyl), $-NH_2$, $-NH(C_{1\sim6}$alkyl), $-N(C_{1\sim6}$alkyl) ($C_{1\sim6}$alkyl), $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring);

$R^{Z1}$ is selected from a group consisting of the following groups: hydrogen, $C_{1\sim6}$alkyl, halo-$C_{1\sim6}$alkyl, $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring);

$R^{Z2}$ is selected from a group consisting of the following groups: hydrogen, halogen, cyano, hydroxyl, $-C_{1\sim6}$alkyl, halo-$C_{1\sim6}$alkyl, $-O(C_{1\sim6}$alkyl), -O(halo-$C_{1\sim6}$alkyl), $-NH_2$, $-NH(C_{1\sim6}$alkyl), $-N(C_{1\sim6}$alkyl) ($C_{1\sim6}$alkyl), $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring);

Ri is selected from a group consisting of the following groups: hydrogen, halogen, cyano, nitro, amino, hydroxyl, $-C_{1\sim6}$alkyl, $-O(C_{1\sim6}$alkyl), halo-$C_{1\sim6}$alkyl, $-C_{0\sim2}$alkylene-$OR^{1b}$, $-C_{0\sim2}$alkylene-$C(O)R^{1b}$, $-C_{0\sim2}$alkylene-$C(O)NR^{1b}R^{1c}$, $-C_{0\sim2}$alkylene-$NR^{1b}R^{1c}$, $-C_{0\sim2}$alkylene-$NR^{1b}C(O)R^{1c}$, $-C_{0\sim4}$alkylene-$S(O)_2R^{1b}$, $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring); wherein said alkyl, alkylene, cycloalkyl, heterocycloalkyl, aryl ring, heteroaryl ring is optionally further substituted by one, two or three independent $R^{1b}$;

$R^{1b}$, $R^{1c}$ is each independently selected from a group consisting of the following groups: hydrogen, -OH, $C_{1\sim6}$alkyl, halo-$C_{1\sim6}$alkyl, $-O(C_{1\sim6}$alkyl), -O(halo-$C_{1\sim6}$alkyl), $-NH_2$, $-NH(C_{1\sim6}$alkyl), $-N(C_{1\sim6}$alkyl) ($C_{1\sim6}$alkyl), $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring);

$R_2$ is selected from a group consisting of the following groups: $-C_{0\sim2}$alkylene-(3~10 membered cycloalkyl), $-C_{0\sim2}$alkylene-(3~10 membered heterocycloalkyl); wherein said alkylene, cycloalkyl, heterocycloalkyl is optionally further substituted by one, two or three independent $R^{2b}$;

$R^{2b}$ is each independently selected from a group consisting of the following groups: hydrogen, halogen, cyano, nitro, amino, hydroxyl, $-C_{1\sim6}$alkyl, $-O(C_{1\sim6}$alkyl), halo-$C_{1\sim6}$alkyl, $-C_{0\sim2}$alkylene-$OR^{2c}$, $-C_{0\sim2}$alkylene-$C(O)R^{2c}$, $-C_{0\sim2}$alkylene-$C(O)NR^{2c}R^{2d}$, $-C_{0\sim2}$alkylene-$NR^{2c}R^{2d}$, $-C_{0\sim2}$alkylene-$NR^{2c}C(O)R^{2d}$, $-C_{0\sim4}$alkylene-$S(O)_2R^{2c}$, $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring); wherein alkyl, alkylene, cycloalkyl, heterocycloalkyl, aryl ring, heteroaryl ring is optionally further substituted by one, two or three independent $R^{2c}$;

$R^{2c}$, $R^{2d}$ is each independently selected from a group consisting of hydrogen, cyano, nitro, -OH, halogen, $-C_{1\sim6}$alkyl, halo-$C_{1\sim6}$alkyl, $-C_{0\sim2}$alkylene-$O(C_{1\sim6}$alkyl), -O(halo-$C_{1\sim6}$alkyl), -O(3~10 membered cycloalkyl), -O(3~10 membered heterocycloalkyl), $-NH_2$, $-NH(C_{1\sim6}$alkyl), $-N(C_{1\sim6}$alkyl) ($C_{1\sim6}$alkyl), $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring); wherein said alkyl, alkylene, cycloalkyl, heterocycloalkyl, aryl ring, heteroaryl ring is optionally further substituted by one, two or three independent $R^{2e}$;

$R^{2e}$ is each independently selected from a group consisting of hydrogen, cyano, nitro, -OH, halogen, $-C_{1\sim6}$alkyl, halo-$C_{1\sim6}$alkyl, $-C_{0\sim2}$alkylene-$O(C_{1\sim6}$alkyl), -O(halo-$C_{1\sim6}$alkyl), -O(3~10 membered cycloalkyl), -O(3~10 membered heterocycloalkyl), $-NH_2$, $-NH(C_{1\sim6}$alkyl), $-N(C_{1\sim6}$alkyl) ($C_{1\sim6}$alkyl), $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring);

$R_3$ is selected from a group consisting of the following groups: hydrogen, halogen, cyano, nitro, amino, -OH, $-C_{1\sim6}$alkyl, $-O(C_{1\sim6}$alkyl), halo-$C_{1\sim6}$alkyl, $-C_{0\sim2}$alkylene-$OR^{3b}$, $-C_{0\sim2}$alkylene-$C(O)R^{3b}$, $-C_{0\sim2}$alkylene-$C(O)NR^{3b}R^{3c}$, $-C_{0\sim2}$alkylene-$NR^{3b}R^{3c}$, $-C_{0\sim2}$alkylene-$NR^{3b}C(O)R^{3c}$, $-C_{0\sim4}$alkylene-$S(O)_2R^{3b}$, $-C_{0\sim2}$alkylene-(3~10 membered cycloalkyl), $-C_{0\sim2}$alkylene-(3~10 membered heterocycloalkyl);

$R^{3b}$, $R^{3c}$ is each independently selected from a group consisting of the following groups: hydrogen, halogen, cyano, nitro, -OH, $C_{1\sim6}$alkyl, halo-$C_{1\sim6}$alkyl, $-O(C_{1\sim6}$alkyl), -O(halo-$C_{1\sim6}$alkyl), $-NH_2$, $-NH(C_{1\sim6}$alkyl), $-N(C_{1\sim6}$alkyl) ($C_{1\sim6}$alkyl), $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring);

$R_4$ is selected from a group consisting of the following groups: hydrogen, halogen, cyano, nitro, hydroxyl, $-C_{1\sim6}$alkyl, halo-$C_{1\sim6}$alkyl, $-O(C_{1\sim6}$alkyl), -O(halo-$C_{1\sim6}$alkyl), $-NH_2$, $-NH(C_{1\sim6}$alkyl), $-N(C_{1\sim6}$alkyl) ($C_{1\sim6}$alkyl), $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring); wherein said alkyl, alkylene, cycloalkyl, heterocycloalkyl, aryl ring, heteroaryl ring is optionally further substituted by one, two or three independent $R^{4b}$;

$R^{4b}$ is each independently selected from a group consisting of the following groups: hydrogen, halogen, cyano, nitro,

-OH, $C_{1\sim6}$alkyl, halo-$C_{1\sim6}$alkyl, -O($C_{1\sim6}$alkyl), -O(halo-$C_{1\sim6}$alkyl), $-NH_2$, -NH($C_{1\sim6}$alkyl), -N($C_{1\sim6}$alkyl) ($C_{1\sim6}$alkyl), -$C_{0\sim2}$alkylene-(3~10 membered cycloalkyl), -$C_{0\sim2}$alkylene-(3~10 membered heterocycloalkyl), -$C_{0\sim2}$alkylene-(5~10 membered aryl ring), -$C_{0\sim2}$alkylene-(5~10 membered heteroaryl ring);

Rs is selected from a group consisting of the following groups: hydrogen, halogen, cyano, nitro, hydroxyl, -$C_{1\sim6}$alkyl, halo-$C_{1\sim6}$alkyl, -O($C_{1\sim6}$alkyl), -O(halo-$C_{1\sim6}$alkyl), $-NH_2$, -NH($C_{1\sim6}$alkyl), -N($C_{1\sim6}$alkyl) ($C_{1\sim6}$alkyl), -$C_{0\sim2}$alkylene-(3~10 membered cycloalkyl), -$C_{0\sim2}$alkylene-(3~10 membered heterocycloalkyl), -$C_{0\sim2}$alkylene-(5~10 membered aryl ring), -$C_{0\sim2}$alkylene-(5~10 membered heteroaryl ring);

$R_6$ is selected from a group consisting of the following groups: hydrogen, halogen, cyano, nitro, hydroxyl, -$C_{1\sim6}$alkyl, halo-$C_{1\sim6}$alkyl, -O($C_{1\sim6}$alkyl), -O(halo-$C_{1\sim6}$alkyl), $-NH_2$, -NH($C_{1\sim6}$alkyl), -N($C_{1\sim6}$alkyl) ($C_{1\sim6}$alkyl), -$C_{0\sim2}$alkylene-(3~10 membered cycloalkyl), -$C_{0\sim2}$alkylene-(3~10 membered heterocycloalkyl), -$C_{0\sim2}$alkylene-(5~10 membered aryl ring), -$C_{0\sim2}$alkylene-(5~10 membered heteroaryl ring);

$R_7$ is selected from a group consisting of the following groups: hydrogen, -$C_{1\sim6}$alkyl, halo-$C_{1\sim6}$alkyl, -$C_{0\sim2}$alkylene-(3~10 membered cycloalkyl), -$C_{0\sim2}$alkylene-(3~10 membered heterocycloalkyl), -$C_{0\sim2}$alkylene-(5~10 membered aryl ring), -$C_{0\sim2}$alkylene-(5~10 membered heteroaryl ring);

Rs is selected from a group consisting of the following groups: hydrogen, halogen, cyano, nitro, -OH, Ciealkyl, halo-$C_{1\sim6}$alkyl, -O($C_{1\sim6}$alkyl), -O(halo-$C_{1\sim6}$alkyl), $-NH_2$, -NH($C_{1\sim6}$alkyl), -N($C_{1\sim6}$alkyl) ($C_{1\sim6}$alkyl), -$C_{0\sim2}$alkylene-(3~10 membered cycloalkyl), -$C_{0\sim2}$alkylene-(3~10 membered heterocycloalkyl), -$C_{0\sim2}$alkylene-(5~10 membered aryl ring), -$C_{0\sim2}$alkylene-(5~10 membered heteroaryl ring);

or, $R^7$, $R^8$ and the atom directly bound thereto form a 4~10 membered heterocyclic ring; wherein said heterocyclic ring is optionally further substituted by one, two or three independent $R^{71}$;

each $R^{71}$ is each independently selected from a group consisting of the following groups: hydrogen, halogen, cyano, nitro, -OH, $C_{1\sim6}$alkyl, halo-$C_{1\sim6}$alkyl, -O($C_{1\sim6}$alkyl), -O(halo-$C_{1\sim6}$alkyl), $-NH_2$, -NH($C_{1\sim6}$alkyl), -N($C_{1\sim6}$alkyl) ($C_{1\sim6}$alkyl), -$C_{0\sim2}$alkylene-(3~10 membered cycloalkyl), -$C_{0\sim2}$alkylene-(3~10 membered heterocycloalkyl), -$C_{0\sim2}$alkylene-(5~10 membered aryl ring), -$C_{0\sim2}$alkylene-(5~10 membered heteroaryl ring).

In the first embodiment of the present invention, it provides the compound of the present invention, or a deuterated compound thereof, or a stereoisomer thereof, or a pharmaceutically acceptable salt thereof, wherein said $R_1$, $R_3$, $R_4$, $R_5$, $R_6$, $R_7$, $R_8$ is each independently selected from a group consisting of hydrogen, halogen, -$C_{1\sim3}$alkyl, halo-$C_{1\sim3}$alkyl.

In the first embodiment of the present invention, it provides the compound of the present invention, or a deuterated compound thereof, or a stereoisomer thereof, or a pharmaceutically acceptable salt thereof, wherein said $R_1$, $R_3$, $R_4$, $R_5$, $R_6$, $R_7$, $R_8$ is each independently selected from a group consisting of hydrogen, Cl, methyl, ethyl, $-CF_3$.

In the first embodiment of the present invention, it provides the compound of the present invention, or a deuterated compound thereof, or a stereoisomer thereof, or a pharmaceutically acceptable salt thereof, wherein said $R_2$ is selected from a group consisting of -(3-10 membered cycloalkyl), -(3-10 membered heterocycloalkyl); wherein said cycloalkyl, heterocycloalkyl is optionally further substituted by one, two or three independent $R^{2b}$;

$R^{2b}$ is selected from a group consisting of hydrogen, halogen, -$C_{1\sim6}$alkyl, $-NR^{2c}R^{2d}$, $-NR^{2c}C(O)R^{2d}$, -$C_{0\sim2}$alkylene-(3~10 membered cycloalkyl), -$C_{0\sim2}$alkylene-(3~10 membered heterocycloalkyl); wherein said alkyl, cycloalkyl, heterocycloalkyl is optionally further substituted by one, two or three independent $R^{2c}$;

$R^{2c}$, $R^{2d}$ is each independently selected from a group consisting of hydrogen, halogen, -$C_{1\sim6}$alkyl, halo-$C_{1\sim6}$alkyl, -O($C_{1\sim6}$alkyl), -O(halo-$C_{1\sim6}$alkyl), -O(3~10 membered cycloalkyl), -(3-10 membered cycloalkyl), -(3-10 membered heterocycloalkyl); wherein said alkyl, cycloalkyl, heterocycloalkyl is optionally further substituted by one, two or three independent $R^{2e}$,

$R^{2e}$ is each independently selected from a group consisting of hydrogen, halogen, -O($C_{1\sim6}$alkyl).

In the first embodiment of the present invention, it provides the compound of the present invention, or a deuterated compound thereof, or a stereoisomer thereof, or a pharmaceutically acceptable salt thereof, wherein said $R_2$ is selected from a group consisting of

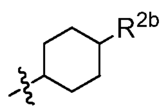

or

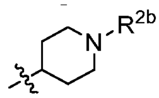

In the first embodiment of the present invention, it provides the compound of the present invention, or a deuterated compound thereof, or a stereoisomer thereof, or a pharmaceutically acceptable salt thereof, wherein said $R_2$ is specifically selected from a group consisting of

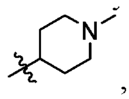,

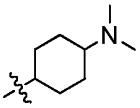, 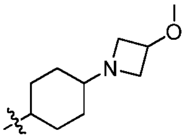, 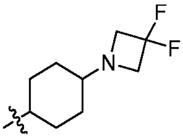, 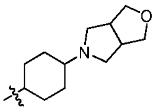, 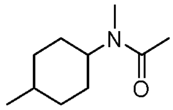, 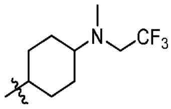,

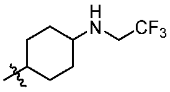, 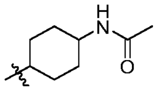, 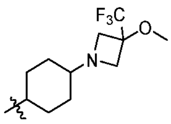, 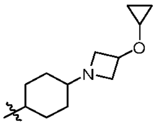, 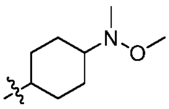, 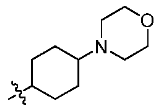,

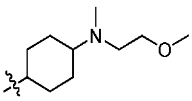, 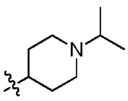, 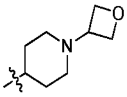, 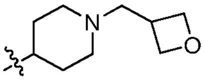, 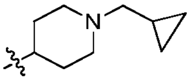, 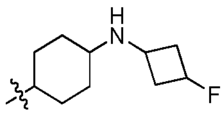;

preferably, said $R_2$ is specifically selected from a group consisting of

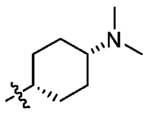, 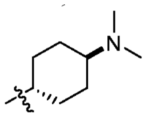,

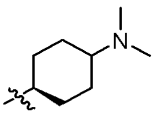, 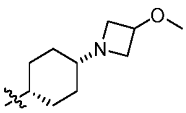, 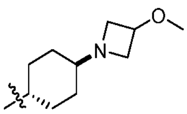, 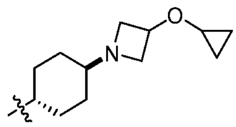, 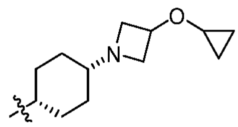,

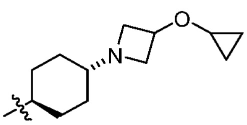, 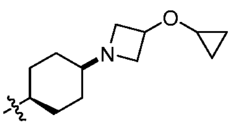, 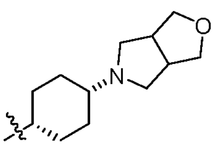, 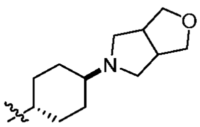, 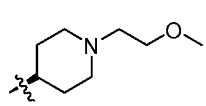,

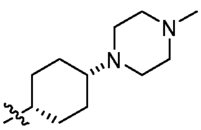, 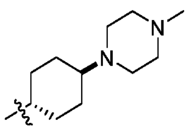, 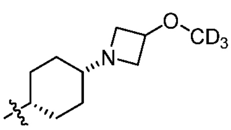, 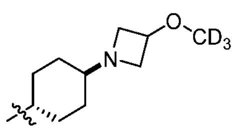, 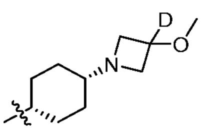,

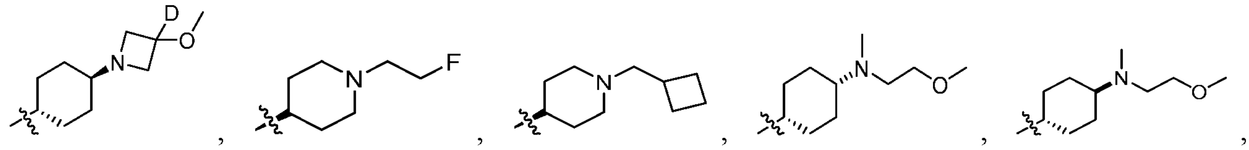
, , , , ,

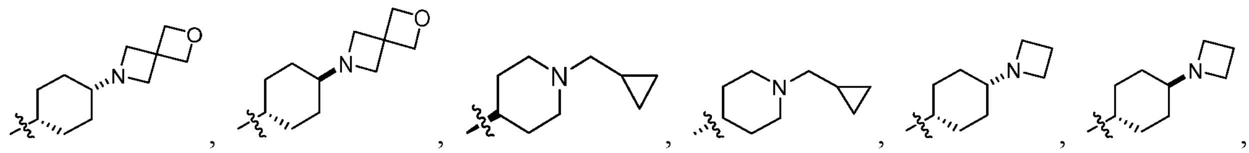
, , , , , ,

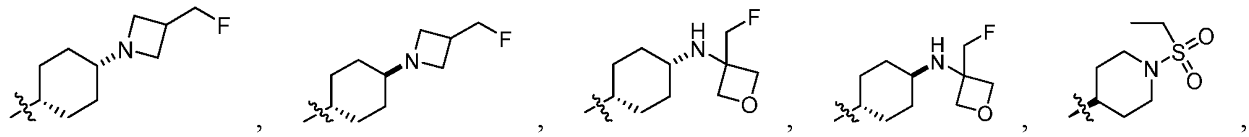
, , , , ,

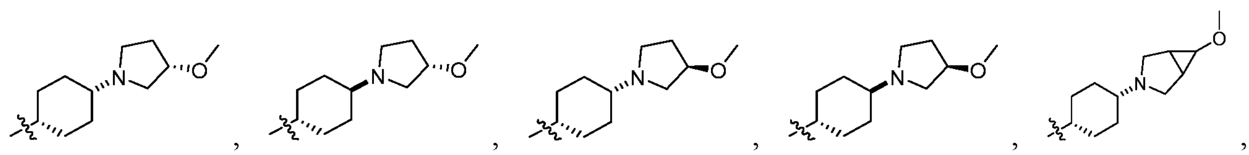
, , , , ,

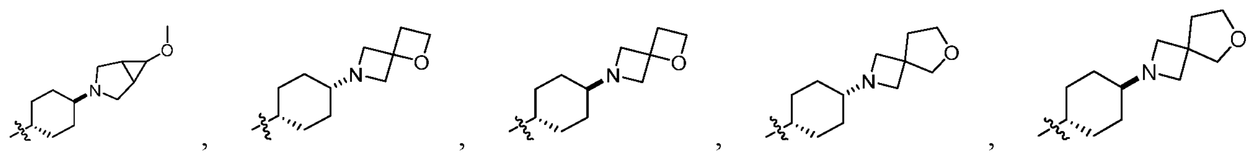
, , , ,

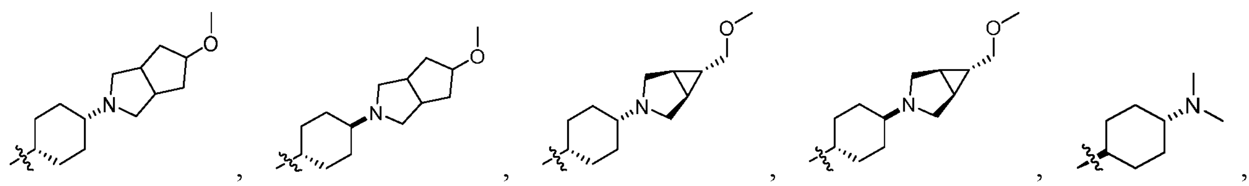
, , , , ,

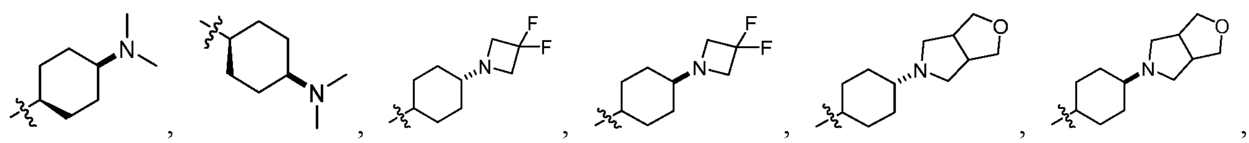
, , , , , ,

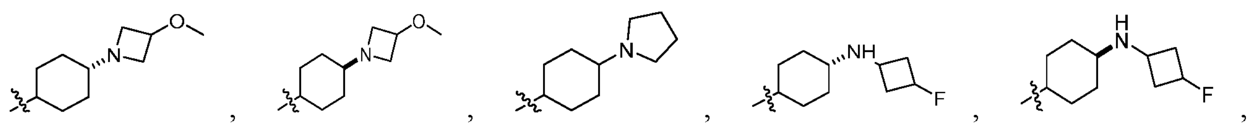
, , , , ,

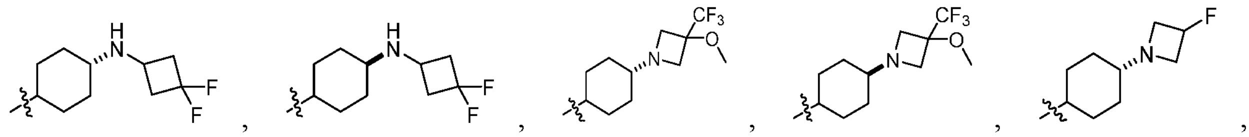
, , , , ,

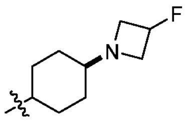, 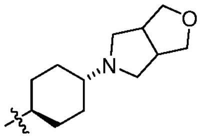, 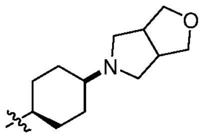, 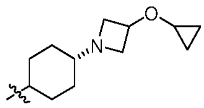, 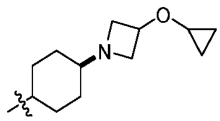,

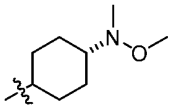, 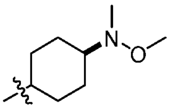, 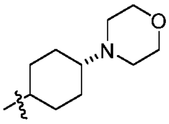, 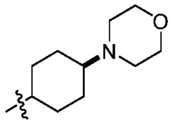, 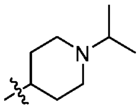, 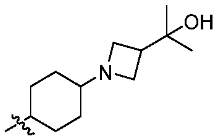,

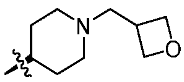, 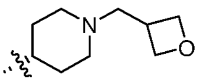, 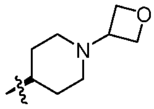, 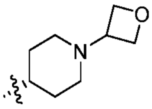, 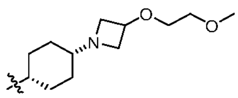, 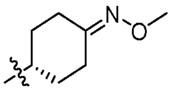,

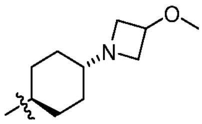, 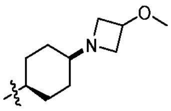, 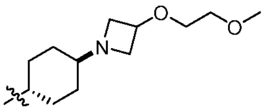, 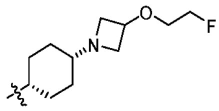, 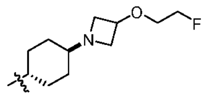,

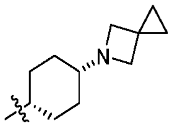, 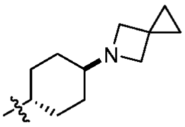, 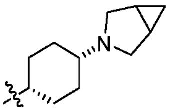, 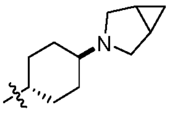, 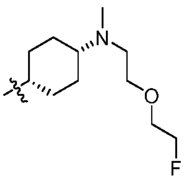, 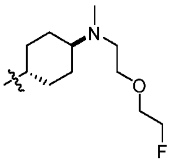,

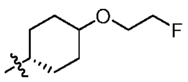, 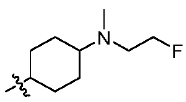, 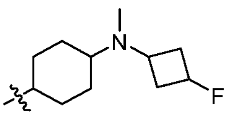, 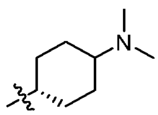, 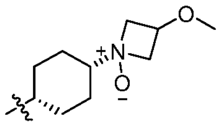, 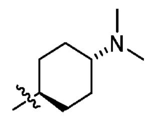,

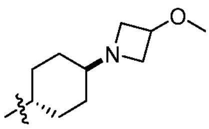, 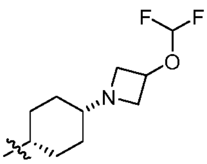, 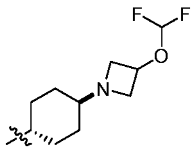, 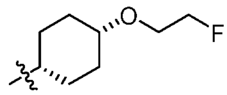, 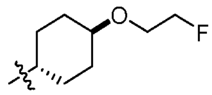

In the first embodiment of the present invention, it provides the compound of the present invention, or a deuterated compound thereof, or a stereoisomer thereof, or a pharmaceutically acceptable salt thereof, wherein, X is selected from a group consisting of single bond, O, $CHR^{X2}$, Y is selected from a group consisting of O, $NR^{Y1}$ or $CHR^{Y2}$; Z is selected from a group consisting of -C=O, O, $NR^{Z1}$ or $CHR^{Z2}$; Q is selected from a group consisting of single bond, O or S;

$R^{X2}$ is selected from a group consisting of hydrogen, -$C_{1\sim3}$alkyl;
$R^{Y1}$ is selected from a group consisting of hydrogen, -$C_{1\sim3}$alkyl, halo-$C_{1\sim3}$alkyl;
$R^{Y2}$ is selected from a group consisting of hydrogen, -$C_{1\sim3}$alkyl, halo-$C_{1\sim3}$alkyl;
$R^{Z1}$ is selected from a group consisting of hydrogen, -$C_{1\sim3}$alkyl, -(3~6 membered cycloalkyl), halo-$C_{1\sim3}$alkyl;
$R^{Z2}$ is selected from a group consisting of hydrogen, -$C_{1\sim3}$alkyl, halo-$C_{1\sim3}$alkyl.

In the first embodiment of the present invention, it provides the compound of the present invention, or a deuterated compound thereof, or a stereoisomer thereof, or a pharmaceutically acceptable salt thereof, wherein said $R^{Y1}$ is selected from a group consisting of methyl; $R^{Y2}$ is selected from a group consisting of hydrogen, methyl; $R^{Z1}$ is selected from a group consisting of hydrogen, methyl, ethyl, cyclopropyl.

In the second embodiment of the present invention, it provides the compound of the present invention, or a deuterated compound thereof, or a stereoisomer thereof, or a pharmaceutically acceptable salt thereof, including the following compounds represented by Formula IIa:

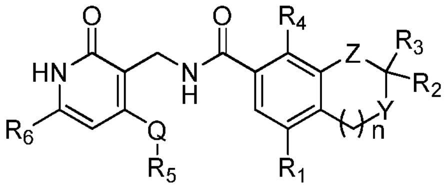

Formula IIa

Q is selected from a group consisting of single bond, O or S;

Z is selected from a group consisting of C=O, O, $NR^{Z1}$ or $CHR^{Z2}$;
Y is selected from a group consisting of O, $NR^{Y1}$ or $CHR^{Y2}$; n is 0 or 1;
when n is 0, Y and Z are not both O;

$R^{Y1}$ is selected from a group consisting of hydrogen, $-C_{1\sim6}$alkyl, halo-$C_{1\sim6}$alkyl, $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring);

$R^{Y2}$ is selected from a group consisting of hydrogen, halogen, cyano, hydroxyl, $-C_{1\sim6}$alkyl, halo-$C_{1\sim6}$alkyl, -O($C_{1\sim6}$alkyl), -O(halo-$C_{1\sim6}$alkyl), $-NH_2$, -NH($C_{1\sim6}$alkyl), -N($C_{1\sim6}$alkyl) ($C_{1\sim6}$alkyl), $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring);

$R^{Z1}$ is selected from a group consisting of hydrogen, $-C_{1\sim6}$alkyl, halo-$C_{1\sim6}$alkyl, $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring);

$R^{Z2}$ is selected from a group consisting of hydrogen, halogen, cyano, hydroxyl, $-C_{1\sim6}$alkyl, halo-$C_{1\sim6}$alkyl, -O($C_{1\sim6}$alkyl), -O(halo-$C_{1\sim6}$alkyl), $-NH_2$, -NH($C_{1\sim6}$alkyl), -N($C_{1\sim6}$alkyl) ($C_{1\sim6}$alkyl), $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring);

$R_1$ is selected from a group consisting of hydrogen, halogen, cyano, nitro, amino, hydroxyl, $-C_{1\sim6}$alkyl, halo-$C_{1\sim6}$alkyl, $-C_{0\sim2}$alkylene-$OR^{1b}$, $-C_{0\sim2}$alkylene-C(O)$R^{1b}$, $-C_{0\sim2}$alkylene-C(O)$NR^{1b}R^{1c}$, $-C_{0\sim2}$alkylene-$NR^{1b}R^{1c}$, $-C_{0\sim2}$alkylene-$NR^{1b}C(O)R^{1c}$, $-C_{0\sim4}$alkylene-$S(O)_2R^{1b}$, $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring); wherein said alkyl, alkylene, cycloalkyl, heterocycloalkyl, aryl ring, heteroaryl ring is optionally further substituted by one, two or three independent $R^{1b}$;

$R^{1b}$, $R^{1c}$ is each independently selected from a group consisting of hydrogen, -OH, $C_{1\sim6}$alkyl, halo-$C_{1\sim6}$alkyl, -O($C_{1\sim6}$alkyl), -O(halo-$C_{1\sim6}$alkyl), $-NH_2$, -NH($C_{1\sim6}$alkyl), -N($C_{1\sim6}$alkyl) ($C_{1\sim6}$alkyl), $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring);

$R_2$ is selected from a group consisting of $-C_{0\sim2}$alkylene-(3~10 membered cycloalkyl), $-C_{0\sim2}$alkylene-(3~10 membered heterocycloalkyl); wherein said alkylene, cycloalkyl, heterocycloalkyl is optionally further substituted by one, two or three independent $R^{2b}$;

$R^{2b}$ is each independently selected from a group consisting of hydrogen, halogen, cyano, nitro, amino, hydroxyl, $-C_{1\sim6}$alkyl, -O($C_{1\sim6}$alkyl), halo-$C_{1\sim6}$alkyl, $-C_{0\sim2}$alkylene-$OR^{2c}$, $-C_{0\sim2}$alkylene-C(O)$R^{2c}$, $-C_{0\sim2}$alkylene-C(O)$NR^{2c}R^{2d}$, $-C_{0\sim2}$alkylene-$NR^{2c}R^{2d}$, $-C_{0\sim2}$alkylene-$NR^{2c}C(O)R^{2d}$, $-C_{0\sim4}$alkylene-$S(O)_2R^{2c}$, $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring); wherein alkyl, alkylene, cycloalkyl, heterocycloalkyl, aryl ring, heteroaryl ring is optionally further substituted by one, two or three independent $R^{2c}$;

$R^{2c}$, $R^{2d}$ is each independently selected from a group consisting of hydrogen, cyano, nitro, -OH, halogen, $-C_{1\sim6}$alkyl, halo-$C_{1\sim6}$alkyl, $-C_{0\sim2}$alkylene-O($C_{1\sim6}$alkyl), -O(halo-$C_{1\sim6}$alkyl), -O(3~10 membered cycloalkyl), -O(3~10 membered heterocycloalkyl), $-NH_2$, -NH($C_{1\sim6}$alkyl), -N($C_{1\sim6}$alkyl) ($C_{1\sim6}$alkyl), $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring); wherein said alkyl, alkylene, cycloalkyl, heterocycloalkyl, aryl ring, heteroaryl ring is optionally further substituted by one, two or three independent $R^{2e}$;

$R^{2e}$ is each independently selected from a group consisting of hydrogen, cyano, nitro, -OH, halogen, -$C_{1\sim6}$alkyl, halo-$C_{1\sim6}$alkyl, -$C_{0\sim2}$alkylene-O($C_{1\sim6}$alkyl), -O(halo-$C_{1\sim6}$alkyl), -O(3~10 membered cycloalkyl), -O(3~10 membered heterocycloalkyl), -$NH_2$, -NH($C_{1\sim6}$alkyl), -N($C_{1\sim6}$alkyl) ($C_{1\sim6}$alkyl), -$C_{0\sim2}$alkylene-(3~10 membered cycloalkyl), -$C_{0\sim2}$alkylene-(3~10 membered heterocycloalkyl), -$C_{0\sim2}$alkylene-(5~10 membered aryl ring), -$C_{0\sim2}$alkylene-(5~10 membered heteroaryl ring);

$R_3$ is selected from a group consisting of hydrogen, halogen, cyano, nitro, amino, -OH, -$C_{1\sim6}$alkyl, -O($C_{1\sim6}$alkyl), halo-$C_{1\sim6}$alkyl, -$C_{0\sim2}$alkylene-$OR^{3b}$, -$C_{0\sim2}$alkylene-C(O)$R^{3b}$, -$C_{0\sim2}$alkylene-C(O)$NR^{3b}R^{3c}$, -$C_{0\sim2}$alkylene-$NR^{3b}R^{3c}$, -$C_{0\sim2}$alkylene-$NR^{3b}C(O)R^{3c}$, -$C_{0\sim4}$alkylene-$S(O)_2R^{3b}$, -$C_{0\sim2}$alkylene-(3~10 membered cycloalkyl), -$C_{0\sim2}$alkylene-(3~10 membered heterocycloalkyl);

$R^{3b}$, $R^{3c}$ is each independently selected from a group consisting of hydrogen, halogen, cyano, nitro, -OH, $C_{1\sim6}$alkyl, halo-$C_{1\sim6}$alkyl, -O($C_{1\sim6}$alkyl), -O(halo-$C_{1\sim6}$alkyl), -$NH_2$, -NH($C_{1\sim6}$alkyl), -N($C_{1\sim6}$alkyl) ($C_{1\sim6}$alkyl), -$C_{0\sim2}$alkylene-(3~10 membered cycloalkyl), -$C_{0\sim2}$alkylene-(3~10 membered heterocycloalkyl), -$C_{0\sim2}$alkylene-(5~10 membered aryl ring), -$C_{0\sim2}$alkylene-(5~10 membered heteroaryl ring);

$R_4$ is selected from a group consisting of hydrogen, halogen, cyano, nitro, hydroxyl, -$C_{1\sim6}$alkyl, halo-$C_{1\sim6}$alkyl, -O($C_{1\sim6}$alkyl), -O(halo-$C_{1\sim6}$alkyl), -$NH_2$, -NH($C_{1\sim6}$alkyl), -N($C_{1\sim6}$alkyl) ($C_{1\sim6}$alkyl), -$C_{0\sim2}$alkylene-(3~10 membered cycloalkyl), -$C_{0\sim2}$alkylene-(3~10 membered heterocycloalkyl), -$C_{0\sim2}$alkylene-(5~10 membered aryl ring), -$C_{0\sim2}$alkylene-(5~10 membered heteroaryl ring); wherein said alkyl, alkylene, cycloalkyl, heterocycloalkyl, aryl ring, heteroaryl ring is optionally further substituted by one, two or three independent $R^{4b}$;

$R^{4b}$ is each independently selected from a group consisting of hydrogen, halogen, cyano, nitro, -OH, Ciealkyl, halo-$C_{1\sim6}$alkyl, -O($C_{1\sim6}$alkyl), -O(halo-$C_{1\sim6}$alkyl), -$NH_2$, -NH($C_{1\sim6}$alkyl), -N($C_{1\sim6}$alkyl) ($C_{1\sim6}$alkyl), -$C_{0\sim2}$alkylene-(3~10 membered cycloalkyl), -$C_{0\sim2}$alkylene-(3~10 membered heterocycloalkyl), -$C_{0\sim2}$alkylene-(5~10 membered aryl ring), -$C_{0\sim2}$alkylene-(5~10 membered heteroaryl ring);

Rs is selected from a group consisting of hydrogen, halogen, cyano, nitro, hydroxyl, -$C_{1\sim6}$alkyl, halo-$C_{1\sim6}$alkyl, -O($C_{1\sim6}$alkyl), -O(halo-Ciealkyl), -$NH_2$, -NH($C_{1\sim6}$alkyl), -N($C_{1\sim6}$alkyl) ($C_{1\sim6}$alkyl), -$C_{0\sim2}$alkylene-(3~10 membered cycloalkyl), -$C_{0\sim2}$alkylene-(3~10 membered heterocycloalkyl), -$C_{0\sim2}$alkylene-(5~10 membered aryl ring), -$C_{0\sim2}$alkylene-(5~10 membered heteroaryl ring);

$R_6$ is selected from a group consisting of hydrogen, halogen, cyano, nitro, hydroxyl, -$C_{1\sim6}$alkyl, halo-$C_{1\sim6}$alkyl, -O($C_{1\sim6}$alkyl), -O(halo-Ciealkyl), -$NH_2$, -NH($C_{1\sim6}$alkyl), -N($C_{1\sim6}$alkyl) ($C_{1\sim6}$alkyl), -$C_{0\sim2}$alkylene-(3~10 membered cycloalkyl), -$C_{0\sim2}$alkylene-(3~10 membered heterocycloalkyl), -$C_{0\sim2}$alkylene-(5~10 membered aryl ring), -$C_{0\sim2}$alkylene-(5~10 membered heteroaryl ring).

In the second embodiment of the present invention, it provides the compound of the present invention, or a deuterated compound thereof, or a stereoisomer thereof, or a pharmaceutically acceptable salt thereof, wherein said $R_1$, $R_3$, $R_4$, $R_5$, $R_6$ is each independently selected from a group consisting of hydrogen, halogen, -$C_{1\sim3}$alkyl;

Q is selected from a group consisting of single bond, O or S; Z is selected from a group consisting of C=O, O, $NR^{Z1}$ or $CHR^{Z2}$; Y is selected from a group consisting of O or $NR^{Y1}$;

$R^{Z1}$ is selected from a group consisting of hydrogen, -$C_{1\sim3}$alkyl, -(3~6 membered cycloalkyl), halo-$C_{1\sim3}$alkyl;

$R^{Z2}$ is selected from a group consisting of hydrogen, -$C_{1\sim3}$alkyl, halo-$C_{1\sim3}$alkyl;

$R^{Y1}$ is selected from a group consisting of hydrogen, -$C_{1\sim3}$alkyl, halo-$C_{1\sim3}$alkyl.

In the second embodiment of the present invention, it provides the compound of the present invention, or a deuterated compound thereof, or a stereoisomer thereof, or a pharmaceutically acceptable salt thereof, wherein said $R_2$ is specifically selected from a group consisting of

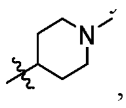,

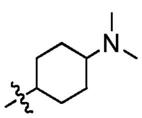, 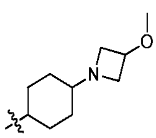, 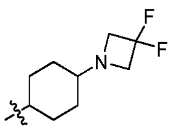, 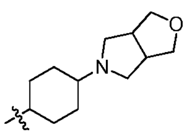, 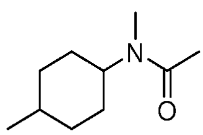, 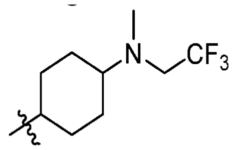,

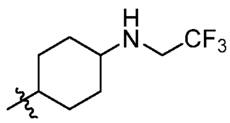, 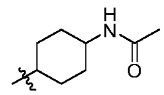, 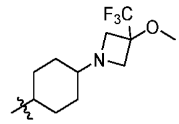, 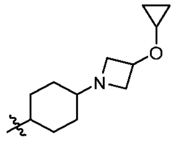, 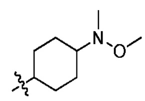, 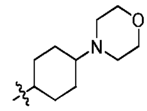,

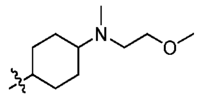, 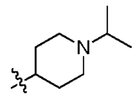, 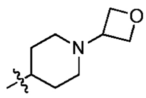, 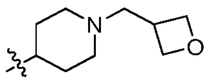, 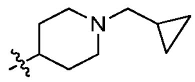, 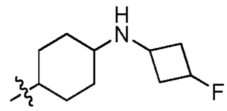;

preferably, said $R_2$ is specifically selected from a group consisting of

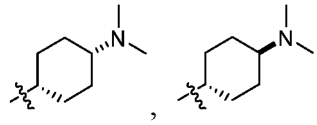,

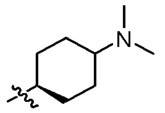, 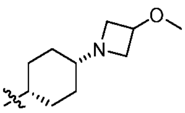, 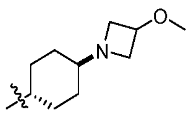, 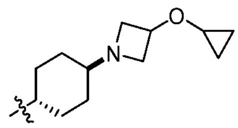, 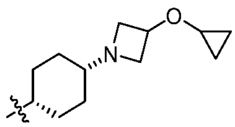,

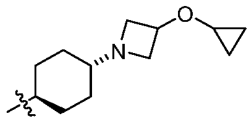, 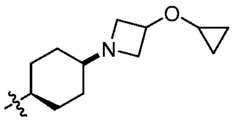, 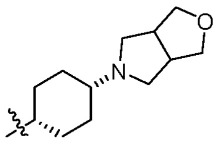, 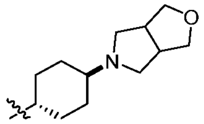, 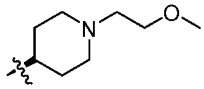,

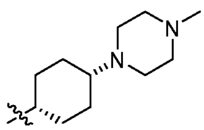, 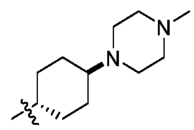, 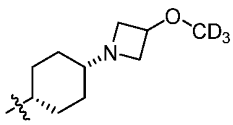, 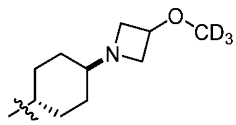, 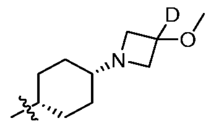,

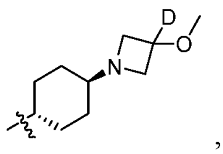, 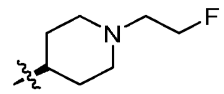, 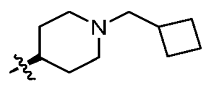, 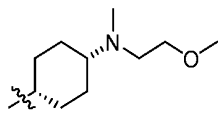, 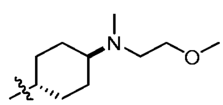,

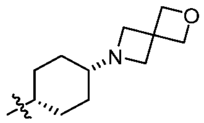, 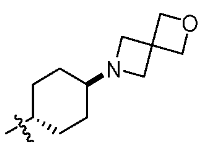, 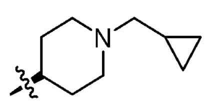, 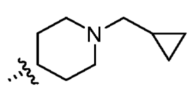, 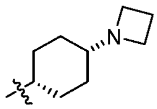, 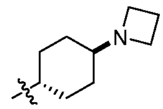,

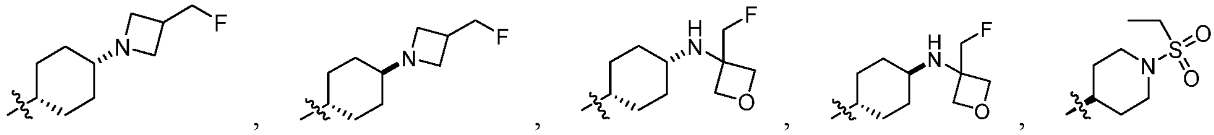,

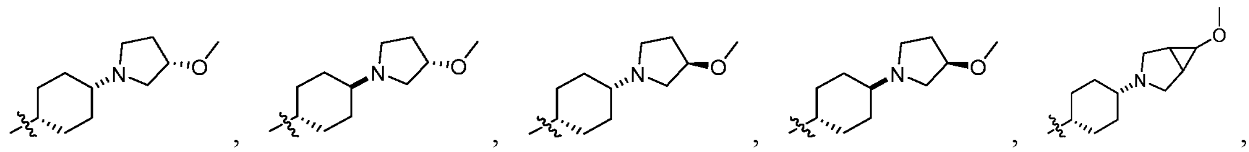

, , , , ,

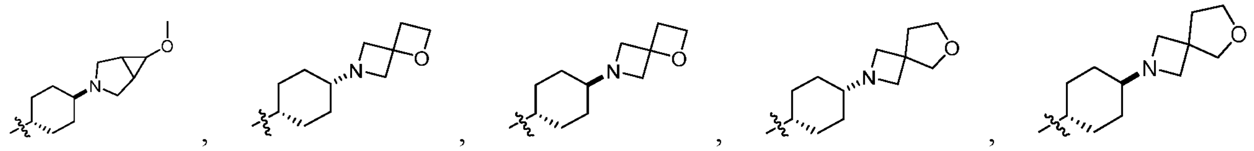

, , , ,

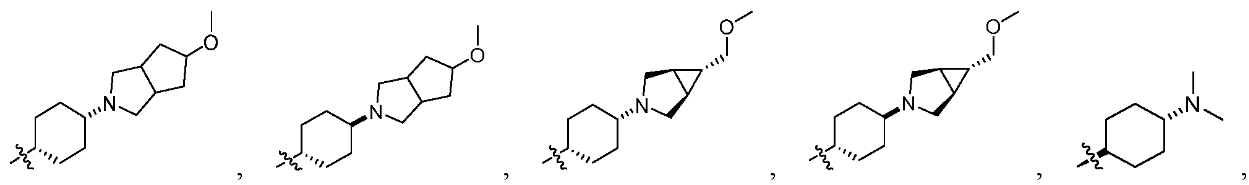

, , , , ,

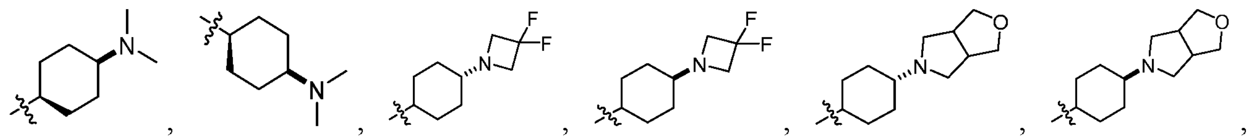

, , , , , ,

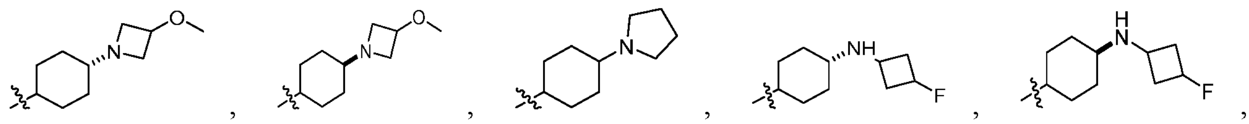

, , , , ,

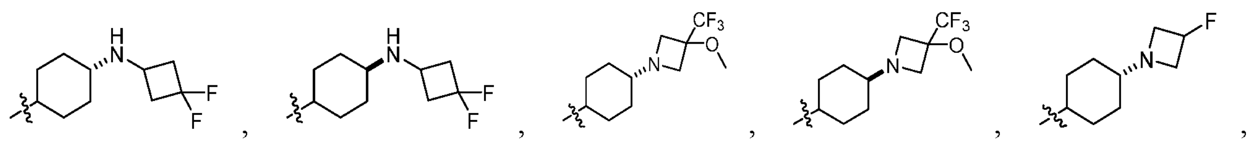

, , , , ,

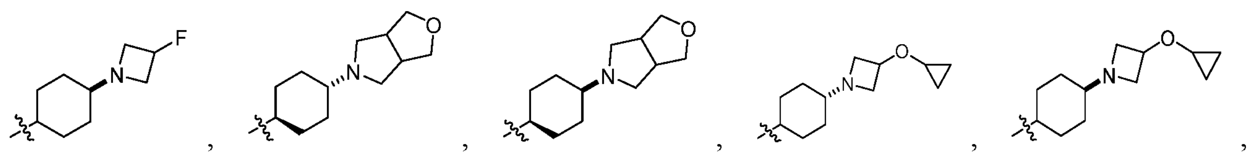

, , , , ,

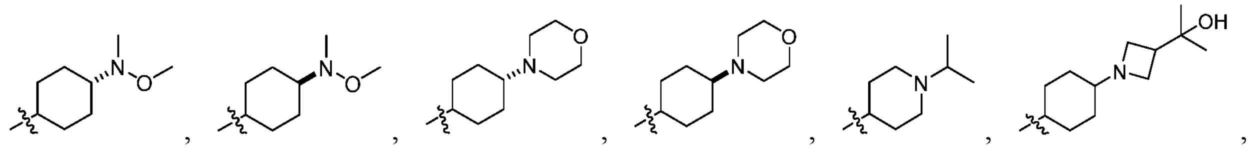

, , , , , ,

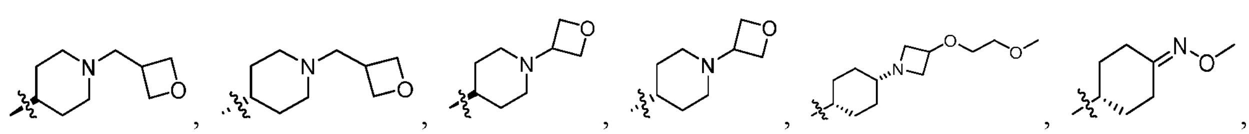

, , , , , ,

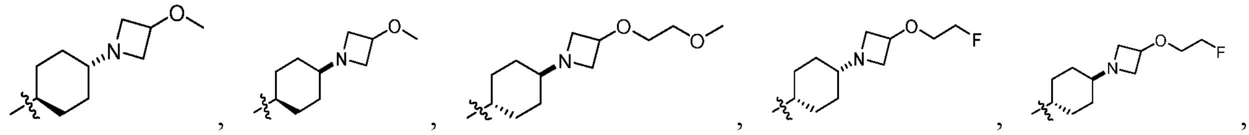

, , , , ,

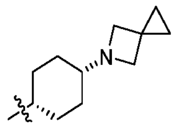, 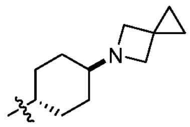, 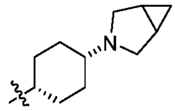, 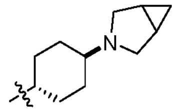, 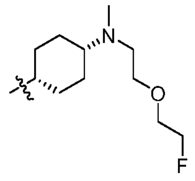, 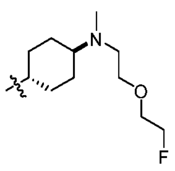,

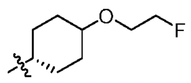, 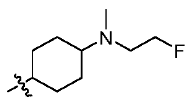, 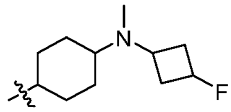, 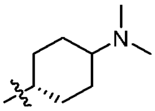, 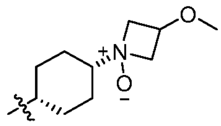, 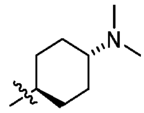,

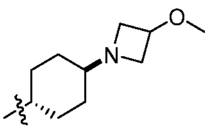, 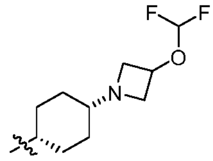, 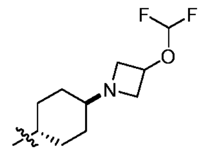, 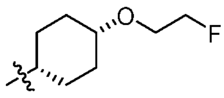, 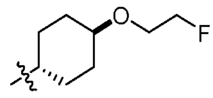

In the third embodiment of the present invention, it provides the compound of the present invention, or a deuterated compound thereof, or a stereoisomer thereof, or a pharmaceutically acceptable salt thereof, including the following compounds represented by Formula IIb:

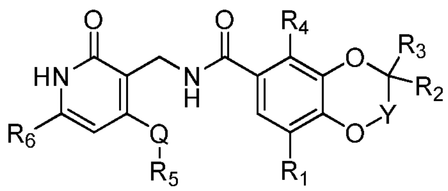

Formula IIb

Q is selected from a group consisting of single bond, O or S; Y is selected from a group consisting of O, $NR^{Y1}$ or $CHR^{Y2}$ ;

$R^{Y1}$ is selected from a group consisting of hydrogen, $-C_{1\sim6}$alkyl, halo-$C_{1\sim6}$alkyl, $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring);

$R^{Y2}$ is selected from a group consisting of hydrogen, halogen, cyano, hydroxyl, $-C_{1\sim6}$alkyl, halo-$C_{1\sim6}$alkyl, $-O(C_{1\sim6}$alkyl), -O(halo-Cialkyl), $-NH_2$, $-NH(C_{1\sim6}$alkyl), $-N(C_{1\sim6}$alkyl) ($C_{1\sim6}$alkyl), $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring);

$R_1$ is selected from a group consisting of hydrogen, halogen, cyano, nitro, amino, hydroxyl, $-C_{1\sim6}$alkyl, $-O(C_{1\sim6}$alkyl), halo-$C_{1\sim6}$alkyl, $-C_{0\sim2}$alkylene-$OR^{1b}$, $-C_{0\sim2}$alkylene-$C(O)R^{1b}$, $-C_{0\sim2}$alkylene-$C(O)NR^{1b}R^{1c}$, $-C_{0\sim2}$alkylene-$NR^{1b}R^{1c}$, $-C_{0\sim2}$alkylene-$NR^{1b}C(O)R^{1c}$, $-C_{0\sim4}$alkylene-$S(O)_2R^{1b}$, $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring); wherein said alkyl, alkylene, cycloalkyl, heterocycloalkyl, aryl ring, heteroaryl ring is optionally further substituted by one, two or three independent $R^{1b}$;

$R^{1b}$, $R^{1c}$ is each independently selected from a group consisting of hydrogen, -OH, $C_{1\sim6}$alkyl, halo-$C_{1\sim6}$alkyl, $-O(C_{1\sim6}$alkyl), -O(halo-Cialkyl), $-NH_2$, $-NH(C_{1\sim6}$alkyl), $-N(C_{1\sim6}$alkyl) ($C_{1\sim6}$alkyl), $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring);

$R_2$ is selected from a group consisting of $-C_{0\sim2}$alkylene-(3~10 membered cycloalkyl), $-C_{0\sim2}$alkylene-(3~10 membered heterocycloalkyl); wherein said alkylene, cycloalkyl, heterocycloalkyl is optionally further substituted by one, two or three independent $R^{2b}$;

$R^{2b}$ is each independently selected from a group consisting of hydrogen, halogen, cyano, nitro, amino, hydroxyl, $-C_{1\sim6}$alkyl, $-O(C_{1\sim6}$alkyl), halo-$C_{1\sim6}$alkyl, $-C_{0\sim2}$alkylene-$OR^{2c}$, $-C_{0\sim2}$alkylene-$C(O)R^{2c}$, $-C_{0\sim2}$alkylene-$C(O)NR^{2c}R^{2d}$, $-C_{0\sim2}$alkylene-$NR^{2c}R^{2d}$, $-C_{0\sim2}$alkylene-$NR^{2c}C(O)R^{2d}$, $-C_{0\sim4}$alkylene-$S(O)_2R^{2c}$, $-C_{0\sim2}$alkylene-(3~10

membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring); wherein alkyl, alkylene, cycloalkyl, heterocycloalkyl, aryl ring, heteroaryl ring is optionally further substituted by one, two or three independent $R^{2c}$;

$R^{2c}$, $R^{2d}$ is each independently selected from a group consisting of hydrogen, cyano, nitro, -OH, halogen, $-C_{1\sim6}$alkyl, halo-$C_{1\sim6}$alkyl, $-C_{0\sim2}$alkylene-O($C_{1\sim6}$alkyl), -O(halo-$C_{1\sim6}$alkyl), -O(3~10 membered cycloalkyl), -O(3~10 membered heterocycloalkyl), $-NH_2$, -NH($C_{1\sim6}$alkyl), -N($C_{1\sim6}$alkyl) ($C_{1\sim6}$alkyl), $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring); wherein said alkyl, alkylene, cycloalkyl, heterocycloalkyl, aryl ring, heteroaryl ring is optionally further substituted by one, two or three independent $R^{2e}$;

$R^{2e}$ is each independently selected from a group consisting of hydrogen, cyano, nitro, -OH, halogen, $-C_{1\sim6}$alkyl, halo-$C_{1\sim6}$alkyl, $-C_{0\sim2}$alkylene-O($C_{1\sim6}$alkyl), -O(halo-$C_{1\sim6}$alkyl), -O(3~10 membered cycloalkyl), -O(3~10 membered heterocycloalkyl), $-NH_2$, -NH($C_{1\sim6}$alkyl), -N($C_{1\sim6}$alkyl) ($C_{1\sim6}$alkyl), $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring);

$R_3$ is selected from a group consisting of hydrogen, halogen, cyano, nitro, amino, -OH, $-C_{1\sim6}$alkyl, -O($C_{1\sim6}$alkyl), halo-$C_{1\sim6}$alkyl, $-C_{0\sim2}$alkylene-$OR^{3b}$, $-C_{0\sim2}$alkylene-C(O)$R^{3b}$, $-C_{0\sim2}$alkylene-C(O)$NR^{3b}R^{3c}$, $-C_{0\sim2}$alkylene-$NR^{3b}R^{3c}$, $-C_{0\sim2}$alkylene-$NR^{3b}$C(O)$R^{3c}$, $-C_{0\sim4}$alkylene-$S(O)_2R^{3b}$, $-C_{0\sim2}$alkylene-(3~10 membered cycloalkyl), $-C_{0\sim2}$alkylene-(3~10 membered heterocycloalkyl);

$R^{3b}$, $R^{3c}$ is each independently selected from a group consisting of hydrogen, halogen, cyano, nitro, -OH, $C_{1\sim6}$alkyl, halo-$C_{1\sim6}$alkyl, -O($C_{1\sim6}$alkyl), -O(halo-$C_{1\sim6}$alkyl), $-NH_2$, -NH($C_{1\sim6}$alkyl), -N($C_{1\sim6}$alkyl) ($C_{1\sim6}$alkyl), $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring);

$R_4$ is selected from a group consisting of hydrogen, halogen, cyano, nitro, hydroxyl, $-C_{1\sim6}$alkyl, halo-$C_{1\sim6}$alkyl, -O($C_{1\sim6}$alkyl), -O(halo-$C_{1\sim6}$alkyl), $-NH_2$, -NH($C_{1\sim6}$alkyl), -N($C_{1\sim6}$alkyl) ($C_{1\sim6}$alkyl), $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring); wherein said alkyl, alkylene, cycloalkyl, heterocycloalkyl, aryl ring, heteroaryl ring is optionally further substituted by one, two or three independent $R^{4b}$;

$R^{4b}$ is each independently selected from a group consisting of hydrogen, halogen, cyano, nitro, -OH, Ciealkyl, halo-$C_{1\sim6}$alkyl, -O($C_{1\sim6}$alkyl), -O(halo-$C_{1\sim6}$alkyl), $-NH_2$, -NH($C_{1\sim6}$alkyl), -N($C_{1\sim6}$alkyl) ($C_{1\sim6}$alkyl), $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring);

$R_5$ is selected from a group consisting of hydrogen, halogen, cyano, nitro, hydroxyl, $-C_{1\sim6}$alkyl, halo-$C_{1\sim6}$alkyl, -O($C_{1\sim6}$alkyl), -O(halo-Ciealkyl), $-NH_2$, -NH($C_{1\sim6}$alkyl), -N($C_{1\sim6}$alkyl) ($C_{1\sim6}$alkyl), $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring);

$R_6$ is selected from a group consisting of hydrogen, halogen, cyano, nitro, hydroxyl, $-C_{1\sim6}$alkyl, halo-$C_{1\sim6}$alkyl, -O($C_{1\sim6}$alkyl), -O(halo-Ciealkyl), $-NH_2$, -NH($C_{1\sim6}$alkyl), -N($C_{1\sim6}$alkyl) ($C_{1\sim6}$alkyl), $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring);

In the third embodiment of the present invention, it provides the compound of the present invention, or a deuterated compound thereof, or a stereoisomer thereof, or a pharmaceutically acceptable salt thereof, wherein said $R_1$, $R_3$, $R_4$, $R_5$, $R_6$ is each independently selected from a group consisting of hydrogen, halogen, $-C_{1\sim3}$alkyl;
Q is selected from a group consisting of single bond; Y is selected from a group consisting of $CHR^{Y2}$; $R^{Y2}$ is selected from a group consisting of hydrogen, $-C_{1\sim3}$alkyl.

In the third embodiment of the present invention, it provides the compound of the present invention, or a deuterated compound thereof, or a stereoisomer thereof, or a pharmaceutically acceptable salt thereof, wherein said $R_2$ is specifically selected from a group consisting of

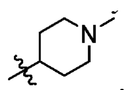,

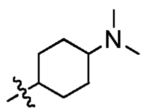, 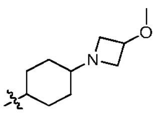, 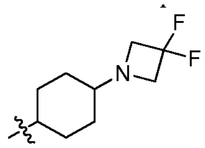, 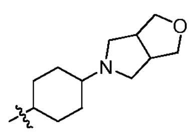, 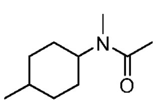, 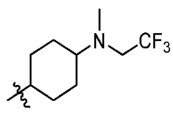,

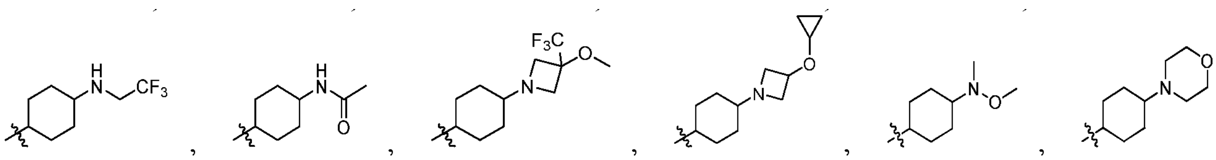, , , , , ,

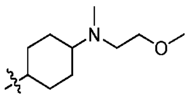, 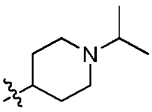, 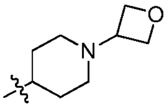, 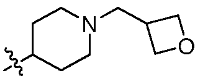, 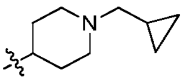, 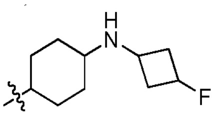;

preferably, said $R_2$ is specifically selected from a group consisting of

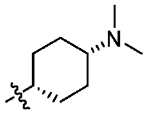, 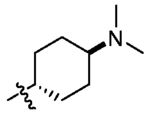,

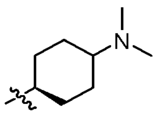, 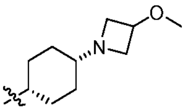, 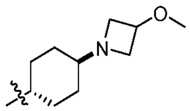, 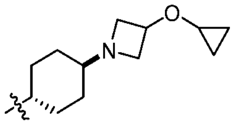, 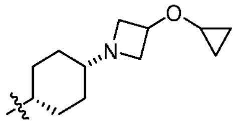,

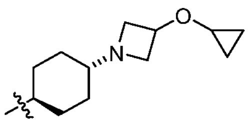, 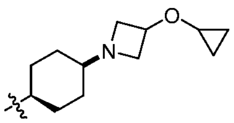, 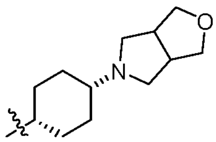, 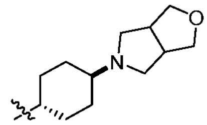, 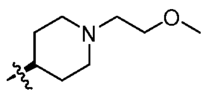,

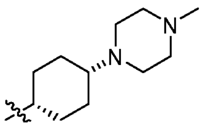, 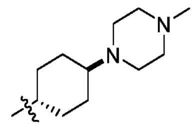, 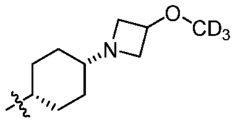, 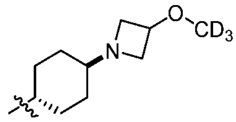, 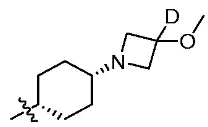,

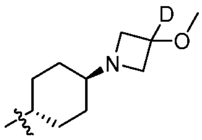, 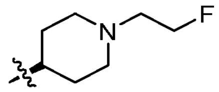, 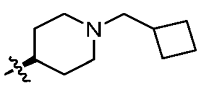, 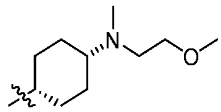, 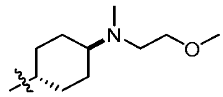,

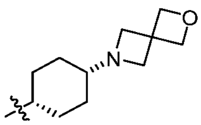, 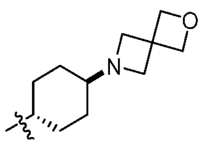, 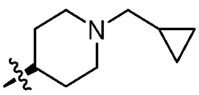, 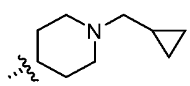, 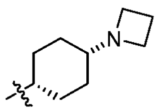, 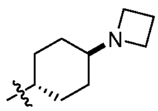,

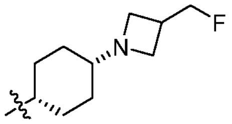, 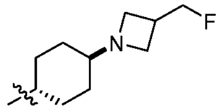, 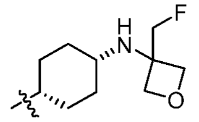, 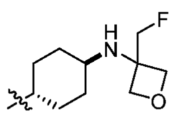, 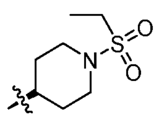,

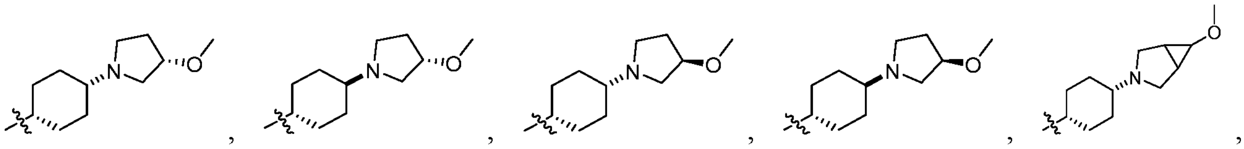 , , , , ,

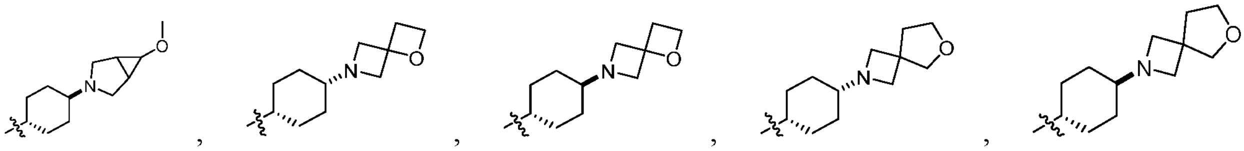 , , , ,

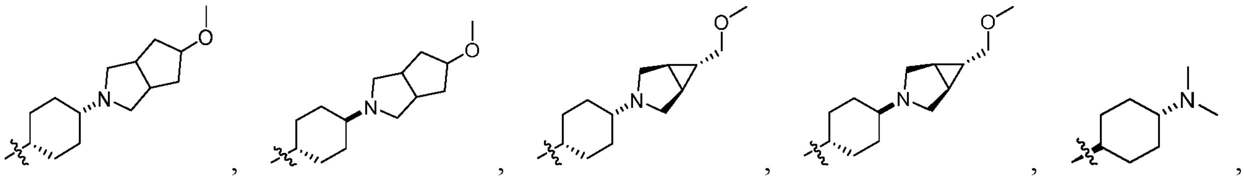 , , , , ,

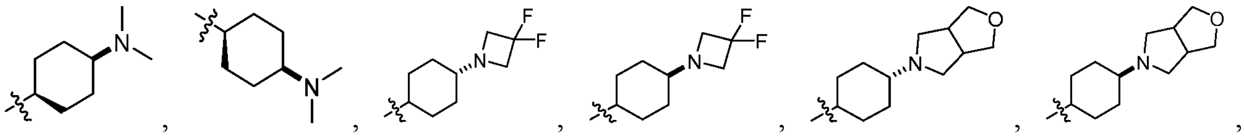 , , , , , ,

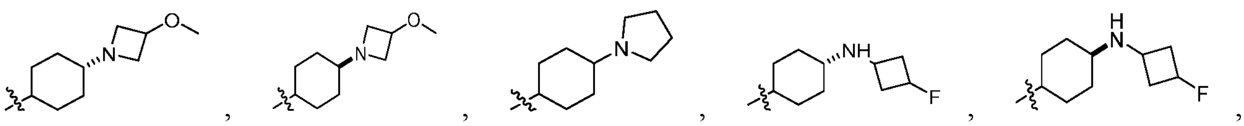 , , , , ,

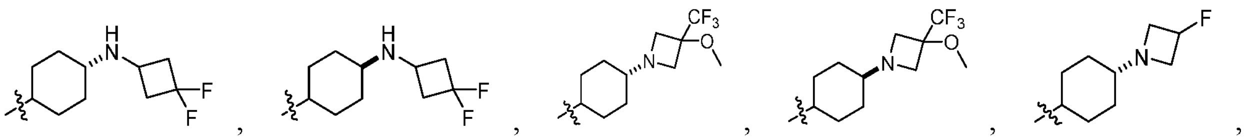 , , , , ,

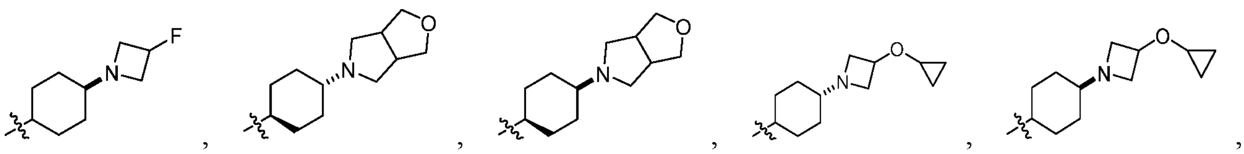 , , , , ,

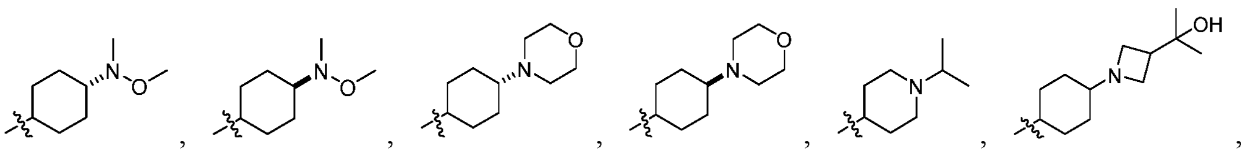 , , , , , ,

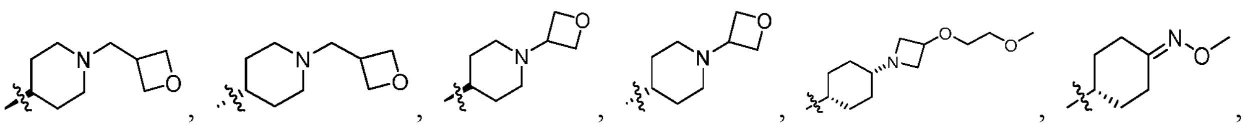 , , , , , ,

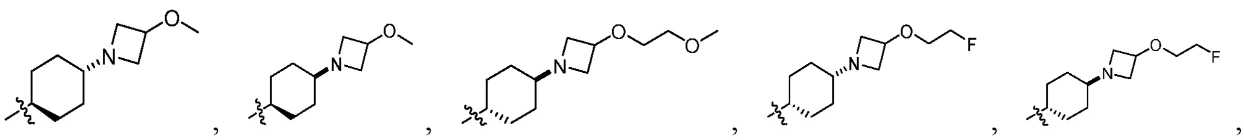 , , , , ,

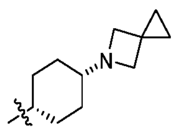, 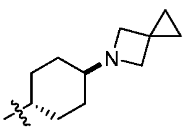, 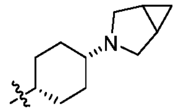, 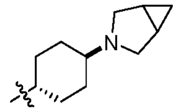, 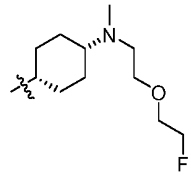, 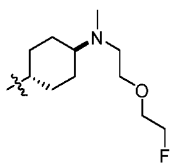,

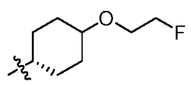, 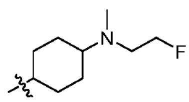, 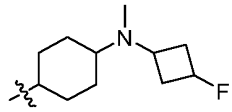, 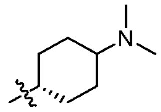, 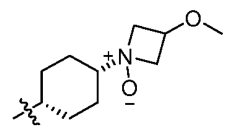, 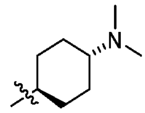,

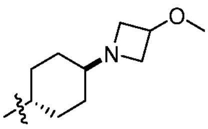, 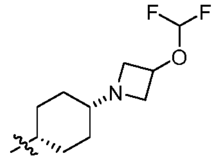, 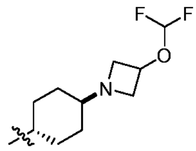, 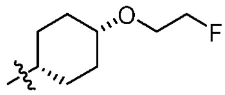, 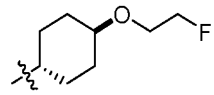,

In the fourth embodiment of the present invention, it provides the compound of the present invention, or a deuterated compound thereof, or a stereoisomer thereof, or a pharmaceutically acceptable salt thereof, including the following compounds represented by Formula IIc:

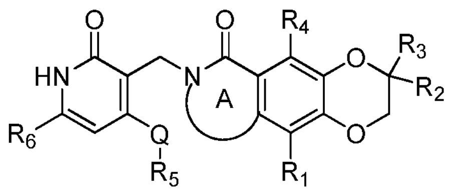

Formula IIc

Q is selected from a group consisting of single bond, O or S;

A-ring is selected from a group consisting of 4~10 membered heterocyclic ring; wherein said heterocyclic ring is optionally further substituted by one, two or three independent $R^{71}$;

each $R^{71}$ is each independently selected from a group consisting of hydrogen, halogen, cyano, nitro, -OH, $C_{1\sim6}$alkyl, halo-$C_{1\sim6}$alkyl, -O($C_{1\sim6}$alkyl), -O(halo-$C_{1\sim6}$alkyl), -$NH_2$, -NH($C_{1\sim6}$alkyl), -N($C_{1\sim6}$alkyl) ($C_{1\sim6}$alkyl), -$C_{0\sim2}$alkylene-(3~10 membered cycloalkyl), -$C_{0\sim2}$alkylene-(3~10 membered heterocycloalkyl), -$C_{0\sim2}$alkylene-(5~10 membered aryl ring), -$C_{0\sim2}$alkylene-(5~10 membered heteroaryl ring);

$R_1$ is selected from a group consisting of hydrogen, halogen, cyano, nitro, amino, hydroxyl, -$C_{1\sim6}$alkyl, -O($C_{1\sim6}$alkyl), halo-$C_{1\sim6}$alkyl, -$C_{0\sim2}$alkylene-$OR^{1b}$, -$C_{0\sim2}$alkylene-C(O)$R^{1b}$, -$C_{0\sim2}$alkylene-C(O)$NR^{1b}R^{1c}$, -$C_{0\sim2}$alkylene-$NR^{1b}R^{1c}$, -$C_{0\sim2}$alkylene-$NR^{1b}$C(O)$R^{1c}$, -$C_{0\sim4}$alkylene-$S(O)_2R^{1b}R^{1c}$, -$C_{0\sim2}$alkylene-(3~10 membered cycloalkyl), -$C_{0\sim2}$alkylene-(3~10 membered heterocycloalkyl), -$C_{0\sim2}$alkylene-(5~10 membered aryl ring), -$C_{0\sim2}$alkylene-(5~10 membered heteroaryl ring); wherein said alkyl, alkylene, cycloalkyl, heterocycloalkyl, aryl ring, heteroaryl ring is optionally further substituted by one, two or three independent $R^{1b}$;

$R^{1b}$, $R^{1c}$ is each independently selected from a group consisting of hydrogen, -OH, $C_{1\sim6}$alkyl, halo-$C_{1\sim6}$alkyl, -O($C_{1\sim6}$alkyl), -O(halo-Ciealkyl), -$NH_2$, -NH($C_{1\sim6}$alkyl), -N($C_{1\sim6}$alkyl) ($C_{1\sim6}$alkyl), -$C_{0\sim2}$alkylene-(3~10 membered cycloalkyl), -$C_{0\sim2}$alkylene-(3~10 membered heterocycloalkyl), -$C_{0\sim2}$alkylene-(5~10 membered aryl ring), -$C_{0\sim2}$alkylene-(5~10 membered heteroaryl ring);

$R_2$ is selected from a group consisting of -$C_{0\sim2}$alkylene-(3~10 membered cycloalkyl), -$C_{0\sim2}$alkylene-(3~10 membered heterocycloalkyl); wherein said alkylene, cycloalkyl, heterocycloalkyl is optionally further substituted by one, two or three independent $R^{2b}$;

$R^{2b}$ is each independently selected from a group consisting of hydrogen, halogen, cyano, nitro, amino, hydroxyl, -$C_{1\sim6}$alkyl, -O($C_{1\sim6}$alkyl), halo-$C_{1\sim6}$alkyl, -$C_{0\sim2}$alkylene-$OR^{2c}$, -$C_{0\sim2}$alkylene-C(O)$R^{2c}$, -$C_{0\sim2}$alkylene-C(O)$NR^{2c}R^{2d}$, -$C_{0\sim2}$alkylene-$NR^{2c}R^{2d}$, -$C_{0\sim2}$alkylene-$NR^{2c}$C(O)$R^{2d}$, -$C_{0\sim4}$alkylene-$S(O)_2R^{2c}$, -$C_{0\sim2}$alkylene-(3~10 membered cycloalkyl), -$C_{0\sim2}$alkylene-(3~10 membered heterocycloalkyl), -$C_{0\sim2}$alkylene-(5~10 membered aryl ring), -$C_{0\sim2}$alkylene-(5~10 membered heteroaryl ring); wherein alkyl, alkylene, cycloalkyl, heterocycloalkyl, aryl ring,

heteroaryl ring is optionally further substituted by one, two or three independent $R^{2c}$;

$R^{2c}$, $R^{2d}$ is each independently selected from a group consisting of hydrogen, cyano, nitro, -OH, halogen, -$C_{1\sim6}$alkyl, halo-$C_{1\sim6}$alkyl, -$C_{0\sim2}$alkylene-O($C_{1\sim6}$alkyl), -O(halo-$C_{1\sim6}$alkyl), -O(3~10 membered cycloalkyl), -O(3~10 membered heterocycloalkyl), -$NH_2$, -NH($C_{1\sim6}$alkyl), -N($C_{1\sim6}$alkyl) ($C_{1\sim6}$alkyl), -$C_{0\sim2}$alkylene-(3~10 membered cycloalkyl), -$C_{0\sim2}$alkylene-(3~10 membered heterocycloalkyl), -$C_{0\sim2}$alkylene-(5~10 membered aryl ring), -$C_{0\sim2}$alkylene-(5~10 membered heteroaryl ring); wherein said alkyl, alkylene, cycloalkyl, heterocycloalkyl, aryl ring, heteroaryl ring is optionally further substituted by one, two or three independent $R^{2e}$;

$R^{2e}$ is each independently selected from a group consisting of hydrogen, cyano, nitro, -OH, halogen, -$C_{1\sim6}$alkyl, halo-$C_{1\sim6}$alkyl, -$C_{0\sim2}$alkylene-O($C_{1\sim6}$alkyl), -O(halo-$C_{1\sim6}$alkyl), -O(3~10 membered cycloalkyl), -O(3~10 membered heterocycloalkyl), -$NH_2$, -NH($C_{1\sim6}$alkyl), -N($C_{1\sim6}$alkyl) ($C_{1\sim6}$alkyl), -$C_{0\sim2}$alkylene-(3~10 membered cycloalkyl), -$C_{0\sim2}$alkylene-(3~10 membered heterocycloalkyl), -$C_{0\sim2}$alkylene-(5~10 membered aryl ring), -$C_{0\sim2}$alkylene-(5~10 membered heteroaryl ring);

$R_3$ is selected from a group consisting of hydrogen, halogen, cyano, nitro, amino, -OH, -$C_{1\sim6}$alkyl, -O($C_{1\sim6}$alkyl), halo-$C_{1\sim6}$alkyl, -$C_{0\sim2}$alkylene-$OR^{3b}$, -$C_{0\sim2}$alkylene-C(O)$R^{3b}$, -$C_{0\sim2}$alkylene-C(O)$NR^{3b}R^{3c}$, -$C_{0\sim2}$alkylene-$NR^{3b}R^{3c}$, -$C_{0\sim2}$alkylene-$NR^{3b}$C(O)$R^{3c}$, -$C_{0\sim4}$alkylene-$S(O)_2R^{3b}$, -$C_{0\sim2}$alkylene-(3~10 membered cycloalkyl), -$C_{0\sim2}$alkylene-(3~10 membered heterocycloalkyl);

$R^{3b}$, $R^{3c}$ is each independently selected from a group consisting of hydrogen, halogen, cyano, nitro, -OH, $C_{1\sim6}$alkyl, halo-$C_{1\sim6}$alkyl, -O($C_{1\sim6}$alkyl), -O(halo-$C_{1\sim6}$alkyl), -$NH_2$, -NH($C_{1\sim6}$alkyl), -N($C_{1\sim6}$alkyl) ($C_{1\sim6}$alkyl), -$C_{0\sim2}$alkylene-(3~10 membered cycloalkyl), -$C_{0\sim2}$alkylene-(3~10 membered heterocycloalkyl), -$C_{0\sim2}$alkylene-(5~10 membered aryl ring), -$C_{0\sim2}$alkylene-(5~10 membered heteroaryl ring);

$R_4$ is selected from a group consisting of hydrogen, halogen, cyano, nitro, hydroxyl, -$C_{1\sim6}$alkyl, halo-$C_{1\sim6}$alkyl, -O($C_{1\sim6}$alkyl), -O(halo-$C_{1\sim6}$alkyl), -$NH_2$, -NH($C_{1\sim6}$alkyl), -N($C_{1\sim6}$alkyl) ($C_{1\sim6}$alkyl), -$C_{0\sim2}$alkylene-(3~10 membered cycloalkyl), -$C_{0\sim2}$alkylene-(3~10 membered heterocycloalkyl), -$C_{0\sim2}$alkylene-(5~10 membered aryl ring), -$C_{0\sim2}$alkylene-(5~10 membered heteroaryl ring); wherein said alkyl, alkylene, cycloalkyl, heterocycloalkyl, aryl ring, heteroaryl ring is optionally further substituted by one, two or three independent $R^{4b}$;

$R^{4b}$ is each independently selected from a group consisting of hydrogen, halogen, cyano, nitro, -OH, Ciealkyl, halo-$C_{1\sim6}$alkyl, -O($C_{1\sim6}$alkyl), -O(halo-$C_{1\sim6}$alkyl), -$NH_2$, -NH($C_{1\sim6}$alkyl), -N($C_{1\sim6}$alkyl) ($C_{1\sim6}$alkyl), -$C_{0\sim2}$alkylene-(3~10 membered cycloalkyl), -$C_{0\sim2}$alkylene-(3~10 membered heterocycloalkyl), -$C_{0\sim2}$alkylene-(5~10 membered aryl ring), -$C_{0\sim2}$alkylene-(5~10 membered heteroaryl ring);

Rs is selected from a group consisting of hydrogen, halogen, cyano, nitro, hydroxyl, -$C_{1\sim6}$alkyl, halo-$C_{1\sim6}$alkyl, -O($C_{1\sim6}$alkyl), -O(halo-Ciealkyl), -$NH_2$, -NH($C_{1\sim6}$alkyl), -N($C_{1\sim6}$alkyl) ($C_{1\sim6}$alkyl), -$C_{0\sim2}$alkylene-(3~10 membered cycloalkyl), -$C_{0\sim2}$alkylene-(3~10 membered heterocycloalkyl), -$C_{0\sim2}$alkylene-(5~10 membered aryl ring), -$C_{0\sim2}$alkylene-(5~10 membered heteroaryl ring);

$R_6$ is selected from a group consisting of hydrogen, halogen, cyano, nitro, hydroxyl, -$C_{1\sim6}$alkyl, halo-$C_{1\sim6}$alkyl, -O($C_{1\sim6}$alkyl), -O(halo-Ciealkyl), -$NH_2$, -NH($C_{1\sim6}$alkyl), -N($C_{1\sim6}$alkyl) ($C_{1\sim6}$alkyl), -$C_{0\sim2}$alkylene-(3~10 membered cycloalkyl), -$C_{0\sim2}$alkylene-(3~10 membered heterocycloalkyl), -$C_{0\sim2}$alkylene-(5~10 membered aryl ring), -$C_{0\sim2}$alkylene-(5~10 membered heteroaryl ring);

In the fourth embodiment of the present invention, it provides the compound of the present invention, or a deuterated compound thereof, or a stereoisomer thereof, or a pharmaceutically acceptable salt thereof, wherein said $R_1$, $R_3$, $R_4$, $R_5$, $R_6$ is each independently selected from a group consisting of hydrogen, halogen, -$C_{1\sim3}$alkyl; Q is selected from a group consisting of single bond.

In the fourth embodiment of the present invention, it provides the compound of the present invention, or a deuterated compound thereof, or a stereoisomer thereof, or a pharmaceutically acceptable salt thereof, wherein said $R_2$ is specifically selected from a group consisting of

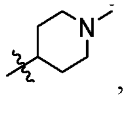,

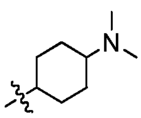, 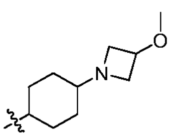, 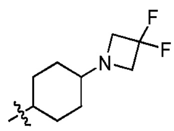, 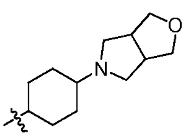, 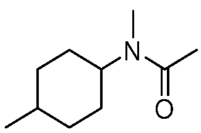, 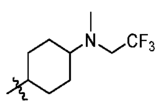,

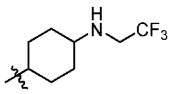, 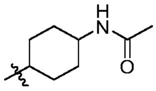, 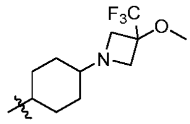, 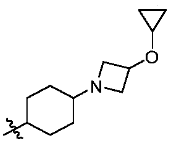, 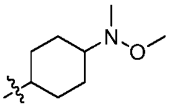, 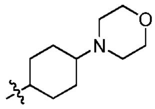,

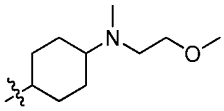, 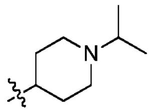, 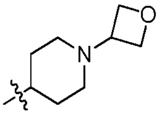, 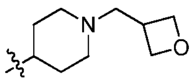, 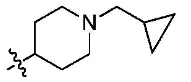,

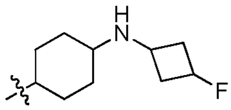;

preferably, said $R_2$ is specifically selected from a group consisting of

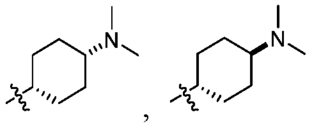, ,

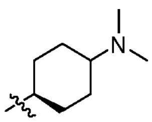, 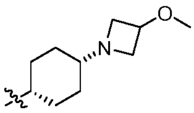, 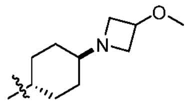, 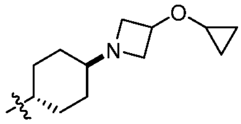, 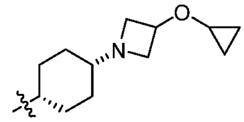,

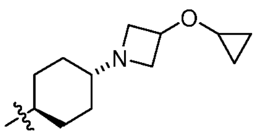, 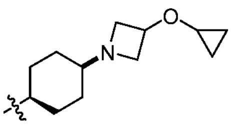, 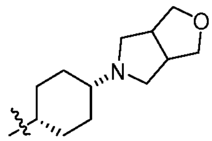, 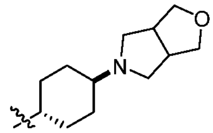, 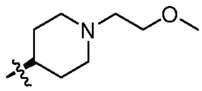,

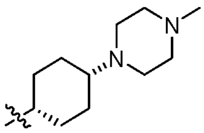, 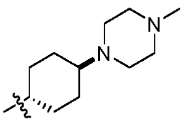, 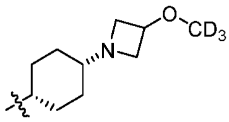, 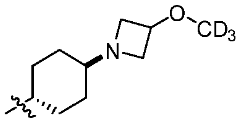, 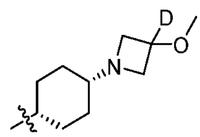,

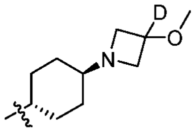, 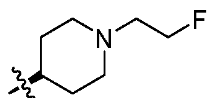, 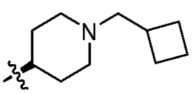, 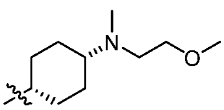, 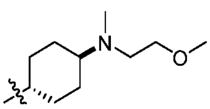,

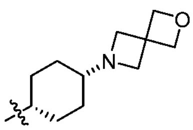, 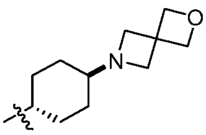, 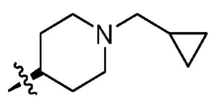, 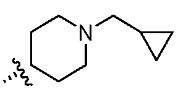, 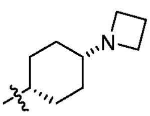, 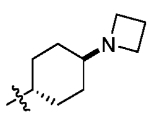,

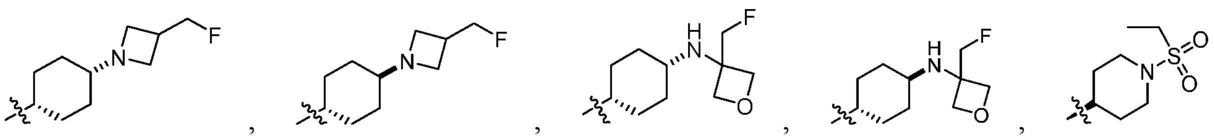, , , , ,

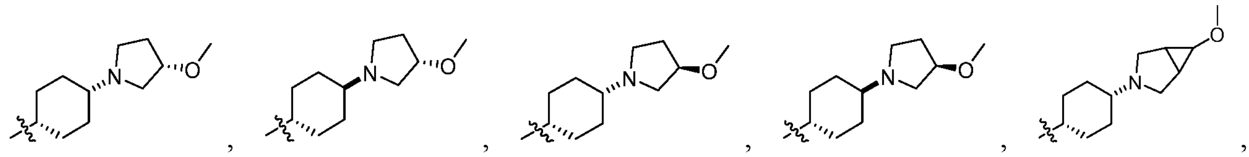

, , , , ,

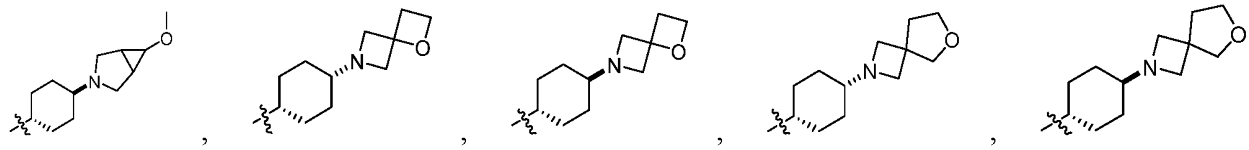

, , , ,

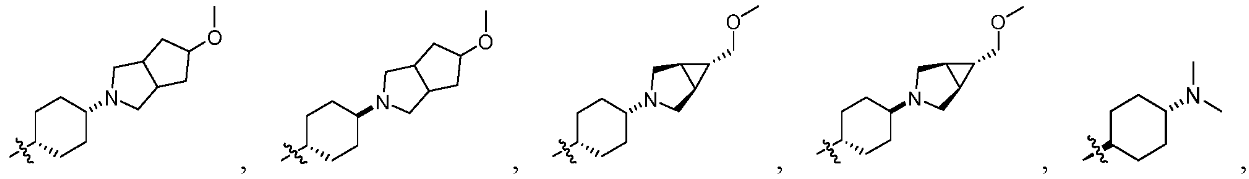

, , , , ,

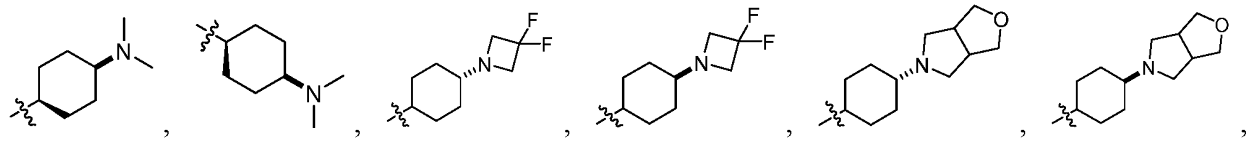

, , , , , ,

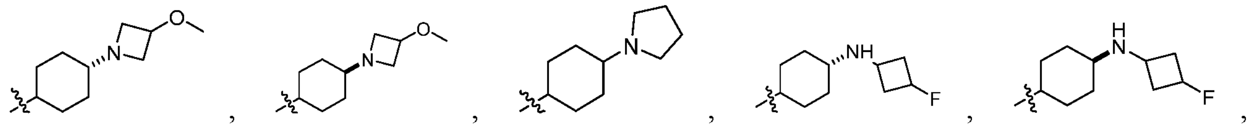

, , , , ,

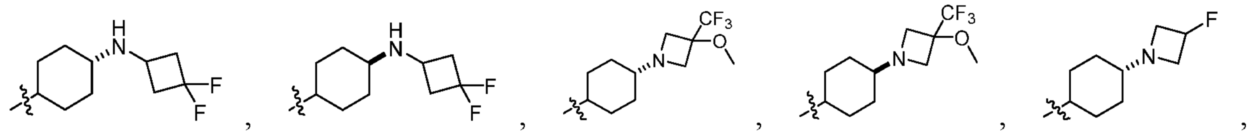

, , , , ,

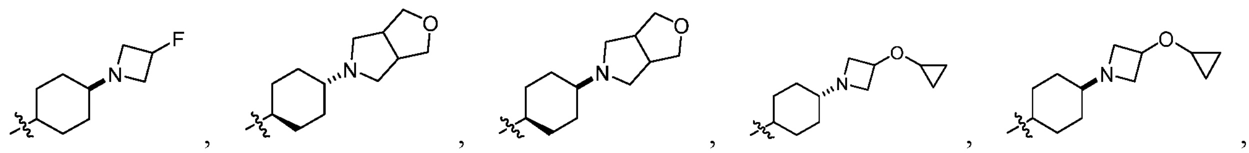

, , , , ,

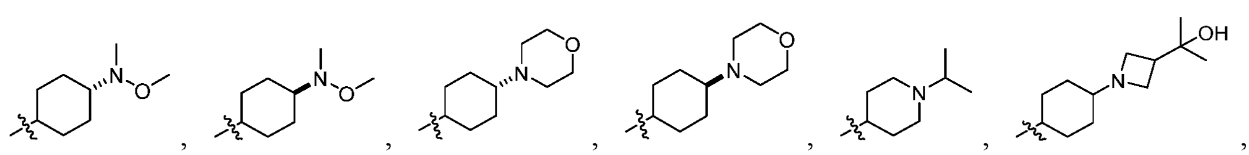

, , , , , ,

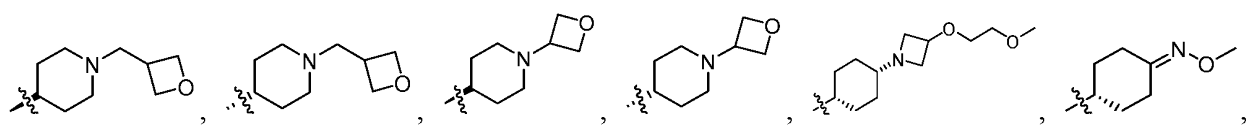

, , , , , ,

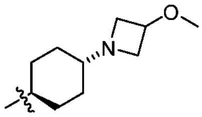, 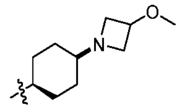, 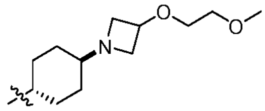, 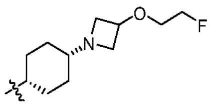, 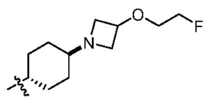,

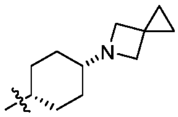, 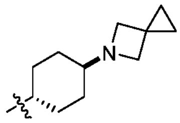, 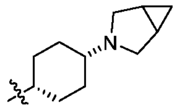, 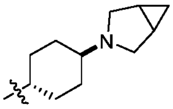, 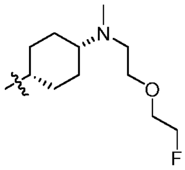, 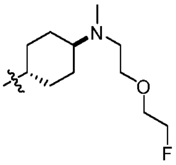,

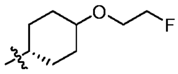, 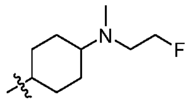, 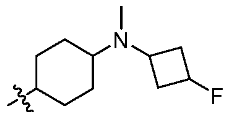, 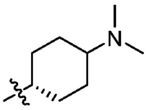, 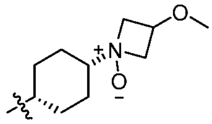, 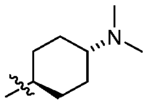,

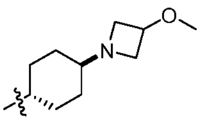, 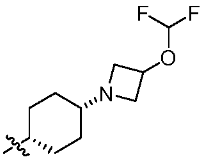, 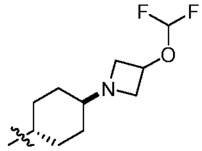, 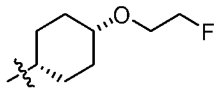, 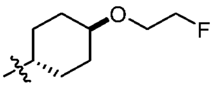.

In the embodiment of the present invention, it provides the compound of the present invention, or a deuterated compound thereof, or a stereoisomer thereof, or a pharmaceutically acceptable salt thereof, wherein the compound represented by Formula I or Formula II is specifically:

| | | |
|---|---|---|
| 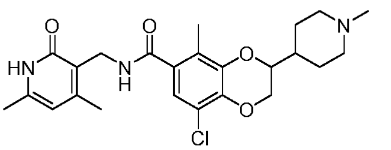 1 | 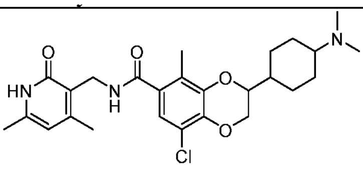 2 | 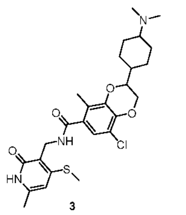 3 |
| 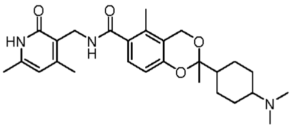 4 | 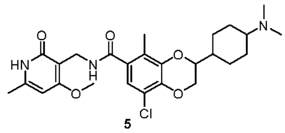 5 | 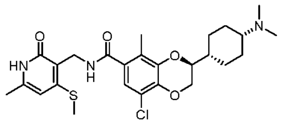 6 |
| 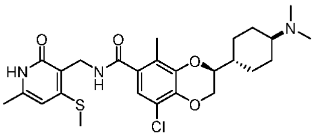 6' | 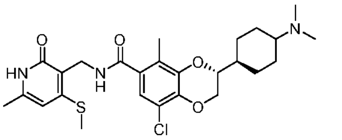 7 | 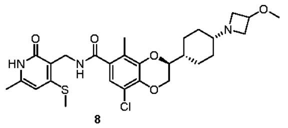 8 |
| 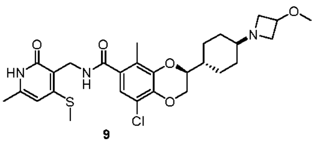 9 | 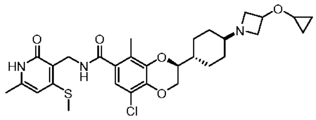 10 | 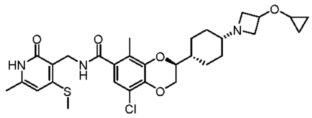 11 |
| 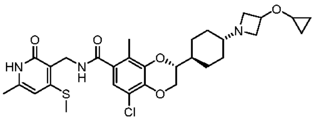 12 | 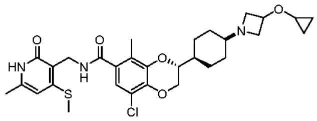 13 | 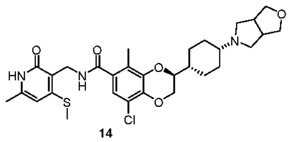 14 |
| 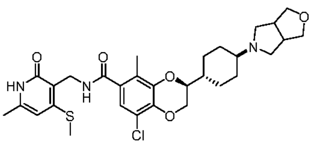 15 | 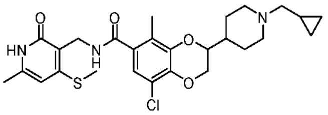 16 | 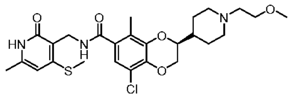 17 |
| 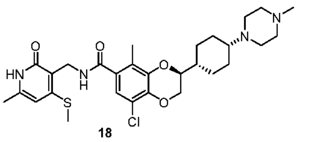 18 | 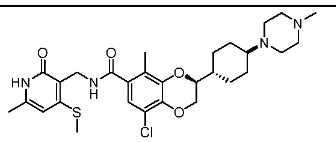 19 | 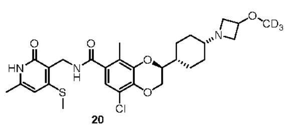 20 |

| | | |
|---|---|---|
| 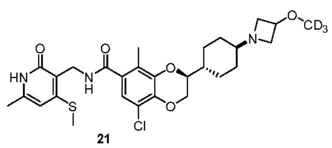<br>21 | 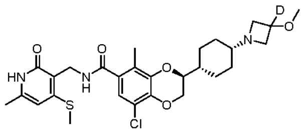<br>22 | 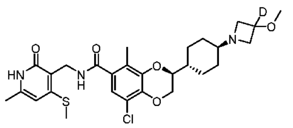<br>23 |
| 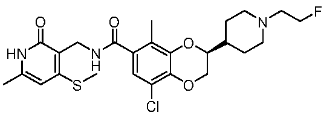<br>24 | 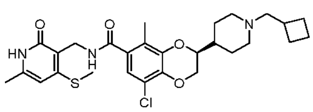<br>25 | 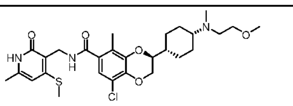<br>26 |
| 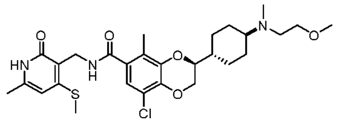<br>27 | 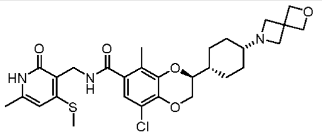<br>28 | 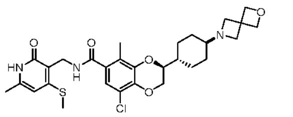<br>29 |
| 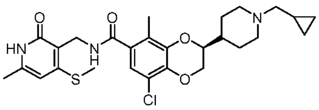<br>30 | 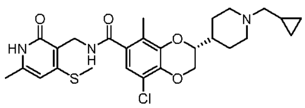<br>31 | 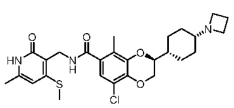<br>32 |
| 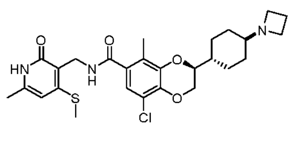<br>33 | 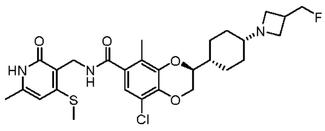<br>34 | 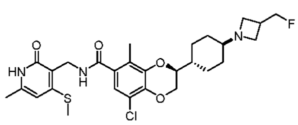<br>35 |
| 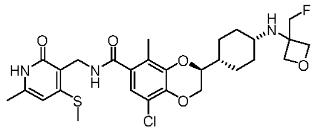<br>36 | 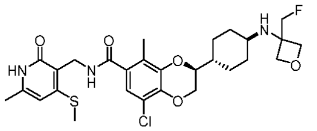<br>37 | 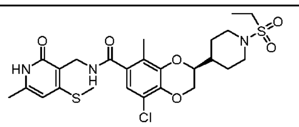<br>38 |
| 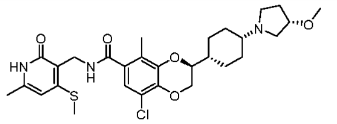<br>39 | 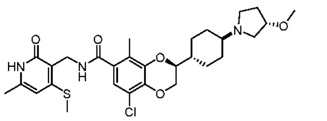<br>40 | 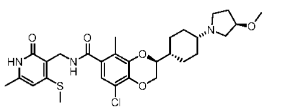<br>41 |
| 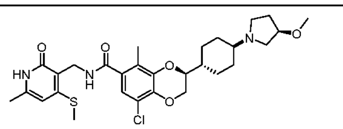<br>42 | 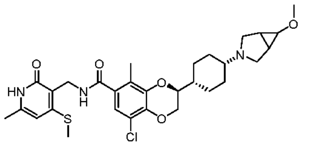<br>43 | 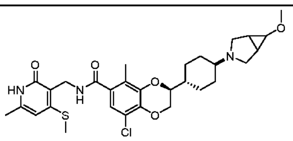<br>44 |
| 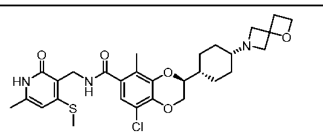<br>45 | 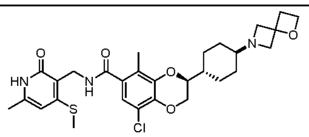<br>46 | 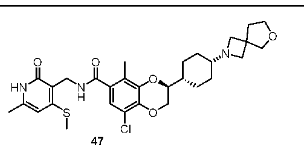<br>47 |
| 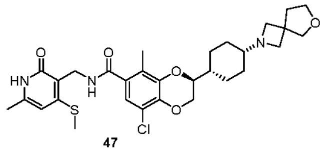<br>47 | 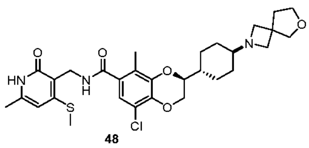<br>48 | 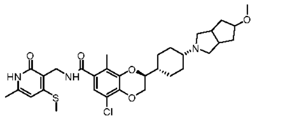<br>49 |

| | | |
|---|---|---|
| 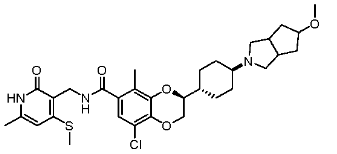 50 | 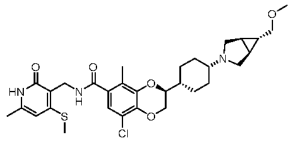 51 | 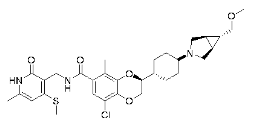 52 |
| 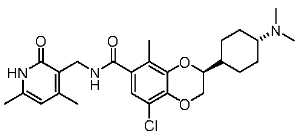 53 | 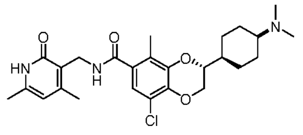 54 | 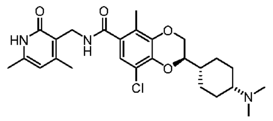 55 |
| 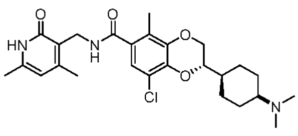 56 | 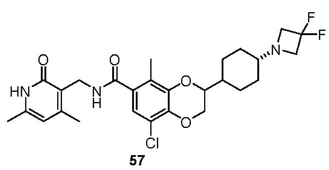 57 | 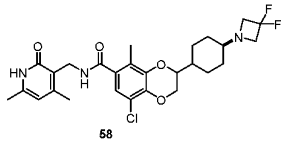 58 |
| 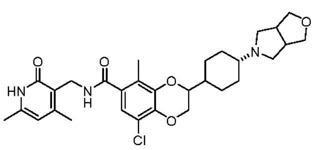 59 | 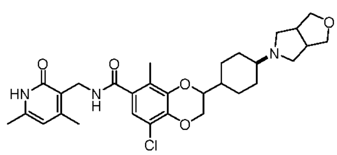 60 | 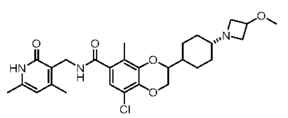 61 |
| 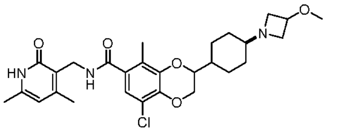 62 | 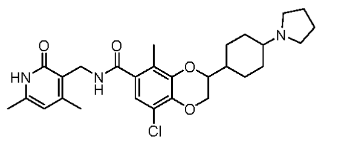 63 | 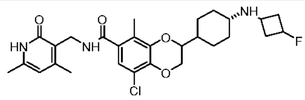 64 |
| 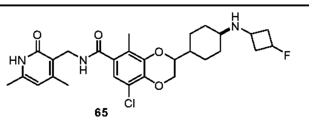 65 | 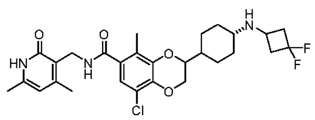 66 | 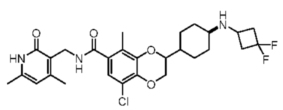 67 |
| 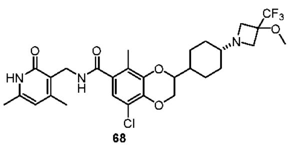 68 | 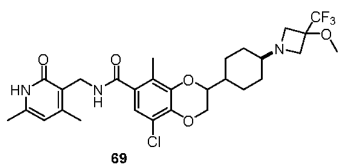 69 | 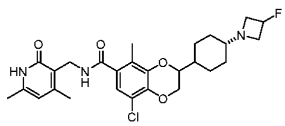 70 |
| 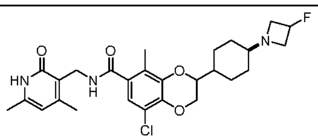 71 | 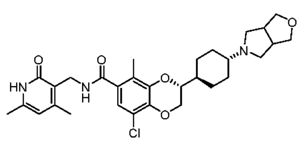 72 | 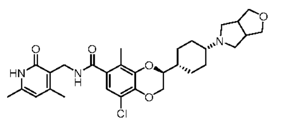 73 |
| 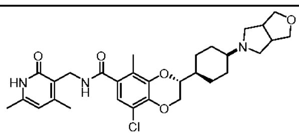 74 | 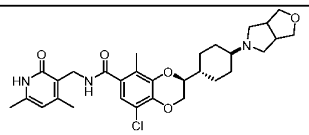 75 | 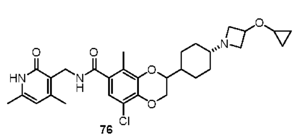 76 |

| | | |
|---|---|---|
| 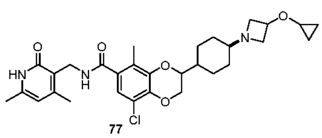 77 | 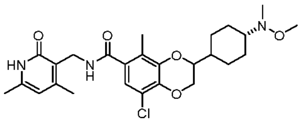 78 | 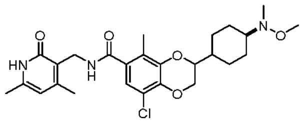 79 |
| 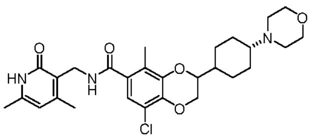 80 | 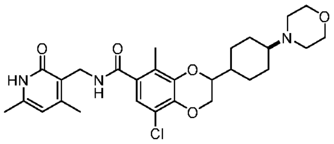 81 | 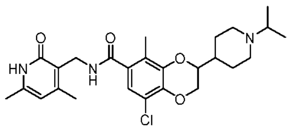 82 |
| 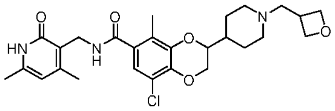 83 | 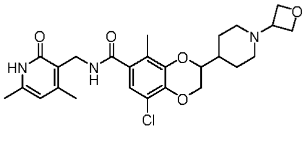 84 | 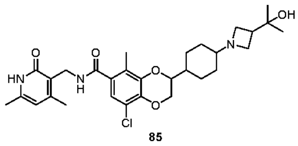 85 |
| 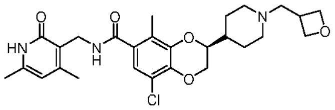 86 | 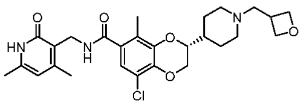 87 | 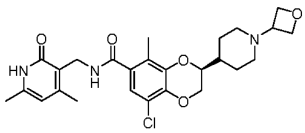 88 |
| 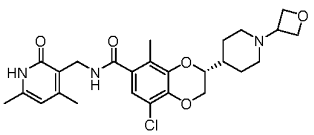 89 | 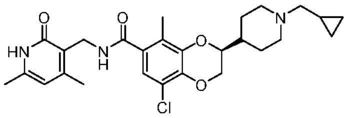 90 | 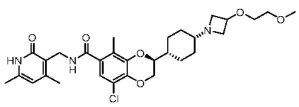 91 |
| 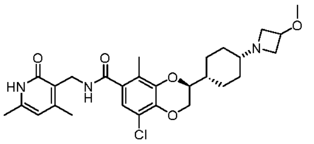 92 | 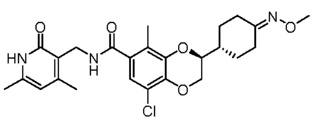 93 | 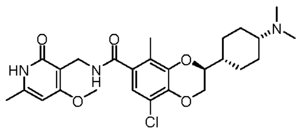 94 |
| 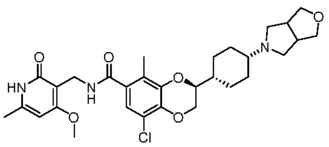 95 | 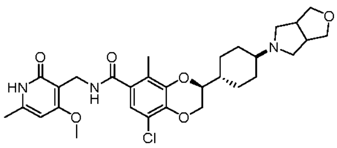 96 | 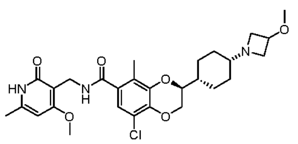 97 |
| 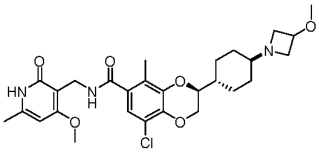 98 | 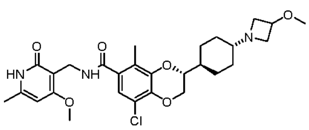 99 | 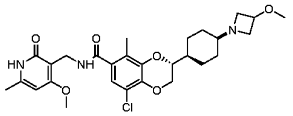 100 |
| 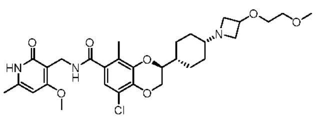 101 | 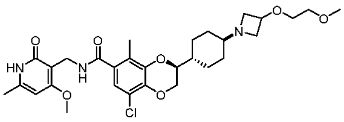 102 | 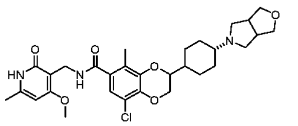 103 |

| | | |
|---|---|---|
| 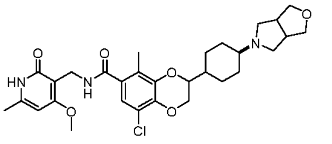 104 | 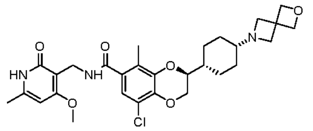 105 | 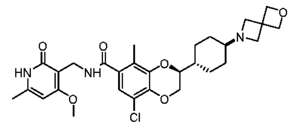 106 |
| 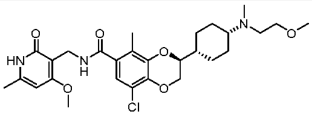 107 | 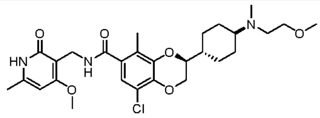 108 | 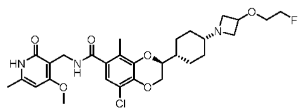 109 |
| 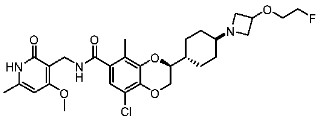 110 | 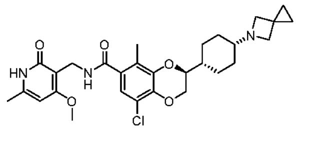 111 | 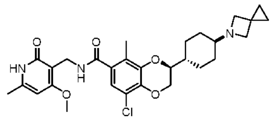 112 |
| 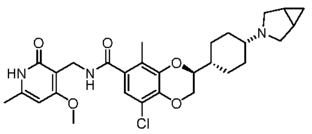 113 | 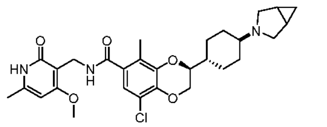 114 | 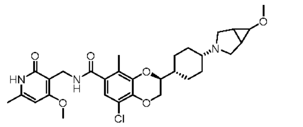 115 |
| 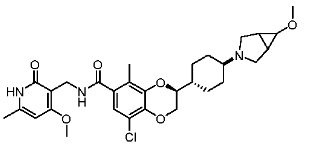 116 | 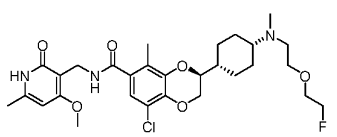 117 | 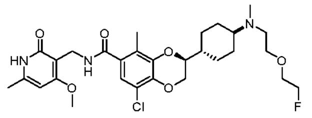 118 |
| 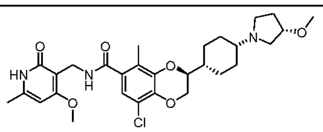 119 | 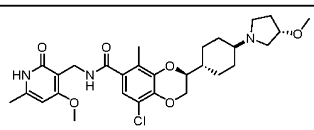 120 | 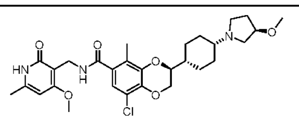 121 |
| 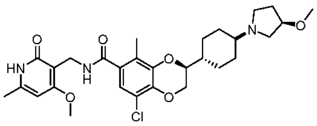 122 | 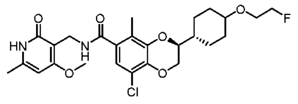 123 | 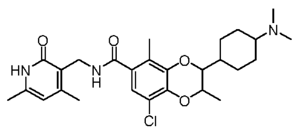 124 |
| 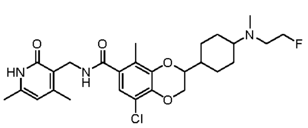 125 | 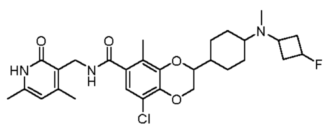 126 | 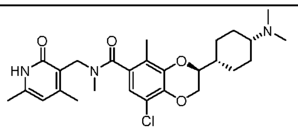 127 |
| 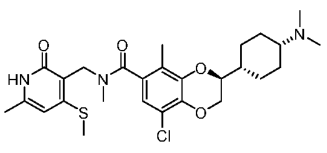 128 | 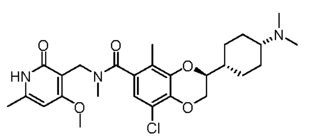 129 | 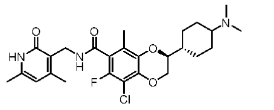 130 |

| | | |
|---|---|---|
| 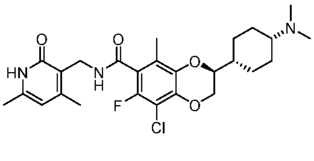 131 | 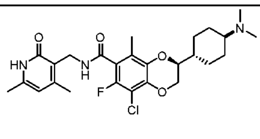 132 | 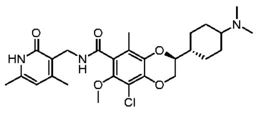 133 |
| 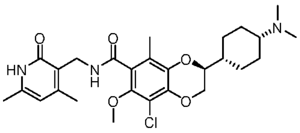 134 | 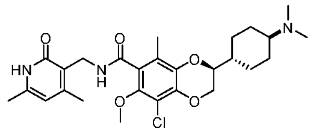 135 | 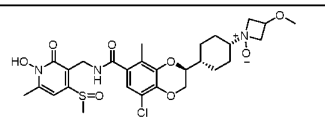 136 |
| 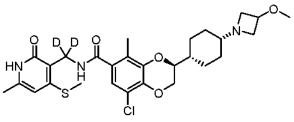 137 | 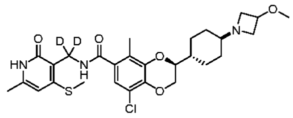 138 | 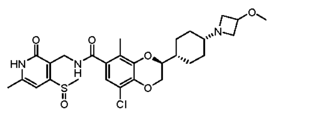 139 |
| 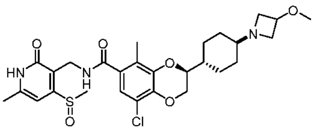 140 | 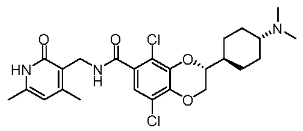 141 | 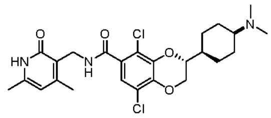 142 |
| 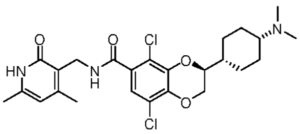 143 | 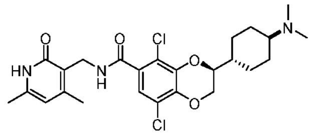 144 | 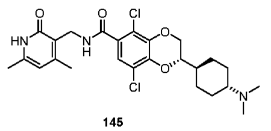 145 |
| 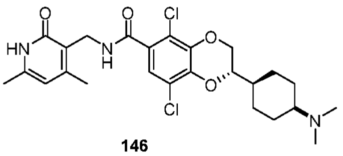 146 | 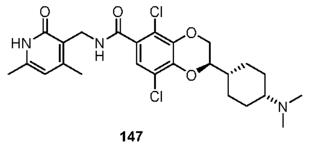 147 | 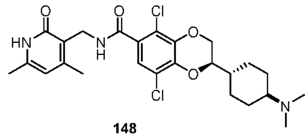 148 |
| 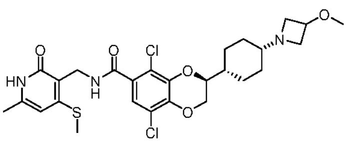 149 | 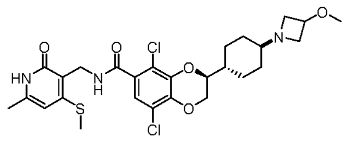 150 | 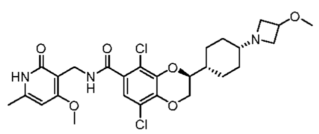 151 |
| 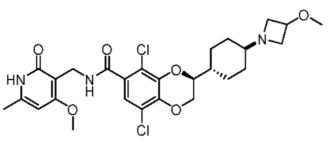 152 | 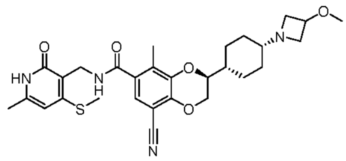 153 | 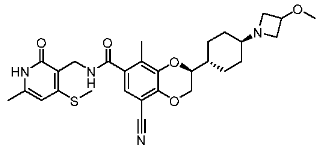 154 |
| 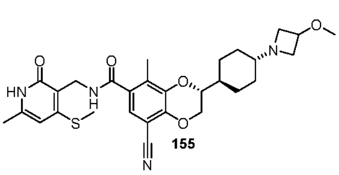 155 | 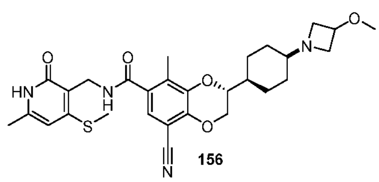 156 | 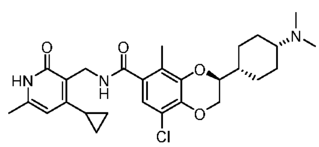 157 |

| | | |
|---|---|---|
| 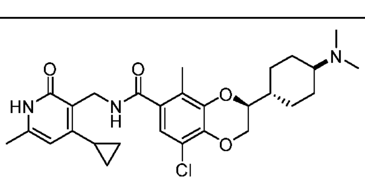 158 | 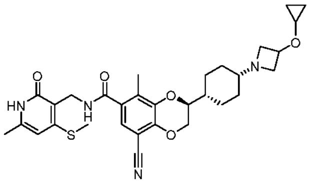 159 | 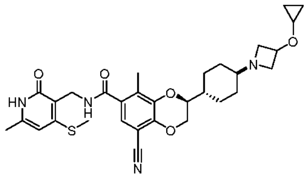 160 |
| 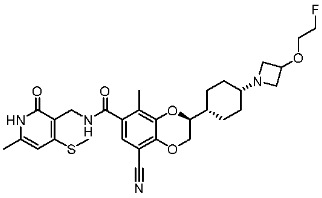 161 | 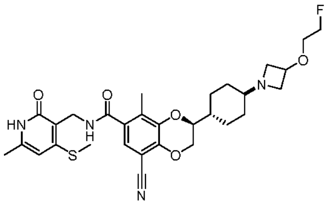 162 | 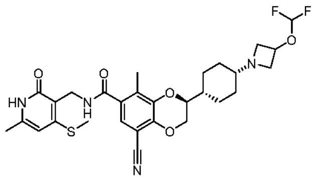 163 |
| 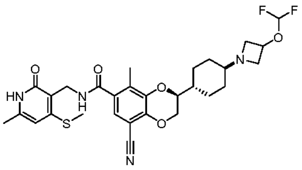 164 | 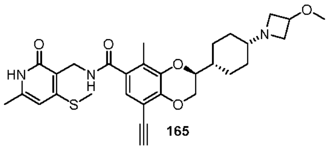 165 | 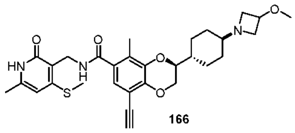 166 |
| 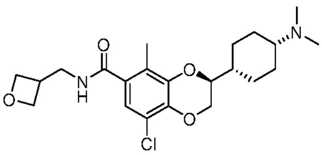 167 | 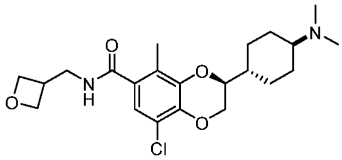 168 | 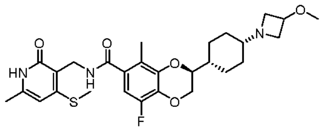 169 |
| 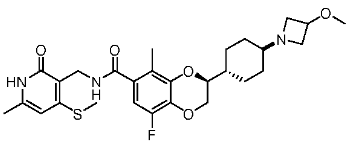 170 | 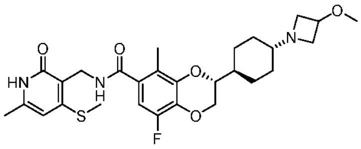 171 | 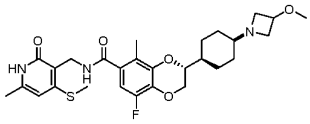 172 |
| 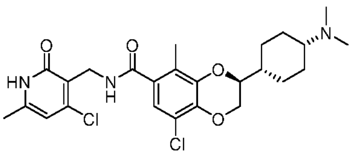 173 | 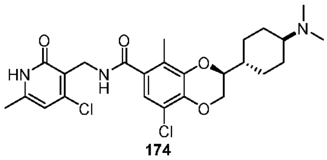 174 | 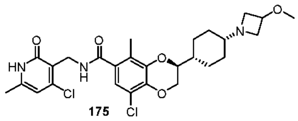 175 |
| 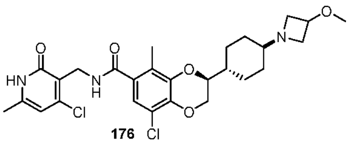 176 | 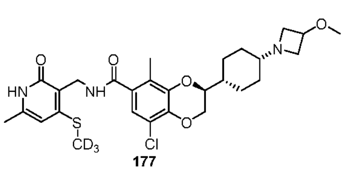 177 | 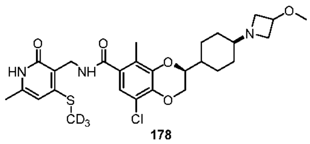 178 |
| 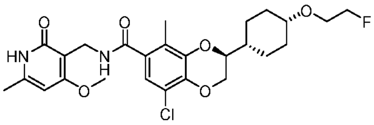 179 | 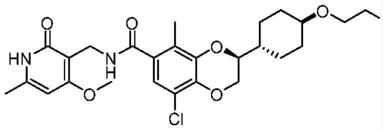 180 | 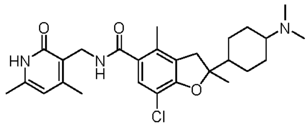 181 |

In the embodiment of the present invention, the compound of the present invention, or a deuterated compound

thereof, or a stereoisomer thereof, or a pharmaceutically acceptable salt thereof, is characterized in that, the compound represented by Formula I or Formula II is specifically:

| | | |
|---|---|---|
| 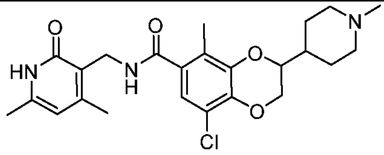<br>182 | 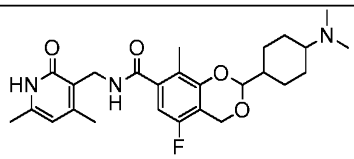<br>183 | 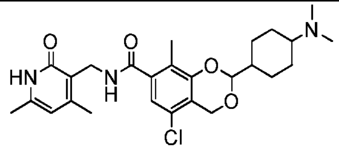<br>184 |
| 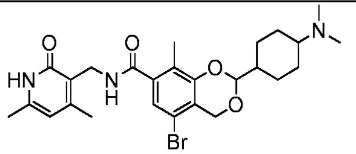<br>185 | 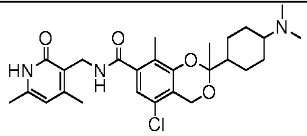<br>186 | 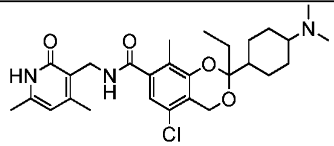<br>187 |
| 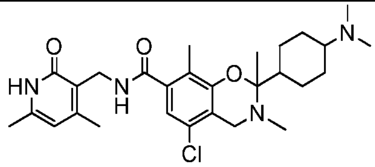<br>188 | 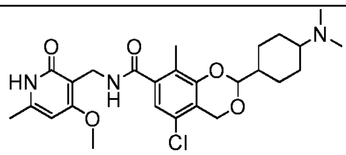<br>189 | 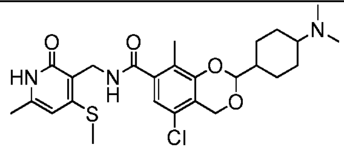<br>190 |
| 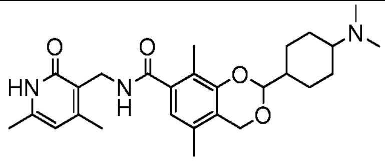<br>191 | 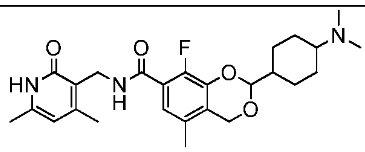<br>192 | 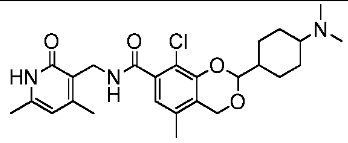<br>193 |
| 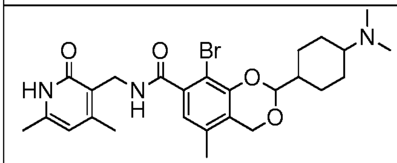<br>194 | 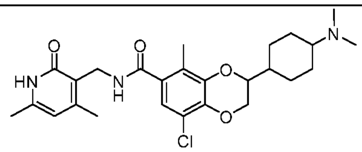<br>195 | 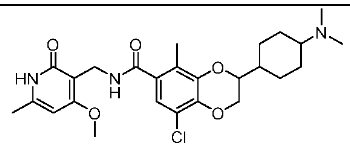<br>196 |
| 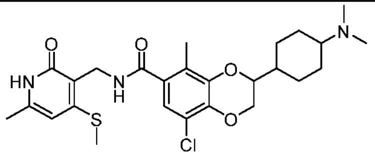<br>197 | 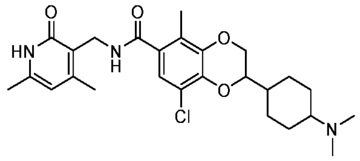<br>198 | 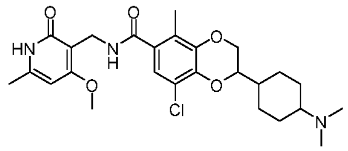<br>199 |
| 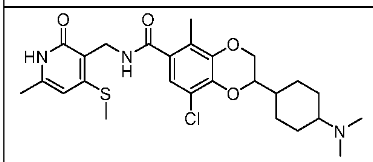<br>200 | 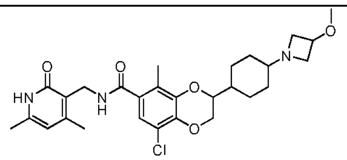<br>201 | 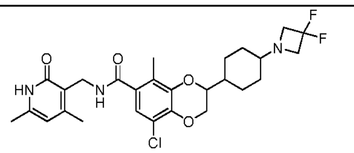<br>202 |

| | | |
|---|---|---|
| 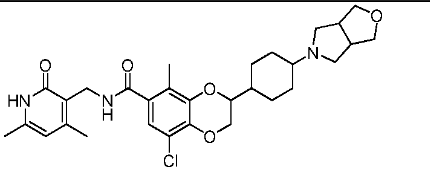<br>203 | 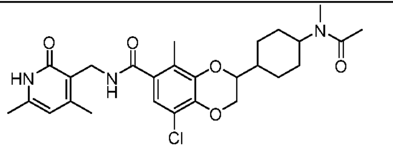<br>204 | 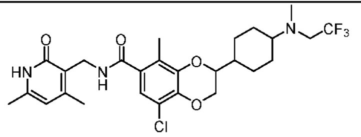<br>205 |
| 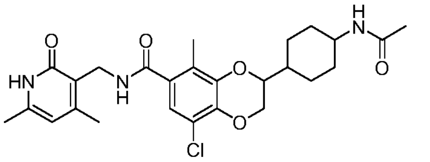<br>206 | 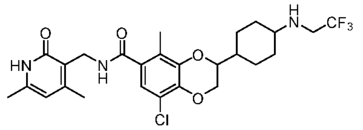<br>207 | 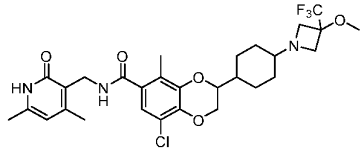<br>208 |
| 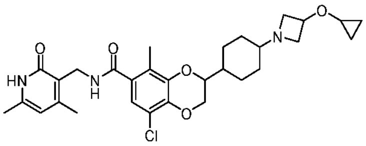<br>209 | 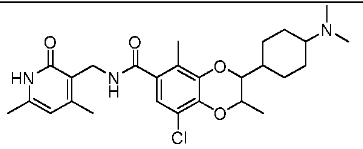<br>210 | 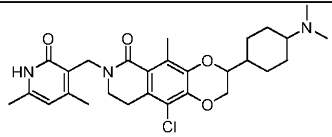<br>211 |
| 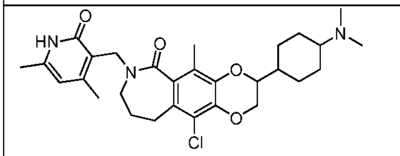<br>212 | 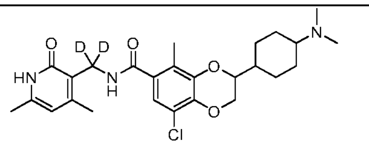<br>213 | 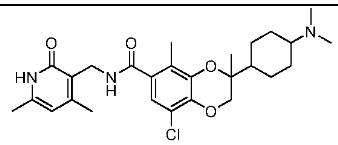<br>214 |
| 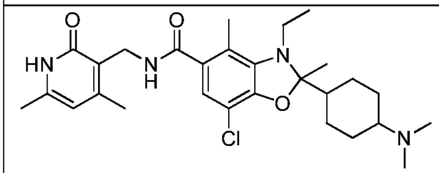<br>215 | 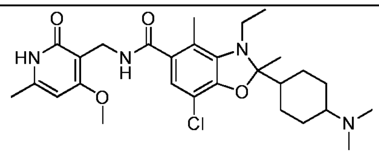<br>216 | 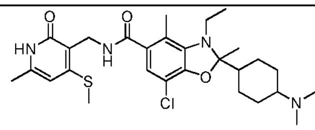 217 |
| 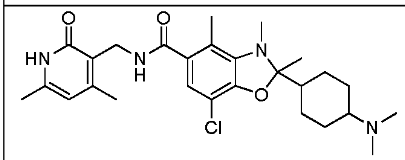<br>218 | 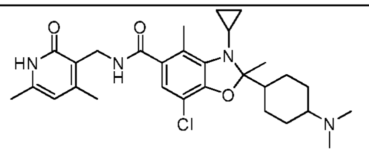<br>219 | 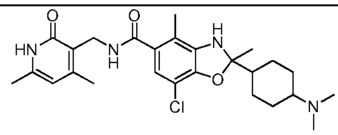<br>220 |
| 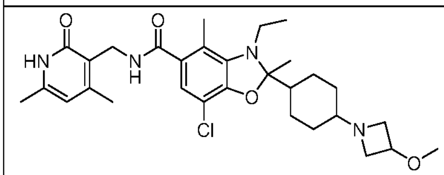<br>221 | 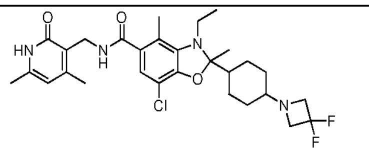<br>222 | 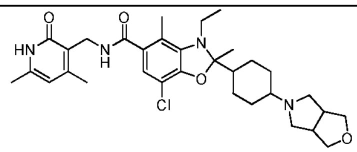<br>223 |

| | | |
|---|---|---|
| 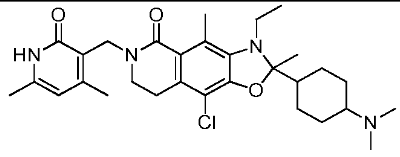 224 | 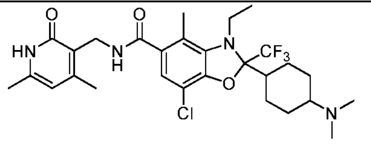 225 | 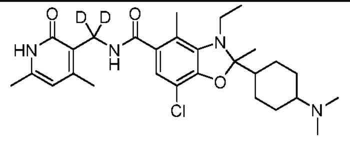 226 |
| 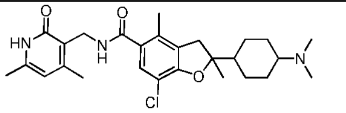 227 | 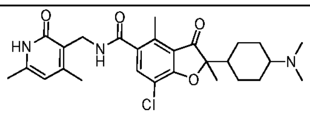 228 | 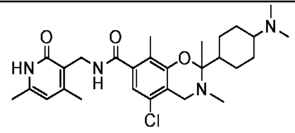 229 |
| 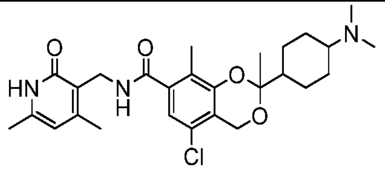 230 | 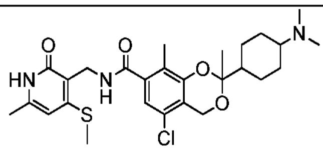 231 | 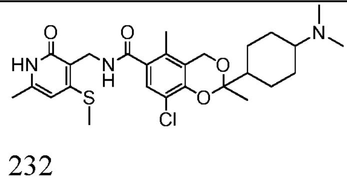 232 |
| 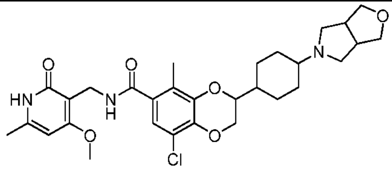 233 | 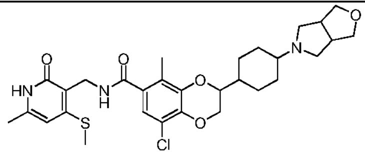 234 | 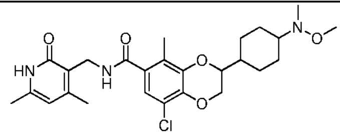 235 |
| 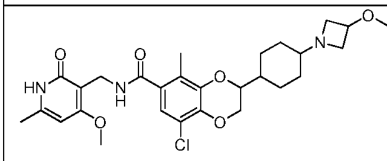 236 | 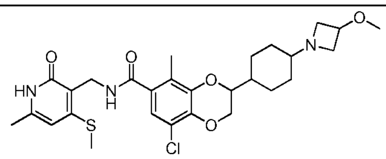 237 | 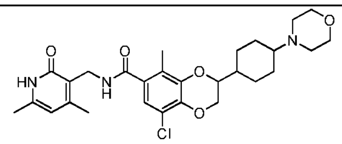 238 |
| 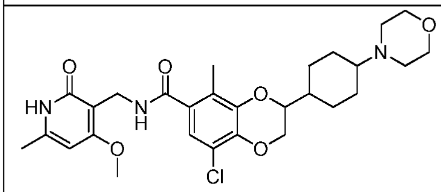 239 | 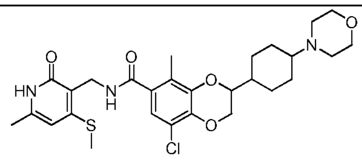 240 | 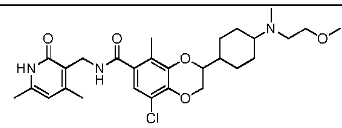 241 |
| 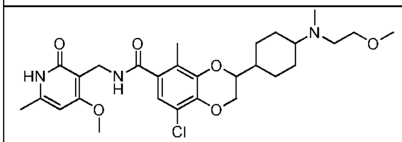 242 | 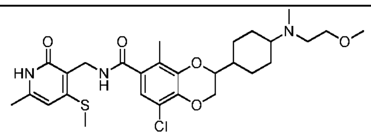 243 | 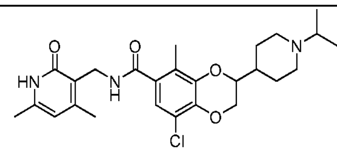 244 |
| 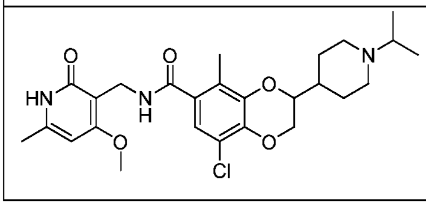 | 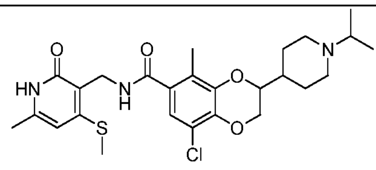 | 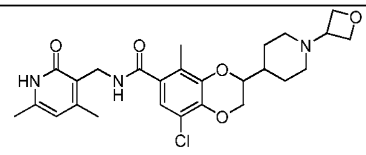 |

| | | |
|---|---|---|
| 245 | 246 | 247 |
| 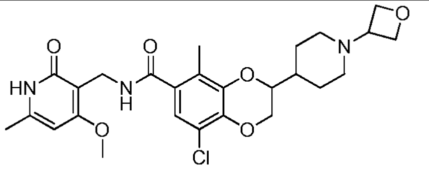<br>248 | 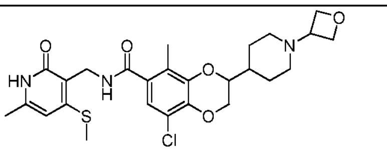<br>249 | 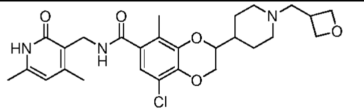<br>250 |
| 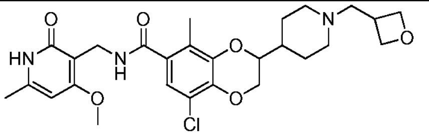<br>251 | 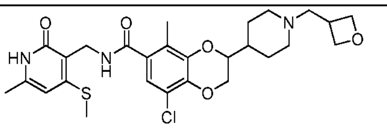<br>252 | 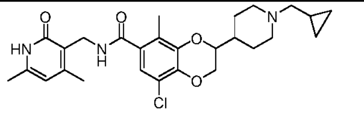<br>253 |
| 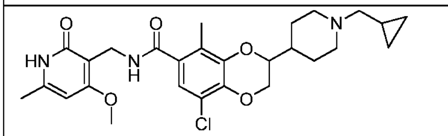<br>254 | 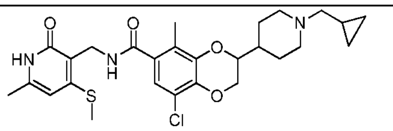<br>255 | 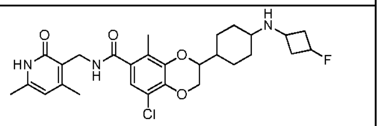<br>256 |
| 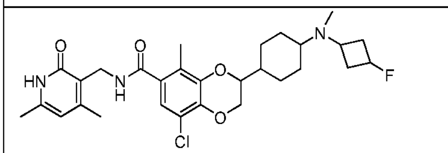<br>257 | 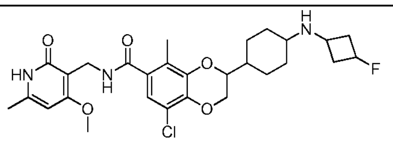<br>258 | 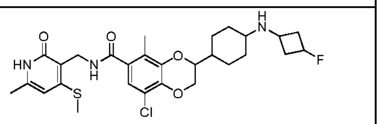<br>259 |
| 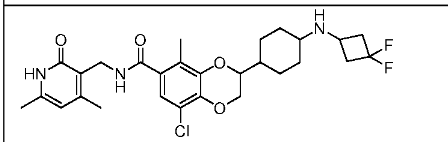<br>260 | 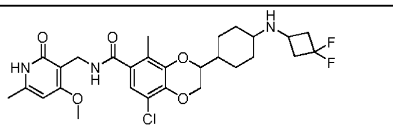<br>261 | 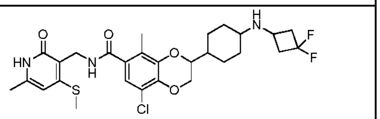<br>262 |
| 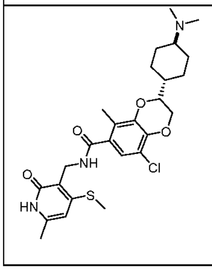<br>263 | 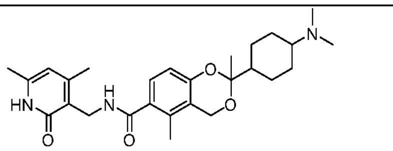<br>264 | |

The present invention further provides an intermediate compound shown as follows:

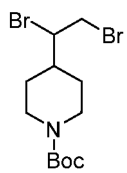

**IM-1** ,

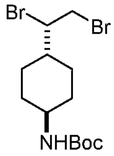
IM-2,
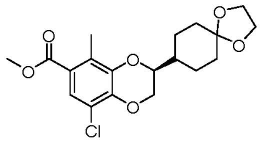
IM-3a,
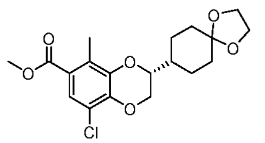
IM-3b,
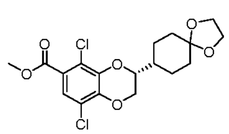
IM-4a,
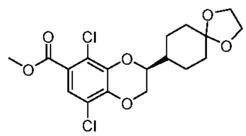
IM-4b,
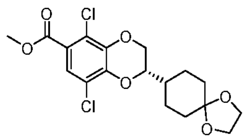
IM-4c,
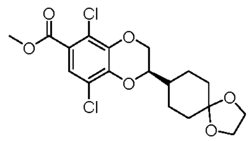
IM-4d,
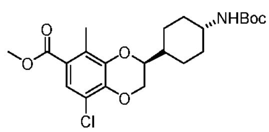
IM-5a,
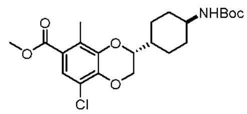
IM-5b,
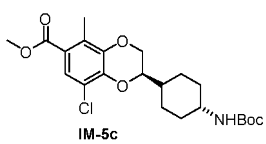
IM-5c,
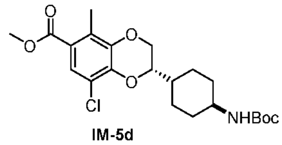
IM-5d

The present invention further provides the use of the compound of the present invention, or a stereoisomer thereof, or a pharmaceutically acceptable salt thereof in the preparation of a medicament for treating an EZH1/2-mediated disease.

Further, the EZH1/2-mediated disease is one or more selected from diseases related to inflammation, autoimmune disease, infectious disease, cancer, and precancerous syndrome.

The present invention further provides a pharmaceutical composition, comprising the compound of the present invention, or a stereoisomer thereof, or a pharmaceutically acceptable salt thereof as an active pharmaceutical ingredient, and if necessary, a pharmaceutically acceptable excipient to prepare a formulation.

The present invention further provides a preparation method of the compound of the present invention, or a deuterated compound thereof, or a stereoisomer thereof, or a pharmaceutically acceptable salt thereof.

In an embodiment of the present invention, the preparation method of the compound of

Formula I 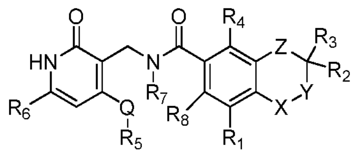

is as follows:

Step a:

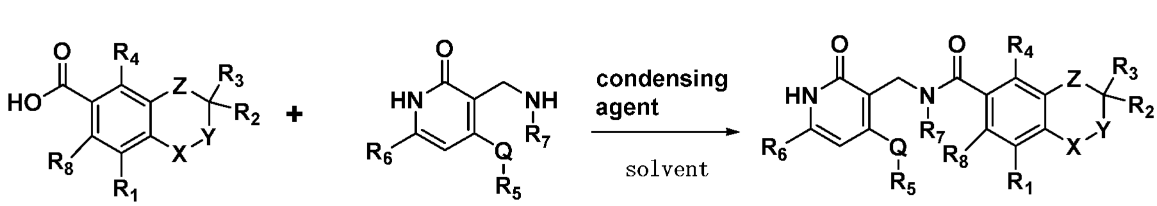


Formula I-a    Formula I-b    Formula I

The compounds of Formula I-a and Formula I-b are subjected to amide condensation reaction under the condition of condensing agent, to prepare the compound of Formula I. The compound of Formula I-b is commercially available or can be synthesized by general organic synthesis method;

Step b:

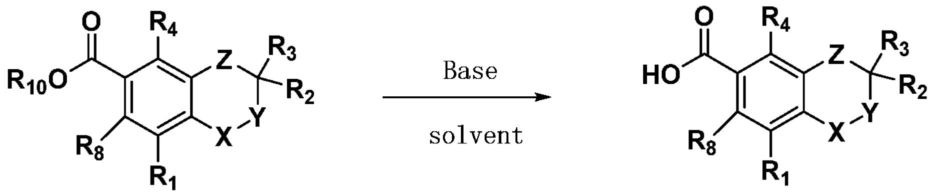


Formula I-c Formula I-a

The compound of Formula I-c is subjected to ester hydrolysis reaction under basic condition to give the compound of Formula I-a, wherein the substituent $R_{10}$ is $C_1$-$C_6$alkyl group, for example, methyl, ethyl, propyl (n-propyl and isopropyl), butyl (n-butyl, isobutyl and t-butyl), pentyl (n-pentyl, isopentyl and neopentyl) and hexyl.

Step c:

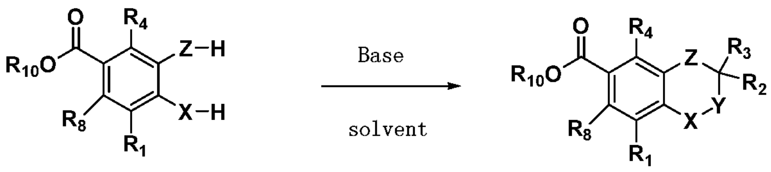


Formula I-d Formula I-c

Formula I-d is subjected to cyclization reaction under basic condition, to prepare the compound of Formula I-c.

In the above Steps a-c, the substituents $R_1$, $R_2$, $R_3$, $R_4$, $R_5$, $R_6$, $R_7$, $R_8$ in each intermediate compound of Formula I-a, Formula I-b, I-c and I-d have the same definition with the definition above.

In Steps a-c, said condensing agent includes but is not limited to O-(7-azabenzotriazole-1-yl)-bis(dimethylamino)carbonium hexafluorophosphate (HATU), O-(benzotriazole-1-yl)-bis(dimethylamino)carbonium hexafluorophosphate (HBTU), O-(5-chlorobenzotriazole-1-yl)-bis(dimethylamino)carbonium hexafluorophosphate (HCTU), O-(benzotriazole-1-yl)-bis(dimethylamino)carbonium tetrafluoroborate (TBTU), O-(N-succinimidyl)-bis(dimethylamino)carbonium tetrafluoroborate (TSTU), O-(N-endo-5-norbornene-2,3-dicarboxylimide)-bis(dimethylamino)carbonium tetrafluoroborate (TNTU), benzotriazole-1-yloxy-tris(tetrahydropyrrolyl)phosphonium hexafluorophosphate (PyBOP) and the like; wherein said base is one or two or more selected from a group consisting of organic base or inorganic base, inorganic base is one or two or more selected from a group consisting of sodium hydride, sodium carbonate, potassium carbonate, cesium carbonate, sodium bicarbonate, potassium bicarbonate, sodium hydroxide, potassium hydroxide, lithium hydroxide or barium hydroxide; organic base is one or two or more selected from a group consisting of N,N-diisopropylethylamine, triethylamine, lithium diisopropylamide, n-($C_{1-6}$alkyl)lithium, lithium hexamethyldisilazane, potassium hexamethyldisilazane, sodium hexamethyldisilazane, lithium tetramethylpiperidine, potassium butoxide, potassium pentoxide, potassium pentanolate. Among others, said solvent is one or two or more of dichloromethane, chloroform, ethyl acetate, n-hexane, methyl t-butyl ether, ethyl ether, DMF, water, methanol, ethanol, propanol, butanol.

In an embodiment of the present invention, the preparation method of Compound II

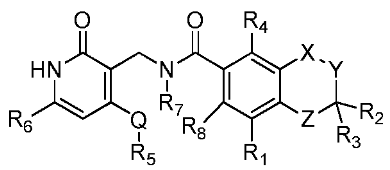

is as follows.

Step a:

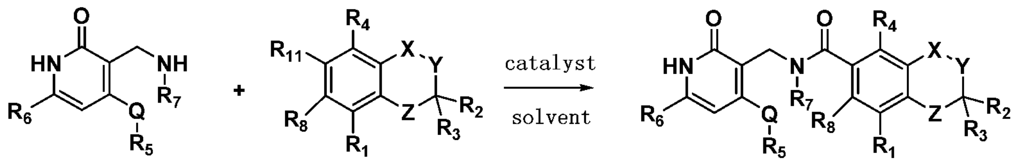


Formula II-a Formula II-b Formula II

The compounds of Formula II-a and Formula II-b are subjected to coupling reaction via insertion of carbonyl group under catalyst and solvent condition, to prepare the compound of Formula II. The compound of Formula II-a is commercially available or can be synthesized by general organic synthesis method. Among others, the catalyst is molybdenum hexacarbonyl, Pd(dppf)Cl2.DCM, K2CO3; the solvent is one or more or two of dichloromethane, chloroform, ethyl acetate, n-hexane, methyl t-butyl ether, ethyl ether, DMF, water, methanol, ethanol, propanol, butanol, toluene, 1, 4-dioxane; and the substituent $R_{11}$ is halogen group, including F, Cl, Br or I.

Step b:

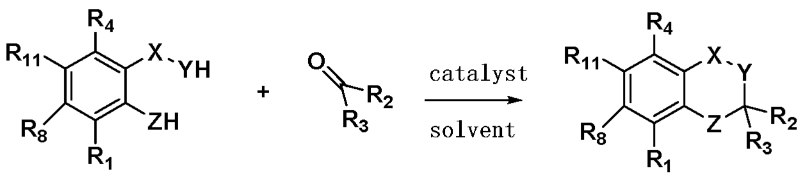


Formula II-c Formula II-d Formula II-b

The compounds of Formula II-c and Formula II-d are subjected to cyclization reaction under catalyst and solvent condition, to prepare the compound of Formula II-b. The compound of Formula II-c is commercially available or can be synthesized by general organic synthesis method. Among others, in the cyclization step, the catalyst is selected from a catalyst to catalyze the acetal formation reaction of alcohol and aldehyde, including but not limited to one or two or more of PTSA (p-tosylic acid), PPTS (pyridium p-tosylate) + molecular sieve, Bronsted acid, hydrochloric acid, and sulfuric acid. The solvent is one or two or more of dichloromethane, chloroform, ethyl acetate, n-hexane, methyl t-butyl ether, ethyl ether, DMF, water, methanol, ethanol, propanol, butanol, toluene, 1,4-dioxane.

In the above Steps a-b, the substituents $R_1$, $R_2$, $R_3$, $R_4$, $R_5$, $R_6$, $R_7$, $R_8$ in each intermediate compound of Formula II-a, Formula II-b, II-c and II-d have the same definition with the definition above.

In an embodiment of the present invention, the preparation method of Compound IIb

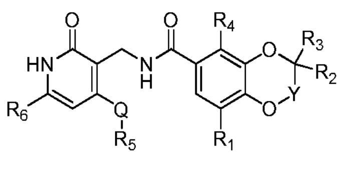

is as follows.

Step a:

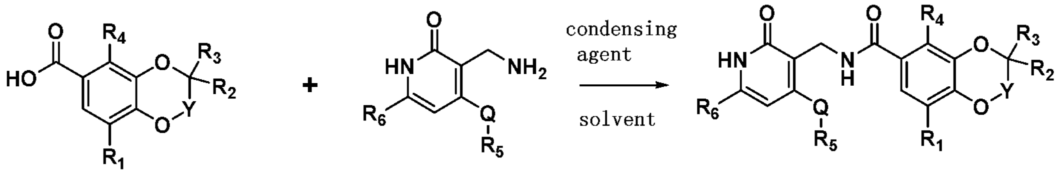


Formula IIb-a Formula IIb-b Formula IIb

The compounds of Formula IIb-a and Formula IIb-b are subject to amide condensation reaction under the condition of condensing agent, to prepare the compound of Formula lib. The compound of Formula IIb-b is commercially available or can be synthesized by general organic synthesis method.

Step b:

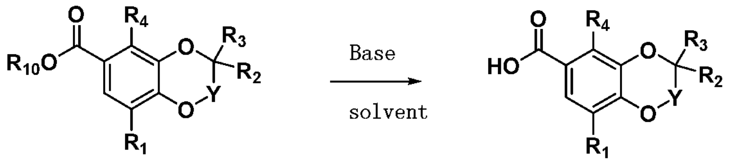


Formula IIb-c Formula IIb-a

The compound of Formula IIb-c is subjected to ester hydrolysis reaction under basic condition to give the compound of Formula IIb-a, wherein the substituent $R_{10}$ is $C_1$-$C_6$alkyl group, for example, methyl, ethyl, propyl (n-propyl and isopropyl), butyl (n-butyl, isobutyl and t-butyl), pentyl (n-pentyl, isopentyl and neopentyl) and hexyl.

Step c:

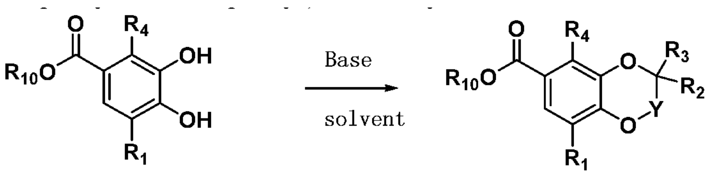


Formula IIb-d Formula IIb-c

Formula IIb-d is subjected to cyclization reaction under basic condition, to prepare the compound of Formula IIb-c.

In the above Steps a-c, the substituents $R_1$, $R_2$, $R_3$, $R_4$, $R_5$, $R_6$, $R_7$, $R_8$ in each intermediate compound of Formula IIb-a, Formula IIb-b, IIb-c and IIb-d have the same definition with the definition above.

In Steps a-c, the condensing agent includes but is not limited to O-(7-azabenzotriazole-1-yl)-bis(dimethylamino) carbonium hexafluorophosphate (HATU), O-(benzotriazole-1-yl)-bis(dimethylamino)carbonium hexafluorophosphate (HBTU), O-(5-chlorobenzotriazole-1-yl)-bis(dimethylamino)carbonium hexafluorophosphate (HCTU), O-(benzotriazole-1-yl)-bis(dimethylamino)carbonium tetrafluoroborate (TBTU), O-(N-succinimidyl)-bis(dimethylamino)carbonium tetrafluoroborate (TSTU), O-(N-endo-5-norbornene-2,3-dicarboxylimide)-bis(dimethylamino)carbonium tetrafluoroborate (TNTU), benzotriazole-1-yloxy-tris(tetrahydropyrrolyl)phosphonium hexafluorophosphate (PyBOP) and the like. Among others, the base is selected from a group consisting of organic base or inorganic base, and the inorganic base is one or two or more selected from a group consisting of sodium hydride, sodium carbonate, potassium carbonate, cesium carbonate, sodium bicarbonate, potassium bicarbonate, sodium hydroxide, potassium hydroxide, lithium hydroxide or barium hydroxide; and the organic base is one or two or more selected from a group consisting of N,N-diisopropylethylamine, triethylamine, lithium diisopropylamide, n-($C_{1-6}$alkyl)lithium, lithium hexamethyldisilazane, potassium hexamethyldisilazane, sodium hexamethyldisilazane, lithium tetramethylpiperidine, potassium butoxide, potassium pentoxide, potassium pentanolate. The solvent is one or two or more of dichloromethane, chloroform, ethyl acetate, n-hexane, methyl t-butyl ether, ethyl ether, DMF, water, methanol, ethanol, propanol, butanol.

In another two embodiments of the present invention, the preparation methods of Compound

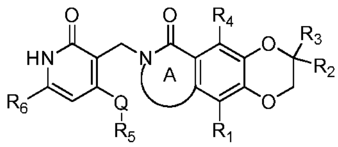 IIc , Compound IIa 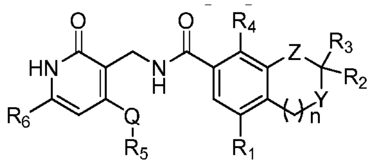

are similar to the preparation methods of Compound I and Compound lib.

The followings are the explanation and definition to the terms used in the present invention.

"Cancer" or "malignant tumor" means any of a variety of diseases characterized by uncontrolled abnormal proliferation of cells, the ability of affected cells to spread to other sites locally or through the bloodstream and lymphatic system body (i.e. metastases) and any of a number of characteristic structural and/or molecular features. "Cancer cell" refers to cells that undergo early, intermediate or late stages of multi-step tumor progression. Cancers include sarcomas, breast cancer, lung cancer, brain cancer, bone cancer, liver cancer, kidney cancer, colon cancer and prostate cancer. In some embodiments, the compound of Formula I or Formula II is used to treat a cancer selected from colon cancer, brain cancer, breast cancer, fibrosarcoma, and squamous cell carcinoma. In some embodiments, the cancer is selected from melanoma, breast cancer, colon cancer, lung cancer, and ovarian cancer. In some embodiments, the cancer treated is a metastatic cancer.

Autoimmune diseases are caused by the body's immune response to substances and tissues normally present in the body. Examples of autoimmune diseases include myocarditis, lupus nephritis, primary biliary cirrhosis, psoriasis, type 1 diabetes, Graves' disease, celiac disease, Crohn's disease, autoimmune neutropenia, juvenile arthritis, rheumatoid arthritis, fibromyalgia, Guillain-Barre syndrome, multiple sclerosis, and autoimmune retinopathy. Some embodiments of the invention relate to the treatment of autoimmune diseases such as psoriasis or multiple sclerosis.

Inflammatory diseases include a variety of conditions characterized by histopathic inflammation. Examples of inflammatory diseases include acne vulgaris, asthma, celiac disease, chronic prostatitis, glomerulonephritis, inflammatory bowel disease, pelvic inflammatory disease, reperfusion injury, rheumatoid arthritis, sarcoidosis, vasculitis, airway inflammation and interstitial cystitis caused by house dust mites. Significant overlap exists between inflammatory and autoimmune diseases. Some embodiments of the invention relate to the treatment of the inflammatory disease asthma. The immune system is often involved in inflammatory diseases, as seen in anaphylaxis and some myopathies, and many immune system disorders result in abnormal inflammation. EZH1/2-mediated diseases also include autoimmune inflammatory diseases.

The compounds and derivatives provided in the present invention may be named according to the IUPAC (International Union of Pure and Applied Chemistry) or CAS (Chemical Abstracts Service, Columbus, OH) nomenclature system.

Definition of terms used in the present invention: Unless otherwise stated, the initial definition provided by a group or term herein applies to the group or term throughout the specification. For terms that are not specifically defined herein, they should be interpreted based on the disclosure and context, with the meanings which a person skilled in the art can assign to them.

"Substitution" means that a hydrogen atom in a molecule is replaced by a different atom or molecule.

The minimum and maximum carbon atom content in a hydrocarbon group is indicated by a prefix, for example, the prefix $C_{a\sim b}$ alkyl indicates any alkyl group containing "a" to "b" carbon atoms. Thus, for example, "$C_{1\sim 4}$ alkyl" refers to an alkyl group containing 1 to 4 carbon atoms.

"Alkyl" means a saturated hydrocarbon chain having the indicated number of member atoms. For example, $C_{1\sim 6}$ alkyl refers to an alkyl group having 1 to 6 member atoms, for example, 1 to 4 member atoms. Alkyl groups can be straight or branched. Representative branched alkyl groups have one, two or three branches. Alkyl groups may be optionally substituted with one or more substituents as defined herein. Alkyl groups include methyl, ethyl, propyl (n-propyl and isopropyl), butyl (n-butyl, isobutyl and t-butyl), pentyl (n-pentyl, isopentyl and neopentyl) and hexyl. Alkyl groups may also be part of other groups such as $C_1$~$C_6$ alkoxy groups.

"Cycloalkyl" and "cycloalkane" means a saturated or partially saturated cyclic group having carbon atoms and no ring heteroatoms, and having a single ring or multiple rings (including fused and combined). For polycyclic ring systems having aromatic and non-aromatic rings containing no ring heteroatoms, the term "cycloalkyl" is applied when the binding site is on the non-aromatic carbon (for example. 5,6,7,8-tetralin-5-yl). The term "cycloalkyl" includes cycloalkenyl groups such as cyclohexenyl. Examples of cycloalkyl groups include, for example, adamantyl, cyclopropyl, cyclobutyl, cyclohexyl, cyclopentyl, cyclooctyl, cyclopentenyl, and cyclohexenyl. Examples of cycloalkyl groups including multiple bicycloalkyl ring systems are bicyclohexyl, bicyclopentyl, bicyclooctyl and the like, for example

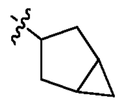, 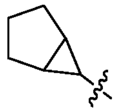, 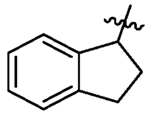

"Alkenyl" means a straight-chain or branched-chain hydrocarbon group having 2 to 10 carbon atoms and in some embodiments 2 to 6 carbon atoms or 2 to 4 carbon atoms, and having at least one ethylenic unsaturated site (>C=C<). For example, $(C_a-C_b)$alkenyl refers to an alkenyl group having a to b carbon atoms and is intended to include, for example, ethenyl, propenyl, isopropenyl, 1,3-butadienyl, and the like.

"Alkynyl" means a straight-chain or branched-chain monovalent hydrocarbon group containing at least one triple bond. The term "alkynyl" is also intended to include those hydrocarbyl groups having one triple bond and one double bond. For example, $(C_2-C_6)$alkynyl is intended to include ethynyl, propynyl, and the like.

"Halogen" is fluorine, chlorine, bromine or iodine.

"Haloalkyl" means that the hydrogen atoms in the alkyl group may be replaced by one or more halogen atoms. For example, $C_{1\sim4}$ haloalkyl refers to an alkyl group containing 1-4 carbon atoms whose hydrogen atoms are replaced by one or more halogen atoms.

"Heterocyclic ring", "heterocycloalkyl", and "heterocycloalkane" refer to a saturated ring or a non-aromatic unsaturated ring containing at least one heteroatom; wherein the heteroatom refers to nitrogen atom, oxygen atom, or sulfur atom;

"Heteroaryl ring" refers to an aromatic unsaturated ring containing at least one heteroatom; wherein the heteroatom refers to nitrogen atom, oxygen atom, sulfur atom;

The compounds of the present invention may also contain unnatural proportions of atomic isotopes such as deuterium ($^2H$), tritium ($^3H$), for example, at one or more atoms constituting the compound. Deuterated compounds generally retain comparable activity to non-deuterated compounds, and can achieve better metabolic stability when deuterated at certain sites, resulting in certain therapeutic advantages, such as increased in vivo half-life or dosage reduced demand and the like. Therefore, in the present invention, the number of deuterium atoms in the deuterated substance can be 1, 2, 3, 4, 5, 6, 7 or more;

"Stereoisomer" includes enantiomers and diastereomers. The compound of the present invention may contain asymmetric centers or chiral centers and thus exist as different stereoisomers. All stereoisomeric forms of the compounds of the present invention, including but not limited to, diastereomers, enantiomers, atropisomers, and mixtures thereof, such as racemic mixtures, constitute a part of the present invention. Many organic compounds exist in optically active forms, that is, they have the ability to rotate the plane of plane-polarized light. When describing optically active compounds, the prefixes D, L or R, S are used to indicate the absolute configuration of the molecular chiral center. The chemical structures of these stereoisomers are the same, but their three-dimensional structures are different. A particular stereoisomer may be an enantiomer, and a mixture of isomers is often referred to as an enantiomeric mixture. A 50:50 mixture of enantiomers is known as a racemic mixture or racemate, which can result in no stereoselectivity or stereospecificity during a chemical reaction. The terms "racemic mixture" and "racemate" refer to an equimolar mixture of two enantiomers, devoid of optical activity.

The term "pharmaceutically acceptable" means that a certain carrier, vector, diluent, excipient, and/or formed salt are generally chemically or physically compatible with other ingredients that constitute a pharmaceutical dosage form, and are physiologically compatible with receptors.

The pharmaceutical composition of the present invention can be in any form of reusable pharmaceutical preparations, such as: oral, injection, external preparations, and the like. The oral dosage forms include but not limited to: tablets, capsules, oral liquids, granules, pills, suspensions, and injections are selected from water injections and powder injections, and external preparations are selected from patches and ointments. All preparations can be prepared according to conventional techniques of pharmacy, such as using any one of the compound of the present invention, or a stereoisomer thereof, or a pharmaceutically acceptable salt thereof as a pharmaceutically active ingredient, and adding pharmaceutically acceptable carrier to prepare into the above-mentioned pharmaceutical dosage form suitable for taking, wherein the unit dose of the active ingredient can be 0.1mg-1000mg, such as each tablet contains 0.1mg-1000mg, preferably 5-500mg of the active ingredient.

The terms "salt" and "pharmaceutically acceptable salt" refer to an acidic and/or basic salt formed with the above-mentioned compound or a stereoisomer thereof and inorganic and/or organic acids and bases, and also include zwitterionic salts (internal salts), also include quaternary ammonium salts, such as alkyl ammonium salts. These salts may be obtained directly in the final isolation and purification of the compounds. It can also be obtained by mixing the above-mentioned compound, or a stereoisomer thereof, with a certain amount of acid or base as appropriate (for example, equivalent). These salts may form precipitates in solution and be collected by filtration, or may be recovered after evaporation of the solvent, or may be obtained by freeze-drying after reaction in an aqueous medium. Said salt in the present invention can be hydrochloride, sulfate, citrate, benzene sulfonate, hydrobromide, hydrofluoride, phosphate,

acetate, propionate, dibutyl salt, oxalate, malate, succinate, fumarate, maleate, tartrate, or trifluoroacetate.

In certain embodiments, one or more compounds of the invention may be used in combination with each other. Alternatively, the compound of the present invention may be used in combination with any other active agents for the preparation of drugs or pharmaceutical compositions for regulating cell functions or treating diseases. If a group of compounds is used, the compounds may be administered to the subject simultaneously, separately or sequentially.

Apparently, according to the above content of the present invention, according to common technical knowledge and conventional means in this field, without departing from the above basic technical idea of the present invention, other various forms of modification, replacement or change can also be made.

**The beneficial effects of the present invention are:**

The EZH1/2 inhibitor of the present invention is superior to existing EZH2 inhibitors in both in vivo and in vitro activities. The above-mentioned content of the present invention will be further described in detail below through specific implementation in the form of examples. However, this should not be construed as limiting the scope of the above-mentioned subject matter of the present invention to the following examples. All technologies realized based on the above contents of the present invention belong to the scope of the present invention.

## DESCRIPTION OF DRAWINGS

Figure 1 is a graph showing the inhibition of compound DS-3201 on tumor growth in mice;
Figure 2 is a graph showing the inhibition of Compound 8 on tumor growth in mice;
Figure 3 is a graph showing the inhibition of Compound 97 on tumor growth in mice;
Figure 4 is the inhibition of Compound 97 and 169 on tumor growth in mice;

## DETAILED DESCRIPTION OF THE INVENTION

The structure of the compound was determined by nuclear magnetic resonance (NMR) and mass spectrometry (MS). The NMR shift (δ) was given in units of $10^{-6}$(ppm). The NMR was measured by (Bruker Avance III 400 and Bruker Avance 300) nuclear magnetic apparatus. Deuterated methyl sulfoxide (DMSO-$d_6$), deuterated chloroform ($CDCl_3$) and deuterated methanol ($CD_3OD$) were used as the charaterization solvents, and tetramethylsilane (TMS) was used as the internal standard.

The LC-MS was determined by Shimadzu LC-MS 2020 (ESI). The HPLC was determined by Shimadzu LC-20A. MPLC (medium performance liquid chromatography) was conducted by Gilson GX-281 reverse phase preparative chromatography. Yantai Huanghai HSGF254 or Qingdao GF254 silica gel plate was used as the silica gel plate for thin-layer chromatography, and the specification of thin-layer chromatography separation and purification products was 0.4 mm~0.5 mm. Column chromatography generally used Yantai Huanghai silica gel 200~300 mesh silica gel as carrier.

The known starting materials of the present invention can be synthesized by or according to the methods known in the field, or can be purchased from Anneiji Chemical, Chengdu Kelon Chemical, Shaoyuan Chemical Technology, Bailingwei Technology etc.

Unless otherwise specified in Examples, the reaction was carried out under $N_2$ atmosphere. Unless otherwise specified in the examples, the solution refers to an aqueous solution. Unless otherwise specified in Examples, the reaction temperature was room temperature. Unless otherwise specified in Examples, M refers to mole per liter.

DCM: dichloromethane; THF: tetrahydrofuran; DMF: N,N-dimethylformamide;
DIEA: N,N-diisopropylethylamine;
HATU: 2-(7-azobenzotriazole)-N,N,N',N'-tetramethyluronium hexafluorophosphate;
TFA: trifluoroacetic acid; DIPEA: N, N-diisopropylethylamine.

**Intermediate Example 1: Intermediate IM-1 Synthesis:**

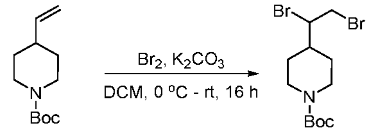


IM-1-1 IM-1

**Step 1, Compound IM-1 Synthesis:**

A single-necked flask was added with substrate **IM-1-1** (1.0 g, 4.7 mmol). The mixture was dissolved with DCM (15 mL) under stirring. Then, the mixture was added at 0 °C with **$K_2CO_3$** (1.4 g, 10.1 mmol) and **$Br_2$** (1.5 g, 9.4 mmol), and reacted at room temperature for 16 hours, with TLC monitoring. After the completion of the reaction, the mixture was added with water and DCM to extract three times. The organic phase was combined, washed with saturated saline solution, and dried with anhydrous sodium sulfate, and then purified by medium pressure liquid chromatography, to give the product **IM-1** (1.2 g, 3.2 mmol, 68.8% yield).

**Intermediate Example 2: Intermediate IM-2 Synthesis:**

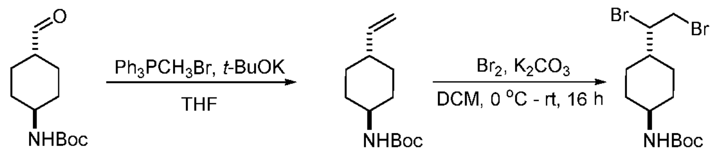


IM-2-1 IM-2-2 IM-2

**Step 1, Compound IM-2-2 Synthesis:**

A single-necked flask was added with substrate $Ph_3PCH_3Br$ (37.7 g, 105.6 mmol). The mixture was dissolved with ultra dry THF (150 mL) under stirring. Then, the mixture was added at 0 °C with ***t*-BuOK** (29.6 g, 264.3 mmol), stirred for 15 mins, and then added with **IM-2-1** (20.0 g, 88.1 mmol), and reacted at room temperature for 1 hour, with TLC monitoring. After the completion of the reaction, the mixture was added with water to quench, and condensated under reduced pressure. The residue was dissolved with petroleum ether, and filtered. The filtrate was washed with saturated saline solution, and dried with anhydrous sodium sulfate and then condensated under reduced pressure, to give the crude product **IM-2-2** (25.0 g, crude).

**Step 2, Compound IM-2 Synthesis:**

A single-necked flask was added with substrate **IM-2-2** (25.0 g, crude). The mixture was dissolved with DCM (200 mL) under stirring. Then, the mixture was added at 0 °C with **$K_2CO_3$** (25.5 g, 185.0 mmol) and **$Br_2$** (28.2 g, 176.2 mmol), and reacted at room temperature for 16 hours, with TLC monitoring. After the completion of the reaction, the mixture was added with water and DCM to extract three times. The organic phase was combined, washed with saturated saline solution, and dried with anhydrous sodium sulfate, and then purified by medium pressure liquid chromatography, to give the product IM-2 (28.6 g, 74.3 mmol, 84.3% yield).

**Intermediate Example 3: Intermediate IM-3a and IM-3b Synthesis:**

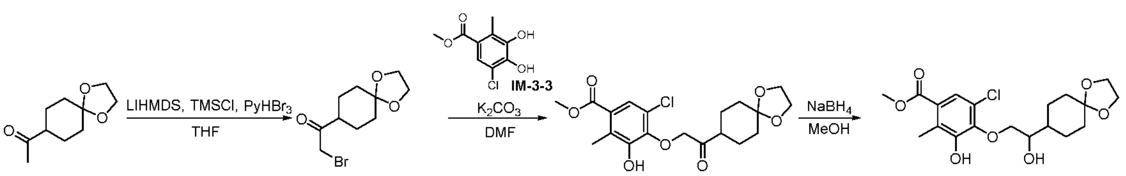


IM-3-1 IM-3-2 IM-3-4 IM-3-5

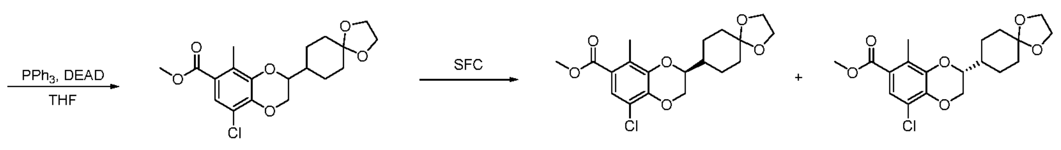


IM-3-6 IM-3a IM-3b

**Step 1, Compound IM-3-2 Synthesis:**

Under nitrogen protection, a three-necked flask was added with substrate **IM-3-1** (20.0 g, 108.7 mmol). The mixture was dissolved with THF (400 mL) under stirring, and then under nitrogen protection and at 0°C added dropwise with **LIHMDS** (218.0 mmol, 167.7 mL), stirred at 0°C for 30 mins, and then added with **TMSCI** (24.0 g, 221.0 mmol), further stirred at 0°C for 1 hour, and then added with $PyHBr_3$ (53.0 g, 166.2 mmol), reacted at 0 °C for 1 hour, with TLC(PE: EA = 10: 1) monitoring. After the completion of the reaction, the reaction mixture was added to saturated sodium bicarbonate solution, and added with ethyl acetate to extract three times. The organic phase was combined, washed with saturated saline solution, and dried with anhydrous sodium sulfate, to give the crude product **IM-3-2** (30.0 g, crude). LCMS ($ESI^+$) m/z: 263.0, 265.0 $[M+H]^+$

**Step 2, Compound IM-3-4 Synthesis:**

A single-necked flask was added with substrate **IM-3-3** (19.0 g, 87.7 mmol). The mixture was dissolved with DMF (300 mL) under stirring, and then added with **IM-3-2** (26.0 g, 98.8 mmol) and $K_2CO_3$ (40.0 g, 289.9 mmol), stirred at 50°C to react for 1 hour, with LC-MS monitoring. After the completion of the reaction, the reaction mixture was adjusted with 1 N HCl solution to pH = 6, added with ethyl acetate to extract three times. The organic phase was combined, washed with saturated saline solution, and dried with anhydrous sodium sulfate, to give the crude product **IM-3-4** (39.6 g, crude). LCMS ($ESI^+$) m/z: 397.0 $[M-H]^-$

**Step 3, Compound IM-3-5 Synthesis:**

A single-necked flask was added with substrate **IM-3-4** (39.0 g, 97.8 mmol). The mixture was dissolved with MeOH (300 mL) under stirring, and then at 0°C added with $NaBH_4$ (4.0 g, 105.8 mmol), and stirred to react for 0.5 hours, with LC-MS monitoring. After the completion of the reaction, the reaction mixture was condensated under reduced pressure. Water was added to dissolve the crude product. The reaction mixture was adjusted with 1 N HCl solution to pH = 6-7, added with ethyl acetate to extract three times. The organic phase was combined, washed with saturated saline solution, and dried with anhydrous sodium sulfate, and then purified by medium pressure liquid chromatography to give the product **IM-3-5** (26.0 g, 64.9 mmol, 66.3% yield). LCMS ($ESI^+$) m/z: 401.8 $[M+H]^+$

**Step 4, Compound IM-3-6 Synthesis:**

A single-necked flask was added with substrate **IM-3-5** (26.0 g, 64.9 mmol). The mixture was dissolved with THF (300 mL) under stirring, and then at 0°C added with $PPh_3$ (21.0 g, 80.2 mmol) and DIAD (16.0 g, 79.1 mmol), stirred at 0°C to react for 0.5 hours and then reacted at room temperature overnight, with LC-MS monitoring. After the completion of the reaction, the reaction mixture was condensated under reduced pressure, purified by medium pressure liquid chromatography to give the crude product **IM-3-6** (26.0 g, crude). LCMS ($ESI^+$) m/z: 383.8 $[M+H]^+$

**IM-3-6** can be resolved by SFC to give **IM-3a** and **IM-3b, LCMS** ($ESI^+$) m/z: 383.1 $[M+H]^+$.

**IM-3a NMR: $^1$H NMR** (600 MHz, DMSO- $d_6$) $\delta$ 7.44 (s, 1H), 4.56 (dd, $J$ = 11.4, 2.2 Hz, 1H), 4.17-4.09 (m, 1H), 4.02-3.98 (m, 1H), 3.88-3.84 (m, 4H), 3.79 (s, 3H), 2.34 (s, 3H), 19.4-1.92 (m, 1H), 1.72-1.65 (m, 4H), 1.50-1.39 (m, 4H).

**Intermediate Example 4: Intermediate IM-4a, IM-4b, IM-4c and IM-4d Synthesis**

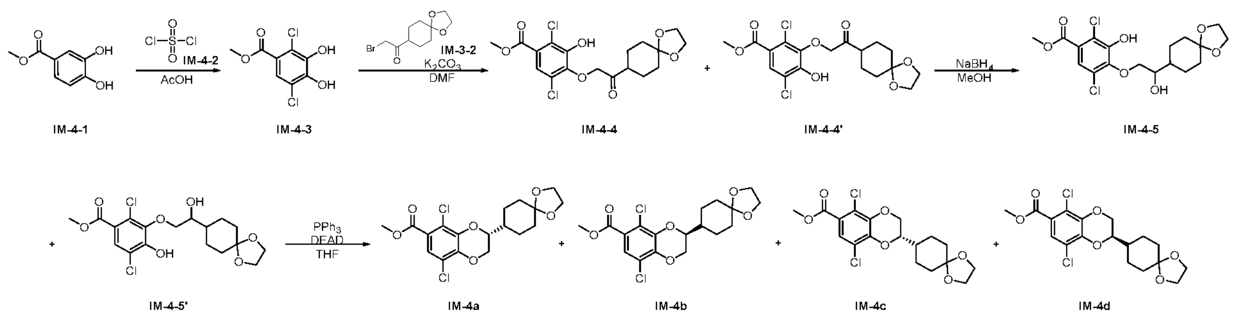

**Step 1, Compound IM-4-3 Synthesis:**

A single-necked flask was added with substrate **IM-4-1** (2.0 g, 11.9 mmol). The mixture was dissolved with AcOH (20 mL) under stirring, and then added dropwise with **IM-4-2** (2.0 g, 14.8 mmol, 1.2 mL), stirred at 60°C for 2 hours, with LC-MS monitoring. After the completion of the reaction, the reaction mixture was condensated under reduced pressure, added with water and ethyl acetate to extract three times. The organic phase was combined, washed with saturated saline solution, and dried with anhydrous sodium sulfate, to give the crude product **IM-4-3** (2.1 g, crude).
LCMS ($ESI^+$) m/z: 237.0 $[M+H]^+$

**Step 2, Compound IM-4-4 and IM-4-4' Synthesis:**

Following the synthesis method of Example **IM-3,** Step **2,** except replacing Step **2, IM-3-3** with **IM-4-3** (2.1 g, 8.9 mmol), the same synthesis method was applied, to give the compound **IM-4-4** and **IM-4-4'**(1.8 g, 4.3 mmol, 48.3% yield), LCMS ($ESI^+$) m/z: 419.3 $[M+H]^+$.

**Step 3, Compound IM-4-5 and IM-4-5' Synthesis:**

Following the synthesis method of Example **IM-3,** Step **3,** except replacing Step **3, IM-3-4** with **IM-4-4** and **IM-4-4** '(1.8 g, 4.3 mmol), the same synthesis method was applied, to give the compound **IM-4-5** and **IM-4-5'**(1.3 g, 3.1 mmol, 71.8% yield), LCMS ($ESI^+$) m/z: 421.3 $[M+H]^+$.

**Step 4, Compound IM-4a, IM-4b, IM-4c and IM-4d Synthesis:**

Following the synthesis method of Example **IM-3,** Step **4,** except replacing Step **4, IM-3-5** with **IM-4-5 and IM-4-5'** (1.3 g, 3.1 mmol), the same synthesis method was applied, and the obtained compound was resolved by SFC to give **IM-4a** (72.0 mg, 178.5 μmol), **IM-4b** (170.0 mg, 421.5 μmol), **IM-4c** (74.0 mg, 183.5 μmol) and **IM-4d** (166.0 mg, 411.6 μmol), LCMS ($ESI^+$) m/z: 403.3 $[M+H]^+$.

**Intermediate Example 5: Intermediate IM-5a, IM-5b, IM-5c and IM-5d**

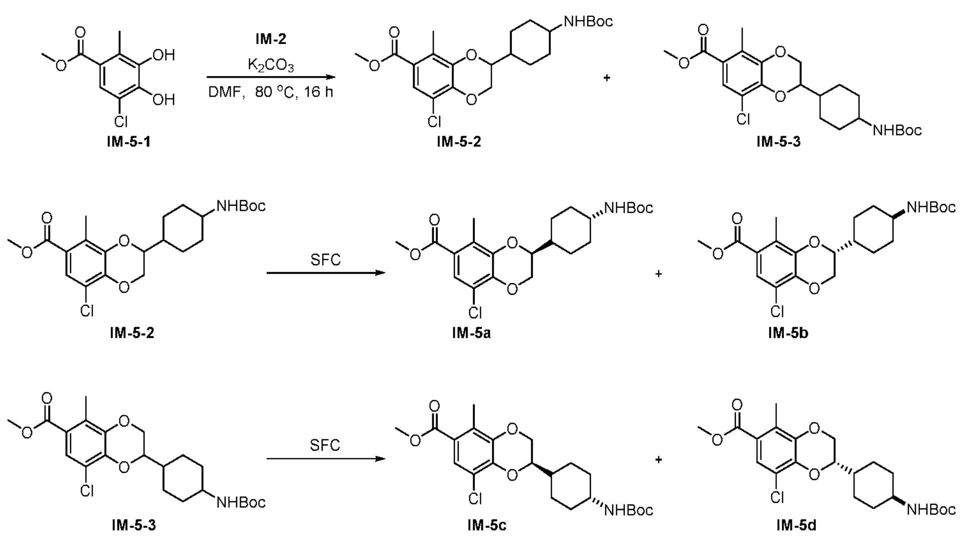


IM-5-1 IM-5-2 + IM-5-3

IM-5-2 IM-5a + IM-5b

IM-5-3 IM-5c + IM-5d

**Step 1, Compound IM-5-2 and IM-5-3 Synthesis:**

Following the synthesis method of Example **IM-3,** Step **2,** except replacing Step **2, IM-3-3** with **IM-5-1** (2.0 g, 9.2 mmol), the same synthesis method was applied, to give the compound **IM-5-2** (1.0 g, 2.3 mmol, 24.7% yield), **IM-5-3** (1.0 g, 2.3 mmol, 24.7% yield), LCMS ($ESI^+$) m/z: 439.2 $[M+H]^+$.

**Step 2, Compound IM-5a and IM-5b Synthesis:**

Compound **IM**-5a and **IM**-5b can be obtained by SFC resolution from Compound **IM-5-2.**

**Step 3, Compound IM-5c and IM-5d Synthesis:**

Compound **IM-5c** and **IM**-5d can be obtained by SFC resolution from Compound **IM**-5-3.

**Example 1, Compound 1 Synthesis:**

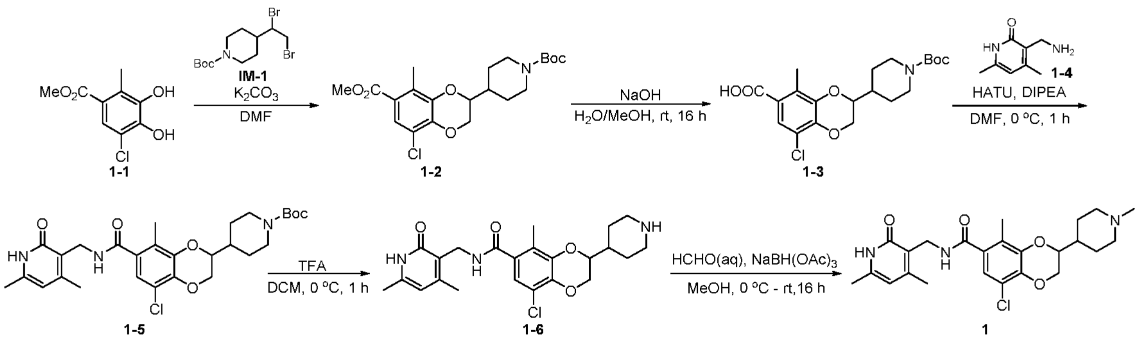


**Step 1, Compound 1-2 Synthesis:**

A single-necked flask was added with substrate **1-1** (200.0 mg, 0.9 mmol). The mixture was dissolved with DMF (5 mL) under stirring, and then added with **IM-1** (411.2 mg, 1.1 mmol), heated to 80 °C to react for 16 hours, with TLC monitoring. After the completion of the reaction, the reaction mixture was cooled to room temperature, added with water and ethyl acetate to extract three times. The organic phase was combined, washed with saturated saline solution, and dried with anhydrous sodium sulfate, and then purified by medium pressure liquid chromatography, to give the product **1-2** (61.0 mg, 143.2 μmol, 15.9% yield).

**Step 2, Compound 1-3 Synthesis:**

A single-necked flask was added with substrate **1-2** (61.0 mg, 143.2 μmol), **NaOH** (11.5 mg, 287.5 μmol). The mixture was dissolved with MeOH (2 mL) and water (2 mL) under stirring. Then, reacted at room temperature for 16 hours, with LC-MS monitoring. After the completion of the reaction, the reaction mixture was condensated under reduced pressure, added with water, and further extracted 6 times with dichloromethane/methanol (10/1 volume ratio) solution. The organic phase was combined, washed with saturated saline solution, and dried with anhydrous sodium sulfate, and then purified by medium pressure liquid chromatography, to give the product **1-3** (52.0 mg, 126.2 μmol, 88.3 % yield). LCMS ($ESI^+$) m/z: 412.1 $[M+H]^+$

**Step 3, Compound 1-5 Synthesis:**

A single-necked flask was added with substrate **1-3** (52.0 mg, 126.2 μmol). The mixture was dissolved with DMF (2 mL) under stirring. Then, the mixture was added at 0 °C with DIPEA (49.0 mg, 0.4 mmol, 66.0 μL), stirred for 5 mins, and then added with HATU (72.0 mg, 189.5 μmol), further stirred for 5 mins, and then added with **1-4** (19.2 mg, 126.1 μmol) to react for 1 hour, with LC-MS monitoring. After the completion of the reaction, the mixture was added with water to quench, the reaction mixture was condensated under reduced pressure, extracted with ethyl acetate three times. The organic phase was combined, washed with saturated saline solution, and dried with anhydrous sodium sulfate, and then purified by medium pressure liquid chromatography, to give the product **1-5** (67.8 mg, 124.2 μmol). LCMS ($ESI^+$) m/z: 546.2 $[M+H]^+$

**Step 4, Compound 1-6 Synthesis:**

A single-necked flask was added with substrate **1-5** (67.8 mg, 124.2 μmol). The mixture was dissolved with DCM (3 mL) under stirring. Then, the mixture was added at 0 °C with TFA (3 mL), under ice bath stirred for 1 hour, with LC-MS monitoring. After the completion of the reaction, the reaction mixture was condensated under reduced pressure, added

with saturated sodium bicarbonate to quench, extracted with ethyl acetate three times. The organic phase was combined, washed with saturated saline solution, and dried with anhydrous sodium sulfate, and then purified by medium pressure liquid chromatography, to give the product **1-6** (20.0 mg, 44.9 μmol, 36.2% yield). LCMS ($ESI^+$) m/z: 446.2 $[M+H]^+$

**Step 5, Compound 1 Synthesis:**

A single-necked flask was added with substrate **1-6** (20.0 mg, 44.9 μmol). The mixture was dissolved with MeOH (2 mL) under stirring. Then, the mixture was added at 0 °C with $NaBH(OAc)_3$ (19.1 mg, 0.09 mmol), formaldehyde (45.0 mg, 37%wt, 1.5 mmol), stirred at room temperature for 16 hours, with LC-MS monitoring. After the completion of the reaction, the mixture was added with water to quench, the reaction mixture was condensated under reduced pressure, extracted with ethyl acetate three times. The organic phase was combined, washed with saturated saline solution, and dried with anhydrous sodium sulfate, and then purified by medium pressure liquid chromatography, to give the product **1** (3.0 mg, 6.5 μmol, 14.5% yield). LCMS ($ESI^+$) m/z: 460.2 $[M+H]^+$

**$^1$H NMR** (600 MHz, Methanol-$d_4$) δ 6.93 (m, 1H), 6.10 (s, 1H), 4.58-4.56 (m, 1H), 4.43-4.41 (m, 3H), 4.10-4.05 (m, 1H), 3.98-3.92 (m, 1H), 3.02-2.96 (m, 2H), 2.35 (s, 3H), 2.33-2.31 (m, 3H), 2.23 (s, 3H), 2.17 (s, 3H), 2.02-1.95 (m, 1H), 1.80-1.75 (m, 1H), 1.72-1.68 (m, 1H), 1.62-1.56 (m, 2H).

**Example 2, Compound 2 Synthesis:**

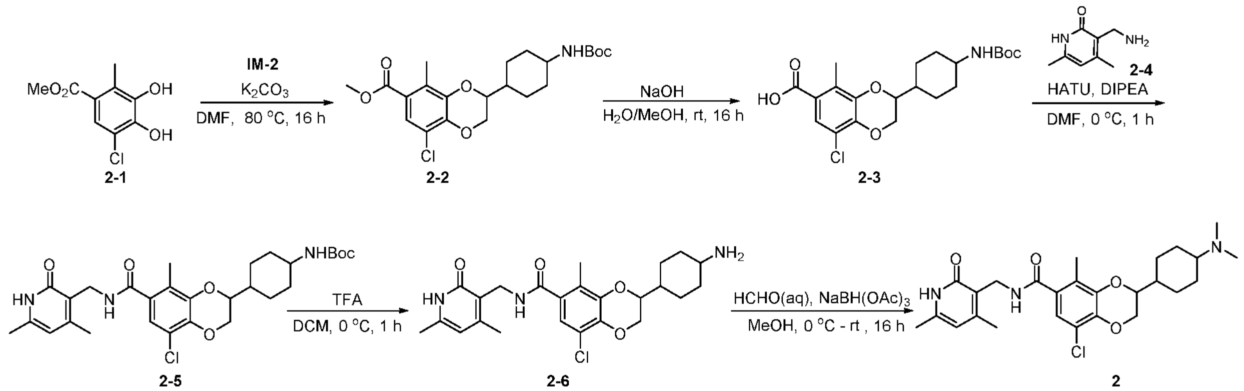


2-1 2-2 2-3 2-5 2-6 2

**Step 1, Compound 2-2 Synthesis:**

A single-necked flask was added with substrate **2-1** (1.2 g, 5.5 mmol). The mixture was dissolved with DMF (20 mL) under stirring, and then added with **IM-2** (2.6 g, 6.8 mmol), heated to 80 °C to react for 16 hours, with TLC monitoring. After the completion of the reaction, the reaction mixture was returned to room temperature, added with water and ethyl acetate to extract three times. The organic phase was combined, washed with saturated saline solution, and dried with anhydrous sodium sulfate, and then purified by medium pressure liquid chromatography, to give the product **2-2** (220.0 mg, 0.5 mmol, 9.2% yield).

**Step 2, Compound 2-3 Synthesis:**

A single-necked flask was added with substrate **2-2** (220.0 mg, 0.5 mmol), **NaOH** (40.0 mg, 1.0 mmol). The mixture was dissolved with MeOH (4 mL) and water (4 mL) under stirring. Then, reacted at room temperature for 16 hours, with LC-MS monitoring. After the completion of the reaction, the reaction mixture was condensated under reduced pressure, added with water, and further extracted 6 times with dichloromethane/methanol (10/1 volume ratio) solution. The organic phase was combined, washed with saturated saline solution, and dried with anhydrous sodium sulfate, and then purified by medium pressure liquid chromatography, to give the product **2-3** (212.0 mg, 0.5 mmol, 99.5% yield). LCMS ($ESI^+$) m/z: 426.2 $[M+H]^+$

**Step 3, Compound 2-5 Synthesis:**

A single-necked flask was added with substrate **2-3** (212.0 mg, 0.5 mmol). The mixture was dissolved with DMF (5 mL) under stirring. Then, the mixture was added at 0 °C with DIPEA (66.8 mg, 0.5 mmol, 90.0 μL), stirred for 5 mins, and

then added with HATU (196.3 mg, 0.5 mmol), further stirred for 5 mins, and then added with **2-4** (94.3 mg, 0.6 mmol) to react for 1 hour, with LC-MS monitoring. After the completion of the reaction, the mixture was added with water to quench, and the reaction mixture was condensated under reduced pressure, extracted with ethyl acetate three times. The organic phase was combined, washed with saturated saline solution, and dried with anhydrous sodium sulfate, and then purified by medium pressure liquid chromatography, to give the product **2-5** (110.0 mg, 0.2 mmol, 39.3% yield). LCMS ($ESI^+$) m/z: 560.2 $[M+H]^+$

**Step 4, Compound 2-6 Synthesis:**

A single-necked flask was added with substrate **2-5** (110.0 mg, 0.2 mmol). The mixture was dissolved with DCM (5 mL) under stirring. Then, the mixture was added at 0 °C with TFA (5 mL), stirred at room temperature for 1 hour, with LC-MS monitoring. After the completion of the reaction, the reaction mixture was condensated under reduced pressure, added with saturated sodium bicarbonate to quench, extracted with ethyl acetate three times. The organic phase was combined, washed with saturated saline solution, and dried with anhydrous sodium sulfate, and then purified by medium pressure liquid chromatography, to give the product **2-6** (40.0 mg, 0.09 mmol, 43.5% yield). LCMS ($ESI^+$) m/z: 460.2 $[M+H]^+$

**Step 5, Compound 2 Synthesis:**

A single-necked flask was added with substrate **2-6** (40.0 mg, 0.09 mmol). The mixture was dissolved with MeOH (3 mL) under stirring. Then, the mixture was added at 0 °C with $NaBH(OAc)_3$ (36.9 mg, 0.2 mmol), formaldehyde (87.0 mg, 37%wt, 0.9 mmol), stirred at room temperature for 16 hours, with LC-MS monitoring. After the completion of the reaction, the mixture was added with water to quench, the reaction mixture was condensated under reduced pressure, extracted with ethyl acetate three times. The organic phase was combined, washed with saturated saline solution, and dried with anhydrous sodium sulfate, and then purified by medium pressure liquid chromatography, to give the product **2** (2.0 mg, 0.04 mmol, 4.6% yield). LCMS ($ESI^+$) m/z: 488.3 $[M+H]^+$

**$^1$H NMR** (600 MHz, $CDCl_3$) $\delta$ 9.51 (s, 1H), 7.08-7.03 (m, 1H), 6.97 (s, 1H), 5.92 (s, 1H), 4.50-4.46 (m, 2H), 4.36-4.32 (m, 1H), 4.08-4.01 (m, 1H), 3.91-3.85 (m, 1H), 2.91-2.89 (m, 1H), 2.68-2.66 (m, 6H), 2.38 (s, 3H), 2.24 (s, 3H), 2.20 (s, 3H), 2.12-2.08 (m, 1H), 1.97-1.93 (m, 1H), 1.80-1.60 (m, 7H).

**Example 3, Compound 3 Synthesis:**

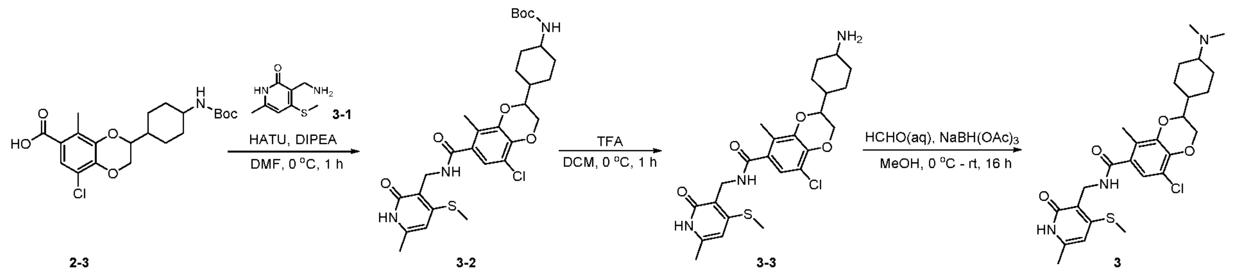


2-3 3-2 3-3 3

**Step 1, Compound 3-2 Synthesis:**

A single-necked flask was added with substrate **2-3** (125.0 mg, 0.3 mmol). The mixture was dissolved with DMF (5 mL) under stirring. Then, the mixture was added at 0 °C with DIPEA (113.8 mg, 0.9 mmol, 153.4 μL), stirred for 5 mins, and then added with HATU (111.5 mg, 0.3 mmol), further stirred for 5 mins, and then added with **3-1** (64.9 mg, 0.4 mmol) to react for 1 hour, with LC-MS monitoring. After the completion of the reaction, the mixture was added with water to quench, the reaction mixture was condensated under reduced pressure, extracted with ethyl acetate three times. The organic phase was combined, washed with saturated saline solution, and dried with anhydrous sodium sulfate, and then purified by medium pressure liquid chromatography, to give the product **3-2** (50.0 mg, 84.4 μmol, 28.8% yield). LCMS ($ESI^+$) m/z: 592.2 $[M+H]^+$

**Step 2, Compound 3-3 Synthesis:**

A single-necked flask was added with substrate **3-2** (50.0 mg, 0.08 mmol). The mixture was dissolved with DCM (2 mL) under stirring. Then, the mixture was added at 0 °C with TFA (2 mL), under ice bath stirred for 1 hour, with LC-MS

monitoring. After the completion of the reaction, the reaction mixture was condensated under reduced pressure, added with saturated sodium bicarbonate to quench, extracted with ethyl acetate three times. The organic phase was combined, washed with saturated saline solution, and dried with anhydrous sodium sulfate, and then purified by medium pressure liquid chromatography, to give the product **3-3** (31.0 mg, 63.0 μmol, 74.7% yield). LCMS ($ESI^+$) m/z: 492.2 $[M+H]^+$

**Step 3, Compound 3 Synthesis:**

A single-necked flask was added with substrate **3-3** (31.0 mg, 63.0 μmol). The mixture was dissolved with MeOH (2 mL) under stirring. Then, the mixture was added at 0 °C with $NaBH(OAc)_3$ (35.7 mg, 168.5 μmol), formaldehyde (84.4 mg, 37%wt, 844 μmol) stirred at room temperature for 1 hour, with LC-MS monitoring. After the completion of the reaction, the mixture was added with water to quench, the reaction mixture was condensated under reduced pressure, extracted with ethyl acetate three times. The organic phase was combined, washed with saturated saline solution, and dried with anhydrous sodium sulfate, and then purified by medium pressure liquid chromatography, to give the product **3** (20.0 mg, 38.5 μmol, 61.0% yield). LCMS ($ESI^+$) m/z: 520.3 $[M+H]^+$

**$^1$H NMR** (600 MHz, Methanol-$d_4$) δ 6.96 (s, 1H), 6.28 (s, 1H), 4.51-4.49 (m, 2H), 4.43-4.40 (m, 1H), 4.10-4.05 (m, 1H), 3.98-3.93 (m, 1H), 3.26-3.20 (m, 1H), 2.85 (s, 6H), 2.53 (s, 3H), 2.28 (s, 3H), 2.28-2.22 (m, 1H), 2.21 (s, 3H), 2.18-2.14 (m, 2H), 2.04-1.98 (m, 1H), 1.76-1.71 (m, 1H), 1.61-1.49 (m, 2H), 1.48-1.35 (m, 2H).

**Example 4, Compound 4 Synthesis:**

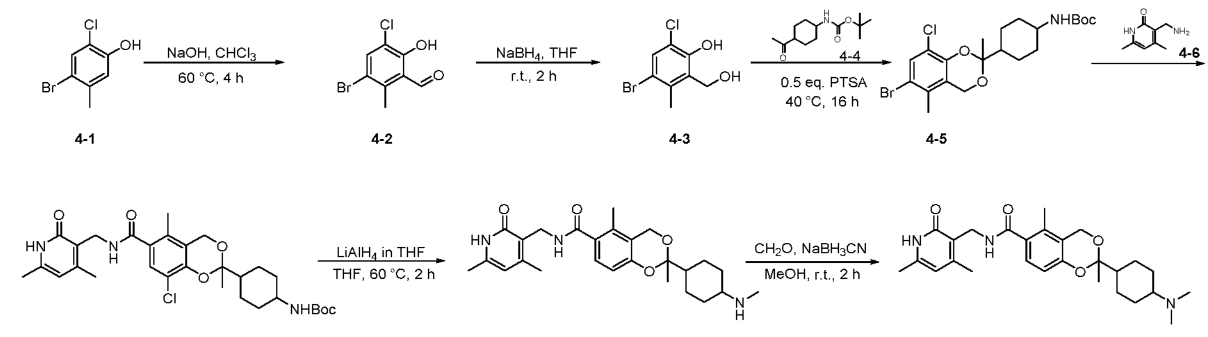


4-1 4-2 4-3 4-5

4-7 4-8 4

**Step 1, Compound 4-2 Synthesis:**

A single-necked flask was added with substrate **4-1** (2.2 g, 10.0 mmol), dissolved with chloroform (20 mL), added with ethanol (20 mL) and 4 M NaOH solution (20 mL), heated to 60 °C and stirred to react for 4 hours. After cooling, the reaction mixture was adjusted by diluted hydrochloric acid to pH = 6, added with water and DCM to extract three times. The organic phase was combined, washed with saturated saline solution, and dried with anhydrous sodium sulfate, and then purified by column chromatography ( PE: EA = 1: 1 ), to give the product **4-2** (1.2 g, 51.0% yield) as yellow solid.

**Step 2, Compound 4-3 Synthesis:**

A single-necked flask was added with substrate **4-2** (1.2 g, 5.0 mmol), dissolved with THF (10 mL), slowly added with $NaBH_4$, stirred at room temperature for 2 hours. After the completion of the reaction, the reaction mixture was added with saturated ammounium chloride solution to quench the reaction, and extracted with DCM two times. The organic phase was combined and dried with anhydrous sodium sulfate, and then filtered. The filtrate was concentrated. The residue was purified by column chromatography ( DCM: MeOH = 10: 1) to give **4-3** (1.1 g, 91.0% yield) as yellow solid.

**Step 3, Compound 4-5 Synthesis:**

A single-necked flask was added with substrate **4-3** (500.0 mg, 2.0 mmol), **4-4** (580.0 mg, 2.4 mmol), PTSA (172.0 mg, 1.0 mmol) to dissolve in toluene (5 mL). The reaction mixture was heated to 40 °C and reacted overnight. After the completion of the reaction, the reaction mixture was filtered. The filtrate was concentrated. The residue was purified by column chromatography (DCM: MeOH = 20: 1) to give **4-5** (120.0 mg, 13.0% yield) as yellow solid.

**Step 4, Compound 4-7 Synthesis:**

A single-necked flask was added with substrate **4-5** (100.0 mg, 0.2 mmol), **4-6** (94.0 mg, 0.6 mmol), Molybdenum hexacarbonyl (55.0 mg, 0.2 mmol), Pd(dppf)$Cl_2$.DCM (20.0 mg, 0.02 mmol), $K_2CO_3$(50.0 mg, 0.4 mmol), to dissolve in 1,4-dioxane (20 mL). Under nitrogen protection, the reaction mixture was heated to 110 °C and reacted overnight. After the cooling of reaction mixture, the reaction mixture was filtered. The filtrate was concentrated. The residue was purified by column chromatography to give **4-7** (65.0 mg, 53.0% yield) as brown solid.

**Step 5, Compound 4-8 Synthesis:**

A single-necked flask was added with substrate **4-7** (50.0 mg, 0.09 mmol) to dissolve in THF (10 mL), and added with 1 N $LiAlH_4$ solution (10 mL), heated to 60 °C to react for 2 hours. After cooling of the reaction mixture under ice bath condition, the reaction mixture was added with water (0.5 mL), 15% NaOH solution (0.5 mL), water (1.5 mL). The reaction mixture was sufficiently stirred, and then filtered. The filtrate was concentrated. The residue was purified by column chromatography (DCM: MeOH = 10: 1) to give **4-8** (15.0 mg, 35.0% yield) as off-white solid.

**Step 6, Compound 4 Synthesis:**

A single-necked flask was added with substrate **4-8** (10.0 mg, 0.02 mmol) to dissolve in methanol (5 mL), added with formaldehyde solution (20 mg). The reaction mixture was sufficiently stirred, and then added with $NaBH_3CN$ (6.0 mg, 0.1 mmol), stirred at room temperature for 2 小时. After the completion of the reaction, the reaction was quenched with water, added with DCM to extract two times. The organic phase was combined, and dried with anhydrous sodium sulfate, and then filtered. The filtrate was concentrated. The residue was purified by Prep-MPLC to give **4** (3.0 mg, 27.0% yield) as white solid product. LCMS ($ESI^+$) m/z: 502.2 $[M+H]^+$

**$^1$H NMR** (600 MHz, Methanol-*$d_4$*) $\delta$ $^1$H NMR (600 MHz, Methanol-*$d_4$*) $\delta$ 7.15 (d, *J* = 8.4 Hz, 1H), 6.63 (d, *J* = 8.4 Hz, 1H), 6.10 (s, 1H), 4.81 - 4.73 (m, 2H), 4.44 (s, 2H), 2.36 (s, 3H), 2.35 - 2.28 (m, 7H), 2.23 (s, 3H), 2.15 (s, 3H), 2.04 - 1.97 (m, 4H), 1.73-1.70 (m, 1H), 1.36 (s, 3H), 1.33 - 1.18 (m, 4H).

**Example 5, Compound 5 Synthesis:**

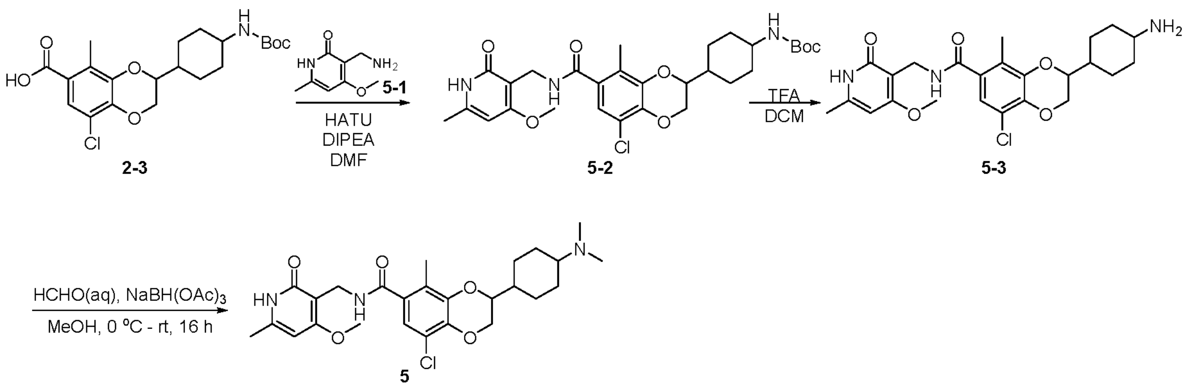


2-3, 5-2, 5-3, 5

**Step 1, Compound 5-2 Synthesis:**

Following the synthesis method of Example **3**, Step **1**, except replacing Step **1, 3-1** with **5-1** (26.0 mg, 154.6 μmol), the same synthesis method was applied, to give Compound **5-2** (51.0 mg, 88.5 μmol, 86.0% yield), LCMS ($ESI^+$) m/z: 576.1 $[M+H]^+$

**Step 2, Compound 5-3 Synthesis:**

Following the synthesis method of Example **3**, Step **2**, except replacing Step **2, 3-2** with **5-2** (41.0 mg, 71.2 μmol), the same synthesis method was applied, to give Compound **5-3** (33.0 mg, 69.3 μmol, 97.3% yield), LCMS ($ESI^+$) m/z: 476.0 $[M+H]^+$

**Step 3, Compound 5 Synthesis:**

Following the synthesis method of Example **3**, Step **3**, except replacing Step **3, 3-3** with **5-3** (33.0 mg, 69.3 μmol), the same synthesis method was applied, to give Compound **5** (15.0 mg, 29.8 μmol, 43.0% yield), LCMS (ESI$^+$) m/z: 468.2 [M+H]$^+$.

**$^1$H NMR** (600 MHz, DMSO-$d_6$) δ 11.42 (s, 1H), 7.92 (t, *J* = 4.8 Hz, 1H), 6.87 (s, 1H), 6.87 (s, 1H), 6.08 (s, 1H), 4.46-4.43 (m, 1H), 4.17 (s, *J* = 4.8 Hz, 2H), 4.08-4.04 (m, 1H), 3.94-3.89 (m, 1H), 3.80 (s, 3H), 2.23-2.21 (m, 6H), 2.17 (s, 3H), 2.12 (s, 3H), 2.08-1.95 (m, 1H), 1.80-1.73 (m, 2H), 1.58-1.49 (m, 1H), 1.48-1.46 (m, 1H), 1.25-1.12 (m, 4H).

**Example 6, Compound 6 and 6' Synthesis:**

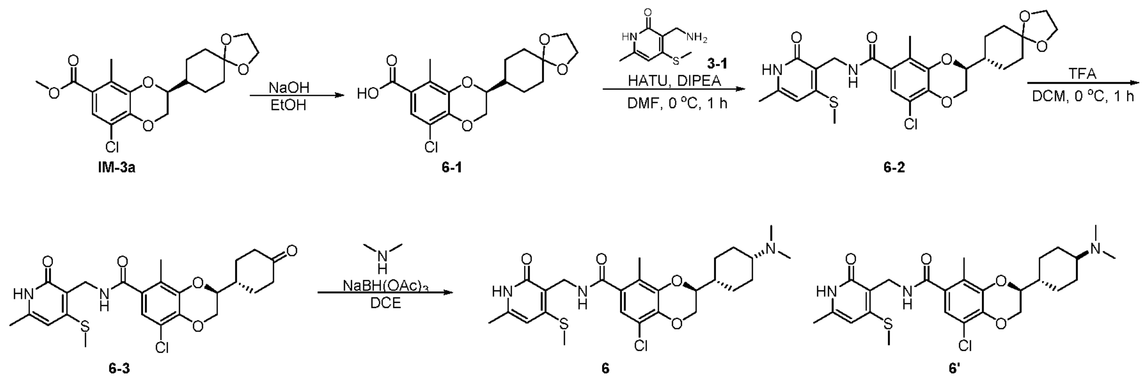


IM-3a, 6-1, 6-2, 6-3, 6, 6'

**Step 1, Compound 6-1 Synthesis:**

A single-necked flask was added with substrate **IM-3a** (1.5 g, 3.9 mmol). The mixture was dissolved with EtOH (10 mL) to dissolve, and then added with 2 N **NaOH** (10 mL), 80°C to react for 1 hour, with LC-MS monitoring. After the completion of the reaction, the mixture was added with 1 N HCl to adjust to pH = 6-7, and then added with EA solution to extract three times. The organic phase was combined, washed with saturated saline solution, and dried with anhydrous sodium sulfate and then condensated under reduced pressure, to give the crude product **6-1** (1.5 g, crude). LCMS (ESI$^+$) m/z: 369.1 [M+H]$^+$

**Step 2, Compound 6-2 Synthesis:**

Following the synthesis method of Example **3**, Step **1**, except replacing Step **1, 2-3** with **6-1** (250.0 mg, 1.4 mmol), **2-4** with **3-1**, the same synthesis method was applied, to give Compound 6-2 (292.0 mg, 545.7 μmol), LCMS (ESI$^+$) m/z: 535.1 [M+H]$^+$.

**Step 3, Compound 6-3 Synthesis:**

Following the synthesis method of Example **3**, Step **2**, except replacing Step **2, 3-2** with **6-2** (292.0 mg, 545.7 μmol), the same synthesis method was applied, to give Compound **6-3** (250.0 mg, crude), LCMS (ESI$^+$) m/z: 491.0 [M+H]$^+$.

**Step 4, Compound 6 and 6' Synthesis:**

A single-necked flask was added with substrate **6-3** (48.0 mg, 97.8 μmol). The mixture was dissolved with DCE (2 mL) to dissolve, and then at 0°C added with NaBH(OAc)$_3$ (103.0 mg, 0.5 mmol) and dimethylamine (22.0 mg, 0.5 mmol), and reacted at room temperature for 2 hours, with LC-MS monitoring. After the completion of the reaction, the mixture was added with water to quench, and then added with EA solution to extract three times. The organic phase was combined, washed with saturated saline solution, and dried with anhydrous sodium sulfate and then condensated under reduced pressure. The residue was purified by Prep-MPLC, to give the product **6** (6.3 mg, 0.01 mmol, 12.4% yield) and **6'**(6.3 mg, 0.01 mmol, 12.4% yield). LCMS (ESI$^+$) m/z: 520.5 [M+H]$^+$

**Compound 6 NMR: $^1$H NMR** (600 MHz, DMSO- $d_6$) δ 11.52 (s, 1H), 8.01 (t, *J* = 4.8 Hz, 1H), 6.90 (s, 1H), 6.08 (s, 1H), 4.48 (dd, *J* = 11.4, 1.8 Hz, 1H), 4.28 (d, *J* = 11.4, 4.2 Hz, 2H), 4.09-4.05 (m, 1H), 3.94-3.91 (m, 1H), 2.45 (s, 3H),

2.42-2.35 (m, 1H), 2.32-2.30 (m, 6H), 2.16 (s, 3H), 2.14 (s, 3H), 2.05-2.01 (m, 1H), 1.92-1.85 (m, 2H), 1.82-1.88 (m, 1H), 1.59-1.52 (m, 1H), 1.26-1.18 (m, 4H).

**Example 7, Compound 7 Synthesis:**

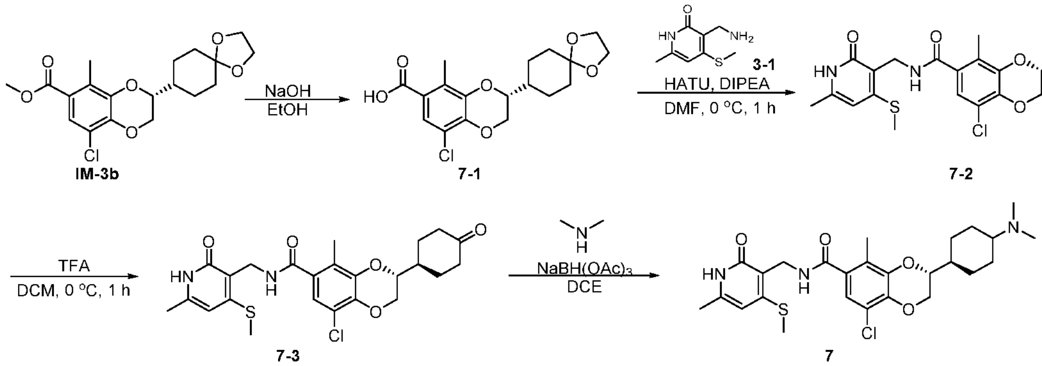


**Step 1, Compound 7-1 Synthesis:**

A single-necked flask was added with substrate **IM-3b** (1.5 g, 3.9 mmol). The mixture was dissolved with EtOH (10 mL) to dissolve, and then added with 2 N **NaOH** (10 mL), 80°C to react for 1 hour, with LC-MS monitoring. After the completion of the reaction, the mixture was added with 1 N HCl to adjust to pH = 6-7, and then added with EA solution to extract three times. The organic phase was combined, washed with saturated saline solution, and dried with anhydrous sodium sulfate and then condensated under reduced pressure, to give the crude product **7-1** (1.5 g, crude). LCMS (ESI$^+$) m/z: 369.1 [M+H]$^+$

**Step 2, Compound 7-2 Synthesis:**

Following the synthesis method of Example **3**, Step **1**, except replacing Step **1, 2-3** with **7-1** (250.0 mg, 677.9 μmol), the same synthesis method was applied, to give Compound **7-2** (292.0 mg, 546.0 μmol), LCMS (ESI$^+$) m/z: 535.1 [M+H]$^+$.

**Step 3, Compound 7-3 Synthesis:**

Following the synthesis method of Example **3**, Step **2**, except replacing Step **2, 3-2** with **7-2** (292.0 mg, 546.0 μmol), the same synthesis method was applied, to give Compound **7-3** (250.0 mg, crude), LCMS (ESI$^+$) m/z: 491.0 [M+H]$^+$.

**Step 4, Compound 7 Synthesis:**

A single-necked flask was added with substrate **7-3** (48.0 mg, 97.8 μmol). The mixture was dissolved with DCE (2 mL) to dissolve, and then at 0°C added with NaBH(OAc)$_3$ (103.0 mg, 0.5 mmol) and dimethylamine (22.0 mg, 0.5 mmol), and reacted at room temperature for 2 hours, with LC-MS monitoring. After the completion of the reaction, the mixture was added with water to quench, and then added with EA solution to extract three times. The organic phase was combined, washed with saturated saline solution, and dried with anhydrous sodium sulfate and then condensated under reduced pressure. The residue was purified by medium pressure liquid chromatography, to give the product 7 (12.6 mg, 24.2 μmol, 24.8% yield). LCMS (ESI$^+$) m/z: 520.3 [M+H]$^+$

**Compound 7$^1$H NMR** (600 MHz, DMSO-*d*$_6$) δ 11.53 (s, 1H), 8.03-8.04 (m, 1H), 6.91 (s, 1H), 6.08 (s, 1H), 4.45-4.42 (dd, *J* =9.6, 1.8 Hz, 1H), 4.27 (d, *J* = 4.8 Hz, 2H), 4.12-4.08 (m, 1H), 3.96-3.92 (m, 1H), 2.88-2.71 (m, 1H), 2.58-2.51 (m, 6H), 2.45 (s, 3H), 2.18-2.14 (m, 6H), 2.08-2.02 (m, 1H), 1.98-1.96 (m, 2H), 1.88-1.84 (m, 1H), 1.64-1.66 (m, 1H), 1.38-1.30 (m, 2H), 1.28-1.21 (m, 2H).

**Example 8, Compound 8 and 9 Synthesis:**

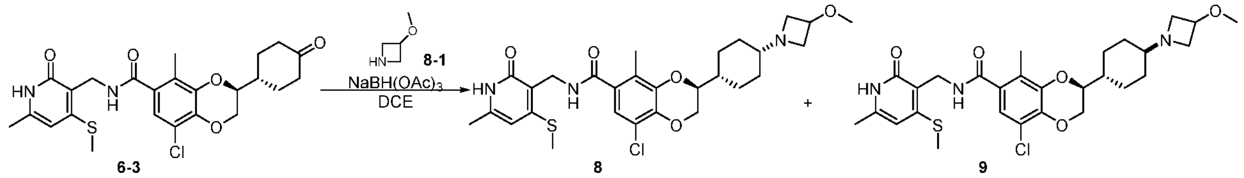


6-3 8 + 9

**Step 1, Compound 8 and 9 Synthesis:**

Following the synthesis method of Example **6**, Step **4**, except replacing Step **4, dimethylamine** with **8-1** (35.5 mg, 0.4 mmol), the same synthesis method was applied, and further resolved by Prep-MPLC to give Compound **8** (21.0 mg, 37.4 μmol, 18.0% yield) and **9** (20.0 mg, 35.6 μmol, 17.0% yield), LCMS (ESI$^+$) m/z: 562.2 [M+H]$^+$.

**Compound 8 NMR: $^1$H NMR** (600 MHz, DMSO-$d_6$) δ 11.50 (s, 1H), 8.03-8.01 (m, 1H), 6.89 (s, 1H), 6.08 (s, 1H), 4.47-4.42 (m, 1H), 4.27 (d, *J* = 4.2 Hz, 2H), 4.16-4.12 (m, 1H), 3.91-3.86 (m, 2H), 3.46-3.42 (m, 2H), 3.13 (s, 3H), 2.72-2.70 (m, 2H), 2.45 (s, 3H), 2.16 (s, 3H), 2.13 (s, 3H), 1.97-1.93 (m, 1H), 1.90-1.84 (m, 1H), 1.79-1.69 (m, 2H), 1.59-1.50 (m, 2H), 1.20-1.11 (m, 2H), 0.89-0.79 (m, 2H).

Compound 9 NMR: **$^1$H NMR** (600 MHz, DMSO-$d_6$) δ 11.52 (s, 1H), 8.03-8.01 (m, 1H), 6.89 (s, 1H), 6.08 (s, 1H), 4.44-4.40 (m, 1H), 4.27 (d, *J* = 4.6 Hz, 2H), 4.09-4.05 (m, 1H), 3.95-3.90 (m, 2H), 3.46-3.43 (m, 2H), 3.15 (s, 3H), 2.68-2.62 (m, 2H), 2.45 (s, 3H), 2.38-2.16 (s, 3H), 2.13 (s, 3H), 1.65-1.55 (m, 5H), 1.51-1.38 (m, 3H), 1.32-1.29 (m, 2H).

**Example 9, Compound 10 and 11 Synthesis:**

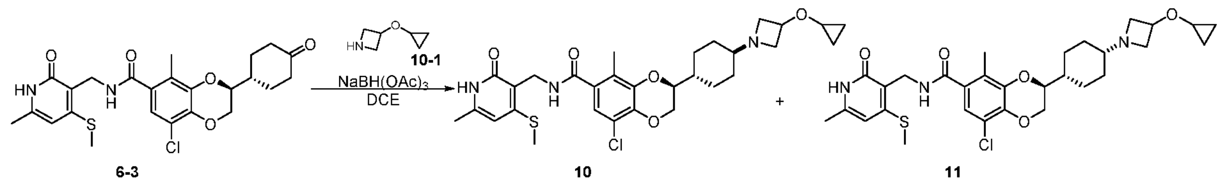


6-3 10 + 11

**Step 1, Compound 10 and 11 Synthesis:**

Following the synthesis method of Example **6**, Step **4**, except replacing Step **4, dimethylamine** with **10-1** (14.0 mg, 123.7 μmol), the same synthesis method was applied, and further resolved by Prep-MPLC to give Compound **10** (2.0 mg, 0.003 mmol, 20.0% yield) and **11** (2.0 mg, 0.003 mmol, 20.0% yield), LCMS (ESI$^+$) m/z: 588.2 [M+H]$^+$.

**Compound 10 NMR: $^1$H NMR** (600 MHz, DMSO-$d_6$) δ 11.52 (s, 1H), 8.01 (t, *J* = 4.2 Hz, 1H), 6.89 (s, 1H), 6.08 (s, 1H), 4.40-4.42 (m, 1H), 4.27 (d, *J* = 4.8 Hz, 2H), 4.09-4.06 (m, 1H), 3.99-3.97 (m, 1H), 3.87-3.84 (m, 2H), 3.31-3.30 (m, 2H), 2.66 (s, 2H), 2.48-2.46 (m, 3H), 2.44 (s, 3H), 2.40-2.29 (m, 2H), 2.16 (s, 3H), 2.13 (s, 3H), 1.80-1.78 (m, 2H), 1.67-1.58 (m, 4H), 1.43-1.40 (m, 3H).

**Compound 11 NMR: $^1$H NMR** (600 MHz, DMSO-$d_6$) δ 11.52 (s, 1H), 8.01 (t, *J* = 4.6 Hz, 1H), 6.89 (s, 1H), 6.08 (s, 1H), 4.40-4.42 (m, 1H), 4.27 (d, *J* = 4.8 Hz, 2H), 4.09-4.06 (m, 1H), 3.92-3.89 (m, 1H), 3.72-3.68 (m, 2H), 3.19-3.30 (m, 3H), 2.70-2.60 (m, 4H), 2.50 (s, 3H), 2.40-2.29 (m, 2H), 2.16 (s, 3H), 2.13 (s, 3H), 2.01-1.90 (m, 3H), 1.80-1.75 (m, 1H), 1.60-1.50 (m, 1H), 1.01-1.25 (m, 4H).

**Example 10, Compound 12 and 13 Synthesis:**

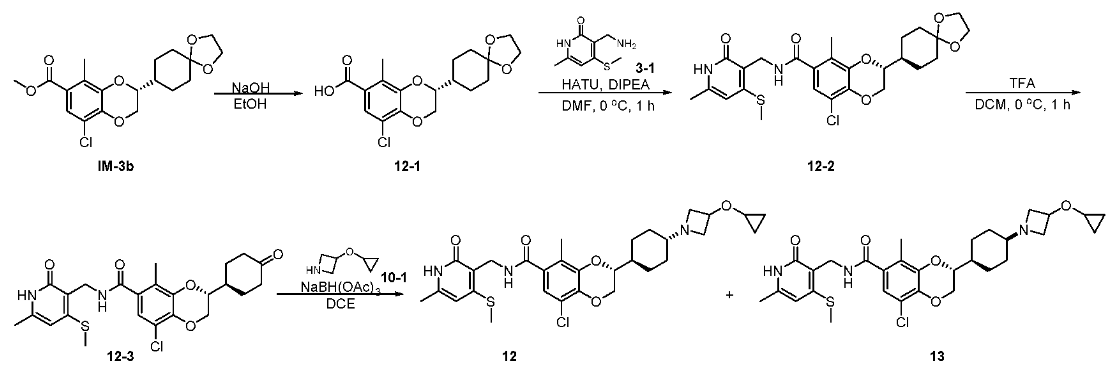


IM-3b 12-1 12-2 12-3 12 13

**Step 1, Compound 12-1 Synthesis:**

Following the synthesis method of Example **6**, Step **1**, except replacing Step **1**, **IM-3a** with **IM-3b** (1.5 g, 3.9 mmol), the same synthesis method was applied, to give Compound **12-1** (1.5 g, crude), LCMS (ESI+) m/z: 369.1 [M+H]+.

**Step 2, Compound 12-2 Synthesis:**

Following the synthesis method of Example **3**, Step **1**, except replacing Step **1, 2-3** with **12-1** (250.0 mg, 677.9 μmol), the same synthesis method was applied, to give Compound **12-2** (292.0 mg, 545.7 μmol), LCMS (ESI+) m/z: 535.1 [M+H]+.

**Step 3, Compound 12-3 Synthesis:**

Following the synthesis method of Example **3**, Step **2**, except replacing Step **2, 3-2** with **12-2** (292.0 mg, 545.7 μmol), the same synthesis method was applied, to give Compound **12-3** (250.0 mg, crude), LCMS (ESI+) m/z: 491.0 [M+H]+.

**Step 4, Compound 12 and 13 Synthesis:**

Following the synthesis method of Example **6**, Step **4**, except replacing Step **4, dimethylamine** with **10-1** (14.0 mg, 0.1 mmol), the same synthesis method was applied, and further resolved by Prep-MPLC to give Compound **12** (2.0 mg, 0.03 mmol, 20.0% yield) and **13** (2.0 mg, 0.003 mmol, 20.0% yield), LCMS (ESI+) m/z: 588.2 [M+H]+.

**Compound 12 NMR: $^1$H NMR** (600 MHz, DMSO-$d_6$) δ 11.52 (s, 1H), 8.01 (t, *J* = 4.6 Hz, 1H), 6.89 (s, 1H), 6.08 (s, 1H), 4.50-4.45 (m, 1H), 4.27-4.25 (m, 2H), 4.07-4.02 (m, 1H), 3.96-3.87 (m, 1H), 3.72-3.66 (m, 2H), 3.27-3.20 (m, 3H), 2.68-2.52 (m, 4H), 2.45 (s, 3H), 2.40-2.30 (m, 2H), 2.16 (s, 3H), 2.13 (s, 3H), 2.20-1.91 (m, 3H), 1.78-1.72 (m, 1H), 1.52-1.60 (m, 1H), 1.25-1.06 (m, 4H).

**Compound 13 NMR: $^1$H NMR** (600 MHz, DMSO-$d_6$) δ 11.52 (s, 1H), 8.01 (t, *J* = 4.2 Hz, 1H), 6.89 (s, 1H), 6.08 (s, 1H), 4.44-4.41 (m, 1H), 4.27 (d, *J* = 4.8 Hz, 2H), 4.08-4.05 (m, 1H), 3.97-3.93 (m, 1H), 3.88-3.83 (m, 2H), 3.30-3.25 (m, 2H), 2.66 (m, 2H), 2.46 (s, 3H), 2.45 (s, 3H), 2.36 (s, 2H), 2.16 (s, 3H), 2.13 (s, 3H), 1.92-1.88 (m, 2H), 1.70-1.56 (m, 4H), 1.46-1.37 (m, 3H).

**Example 11, Compound 14 and 15 Synthesis:**

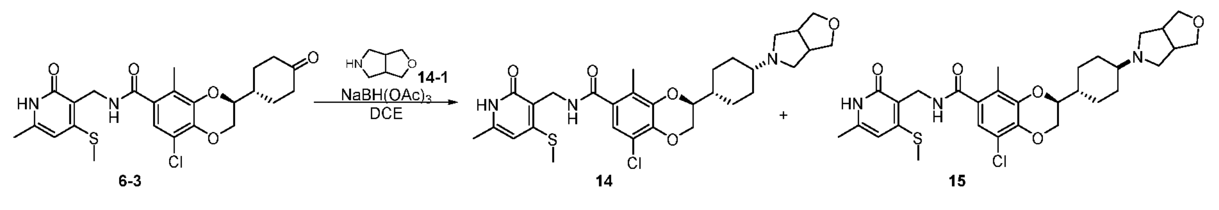


6-3 14 15

**Step 1, Compound 8 and 9 Synthesis:**

Following the synthesis method of Example **6**, Step **4**, except replacing Step **4, dimethylamine** with **14-1** (83.0

mg, 733.2 μmol), the same synthesis method was applied, and further resolved by Prep-MPLC to give Compound **14** (20.0 mg, 34.0 μmol, 8.4% yield) and **15** (20.0 mg, 34.0 μmol, 8.4% yield), LCMS (ESI$^+$) m/z: 588.2 $[M+H]^+$.

**Compound 14 NMR**: **$^1$H NMR** (400 MHz, DMSO-$d_6$) δ 11.52 (s, 1H), 8.01 (t, *J* = 4.4 Hz, 1H), 6.90 (s, 1H), 6.08 (s, 1H), 4.46 (dd, *J* = 11.2, 2.0 Hz, 1H), 4.27 (d, *J* = 4.4 Hz, 2H), 4.07-4.02 (m, 1H), 3.92-3.88 (m, 1H), 3.72-3.69 (m, 2H), 3.38-3.34 (m, 2H), 2.67-2.61 (m, 2H), 2.61-2.57 (m, 1H), 2.45 (s, 3H), 2.37-2.31 (m, 2H), 2.16 (s, 3H), 2.14 (s, 3H), 1.99-1.90 (m, 4H), 1.76-1.72 (m, 1H), 1.58-1.54 (m, 1H), 1.22-1.05 (m, 5H).

**Compound 15 NMR**: **$^1$H NMR** (400 MHz, DMSO-$d_6$) δ 11.52 (s, 1H), 8.01 (t, *J* = 4.4 Hz, 1H), 6.89 (s, 1H), 6.08 (s, 1H), 4.43 (dd, *J* = 11.2, 2.0 Hz, 1H), 4.27 (d, *J* = 4.4 Hz, 2H), 4.10-4.05 (m, 1H), 3.98-3.94 (m, 1H), 3.87-3.83 (m, 2H), 3.30-3.25 (m, 2H), 2.68-2.64 (s, 2H), 2.45 (s, 3H), 2.37-2.26 (m, 3H), 2.16 (s, 3H), 2.13 (s, 3H), 1.82-1.78 (m, 2H), 1.65-1.59 (m, 5H), 1.43-1.37 (m, 4H).

**Example 12, Compound 16 Synthesis:**

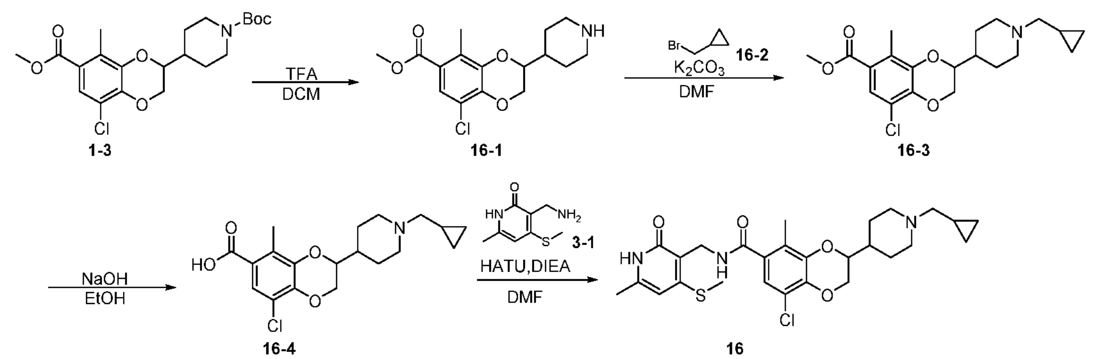


**Step 1, Compound 16-1 Synthesis:**

Following the synthesis method of Example **1**, Step **4**, except replacing Step **4, 1-5** with **1-3** (340.0 mg, 798.3 μmol), the same synthesis method was applied, to give Compound **16-1** (300.0 mg, crude), LCMS (ESI$^+$) m/z: 326.1 $[M+H]^+$.

**Step 2, Compound 16-3 Synthesis:**

A single-necked flask was added with substrate **16-1** (300.0 mg, crude). The mixture was dissolved with DMF (5 mL) under stirring, and then added with **16-2** (147.4 mg, 1.1 mmol), heated to 90 °C to react for 16 hours, with LC-MS monitoring. After the completion of the reaction, the reaction mixture was cooled to room temperature, added with water and ethyl acetate to extract three times. The organic phase was combined, washed with saturated saline solution, and dried with anhydrous sodium sulfate, and then purified by medium pressure liquid chromatography, to give the product **16-3** (247.0 mg, 650.2 μmol, 81.4% yield), LCMS (ESI$^+$) m/z: 380.2 $[M+H]^+$.

**Step 3, Compound 16-4 Synthesis:**

Following the synthesis method of Example **6**, Step **1**, except replacing Step **1**, **IM-3a** with **16-3** (247.0 mg, 650.2 μmol,), the same synthesis method was applied, to give Compound **16-4** (240.0 mg, crude), LCMS (ESI$^+$) m/z: 366.1 $[M+H]^+$.

**Step 4, Compound 16 Synthesis:**

Following the synthesis method of Example **3**, Step **1**, except replacing Step **1, 2-3** with **16-4** (80.0 mg, 218.6 μmol,), the same synthesis method was applied, to give Compound **16** (15.0 mg, 28.2 μmol,, 12.9% yield), LCMS (ESI$^+$) m/z: 532.1 $[M+H]^+$.

**$^1$H NMR** (400 MHz, DMSO-$d_6$) δ 11.51 (s, 1H), 8.02 (t, *J* = 4.4 Hz, 1H), 6.90 (s, 1H), 6.08 (s, 1H), 4.48-4.42 (m, 1H), 4.27 (d, *J* = 4.4 Hz, 2H), 4.07 (dd, *J* = 11.4, 7.2 Hz, 1H), 4.08-4.01 (m, 1H), 3.99-3.92 (m, 1H), 3.00 (d, *J* = 11.0 Hz, 2H), 2.45 (s, 3H), 2.16 (s, 3H), 2.14 (s, 3H), 2.13-2.11 (m, 2H), 1.89-1.79 (m, 3H), 1.66-1.51 (m, 2H), 1.48-1.40 (m, 2H), 0.87-0.77 (m, 1H), 0.47-0.40 (m, 2H), 0.06-0.01 (m, 2H).

**Example 13, Compound 17 Synthesis:**

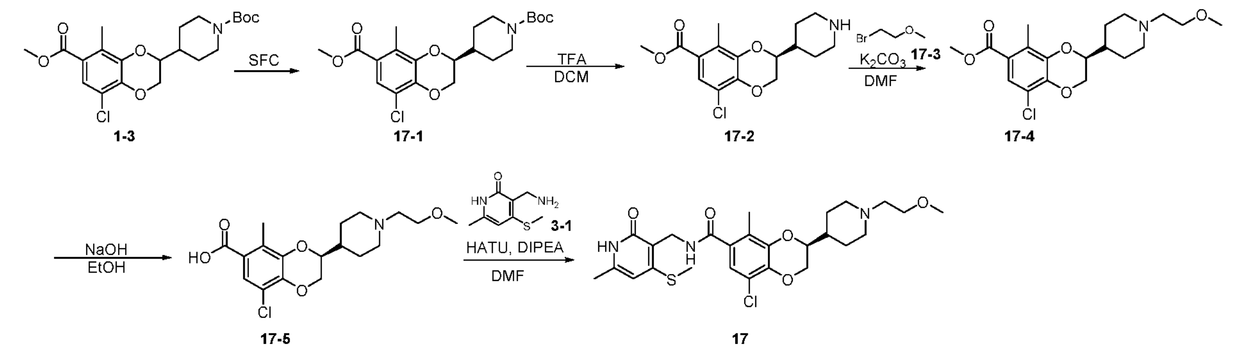


**Step 1, Compound 17-1 Synthesis:**

Compound 17-1 was obtained by SFC resolution of Compound 1-3.

**Step 2, Compound 17-2 Synthesis:**

Following the synthesis method of Example **1**, Step **4**, except replacing Step **4, 1-5** with **17-1** (300.0 mg, 0.7 mmol), the same synthesis method was applied, to give Compound **17-2** (300.0 mg, crude), LCMS (ESI$^+$) m/z: 326.1 $[M+H]^+$.

**Step 3, Compound 17-4 Synthesis:**

Following the synthesis method of Example **12**, Step **2**, except replacing Step **2, 16-1** with **17-2** (100.0 mg, 0.3 mmol), **16-2** with **17-3** (64.0 mg, 0.5 mmol), the same synthesis method was applied, to give Compound **17-4** (132.0 mg, crude), LCMS (ESI$^+$) m/z: 384.1 $[M+H]^+$.

**Step 4, Compound 17-5 Synthesis:**

Following the synthesis method of Example **6**, Step **1**, except replacing Step **1**, **IM-3a** with **17-4** (132.0 mg, crude), the same synthesis method was applied, to give Compound **17-5** (90.0 mg, crude), LCMS (ESI$^+$) m/z: 370.1 $[M+H]^+$.

**Step 5, Compound 17 Synthesis:**

Following the synthesis method of Example **3**, Step **1**, except replacing Step **1, 2-3** with **17-5** (20.0 mg, 54.1 $\mu$mol), the same synthesis method was applied, to give Compound **17** (5.0 mg, 9.0 $\mu$mol, 17.2% yield), LCMS (ESI$^+$) m/z: 536.6 $[M+H]^+$.

**$^1$H NMR** (400 MHz, DMSO-$d_6$) $\delta$ 11.51 (s, 1H), 8.01 (t, $J$= 4.4 Hz, 1H), 6.90 (s, 1H), 6.08 (s, 1H), 4.45 (dd, $J$ = 11.4, 2.0 Hz, 1H), 4.27 (d, $J$ = 4.4 Hz, 2H), 4.07-4.05 (m, 1H), 3.97-3.95 (m, 1H), 3.41 (t, $J$ = 5.8 Hz, 2H), 3.22 (s, 3H), 2.90 (d, $J$ = 10.8 Hz, 2H), 2.45 (s, 3H), 2.44-2.41 (m, 2H), 2.16 (s, 3H), 2.14 (s, 3H), 1.96-1.78 (m, 3H), 1.66-1.51 (m, 2H), 1.46-1.34 (m, 2H).

**Example 14, Compound 18 and 19 Synthesis:**

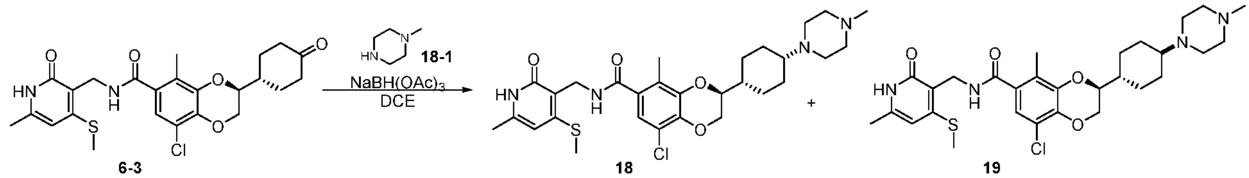

**Step 1, Compound 18 and 19 Synthesis:**

Following the synthesis method of Example **6**, Step **4**, except replacing Step **4, dimethylamine** with **18-1** (17.0 mg, 0.2 mmol), the same synthesis method was applied, and further resolved by Prep-MPLC to give Compound **18** (15.0 mg, 0.03 mmol, 18.3% yield) and **19** (20.0 mg, 0.04 mmol, 24.0% yield), LCMS (ESI$^+$) m/z: 575.2 [M+H]$^+$.

**Compound 18 NMR: $^1$H NMR** (400 MHz, DMSO-$d_6$) δ 11.52 (s, 1H), 8.01 (t, *J* = 4.4 Hz, 1H), 6.90 (s, 1H), 6.08 (s, 1H), 4.47 (d, *J* = 11.2 Hz, 1H), 4.27 (d, *J* = 4.4 Hz, 2H), 4.08-4.06 (m, 1H), 3.92-3.90 (m, 1H), 2.49-2.42 (m, 8H), 2.33-2.31 (m, 4H), 2.16 (s, 3H), 2.14 (s, 3H), 2.13 (s, 3H), 1.85-1.78 (m, 4H), 1.57-1.48 (m, 2H), 1.47-1.36 (m, 3H).

**Compound 19 NMR: $^1$H NMR** (400 MHz, DMSO-$d_6$) δ 11.52 (s, 1H), 8.01 (t, *J* = 4.4 Hz, 1H), 6.90 (s, 1H), 6.08 (s, 1H), 4.47 (d, *J* = 11.2 Hz, 1H), 4.29 (d, *J* = 4.4 Hz, 2H), 4.08-4.03 (m, 1H), 3.95-3.88 (m, 1H), 2.47-2.34 (m, 8H), 2.35-2.23 (m, 4H), 2.16 (s, 3H), 2.14 (s, 3H), 2.12 (s, 3H), 2.04-1.98 (m, 1H), 1.90-1.76 (m, 3H), 1.58-1.49 (m, 1H), 1.23-1.11 (m, 4H).

**Example 15, Compound 20 and 21 Synthesis:**

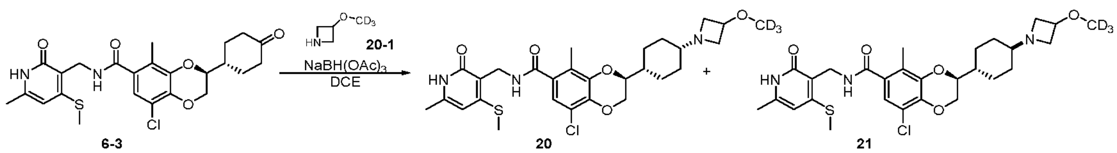


6-3 20 21

**Step 1, Compound 20 and 21 Synthesis:**

Following the synthesis method of Example **6**, Step **4**, except replacing Step **4, dimethylamine** with **20-1** (20.0 mg, 0.2 mmol), the same synthesis method was applied, and further resolved by Prep-MPLC to give Compound **20** (5.0 mg, 9.0 μmol, 8.7% yield) and **21** (9.0 mg, 0.02 mmol, 16.0% yield), LCMS (ESI$^+$) m/z: 565.2 [M+H]$^+$.

**Compound 20 NMR: $^1$H NMR (400 MHz, Methanol-$d_4$)** δ 6.94 (s, 1H), 6.27 (s, 1H), 4.49 (s, 2H), 4.44-4.40 (m, 1H), 4.09-3.99 (m, 2H), 3.90-3.86 (m, 1H), 3.65-3.61 (m, 2H), 3.05-3.01 (m, 2H), 2.52 (s, 3H), 2.29 (s, 3H), 2.20 (s, 3H), 2.15-2.10 (m, 1H), 2.05-2.00 (m, 2H), 1.94-1.90 (m, 2H), 1.85-1.81 (m, 1H), 1.63-1.58 (m, 2H), 1.05-0.98 (m, 2H).

**Compound 21 NMR: $^1$H NMR (400 MHz, Methanol-$d_4$)** δ 6.94 (s, 1H), 6.27 (s, 1H), 4.49 (s, 2H), 4.46-4.42 (m, 1H), 4.09-3.98 (m, 2H), 3.95-3.86 (m, 1H), 3.64-3.60 (m, 2H), 3.10-2.95 (m, 2H), 2.52 (s, 3H), 2.30 (s, 3H), 2.20 (s, 3H), 2.14-2.09 (m, 1H), 2.07-1.94 (m, 2H), 1.96-1.88 (m, 2H), 1.87-1.76 (m, 1H), 1.70-1.50 (m, 2H), 1.09-0.96 (m, 2H).

**Example 16, Compound 22 and 23 Synthesis:**

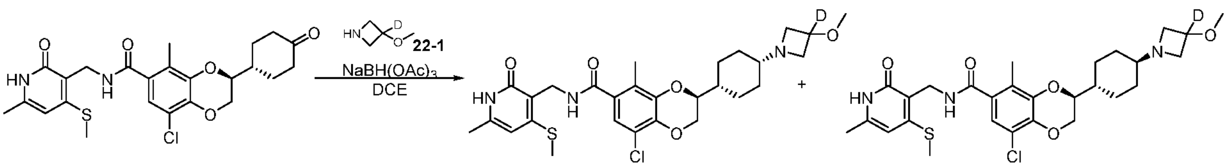


6-3 22 23

**Step 1, Compound 22 and 23 Synthesis:**

Following the synthesis method of Example **6**, Step **4**, except replacing Step **4, dimethylamine** with **22-1** (48.0 mg, 0.5 mmol), the same synthesis method was applied, and further resolved by Prep-MPLC to give Compound **22** (15.0 mg, 0.03 mmol, 25.0% yield) and **23** (15.0 mg, 0.03 mmol, 25.0% yield), LCMS (ESI$^+$) m/z: 563.3 [M+H]$^+$.

**Compound 22 NMR: $^1$H NMR** (600 MHz, DMSO-$d_6$) δ 11.51 (s, 1H), 8.02 (t, *J* = 4.2 Hz, 1H), 6.90 (s, 1H), 6.08 (s, 1H), 4.45-4.44 (m, 1H), 4.27 (d, *J* = 4.8 Hz, 2H), 4.06-4.03 (m, 1H), 3.92-3.89 (m, 1H), 3.43 (d, *J* = 7.2 Hz, 2H), 3.13 (s, 3H), 2.71 (d, *J* = 7.8 Hz, 2H), 2.45 (s, 3H), 2.16 (s, 3H), 2.14 (s, 3H), 1.96-1.93 (m, 1H), 1.89-1.85 (m, 1H), 1.77-1.71 (m, 3H), 1.54-1.52 (m, 1H), 1.18-1.14 (m, 2H), 0.88-0.84 (m, 2H).

**Compound 23 NMR: $^1$H NMR** (600 MHz, DMSO-$d_6$) δ 11.52 (s, 1H), 8.02 (t, *J* = 4.2 Hz, 1H), 6.89 (s, 1H), 6.08 (s, 1H), 4.43 (d, *J* = 9.6 Hz, 1H), 4.27 (d, *J* = 4.2 Hz, 2H), 4.07-4.04 (m, 1H), 3.94-3.92 (m, 1H), 3.45 (t, *J* = 4.8 Hz, 2H), 3.14 (s, 3H), 2.67-2.66 (m, 2H), 2.45 (s, 3H), 2.22-2.20 (m, 1H), 2.16 (s, 3H), 2.13 (s, 3H), 1.62-1.28 (m, 9H).

**Example 17, Compound 24 Synthesis:**

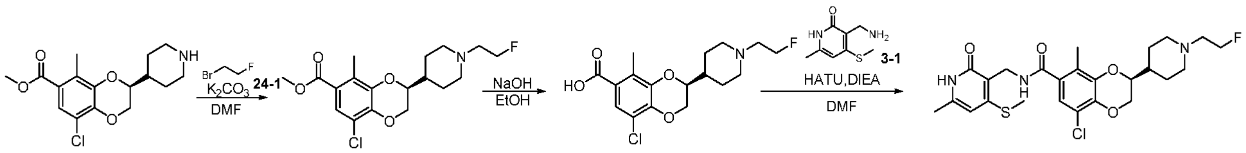


17-2 24-2 24-3 24

**Step 1, Compound 24-2 Synthesis:**

Following the synthesis method of Example **12**, Step **2**, except replacing Step **2, 16-1** with **17-2** (100.0 mg, 0.3 mmol), **16-2** with **24-1** (58.0 mg, 0.5 mmol), the same synthesis method was applied, to give Compound **24-2** (100.0 mg, crude), LCMS (ESI+) m/z: 372.1 [M+H]+.

**Step 2, Compound 24-3 Synthesis:**

Following the synthesis method of Example **6**, Step **1**, except replacing Step **1**, **IM-3a** with **24-2** (100.0 mg, crude), the same synthesis method was applied, to give Compound **24-3** (50.0 mg, crude), LCMS (ESI+) m/z: 358.1 [M+H]+.

**Step 3, Compound 24 Synthesis:**

Following the synthesis method of Example **3**, Step **1**, except replacing Step **1, 2-3** with **24-3** (30.0 mg, 0.08 mmol), the same synthesis method was applied, to give Compound **24** (1.3 mg, 2.5 μmol, 3.0% yield), LCMS (ESI+) m/z: 524.5 [M+H]+.

**$^1$H NMR** (400 MHz, DMSO-$d_6$) δ 11.52 (s, 1H), 8.01 (t, *J* = 4.4 Hz, 1H), 6.90 (s, 1H), 6.08 (s, 1H), 4.57 (d, *J* = 4.8 Hz, 1H), 4.49-4.41 (m, 2H), 4.27 (d, *J* = 4.4 Hz, 2H), 4.07-4.03 (m, 1H), 3.99-3.96 (m, 1H), 2.95-2.91 (m, 2H), 2.63-2.61 (m, 1H), 2.45 (s, 3H), 2.18 (s, 3H), 2.14 (s, 3H), 2.02-1.80 (m, 2H), 1.86-1.80 (m, 2H), 1.67-1.54 (m, 2H), 1.47-1.38 (m, 2H).

**Example 18, Compound 25 Synthesis:**

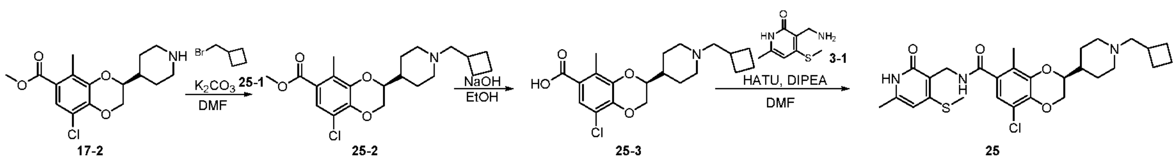


17-2 25-2 25-3 25

**Step 1, Compound 25-2 Synthesis:**

Following the synthesis method of Example **12**, Step **2**, except replacing Step **2, 16-1** with **17-2** (100.0 mg, 0.3 mmol), **16-2** with **25-1** (68.0 mg, 0.5 mmol), the same synthesis method was applied, to give Compound **25-2** (100.0 mg, crude), LCMS (ESI+) m/z: 394.1 [M+H]+.

**Step 2, Compound 25-3 Synthesis:**

Following the synthesis method of Example **6**, Step **1**, except replacing Step **1**, **IM-3a** with **25-2** (100.0 mg, crude), the same synthesis method was applied, to give Compound **25-3** (50.0 mg, crude), LCMS (ESI+) m/z: 380.1 [M+H]+.

**Step 3, Compound 25 Synthesis:**

Following the synthesis method of Example **3**, Step **1**, except replacing Step **1, 2-3** with **25-3** (25.0 mg, 0.07 mmol), the same synthesis method was applied, to give Compound **25** (3.5 mg, 6.4 μmol, 9.7% yield), LCMS (ESI+) m/z: 546.6 [M+H]+.

**$^1$H NMR** (400 MHz, DMSO-$d_6$) δ 11.52 (s, 1H), 8.02 (t, *J* = 4.8 Hz, 1H), 6.90 (s, 1H), 6.08 (s, 1H), 4.46-4.42 (m, 1H),

4.27 (d, $J$ = 4.4 Hz, 2H), 4.08-4.03 (m, 1H), 3.96-3.93 (m, 1H), 2.84-2.81 (m, 2H), 2.45 (s, 3H), 2.30-2.28 (m, 2H), 2.16 (s, 3H), 2.15-2.13 (m, 3H), 2.01-1.97 (m, 2H), 1.84-1.77 (m, 5H), 1.63-1.58 (m, 5H), 1.39-1.36 (m, 2H).

**Example 19, Compound 26 and 27 Synthesis:**

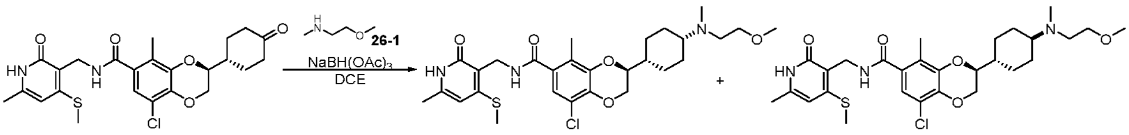


6-3 26 27

**Step 1, Compound 26 and 27 Synthesis:**

Following the synthesis method of Example **6**, Step **4**, except replacing Step **4, dimethylamine** with **26-1** (17.0 mg, 0.2 mmol), the same synthesis method was applied, and further resolved by Prep-MPLC to give Compound **26** (15.0 mg, 0.03 mmol, 28.0% yield) and **27** (15.0 mg, 0.03 mmol, 28.0% yield), LCMS (ESI$^+$) m/z: 564.2 [M+H]$^+$.

**Compound 26 NMR: $^1$H NMR** (600 MHz, DMSO-$d_6$) δ 11.52 (s, 1H), 8.02 (t, $J$ = 4.2 Hz, 1H), 6.90 (s, 1H), 6.08 (s, 1H), 4.47-4.45 (m, 1H), 4.27 (d, $J$ = 4.8 Hz, 2H), 4.18-4.15 (m, 1H), 4.08-4.05 (m, 1H), 3.39 (t, $J$ = 6.0 Hz, 2H), 3.22 (s, 3H), 2.57 (t, $J$ = 6.6 Hz, 2H), 2.45 (s, 3H), 2.37-2.33 (m, 1H), 2.19 (s, 3H), 2.16 (s, 3H), 2.14 (s, 3H), 1.84-1.79 (m, 1H), 1.75-1.63 (m, 4H), 1.52-1.44 (m, 4H).

**Compound 27 NMR: $^1$H NMR** (600 MHz, DMSO-$d_6$) δ 11.52 (s, 1H), 8.02 (t, $J$ = 4.2 Hz, 1H), 6.90 (s, 1H), 6.08 (s, 1H), 4.45-4.43 (m, 1H), 4.27 (d, $J$ = 4.2 Hz, 2H), 4.07-4.04 (m, 1H), 3.92-3.90 (m, 1H), 3.39-3.37 (m, 2H), 3.30-3.29 (m, 1H), 3.18 (s, 3H), 2.63-2.61 (m, 1H), 2.45 (s, 3H), 2.24-2.22 (m, 3H), 2.16-2.14 (m, 7H), 2.03-2.01 (m, 1H), 1.80-1.79 (m, 3H), 1.55-1.54 (m, 1H), 1.26-1.17 (m, 4H).

**Example 20, Compound 28 and 29 Synthesis:**

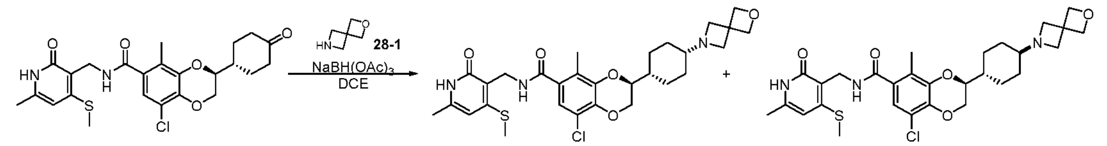


6-3 28 29

**Step 1, Compound 28 and 29 Synthesis:**

Following the synthesis method of Example **6**, Step **4**, except replacing Step **4, dimethylamine** with **28-1** (20.0 mg, 0.2 mmol), the same synthesis method was applied, and further resolved by Prep-MPLC to give Compound **28** (1.1 mg, 2.0 μmol, 1.9% yield) and **29** (1.2 mg, 2.0 μmol, 1.9% yield), LCMS (ESI$^+$) m/z: 574.1 [M+H]$^+$.

**Compound 28 NMR: $^1$H NMR** (400 MHz, DMSO-$d_6$) δ 11.52 (s, 1H), 8.02 (t, $J$ = 4.4 Hz, 1H), 6.90 (s, 1H), 6.08 (s, 1H), 4.59 (s, 4H), 4.45-4.43 (m, 1H), 4.27 (d, $J$ = 4.4 Hz, 2H), 4.07-4.02 (m, 1H), 3.93-3.90 (m, 1H), 3.29 (s, 4H), 2.45 (s, 3H), 2.16 (s, 3H), 2.13 (s, 3H), 1.97-1.91 (m, 1H), 1.75-1.74 (m, 3H), 1.55-1.53 (m, 1H), 1.24-1.09 (m, 3H), 0.89-0.80 (m, 2H).

**Compound 29 NMR: $^1$H NMR** (400 MHz, DMSO-$d_6$) δ 11.51 (s, 1H), 8.02 (t, $J$ = 4.4 Hz, 1H), 6.89 (s, 1H), 6.08 (s, 1H), 4.59 (s, 4H), 4.42-4.40 (m, 1H), 4.27 (d, $J$ = 4.4 Hz, 2H), 4.07-4.02 (m, 1H), 3.96-3.92 (m, 1H), 3.19 (s, 4H), 2.45 (s, 3H), 2.16 (s, 3H), 2.13 (s, 3H), 1.58-1.49 (m, 5H), 1.47-1.24 (m, 5H).

**Example 21, Compound 30 and 31 Synthesis:**

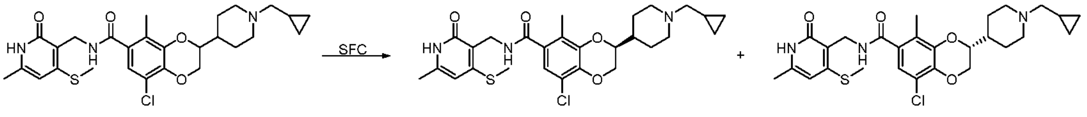


16 30 31

**Step 1, Compound 25-2 Synthesis:**

Compound 30 and 31 was obtained by SFC resolution of Compound 16. LCMS (ESI$^+$) m/z: 532.1 [M+H]$^+$

**Compound 30 NMR: $^1$H NMR** (400 MHz, DMSO-$d_6$) δ 11.52 (s, 1H), 8.02 (t, *J* = 4.4 Hz, 1H), 6.90 (s, 1H), 6.08 (s, 1H), 4.48-4.42 (m, 1H), 4.27 (d, *J* = 4.4 Hz, 2H), 4.10-4.05 (m, 1H), 3.99-3.92 (m, 1H), 3.00 (d, *J* = 11.0 Hz, 2H), 2.45 (s, 3H), 2.17-2.15 (m, 3H), 2.15-2.13 (m, 3H), 2.12-2.10 (m, 1H), 1.90-1.88 (m, 1H), 1.89-1.70 (m, 3H), 1.68-1.62 (m, 1H), 1.60-1.52 (m, 1H), 1.48-1.39 (m, 2H), 0.83-0.78 (m, 1H), 0.48-0.42 (m, 2H), 0.08-0.01 (m, 2H).

**Compound 31 NMR: $^1$H NMR** (400 MHz, DMSO-$d_6$) δ 11.52 (s, 1H), 8.03 (t, *J* = 4.4 Hz, 1H), 6.92 (s, 1H), 6.08 (s, 1H), 4.49-4.47 (m, 1H), 4.27 (d, *J* = 4.4 Hz, 2H), 4.12-4.08 (m, 1H), 4.00-3.98 (m, 1H), 2.96-2.89 (m, 1H), 2.45 (s, 3H), 2.17 (s, 3H), 2.17-2.15 (m, 6H), 2.03-1.89 (m, 2H), 1.80-1.68 (m, 2H), 1.60-1.46 (m, 2H), 1.25-1.21 (m, 3H), 0.95-0.80 (m, 2H), 0.58-0.45 (m, 2H), 0.25-0.10 (m, 2H).

**Example 22, Compound 32 and 33 Synthesis:**

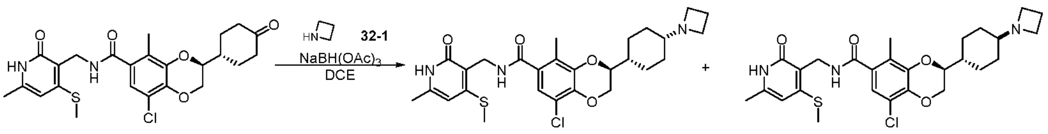


6-3 32 33

**Step 1, Compound 32 and 33 Synthesis:**

Following the synthesis method of Example **6**, Step **4**, except replacing Step **4, dimethylamine** with **32-1** (11.0 mg, 0.2 mmol), the same synthesis method was applied, and further resolved by Prep-MPLC to give Compound **32** (11.0 mg, 0.02 mmol, 22.0% yield) and **33** (5.0 mg, 9.0 μmol, 9.0 % yield), LCMS (ESI$^+$) m/z: 532.1 [M+H]$^+$.

**Compound 32 NMR: $^1$H NMR** (600 MHz, DMSO-$d_6$) δ 11.52 (s, 1H), 8.01 (t, *J* = 4.2 Hz 1H), 6.89 (s, 1H), 6.08 (s, 1H), 4.44-4.40 (m, 1H), 4.27 (d, *J* = 4.2 Hz, 2H), 4.06-4.04 (m, 1H), 3.96-3.90 (m, 1H), 3.02 (t, *J* = 6.0 Hz, 4H), 2.45 (s, 3H),, 2.16 (s, 3H), 2.13 (s, 3H), 1.95-1.91 (m 1H), 1.89-1.83 (m, 2H), 1.76-1.69 (m, 2H), 1.55-1.49 (m, 1H), 1.26-1.12 (m, 4H), 0.89-0.80 (m, 2H).

**Compound 33 NMR: $^1$H NMR** (600 MHz, DMSO-$d_6$) δ 11.52 (s, 1H), 8.01 (t, *J* = 4.2 Hz, 1H), 6.90 (s, 1H), 6.08 (s, 1H), 4.46-4.43 (m, 1H), 4.27 (d, *J* = 4.2 Hz, 2H), 4.07-4.03 (m, 1H), 3.93-3.90 (m, 1H), 3.05 (t, *J* = 6.0 Hz, 4H), 2.45 (s, 3H), 2.16 (s, 3H), 2.14 (s, 3H), 1.96-1.92 (m, 1H), 1.88 (t, *J* = 6.8 Hz, 2H), 1.74-1.69 (m, 2H), 1.56-1.50 (m, 1H), 1.25-1.11 (m, 4H), 0.85-0.81 (m, 2H).

**Example 23, Compound 34 and 35 Synthesis:**

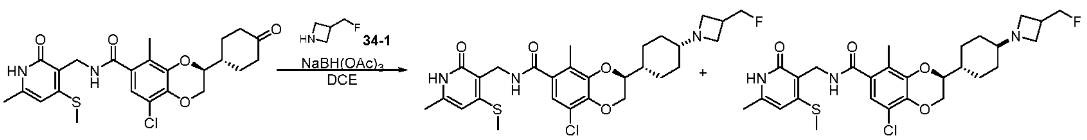


6-3 34 35

**Step 1, Compound 34 and 35 Synthesis:**

Following the synthesis method of Example **6**, Step **4**, except replacing Step **4, dimethylamine** with **34-1** (17.0 mg, 0.2 mmol), the same synthesis method was applied, and further resolved by Prep-MPLC to give Compound **34** (18.0

mg, 0.03 mmol, 29.0% yield) and **35** (18.0 mg, 0.03 mmol, 29.0% yield), LCMS (ESI+) m/z: 564.3 [M+H]+.

**Example 24, Compound 36 and 37 Synthesis:**

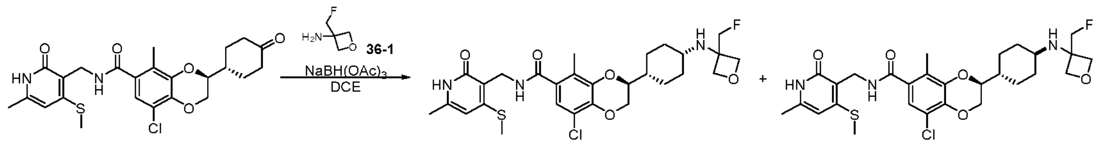


6-3 36 37

**Step 1, Compound 36 and 37 Synthesis:**

Following the synthesis method of Example **6**, Step **4**, except replacing Step **4, dimethylamine** with **36-1** (18.0 mg, 0.2 mmol), the same synthesis method was applied, and further resolved by Prep-MPLC to give Compound **36** (18.0 mg, 0.03 mmol, 31.0% yield) and **37** (18.0 mg, 0.03 mmol, 31.0% yield), LCMS (ESI+) m/z: 580.2 [M+H]+.

**Example 25, Compound 38 Synthesis:**

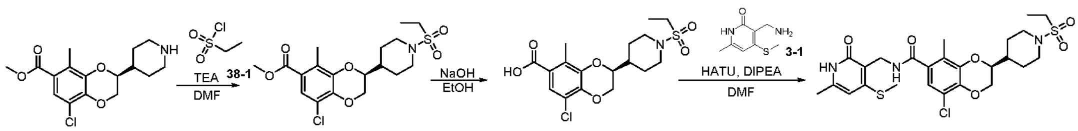


17-2 38-2 38-3 38

**Step 1, Compound 38-2 Synthesis:**

A single-necked flask was added with substrate **17-2** (25.0 mg, 0.08 mmol). The mixture was dissolved with DCM(5 mL) under stirring, and then at 0°C added with **TEA** (23.0 mg, 0.2 mmol), added with **38-1** (10.3 mg, 0.08 mmol), 0 °C to react for 1 hour, with LC-MS monitoring. After the completion of the reaction, condensated under reduced pressure to give the crude product **38-2** (28.0 mg, crude), LCMS (ESI+) m/z: 418.1 [M+H]+.

**Step 2, Compound 38-3 Synthesis:**

Following the synthesis method of Example **6**, Step **1**, except replacing Step **1**, **IM-3a** with **38-2** (28.0 mg, crude), the same synthesis method was applied, to give Compound **38-3** (25.0 mg, crude), LCMS (ESI+) m/z: 404.1 [M+H]+.

**Step 3, Compound 38 Synthesis:**

Following the synthesis method of Example **3**, Step **1**, except replacing Step **1, 2-3** with **38-3** (25 mg, crude), the same synthesis method was applied, to give Compound **38** (21.0 mg, 0.037 mmol, 47.8% yield), LCMS (ESI+) m/z: 570.5 [M+H]+.

**$^1$H NMR** (600 MHz, DMSO-$d_6$) δ 11.52 (s, 1H), 8.02 (t, *J* = 4.4 Hz, 1H), 6.91 (s, 1H), 6.08 (s, 1H), 4.46 (d, *J* = 10.2 Hz, 1H), 4.27 (d, *J* = 4.2 Hz, 2H), 4.15-4.07 (m, 1H), 4.06-4.03 (m, 1H), 3.64 (d, *J* = 11.8 Hz, 2H), 3.02 (q, *J* = 7.4 Hz, 2H), 2.84-2.74 (m, 2H), 2.45 (s, 3H), 2.16 (s, 3H), 2.15 (s, 3H), 1.98-1.93 (m, 1H), 1.83-1.73 (m, 2H), 1.48-1.41 (m, 2H), 1.20 (t, *J* = 7.4 Hz, 3H).

**Example 26, Compound 39 and 40 Synthesis:**

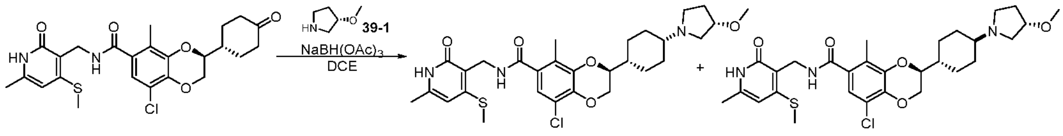


6-3 39 40

**Step 1, Compound 39 and 40 Synthesis:**

Following the synthesis method of Example **6**, Step **4**, except replacing Step **4, dimethylamine** with **39-1** (12.0 mg, 0.1 mmol), the same synthesis method was applied, and further resolved by Prep-MPLC to give Compound **39** (10.0 mg, 0.017 mmol, 17.0% yield) and **40** (10.0 mg, 0.017 mmol, 17.0% yield), LCMS (ESI$^+$) m/z: 576.3 [M+H]$^+$.

**Compound 39 NMR: $^1$H NMR** (600 MHz, Methanol-$d_4$) δ 6.94 (s, 1H), 6.27 (s, 1H), 4.49 (s, 2H), 4.41-4.38 (m, 1H), 4.09-4.05 (m, 1H), 3.98-3.94 (s, 1H), 3.89-3.86 (t, $J$ = 7.2 Hz, 1H), 3.28 (s, 3H), 2.88-2.80 (m, 2H), 2.80-2.75 (m, 1H), 2.63-2.59 (s, 1H), 2.52 (s, 3H), 2.29 (s, 3H), 2.20 (s, 3H), 2.14-2.02 (m, 5H), 1.86-1.80 (m, 2H), 1.68-1.62 (m, 1H), 1.32-1.20 (m, 4H).

**Compound 40 NMR: $^1$H NMR** (600 MHz, Methanol-$d_4$) δ 6.95 (s, 1H), 6.27 (s, 1H), 4.49 (s, 2H), 4.47-4.44 (m, 1H), 4.13-4.10 (t, $J$ =7.8 Hz, 1H), 4.06-4.03 (m, 1H), 3.96 (s, 1H), 3.28 (s, 3H), 2.82-2.74 (m, 2H), 2.71-2.66 (s, 1H), 2.58-2.55 (m, 1H), 2.52 (s, 3H), 2.29 (s, 3H), 2.20 (s, 3H), 2.10-2.06 (m, 1H), 1.98-1.92 (m, 1H), 1.85-1.77 (m, 2H), 1.76-1.63 (m, 7H), 1.58-1.52 (m, 1H).

**Example 27, Compound 41 and 42 Synthesis:**

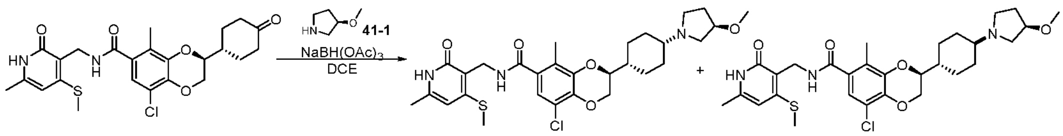


6-3 41 42

**Step 1, Compound 41 and 42 Synthesis:**

Following the synthesis method of Example **6**, Step **4**, except replacing Step **4, dimethylamine** with **41-1** (11.0 mg, 0.1 mmol), the same synthesis method was applied, and further resolved by Prep-MPLC to give Compound **41** (14.0 mg, 0.02 mmol, 24.0% yield) and **42** (6.5 mg, 0.01 mmol, 11.0% yield), LCMS (ESI$^+$) m/z: 576.3 [M+H]$^+$.

**Compound 41 NMR: $^1$H NMR** (600 MHz, Methanol-$d_4$) δ 6.94 (s, 1H), 6.27 (s, 1H), 4.49 (s, 2H), 4.42 (dd, $J$ = 11.4, 1.8 Hz, 1H), 4.07-4.04 (m, 1H), 3.96-3.94 (m, 1H), 3.89 (t, $J$ = 7.2 Hz, 1H), 3.28 (s, 3H), 2.86-2.84 (m, 2H), 2.77-2.75 (m, 1H), 2.63-2.61 (m, 1H), 2.52 (s, 3H), 2.29 (s, 3H), 2.20 (s, 3H), 2.12-2.06 (m, 5H), 1.86-1.81 (m, 2H), 1.65-1.64 (m, 1H), 1.30-1.25 (m, 4H).

**Compound 42 NMR: $^1$H NMR** (600 MHz, Methanol-$d_4$) δ 6.95 (s, 1H), 6.27 (s, 1H), 4.50 (s, 2H), 4.46 (dd, $J$ = 11.4, 1.8 Hz, 1H), 4.15-4.09 (m, 1H), 4.05-4.01 (m, 1H), 3.96 (s, 1H), 3.28 (s, 3H), 2.85-2.74 (m, 2H), 2.74-2.65 (m,1H), 2.58-2.53 (m, 1H), 2.52 (s, 3H), 2.29 (s, 3H), 2.20 (s, 3H), 2.12-2.04 (m, 1H), 1.98-1.90 (s, 1H), 1.85-1.75 (m, 2H), 1.74-1.60 (m, 7H), 1.59-1.54 (m, 1H).

**Example 28, Compound 43 and 44 Synthesis:**

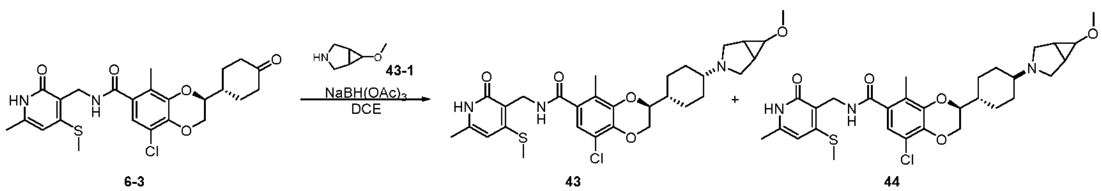


6-3 43 44

**Step 1, Compound 43 and 44 Synthesis:**

Following the synthesis method of Example **6**, Step **4**, except replacing Step **4, dimethylamine** with **43-1** (11.0 mg, 0.1 mmol), the same synthesis method was applied, and further resolved by Prep-MPLC to give Compound **43** (4.0 mg, 7.0 μmol, 9.0% yield) and **44** (6.0 mg, 0.01 mmol, 13.0% yield), LCMS ($ESI^+$) m/z: 588.5 $[M+H]^+$.

**Compound 43 NMR: $^1$H NMR** (600 MHz, $CDCl_3$) δ 6.97 (s, 1H), 6.04 (s, 1H), 4.69-4.51(m, 1H), 4.49-4.42 (m, 1H), 4.41-4.33 (m, 1H), 4.14-4.10 (m, 1H), 4.08-3.91 (m, 2H), 3.89-3.41 (m, 3H), 3.35 (s, 3H), 2.50 (s, 3H), 2.37 (s, 3H), 2.30-2.15 (m, 4H), 2.15-1.94 (m, 6H), 1.90-1.80 (m, 3H), 1.40-0.97 (m, 3H).

**Compound 44 NMR: $^1$H NMR** (600 MHz, $CDCl_3$) δ 6.97 (s, 1H), 6.04 (s, 1H), 4.69-4.51(m, 1H), 4.49-4.35 (m, 1H), 4.35-4.29 (m, 1H), 4.14-4.10 (m, 1H), 4.08-3.91 (m, 2H), 3.90-3.65 (m, 3H), 3.35 (s, 3H), 3.45-3.31 (m, 3H), 2.37 (s, 3H), 2.30-2.15 (m, 4H), 2.15-1.92 (m, 6H), 1.90-1.70 (m, 3H), 1.40-0.97 (m, 3H).

**Example 29, Compound 45 and 46 Synthesis:**

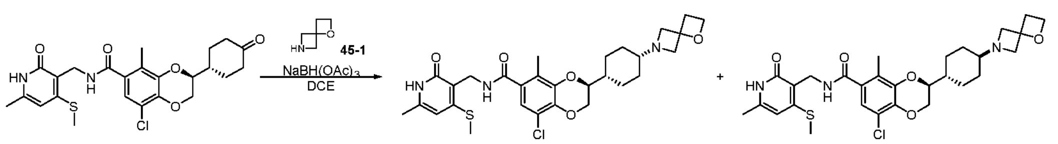


6-3 45 46

**Step 1, Compound 45 and 46 Synthesis:**

Following the synthesis method of Example **6**, Step **4**, except replacing Step **4, dimethylamine** with **45-1** (10.0 mg, 0.1 mmol), the same synthesis method was applied, and further resolved by Prep-MPLC to give Compound **45** (1.5 mg, 2.6 μmol, 3.0% yield) and **46** (1.5 mg, 2.6 μmol, 3.0% yield), LCMS ($ESI^+$) m/z: 574.4 $[M+H]^+$.

**Compound 45 NMR: $^1$H NMR** (600 MHz, DMSO-$d_6$) δ 11.52 (s, 1H), 8.02 (t, *J* = 4.4 Hz, 1H), 6.90 (s, 1H), 6.08 (s, 1H), 4.49-4.41 (dd, *J* = 11.6, 2.0 Hz, 1H), 4.40-4.33 (t, *J* = 7.6 Hz, 2H), 4.30-4.25 (d, *J* = 4.8 Hz, 2H), 4.10-4.01 (m, 1H), 3.93-3.87 (td, *J* = 7.2, 2.0 Hz, 1H), 3.50-3.44 (m, 2H), 2.97-2.95 (m, 2H), 2.72-2.68 (m, 2H), 2.45 (s, 3H), 2.16 (s, 3H), 2.13 (s, 3H), 1.90-1.78 (m, 1H), 1.77-1.69 (m, 3H), 1.55-1.48 (m, 1H), 1.27-1.05 (m, 3H), 0.97-0.81(m, 2H).

**Compound 46 NMR: $^1$H NMR** (600 MHz, DMSO-$d_6$) δ 11.51 (s, 1H), 8.02 (t, *J* = 4.4 Hz, 1H), 6.89 (s, 1H), 6.08 (s, 1H), 4.48-4.34 (m, 3H), 4.32 (d, *J* = 4.4 Hz, 2H), 4.12-3.99 (m, 1H), 3.98-3.89 (m, 1H), 3.46-3.44 (m, 2H), 2.89-2.81 (m, 2H), 2.74 (t, *J* = 7.2 Hz, 2H), 2.45 (m, 3H), 2.16 (s, 3H), 2.13 (s, 3H), 1.64-1.26 (m, 10H).

**Example 30, Compound 47 and 48 Synthesis:**

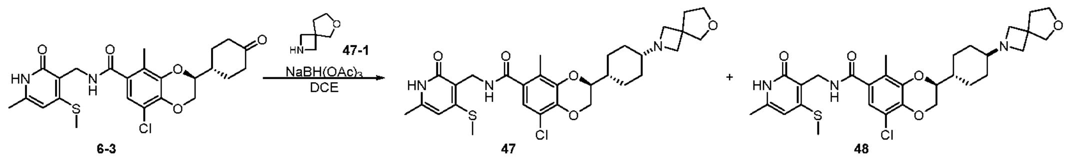


6-3 47 48

**Step 1, Compound 47 and 48 Synthesis:**

Following the synthesis method of Example **6**, Step **4**, except replacing Step **4, dimethylamine** with **47-1** (11.0 mg, 0.1 mmol), the same synthesis method was applied, and further resolved by Prep-MPLC to give Compound **47** (1.0 mg, 1.7 μmol, 2.0% yield) and **46** (1.0 mg, 1.7 μmol, 2.0% yield), LCMS ($ESI^+$) m/z: 588.2 $[M+H]^+$.

**Compound 47 NMR: $^1$H NMR** (600 MHz, DMSO-$d_6$) δ 11.52 (s, 1H), 8.02 (t, *J* = 4.4 Hz, 1H), 6.90 (s, 1H), 6.08 (s, 1H), 4.48 (dd, *J* = 11.6, 2.0 Hz, 1H), 4.32 (d, *J* = 4.4 Hz, 2H), 4.15-4.21 (m, 1H), 3.98-3.85 (td, *J* = 7.6, 2.0 Hz, 1H), 3.67-3.59 (m, 4H), 3.09-3.02(m, 4H), 2.45 (s, 3H), 2.16 (s, 3H), 2.13 (s, 3H), 2.11-1.81 (m, 4H), 1.79-1.69 (m, 3H), 1.58-1.49 (m, 1H), 1.22-1.11 (m, 2H), 0.92-0.81(m, 2H).

**Compound 48 NMR: $^1$H NMR** (600 MHz, DMSO-$d_6$) δ 11.52 (s, 1H), 8.02 (t, *J* = 4.4 Hz, 1H), 6.90 (s, 1H), 6.08 (s, 1H), 4.48 (dd, *J* = 11.6, 2.0 Hz, 1H), 4.29 (d, *J* = 4.4 Hz, 2H), 4.08-4.03 (m, 1H), 3.98 (t, *J* = 5.6 Hz, 1H), 3.68-3.62 (m, 4H), 3.07-3.01(m, 4H), 2.45 (s, 3H), 2.18-2.13 (m, 6H), 2.01 (t, *J*= 7.2 Hz, 2H), 1.58-1.20 (m, 10H).

**Example 31, Compound 49 and 50 Synthesis:**

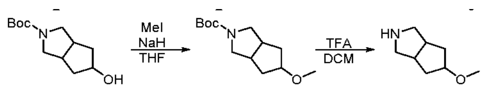


49-1-1 49-1-2 49-1

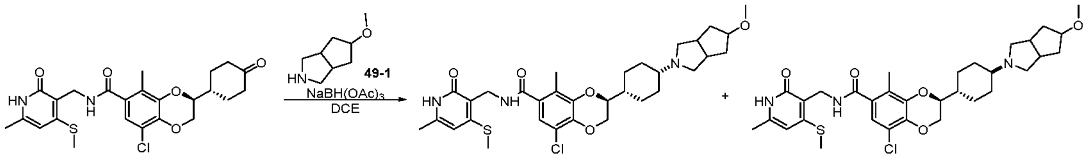


6-3 49 50

**Step 1, Compound 49-1-2 Synthesis**

A single-necked flask was added with substrate **49-1-1** (114.0 mg, 0.5 mmol). The mixture was dissolved with ultra dry THF (2 mL) under stirring, and then at 0°C added with **NaH** (15.0 mg, 0.6 mmol) to react for 0.5 hours, added with iodomethane(356.0 mg, 2.5 mmol), 0 °C to react for 1 hour, with LC-MS monitoring. After the completion of the reaction, the mixture was added with water to quench the reaction, and the reaction mixture was extrated with saturated ammounium chloride solution and EA three times. The organic phase was combined, washed with saturated saline solution, and dried with anhydrous sodium sulfate and then condensated under reduced pressure to give the crude product **49-1-2** (155.0 mg, crude), LCMS (ESI$^+$) m/z: 241.3 [M+H]$^+$.

**Step 2, Compound 49-1 Synthesis**

Following the synthesis method of Example **1**, Step **4**, except replacing Step **4, 1-5** with **49-1-2** (155.0 mg, crude), the same synthesis method was applied, to give Compound **49-1** (70.5 mg, crude), LCMS (ESI$^+$) m/z: 141.2 [M+H]$^+$.

**Step 3, Compound 49 and 50 Synthesis:**

Following the synthesis method of Example **6**, Step **4**, except replacing Step **4, dimethylamine** with **49-1** (70.5 mg, crude), the same synthesis method was applied, and further resolved by Prep-MPLC to give Compound **49** (32.0 mg, 0.05 mmol, 26.0% yield) and **50** (48.0 mg, 0.08 mmol, 39.0% yield), LCMS (ESI$^+$) m/z: 616.2 [M+H]$^+$.

**Compound 49 NMR: $^1$H NMR** (600 MHz, DMSO-$d_6$) δ 11.52 (s, 1H), 8.07 (t, *J* = 4.2 Hz, 1H), 6.90 (s, 1H), 6.08 (s, 1H), 4.51-4.43 (m, 1H), 4.29 (d, *J* = 4.6 Hz, 2H), 4.09-4.02 (m, 1H), 3.94-3.87 (m, 1H), 3.63-3.61 (m, 1H), 3.18 (s, 3H), 2.45 (s, 4H), 2.42-2.32 (m, 2H), 2.16 (s, 3H), 2.14 (s, 3H), 2.06-1.89 (m, 6H), 1.82-1.70 (m, 2H), 1.60-1.52 (m, 1H), 1.34-1.09 (m, 8H).

**Compound 50 NMR: $^1$H NMR** (600 MHz, DMSO-$d_6$) δ 11.52 (s, 1H), 8.07 (t, *J* = 4.2 Hz, 1H), 6.89 (s, 1H), 6.08 (s, 1H), 4.47-4.41 (m, 1H), 4.31 (t, *J* = 8.4 Hz, 2H), 4.12-4.05 (m, 1H), 3.99-3.92 (m, 1H), 3.61-3.50 (m, 1H), 3.18 (s, 3H), 2.61-2.58 (m, 2H), 2.44 (s, 3H), 2.40-2.32 (m, 2H), 2.18-2.07 (m, 10H), 1.82-1.78 (m, 2H), 1.73-1.60 (m, 3H), 1.60-1.54 (m 1H), 1.47-1.35 (m, 3H), 1.31-1.20 (m, 3H).

**Example 32, Compound 51 and 52 Synthesis:**

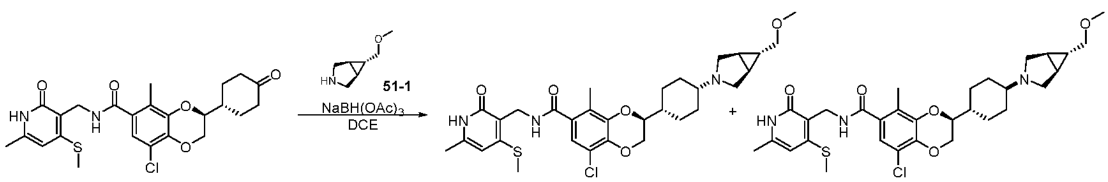


6-3 51 52

**Step 1, Compound 51 and 52 Synthesis:**

Following the synthesis method of Example **6**, Step **4**, except replacing Step **4, dimethylamine** with **51-1** (9.0 mg, 0.07 mmol), the same synthesis method was applied, and further resolved by Prep-MPLC to give Compound **51** (4.0 mg, 7.0 μmol, 12.0% yield) and **52** (6.0 mg, 0.01 mmol, 16.0% yield), LCMS (ESI$^+$) m/z: 602.5 [M+H]$^+$.

**Compound 51 NMR: $^1$H NMR** (400 MHz, DMSO-$d_6$) δ 11.51 (s, 1H), 8.02 (t, *J* = 4.8 Hz, 1H), 6.89 (s, 1H), 6.08 (s, 1H), 4.46-4.44 (m, 1H), 4.27 (d, *J* = 4.4 Hz, 2H), 4.06-4.01 (m, 1H), 3.91-3.87 (m, 1H), 3.19 (s, 3H), 3.13 (d, *J* = 7.2 Hz, 2H), 2.96 (t, *J* = 7.2 Hz, 2H), 2.52.53 (m, 1H), 2.45 (s, 3H), 2.30-2.28 (m, 2H), 2.16 (s, 3H), 2.13 (s, 3H), 2.02-1.96 (m, 2H), 1.92-1.86 (m, 2H), 1.75-1.69 (m, 1H), 1.58-1.52 (m, 1H), 1.26 (s, 2H), 1.21-1.12 (m, 2H), 1.11-1.03 (m. 2H).

**Compound 52 NMR: $^1$H NMR** (400 MHz, DMSO-$d_6$) δ 11.52 (s, 1H), 8.02 (t, *J* = 4.0 Hz, 1H), 6.89 (s, 1H), 6.08 (s, 1H), 4.43-4.40 (m, 1H), 4.27 (d, *J* = 4.4 Hz, 2H), 4.08-4.04 (m, 1H), 3.96-3.93 (m, 1H), 3.20 (s, 3H), 3.15 (d, *J* = 6.4 Hz, 2H), 2.98 (d, *J* = 8.8 Hz, 2H), 2.55-2.53 (m, 1H), 2.45 (s, 3H), 2.21-2.15 (m, 2H), 2.18 (s, 3H), 2.14 (s, 3H), 1.75-1.70 (m, 2H), 1.69-1.59 (m, 1H), 1.58-1.47 (m, 3H), 1.42-1.31 (m, 3H), 1.30-1.28 (m, 3H).

**Example 33, Compound 53, 54, 55 and 56 Synthesis:**

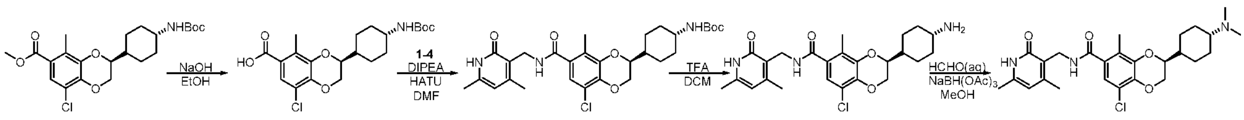


IM-5a 53-1 53-2 53-3 53

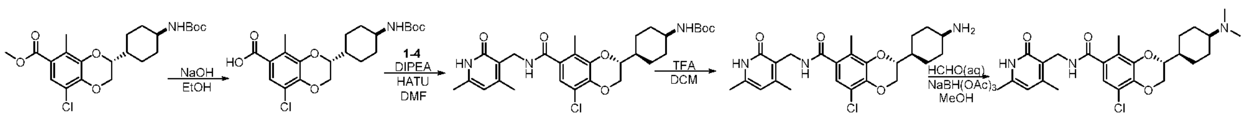


IM-5b 54-1 54-2 54-3 54

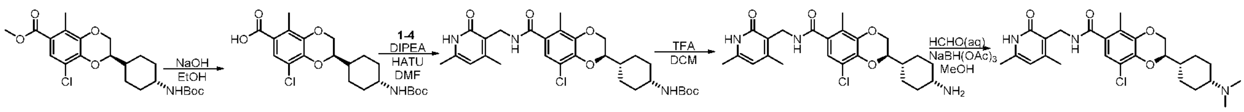


IM-5c 55-1 55-2 55-3 55

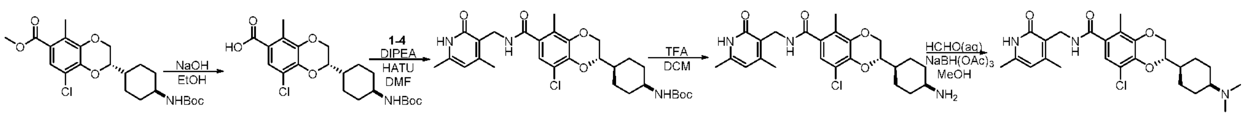


IM-5d 56-1 56-2 56-3 56

**Step 1, Compound 53-1, 54-1, 55-1 and 56-1 Synthesis:**

Taking Compound **53-1** as an example:
Following the synthesis method of Example **6**, Step **1**, except replacing Step **1**, **IM-3a** with **IM-5a** (50.0 mg, 0.11 mmol), the same synthesis method was applied, to give Compound **53-1** (42.0 mg, crude), LCMS (ESI$^+$) m/z: 426.2 [M+H]$^+$.

**Step 2, Compound 53-2, 54-2, 55-2 and 56-2 Synthesis:**

Taking Compound **53-2** as an example:
Following the synthesis method of Example **3**, Step **1**, except replacing Step **1, 2-3** with **53-1** (42.0 mg, crude), **3-1** with **1-4** (22.8 mg, 0.2 mmol)the same synthesis method was applied, to give Compound **53-2** (30.0 mg, 0.05 mmol, 48.7% yield), LCMS (ESI$^+$) m/z: 560.2 [M+H]$^+$.

**Step 3, Compound 53-3, 54-3, 55-3 and 56-3 Synthesis:**

Taking Compound **53-3** as an example:

Following the synthesis method of Example **3**, Step **2**, except replacing Step **2, 3-2** with **53-2** (30.0 mg, 0.05 mmol), the same synthesis method was applied, to give Compound **53-3** (18.0 mg, crude), LCMS (ESI$^+$) m/z: 460.2 [M+H]$^+$.

**Step 4, Compound 53, 54, 55 and 56 Synthesis:**

Taking Compound **53** as an example:
Following the synthesis method of Example **2**, Step **5**, except replacing Step **5, 2-6** with **53-3** (18.0 mg, crude), the same synthesis method was applied, to give Compound **53** (5.0 mg, 0.01 mmol, 20.5% yield), LCMS (ESI$^+$) m/z: 488.2 [M+H]$^+$.

**Compound 53 NMR: $^1$H NMR** (600 MHz, DMSO-$d_6$) δ 11.46 (s, 1H), 8.11 (t, $J$ = 4.8 Hz, 1H), 6.88 (s, 1H), 5.85 (s, 1H), 4.46 (dd, $J$ = 11.4, 2.4 Hz, 1H), 4.22 (d, $J$ = 4.8 Hz, 2H), 4.07-4.05 (m, 1H), 3.93-3.89 (m, 1H), 2.17 (s, 3H), 2.16 (s, 6H), 2.13-2.10 (m, 7H), 2.05-2.01 (m, 1H), 1.88-1.82 (m, 2H), 1.80-1.78 (m, 1H), 1.57-1.51 (m, 1H), 1.20-1.13 (m, 4H).

**Compound 55 NMR: $^1$H NMR** (600 MHz, DMSO-$d_6$) δ 11.46 (s, 1H), 8.11 (t, $J$ = 4.2 Hz, 1H), 6.90 (s, 1H), 5.86 (s, 1H), 4.46-4.43 (m, 1H), 4.22 (d, $J$ = 4.8 Hz, 2H), 4.07-4.05 (m, 1H), 3.93-3.89 (m, 1H), 2.17 (s, 3H), 2.16 (s, 6H), 2.11 (s, 6H), 2.05-1.97 (m, 2H), 1.85-1.81 (m, 2H), 1.80-1.76 (m, 1H), 1.57-1.51 (m, 1H), 1.18-1.12 (m,4H).

**Compound 56 NMR: $^1$H NMR** (600 MHz, DMSO-$d_6$) δ 11.46 (s, 1H), 8.11 (t, $J$ = 4.2 Hz, 1H), 6.90 (s, 1H), 5.86 (s, 1H), 4.46-4.42 (m, 1H), 4.24-4.20 (m, 2H), 4.07-4.02 (m, 1H), 3.97-3.94 (m, 1H), 2.17 (m, 9H), 2.13-2.09 (m, 6H), 2.09-2.07 (m, 1H), 2.07-2.04 (m, 1H), 1.88-1.82 (m, 2H), 1.82-1.76(m, 1H), 1.57-1.51 (m, 1H), 1.20-1.12 (m, 4H).

**Example 34, Compound 57 and 58 Synthesis:**

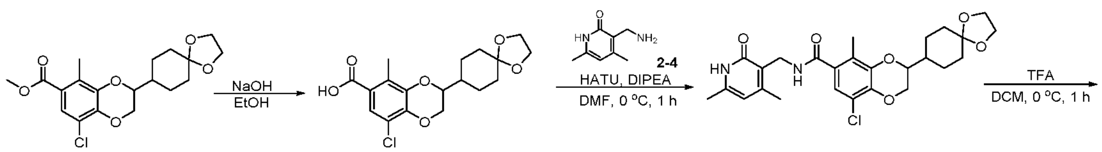


IM-3-6 57-1 57-2

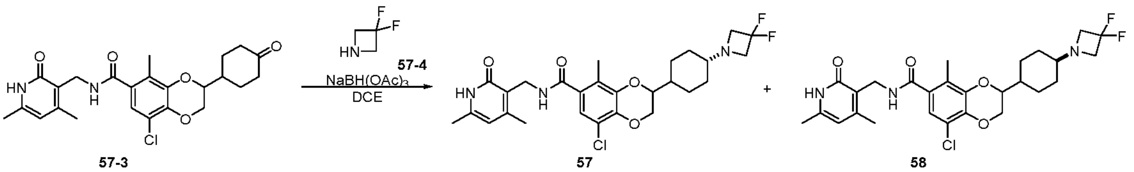


57-3 57 58

**Step 1, Compound 57-1 Synthesis:**

A single-necked flask was added with substrate **IM-3-6** (500.0 mg, 1.3 mmol). The mixture was dissolved with EtOH (3 mL) to dissolve, and then added with 2 N NaOH (3 mL), 80°C to react for 1 hour, with LC-MS monitoring. After the completion of the reaction, the mixture was added with 1 N HCl to adjust to pH = 6-7, and then added with EA solution to extract three times. The organic phase was combined, washed with saturated saline solution, and dried with anhydrous sodium sulfate and then condensated under reduced pressure, to give the crude product **57-1** (502.0 mg, crude). LCMS (ESI$^+$) m/z: 368.8 [M+H]$^+$

**Step 2, Compound 57-2 Synthesis:**

Following the synthesis method of Example **1**, Step **3**, except replacing Step **3, 1-3** with **57-1** (502.0 mg, crude), the same synthesis method was applied, to give Compound **57-2** (705.0 mg, crude), LCMS (ESI$^+$) m/z: 503.1 [M+H]$^+$.

**Step 3, Compound 57-3 Synthesis:**

Following the synthesis method of Example **3**, Step **2**, except replacing Step **2, 3-2** with **57-2** (705.0 mg, crude), the same synthesis method was applied, to give Compound **57-3** (620.0 mg, crude), LCMS (ESI$^+$) m/z: 458.9 [M+H]$^+$.

**Step 4, Compound 57 and 58 Synthesis:**

Following the synthesis method of Example **6**, Step **4**, except replacing Step **4, 6-3** with **57-3** (80.0 mg, 0.17

mmol), **dimethylamine** with **57-4** (49.0 mg, 0.5 mmol), the same synthesis method was applied. The obtained compounds was subjected to medium pressure liquid chromatography to give Compound **57** (20.0 mg, 0.04 mmol, 22.0% yield) and **58** (3.0 mg, 5.6 μmol, 3.3% yield), LCMS ($ESI^+$) m/z: 536.2 $[M+H]^+$.

**Compound 57 NMR: $^1$H NMR** (600 MHz, $CDCl_3$) δ 10.61 (s, 1H), 7.13 (s, 1H), 6.92 (s, 1H), 5.95 (s, 1H), 4.52-4.43 (m, 2H), 4.37 (d, *J* = 4.2 Hz, 1H), 4.02-3.99 (m, 1H), 3.82-3.80 (m, 1H), 3.57 (t, *J* = 11.8 Hz, 4H), 2.37 (s, 3H), 2.23-2.20 (m, 6H), 2.12-2.07 (m, 2H), 1.85-1.81 (m, 2H), 1.79-1.75 (m, 1H), 1.29-0.98 (m, 5H).

**Compound 58 NMR**: **$^1$H NMR** (600 MHz, $CDCl_3$) δ 10.68 (s, 1H), 7.03-7.01 (m, 1H), 6.95 (s, 1H), 5.95 (s, 1H), 4.49-4.46 (m, 2H), 4.38-4.34 (m, 1H), 4.13-4.00 (m, 3H), 3.94-3.68 (m, 3H), 2.90-2.60 (m, 1H), 2.38 (s, 3H), 2.24 (s, 3H), 2.21 (s, 3H), 2.00-1.92 (m, 1H), 1.80-1.69 (m,8H).

**Example 35, Compound 59 and 60 Synthesis:**

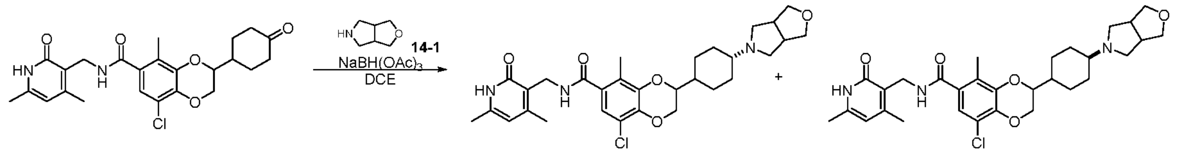


57-3 59 60

**Step 1, Compound 59 and 60 Synthesis:**

Following the synthesis method of Example **6**, Step **4**, except replacing Step **4, 6-3** with **57-3** (50.0 mg, 0.1 mmol), **dimethylamine** with **14-1** (37.0 mg, 0.3 mmol), the same synthesis method was applied. The obtained compounds was subjected to medium pressure liquid chromatography to give Compound **59** (10.0 mg, 0.02 mmol, 16.5% yield) and **60** (10.0 mg, 0.02 mmol, 16.5% yield), LCMS ($ESI^+$) m/z: 556.2 $[M+H]^+$.

**Compound 59 NMR**: **$^1$H NMR** (600 MHz, $CDCl_3$) δ 10.46 (s, 1H), 7.11 (t, *J* = 5.4 Hz, 1H), 6.96 (s, 1H), 5.93 (s, 1H), 4.49 (d, *J* = 6.0 Hz, 2H), 4.37 (dd, *J* = 10.8, 1.8 Hz, 1H), 4.01-3.99 (m, 1H), 3.86-3.82 (m, 1H), 3.74-3.72 (m, 2H), 3.65-3.63 (m, 2H), 3.19-3.01 (m, 2H), 2.45-2.39 (m, 2H), 2.37 (s, 3H), 2.24 (s, 3H), 2.22 (s, 3H), 2.16-2.11 (m, 3H), 1.88-1.84 (m, 1H), 1.74-1.66 (m, 5H), 1.34-1.14 (m, 3H).

**Compound 60 NMR**: **$^1$H NMR** (600 MHz, $CDCl_3$) δ 10.12 (s, 1H), 7.07 (dd, *J* = 11.2, 5.4 Hz, 1H), 6.95 (s, 1H), 5.92 (s, 1H), 4.49 (t, *J* = 5.4 Hz, 2H), 4.34 (d, *J* = 10.8 Hz, 2H), 4.19-4.16 (m, 1H), 4.09-3.95 (m, 2H), 3.86-3.80 (m, 2H), 3.75-3.70 (m, 1H), 3.65-3.55 (m, 2H), 3.48-3.34 (m, 2H), 3.09-3.01 (m, 1H), 2.52-2.48 (m, 2H), 2.50 (s, 3H), 2.37 (s, 3H), 2.24 (s, 3H), 2.10-2.05 (m, 2H), 1.89-1.77 (m, 2H), 1.75-1.68 (m, 2H), 1.63-1.59 (m, 2H).

**Example 36, Compound 61 and 62 Synthesis:**

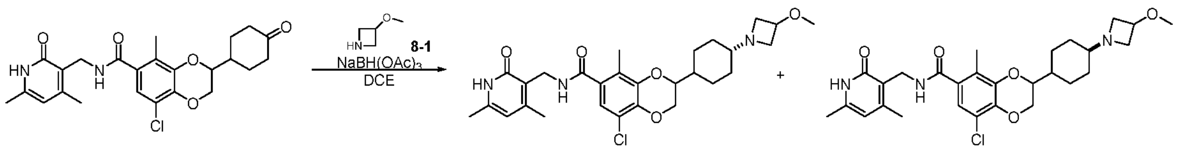


57-3 61 62

**Step 1, Compound 61 and 62 Synthesis:**

Following the synthesis method of Example **6**, Step **4**, except replacing Step **4, 6-3** with **57-3** (20.0 mg, 0.04 mmol), **dimethylamine** with **8-1** (7.6 mg, 0.09 mmol), the same synthesis method was applied. The obtained compounds was subjected to medium pressure liquid chromatography to give Compound **61** (4.4 mg, 8.3 μmol, 19.0% yield) and **62** (4.4 mg, 8.3 μmol, 19.0% yield), LCMS ($ESI^+$) m/z: 530.6 $[M+H]^+$.

**Compound 61 NMR**: **$^1$H NMR** (600 MHz, $CDCl_3$) δ 10.81 (s, 1H), 7.13 (t, *J* = 6.0 Hz, 1H), 6.97 (s, 1H), 5.94 (s, 1H), 4.55 (d, *J* = 5.4 Hz, 2H), 4.40 (dd, *J* = 9.0, 2.4 Hz, 1H), 4.30-4.26 (m, 1H), 4.25-4.10 ( m, 2H), 4.07-3.96 (m, 1H), 3.86-3.82 (m, 1H), 3.46-3.42 (m, 2H), 3.30 (s, 3H), 2.70-2.65 (m, 1H), 2.37 (s, 3H), 2.24 (s, 3H), 2.22 (s, 3H), 2.17-2.12 (m, 1H), 2.05-1.95 (m, 3H), 1.90-1.84 (m, 2H), 1.75-1.69 (m, 3H).

**Compound 62 NMR**: **$^1$H NMR** (600 MHz, $CDCl_3$) δ 12.04 (s, 1H), 7.03 (t, *J* = 6.0 Hz, 1H), 6.94 (s, 1H), 5.94 (s, 1H),

4.65-4.56 (m, 2H), 4.52-4.44 (m, 3H), 4.37-4.29 (m, 1H), 4.28-4.24 (m, 1H), 4.22-4.16 (m, 1H), 3.74-3.72 (m, 1H), 3.60-3.55 (m, 2H), 3.32 (s, 3H), 3.24-3.19 (m, 1H), 2.38 (s, 3H), 2.24 (s, 3H), 2.19 (s, 3H), 2.08-2.05 (m, 1H), 1.95-1.86 (m, 2H), 1.82-1.76 (m, 2H), 1.74-1.60 (m, 3H).

**Example 37, Compound 63 Synthesis:**

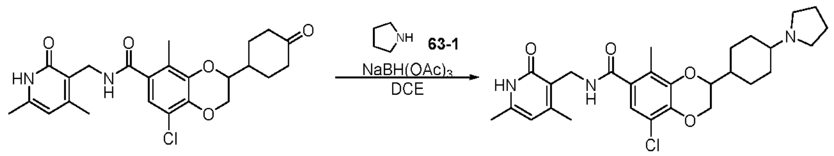


57-3 63

**Step 1, Compound 63 Synthesis:**

Following the synthesis method of Example **6**, Step **4**, except replacing Step **4, 6-3** with **57-3** (46.0 mg, 0.1 mmol), **dimethylamine** with **63-1** (11.0 mg, 0.2 mmol), the same synthesis method was applied to give Compound **63** (40.0 mg, 0.08 mmol, 77.8% yield), LCMS (ESI$^+$) m/z: 514.2 [M+H]$^+$.

**Compound 63 NMR**: **$^1$H NMR** (600 MHz, DMSO-$d_6$) δ 11.46 (s, 1H), 8.12 (t, *J* = 4.8 Hz, 1H), 6.88 (s, 1H), 5.53 (s, 1H), 4.48-4.42 (m, 1H), 4.26 (d, *J* = 5.4 Hz, 2H), 4.11-3.92 (m, 2H), 2.17 (s, 3H), 2.11 (m, 6H), 2.07-1.97 (m, 2H), 1.85-1.43 (m, 12H), 1.28-1.12 (m, 4H).

**Example 38, Compound 64 and 65 Synthesis:**

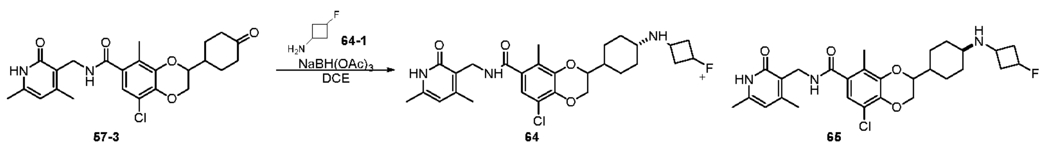


57-3 64 65

**Step 1, Compound 64 and 65 Synthesis:**

Following the synthesis method of Example **6**, Step **4**, except replacing Step **4, 6-3** with **57-3** (46.0 mg, 0.1 mmol), **dimethylamine** with **64-1** (13.0 mg, 0.2 mmol), the same synthesis method was applied. The obtained compounds was subjected to medium pressure liquid chromatography to give Compound **64** (15.0 mg, 0.03 mmol, 28.2 % yield) and **65** (20.0 mg, 0.04 mmol, 37.6 % yield), LCMS (ESI$^+$) m/z: 532.3 [M+H]$^+$.

**Compound 64 NMR**: **$^1$H NMR** (600 MHz, DMSO-$d_6$) δ 11.46 (s, 1H), 8.11 (t, *J* = 4.8 Hz, 1H), 6.88 (s, 1H), 5.86 (s, 1H), 5.35-5.08 (m, 1H), 4.48 (dd, *J* = 12.0, 2.4 Hz, 1H), 4.22 (d, *J* = 5.4 Hz, 2H), 4.06-4.02 (m, 1H), 3.91-3.88 (m, 1H), 3.56-3.51 (m, 1H), 2.32-2.23 (m, 3H), 2.17 (s, 3H), 2.11 (m, 6H), 2.07-2.01 (m, 2H), 1.98-1.92 (m, 1H), 1.89-1.82 (m, 2H), 1.73-1.69 (m, 1H), 1.56-1.51 (m, 1H), 1.28-1.13 (m, 3H), 1.01-0.92 (m, 2H).

**Compound 65 NMR**: **$^1$H NMR** (600 MHz, DMSO-$d_6$) δ 11.46 (s, 1H), 8.11 (t, *J* = 4.8 Hz, 1H), 6.88 (s, 1H), 5.86 (s, 1H), 5.25-5.10 (m, 1H), 4.48 (dd, *J* = 11.4, 1.8 Hz, 1H), 4.22 (d, *J* = 4.8 Hz, 2H), 4.09-4.03 (m, 1H), 4.02-3.98 (m, 1H), 3.52-3.44 (m, 1H), 2.21-2.13 (m, 1H), 2.35-2.26 (m, 2H), 2.17 (s, 3H), 2.11 (m, 8H), 1.68-1.49 (m, 10H).

**Example 39, Compound 66 and 67 Synthesis:**

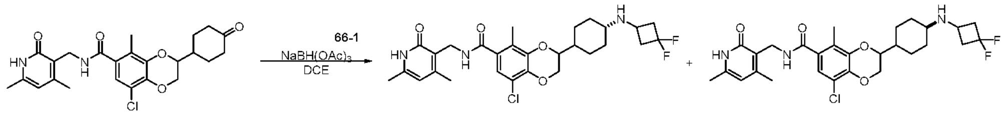


57-3 66 67

**Step 1, Compound 66 and 67 Synthesis:**

Following the synthesis method of Example **6**, Step **4**, except replacing Step **4, 6-3** with **57-3** (46.0 mg, 0.1 mmol), **dimethylamine** with **66-1** (16.0 mg, 0.15 mmol), the same synthesis method was applied. The obtained compounds was subjected to medium pressure liquid chromatography to give Compound **66** (12.0 mg, 0.02 mmol, 21.8 % yield) and **67** (18.0 mg, 0.03 mmol, 32.7 % yield), LCMS ($ESI^+$) m/z: 550.3 $[M+H]^+$.

**Compound 66 NMR**: **$^1$H NMR** (600 MHz, DMSO-$d_6$) δ 11.46 (s, 1H), 8.11 (t, *J* = 4.8 Hz, 1H), 6.88 (s, 1H), 5.86 (s, 1H), 4.48 (dd, *J* = 11.4, 1.8 Hz, 1H), 4.22 (d, *J* = 5.4 Hz, 2H), 4.08-4.04 (m, 1H), 3.92-3.89 (m, 1H), 3.22-3.17 (m, 1H), 2.77-2.68 (m, 2H), 2.35-2.21 (m, 3H), 2.17 (s, 3H), 2.13-2.10 (m, 6H), 1.95-1.93 (m, 1H), 1.88-1.84 (m, 2H), 1.74-1.70 (m, 1H), 1.58-1.49 (m, 1H), 1.25-1.11 (m, 3H), 1.11-0.93 (m, 2H).

**Compound 67 NMR**: **$^1$H NMR** (600 MHz, DMSO-$d_6$) δ 11.46 (s, 1H), 8.11 (t, *J* = 4.8 Hz, 1H), 6.88 (s, 1H), 5.86 (s, 1H), 4.48 (dd, *J* = 12.0, 2.4 Hz, 1H), 4.22 (d, *J* = 4.8 Hz, 2H), 4.08-4.05 (m, 1H), 4.01-3.97 (m, 1H), 3.18-3.11 (m, 1H), 2.78-2.66 (m, 3H), 2.36-2.24 (m, 2H), 2.17 (s, 3H), 2.11 (m, 6H), 1.68-1.48 (m, 10H).

**Example 40, Compound 68 and 69 Synthesis:**

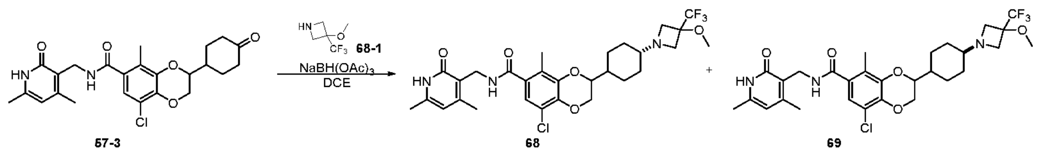


57-3 68 + 69

**Step 1, Compound 68 and 69 Synthesis:**

Following the synthesis method of Example **6**, Step **4**, except replacing Step **4, 6-3** with **57-3** (50.0 mg, 0.1 mmol), **dimethylamine** with **68-1** (85.0 mg, 0.55 mmol), the same synthesis method was applied. The obtained compounds was subjected to medium pressure liquid chromatography to give Compound **68** (9.0 mg, 0.015 mmol, 13.8% yield) and **69** (10.0 mg, 0.017 mmol, 15.3% yield), LCMS ($ESI^+$) m/z: 598.6 $[M+H]^+$.

**Example 41, Compound 70 and 71 Synthesis:**

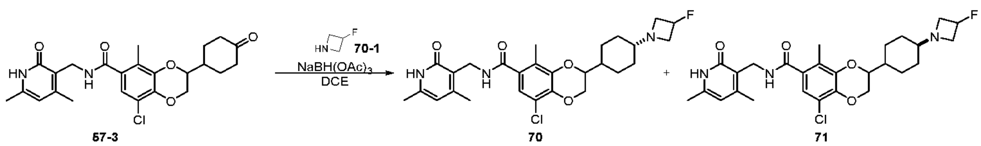


57-3 70 + 71

**Step 1, Compound 70 and 71 Synthesis:**

Following the synthesis method of Example **6**, Step **4**, except replacing Step **4, 6-3** with **57-3** (46.0 mg, 0.1 mmol), **dimethylamine** with **70-1** (9.0 mg, 0.1 mmol), the same synthesis method was applied. The obtained compound was subjected to Prep-MPLC to give Compound **70** (5.0 mg, 0.01 mmol, 9.7% yield) and **71** (12.0 mg, 0.02 mmol, 23.2% yield), LCMS ($ESI^+$) m/z: 518.3 $[M+H]^+$.

**Compound 70 NMR**: **$^1$H NMR** (400 MHz, DMSO-$d_6$) δ 11.47 (s, 1H), 8.12-8.10 (m, 1H), 6.89-6.87 (m, 1H), 5.85 (s, 1H), 5.19-5.04 (m, 1H), 4.50-4.45 (m, 1H), 4.30-4.20 (m, 2H), 4.10-4.02 (m, 1H), 3.98-3.90 (m, 1H), 3.52-3.50 (m, 2H), 3.02-2.98 (m, 2H), 2.17 (s, 3H), 2.13-2.11 (m, 6H), 2.02-1.94 (m, 1H), 1.78-1.72 (m, 2H), 1.60-1.51 (m, 1H), 1.29-1.15 (m, 4H), 0.92-0.85 (m, 2H).

**Compound 71 NMR**: **$^1$H NMR** (400 MHz, DMSO-$d_6$) δ 11.49 (s, 1H), 8.12-8.10 (m, 1H), 6.89-6.87 (m, 1H), 5.85 (s, 1H), 5.20-5.04 (m, 1H), 4.42-4.38 (m, 1H), 4.25-4.18 (m, 2H), 4.10-4.02 (m, 1H), 3.98-3.88 (m, 1H), 3.54-3.46 (m, 2H), 2.99-2.90 (m, 2H), 2.17 (s, 3H), 2.11-2.08 (m, 6H), 1.65-1.49 (m, 6H), 1.42-1.32 (m, 4H).

**Example 42, Compound 72, 73, 74 and 75 Synthesis:**

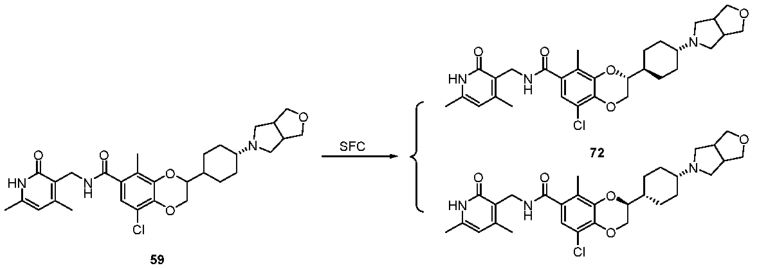


72

73

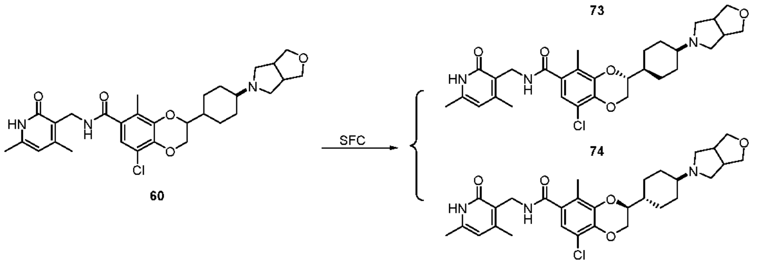


74

75

**Step 1, Compound 72 and 73 Synthesis:**

Compound 72 and 73 could be obtained by SFC resolution of Compound 59., LCMS (ESI$^+$) m/z: 556.3 [M+H]$^+$

**Compound 72 NMR: $^1$H NMR** (400 MHz, $CDCl_3$) δ 12.05 (s, 1H), 7.23-7.21 (m, 1H), 6.96 (s, 1H), 5.95 (s, 1H), 4.50 (d, *J* = 5.4 Hz, 2H), 4.38 (d, *J* = 10.8 Hz, 1H), 4.04-3.98 (m, 1H), 3.84-3.78 (m, 1H), 3.75-3.68 (m, 2H), 3.66-3.62 (m, 2H), 3.12-2.98 (m, 2H), 2.36 (s, 3H), 2.29-2.27 (m, 3H), 2.24-2.22 (m, 6H), 2.12-2.02 (m, 4H), 1.83-1.75 (m, 2H), 1.65-1.58 (m, 2H), 1.31-1.19 (m, 3H).

**Compound 73 NMR: $^1$H NMR** (400 MHz, $CDCl_3$) δ 12.00 (s, 1H), 7.20 (t, *J* = 5.4 Hz, 1H), 6.96 (s, 1H), 5.95 (s, 1H), 4.55-4.46 (m, 2H), 4.39-4.24 (d, *J* = 9.6 Hz, 1H), 4.04-3.92 (m, 4H), 3.58-3.42 (m, 2H), 2.90-2.60 (m, 2H), 2.51-2.45 (m, 3H), 2.37 (s, 3H), 2.24-2.21 (m, 6H), 1.95-1.60 (m, 10H), 1.51-1.48 (m, 1H).

**Step 2, Compound 74 and 75 Synthesis:**

Compound 74 and 75 could be obtained by SFC resolution of Compound 60. LCMS (ESI$^+$) m/z: 556.1 [M+H]$^+$.

**Compound 74 NMR: $^1$H NMR** (400 MHz, $CDCl_3$) δ 11.97 (s, 1H), 7.20 (t, *J* = 6.0 Hz, 1H), 6.95 (s, 1H), 5.95 (s, 1H), 4.55-4.46 (m, 2H), 4.42-4.35 (d, *J* = 9.6 Hz, 1H), 4.10-3.92 (m, 2H), 3.94-3.78 (m, 2H), 3.58-3.47 (m, 2H), 2.51-2.42 (m, 3H), 2.37 (s, 3H), 2.26-2.22 (m, 7H), 1.90-1.63 (m, 9H), 1.50-1.47 (m, 3H).

**Compound 75 NMR: $^1$H NMR** (400 MHz, $CDCl_3$) δ 11.98 (s, 1H), 7.19 (t, *J* = 5.4 Hz, 1H), 6.96 (s, 1H), 5.96 (s, 1H), 4.55 (d, *J* = 5.4 Hz 2H), 4.42 (d, *J* = 10.2 Hz, 1H), 4.07-3.94 (m, 1H), 3.85-3.77 (m, 1H), 3.76-3.51 (m, 4H), 3.18-3.02 (m, 2H), 2.37 (s, 3H), 2.35-2.18 (m, 8H), 2.15-1.98 (m, 5H), 1.82-1.78 (m, 1H), 1.68-1.59 (m, 1H), 1.42-1.22 (m, 5H).

**Example 43, Compound 76 and 77 Synthesis:**

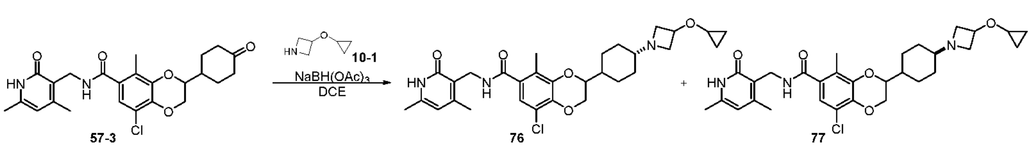


57-3 76 77

**Step 1, Compound 76 and 77 Synthesis:**

Following the synthesis method of Example **6**, Step **4**, except replacing Step **4, 6-3** with **57-3** (40.0 mg, 0.09 mmol), **dimethylamine** with **10-1** (12.0 mg, 0.1 mmol), the same synthesis method was applied. The obtained compound was subjected to Prep-MPLC to give Compound **76** (7.5 mg, 13.5 μmol, 15.5% yield) and 77 (7.5 mg, 13.5 μmol, 15.5% yield), LCMS (ESI$^+$) m/z: 556.3 [M+H]$^+$.

**Compound 76 NMR**: **$^1$H NMR** (600 MHz, DMSO-$d_6$) δ 11.46 (s, 1H), 8.11 (t, *J* = 4.8 Hz, 1H), 6.87 (s, 1H), 5.85 (s, 1H), 4.48-4.43 (m, 1H), 4.22 (d, *J* = 4.8 Hz, 2H), 4.09-4.02 (m, 2H), 3.94-3.88 (m, 1H), 3.49-3.45 (m, 2H), 3.23-3.19 (m, 1H),

2.78-2.72 (m, 2H), 2.16 (s, 3H), 2.13-2.10 (m, 6H), 1.96-1.82 (m, 2H), 1.79-1.69 (m, 3H), 1.56-1.50 (m, 1H), 1.56-1.51 (m, 2H), 0.92-0.80 (m, 2H), 0.46-0.43 (m, 2H), 0.42-0.38 (m, 2H).

**Compound 77 NMR: $^1$H NMR** (600 MHz, DMSO-$d_6$) δ 11.46 (s, 1H), 8.11 (t, *J* = 4.8 Hz, 1H), 6.87 (s, 1H), 5.85 (s, 1H), 4.42 (dd, *J* = 11.4, 1.8 Hz, 1H), 4.22 (d, *J* = 5.4 Hz, 2H), 4.12-4.02 (m, 2H), 3.94-3.92 (m, 1H), 3.50-3.44 (m, 2H), 3.24-3.19 (m, 1H), 2.72 (t, *J* = 6.0 Hz, 2H), 2.24-2.19 (m, 1H), 2.18-2.16 (m, 3H), 2.12-2.09 (m, 6H), 1.62-1.29 (m, 9H), 0.48-0.43 (m, 2H), 0.43-0.38 (m, 2H).

**Example 44, Compound 78 and 79 Synthesis:**

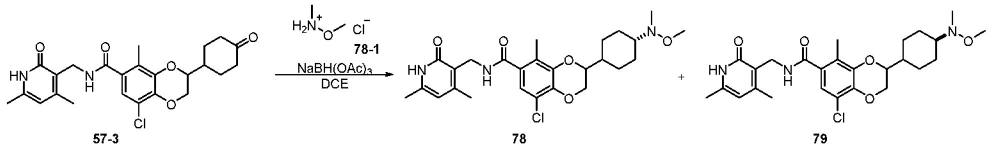


57-3 78 79

**Step 1, Compound 78 and 79 Synthesis:**

Following the synthesis method of Example **6**, Step **4**, except replacing Step **4, 6-3** with **57-3** (100.0 mg, 0.22 mmol), **dimethylamine** with **78-1** (40.0 mg, 0.4 mmol), the same synthesis method was applied. The obtained compound was subjected to Prep-MPLC to give Compound **78** (24.0 mg, 0.05 mmol, 21.8% yield) and **79** (18.0 mg, 0.04 mmol, 16.4% yield), LCMS (ESI$^+$) m/z: 504.2 [M+H]$^+$.

**Compound 78 NMR**: **$^1$H NMR** (600 MHz, DMSO-$d_6$) δ 11.46 (s, 1H), 8.11 (t, *J* = 4.8 Hz, 1H), 6.88 (s, 1H), 5.85 (s, 1H), 4.48 (dd, *J* = 10.8 Hz, 1.8 Hz, 1H), 4.22 (d, *J* = 5.4 Hz, 2H), 4.12-4.06 (m, 1H), 4.05-4.01 (m, 1H), 3.42 (s, 3H), 2.48 (s, 3H), 2.47-2.45 (m, 1H), 2.17 (s, 3H), 2.11 (s, 3H), 2.10 (s, 3H), 1.85-1.52 (m, 6H), 1.52-1.43 (m, 3H).

**Compound 79 NMR**: **$^1$H NMR** (600 MHz, DMSO-$d_6$) δ 11.46 (s, 1H), 8.11 (t, *J* = 5.4 Hz, 1H), 6.88 (s, 1H), 5.85 (s, 1H), 4.48 (dd, *J* = 12.0, 2.4 Hz, 1H), 4.22 (d, *J* = 4.8 Hz, 2H), 4.08-4.02 (m, 1H), 3.93-3.88 (m, 1H), 3.37 (s, 3H), 2.48 (s, 3H), 2.47-2.43 (m, 1H), 2.17 (s, 3H), 2.17 (s, 3H), 2.11 (s, 3H), 2.02-1.91 (m, 3H), 1.81-1.76 (m, 1H), 1.60-1.53 (m, 1H), 1.24-1.10 (m, 4H).

**Example 45, Compound 80 and 81 Synthesis:**

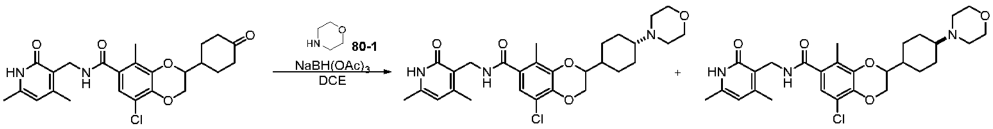


57-3 80 81

**Step 1, Compound 80 and 81 Synthesis:**

Following the synthesis method of Example **6**, Step **4**, except replacing Step **4, 6-3** with **57-3** (50.0 mg, 0.1 mmol), **dimethylamine** with **80-1** (19.0 mg, 0.2 mmol), the same synthesis method was applied. The obtained compound was subjected to Prep-MPLC to give Compound **80** (10.0 mg, 18.9 μmol, 17.3% yield) and **81** (5.0 mg, 9.4 μmol, 8.7% yield), LCMS (ESI$^+$) m/z: 530.6 [M+H]$^+$.

**Compound 80 NMR**: **$^1$H NMR** (600 MHz, $CDCl_3$) δ 10.81 (s, 1H), 7.13 (t, *J* = 6.0 Hz, 1H), 6.97 (s, 1H), 5.94 (s, 1H), 4.51 (d, *J*= 5.4 Hz, 2H), 4.38 (d, *J* = 11.4, 2.4 Hz, 1H), 4.30-4.26 (m, 1H), 4.25-4.19 ( m, 2H), 4.03-3.98 (m, 1H), 3.86-3.82 (m, 1H), 3.46-3.38 (m, 2H), 3.30 (s, 3H), 2.70-2.60 (m, 1H), 2.37 (s, 3H), 2.24 (s, 3H), 2.22 (s, 3H), 2.17-2.12 (m, 1H), 2.05-1.95 (m, 3H), 1.90-1.84 (m, 2H), 1.75-1.69 (m, 3H).

**Compound 81 NMR**: **$^1$H NMR** (600 MHz, $CDCl_3$) δ 12.04 (s, 1H), 7.03 (t, *J* = 6.0 Hz, 1H), 6.94 (s, 1H), 5.94 (s, 1H), 4.65-4.56 (m, 2H), 4.52-4.44 (m, 3H), 4.37-4.29 (m, 1H), 4.28-4.24 (m, 1H), 4.19-4.16 (m, 1H), 3.74-3.72 (m, 1H), 3.60-3.55 (m, 2H), 3.32 (s, 3H), 3.24-3.19 (m, 1H), 2.38 (s, 3H), 2.24 (s, 3H), 2.19 (s, 3H), 2.08-2.05 (m, 1H), 1.95-1.86 (m, 2H), 1.82-1.76 (m, 2H), 1.74-1.60 (m, 3H).

**Example 46, Compound 82 Synthesis:**

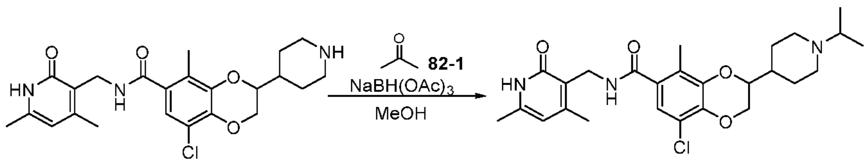


1-6 82

**Step 1, Compound 82 Synthesis:**

Following the synthesis method of Example **6**, Step **4**, except replacing Step **4, 6-3** with **1-6** (50.0 mg, 0.1 mmol), **dimethylamine** with **82-1** (20.0 mg, 0.3 mmol), DCE with MeOH, the same synthesis method was applied, to give Compound **82** (5.0 mg, 10.2 μmol, 9.1 % yield), LCMS (ESI$^+$) m/z: 488.2 $[M+H]^+$.

**$^1$H NMR** (400 MHz, DMSO-$d_6$) δ 11.47 (s, 1H), 8.15 (t, *J* = 4.8 Hz, 1H), 6.92 (m, 1H), 5.86 (s, 1H), 4.46-4.44 (m, 1H), 4.24-4.21 (m, 2H), 4.17-4.12 (m, 1H), 4.10-4.03 (m, 1H), 3.47-3.41(m, 2H), 3.03-2.90 (m, 3H), 2.17 (s, 3H), 2.14 (s, 3H), 2.11 (s, 3H), 2.10-2.01 (m, 1H), 1.96-1.91 (m, 2H), 1.74-1.59 (m, 2H), 1.25-1.15 (m, 6H).

**Example 47, Compound 83 Synthesis:**

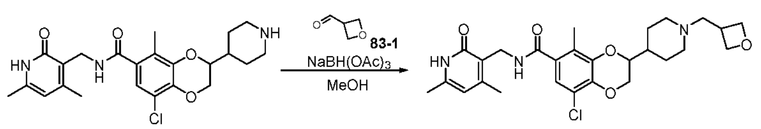


1-6 83

**Step 1, Compound 83 Synthesis:**

Following the synthesis method of Example **6**, Step **4**, except replacing Step **4, 6-3** with **1-6** (20.0 mg, 0.04 mmol), **dimethylamine** with **83-1** (8.0 mg, 0.09 mmol), DCE with MeOH, the same synthesis method was applied, to give Compound **83** (5.0 mg, 9.7 μmol, 21.6% yield), LCMS (ESI$^+$) m/z: 516.2 $[M+H]^+$.

**$^1$H NMR** (400 MHz, DMSO-$d_6$) δ 11.46 (s, 1H), 8.12 (t, *J* = 5.0 Hz, 1H), 6.89 (d, *J* = 5.0 Hz, 1H), 5.85 (s, 1H), 4.62 (dd, *J* = 7.8, 5.8 Hz, 2H), 4.23 (q, *J* = 5.4, 4.8 Hz, 4H), 2.81 (s, 2H), 2.61 (s, 1H), 2.17 (s, 3H), 2.11 (s, 6H), 1.84 (d, *J*= 9.2 Hz, 3H), 1.61 (s, 2H), 1.42-1.35 (m, 3H), 1.31-1.23 (m, 4H).

**Example 48, Compound 84 Synthesis:**

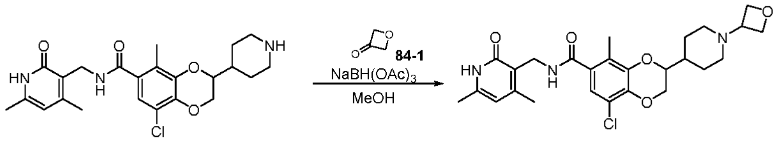


1-6 84

**Step 1, Compound 84 Synthesis:**

Following the synthesis method of Example **6**, Step **4**, except replacing Step **4, 6-3** with **1-6** (50.0 mg, 0.1 mmol), **dimethylamine** with **84-1** (16.0 mg, 0.2 mmol), DCE with MeOH, the same synthesis method was applied, to give Compound **84** (6.0 mg, 12.0 μmol, 10.7% yield), LCMS (ESI$^+$) m/z: 502.3 $[M+H]^+$.

**$^1$H NMR** (400 MHz, Methanol-$d_4$) δ 6.96-6.91 (m, 1H), 6.09 (s, 1H), 4.42 (s, 2H), 3.98-3.96 (m, 1H), 2.36 (s, 3H), 2.34-2.29 (m, 6H), 2.24 (m, 3H), 2.21-2.18 (m, 3H), 2.11-1.96 (m, 1H), 1.89-1.72 (m, 3H), 1.71-1.51 (m, 3H), 1.41-1.36 (m,

2H), 1.20-1.19 (m, 1H).

**Example 49, Compound 85 Synthesis:**

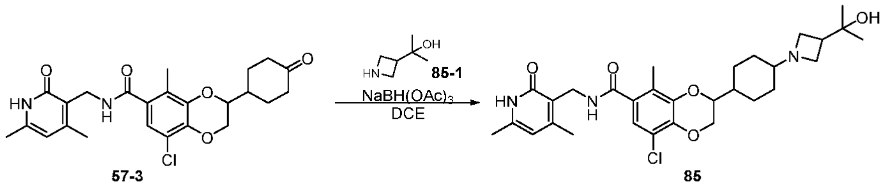


57-3 85

**Step 1, Compound 85 Synthesis:**

Following the synthesis method of Example **6**, Step **4**, except replacing Step **4, 6-3** with **57-3** (80.0 mg, 0.17 mmol), **dimethylamine** with **85-1** (80.0 mg, 0.7 mmol), the same synthesis method was applied. The obtained compound was resolved by SFC to give **85** (60.0 mg, 0.1 mmol, 61.8% yield), LCMS (ESI$^+$) m/z: 558.3 $[M+H]^+$.

**$^1$H NMR** (600 MHz, DMSO-$d_6$) δ 11.48 (s, 1H), 8.13 (t, *J* = 4.8 Hz, 1H), 6.87 (s, 1H), 5.85 (s, 1H), 4.42 (dd, *J* = 11.4, 1.8 Hz, 1H), 4.26-4.18 (m, 3H), 4.08-4.03 (m, 1H), 3.95-3.90 (m, 1H), 3.14-3.06 (m, 2H), 2.88-2.82 (m, 2H), 2.32-2.28 (m, 1H), 2.21-2.18 (m, 4H), 2.14-2.09 (m, 6H), 1.61-1.45 (m, 6H), 1.38-1.32 (m, 1H), 1.31-1.22 (m, 2H), 1.01 (m, 6H).

**Example 50, Compound 86 and 87 Synthesis:**

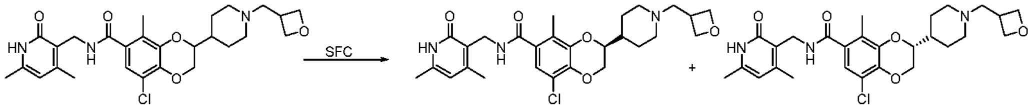


83 86 87

**Step 1, Compound 86 and 87 Synthesis:**

Compound 86 and 87 was obtained by SFC resolution of Compound 83.LCMS (ESI$^+$) m/z: 516.2 $[M+H]^+$, 538.1 $[M+Na]^+$

**Compound 86 NMR: $^1$H NMR** (400 MHz, DMSO-$d_6$) δ 11.49 (s, 1H), 8.13-8.11 (m, 1H), 6.90-6.87 (m, 1H), 5.75 (s, 1H), 4.65-4.62 (m, 2H), 4.26-4.21 (m, 4H), 2.83-2.78 (m, 2H), 2.63-2.58 (m, 1H), 2.17 (s, 3H), 2.11 (s, 6H), 1.90-1.80 (m, 3H), 1.64-1.58 (m, 2H), 1.42-1.35 (m, 3H), 1.38-1.35 (m, 4H).

**Compound 87 NMR: $^1$H NMR** (400 MHz, DMSO-$d_6$) δ 11.49 (s, 1H), 8.13-8.11 (m, 1H), 6.90-6.87 (m, 1H), 5.75 (s, 1H), 4.65-4.62 (m, 2H), 4.26-4.21 (m, 4H), 2.83-2.78 (m, 2H), 2.63-2.58 (m, 1H), 2.17 (s, 3H), 2.11 (s, 6H), 1.90-1.80 (m, 3H), 1.64-1.58 (m, 2H), 1.42-1.35 (m, 3H), 1.38-1.35 (m, 4H).

**Example 51, Compound 88 and 89 Synthesis:**

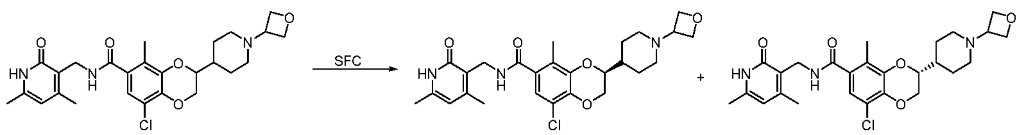


84 88 89

**Step 1, Compound 88 and 89 Synthesis:**

Compound 88 and 89 was obtained by SFC resolution of Compound 84.

**Compound 88 NMR: $^1$H NMR** (400 MHz, methanol-$d_4$) δ 6.95-6.91 (m, 1H), 6.09 (s, 1H), 4.43-4.41 (m, 2H),

4.42-3.94 (m, 1H), 2.36 (s, 3H), 2.38-2.27 (m, 6H), 2.23 (s, 3H), 2.22-2.18 (m, 3H), 2.12-1.95 (m, 1H), 1.89-1.72 (m, 3H), 1.72-1.54 (m, 3H), 1.39-1.36 (s, 2H), 1.21-1.19 (m, 1H).

**Compound 89 NMR: $^1$H NMR** (400 MHz, DMSO-$d_6$) δ 11.46 (m, 1H), 8.13 (t, *J* = 4.8 Hz, 1H), 6.89 (s, 1H), 5.85 (s, 1H), 4.51-4.50 (m, 2H), 4.44-4.39 (m, 3H), 4.22-4.21 (m, 2H), 4.10-3.98 (m, 2H), 2.74-2.72 (m, 1H), 2.17 (s, 3H), 2.12 (s, 3H), 2.11 (s, 3H), 1.94-1.84 (m, 4H), 1.61-1.51 (m, 5H).

**Example 52, Compound 90 Synthesis:**

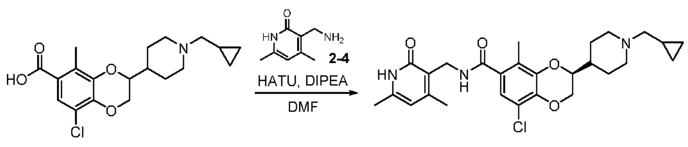


**16-4** **90**

**Step 1, Compound 90 Synthesis:**

Following the synthesis method of Example **3**, Step **1**, except replacing Step **1, 2-3** with **16-4** (50.0 mg, 0.14 mmol), **3-1** with **2-4** (25.0 mg, 0.2 mmol), the same synthesis method was applied, to give Compound **90** (15.0 mg, 0.03 mmol, 22.0% yield), LCMS (ESI$^+$) m/z: 500.3 [M+H]$^+$.

**$^1$H NMR** (400 MHz, DMSO-$d_6$) δ 11.46 (s, 1H), 8.13 (t, *J* = 5.2 Hz, 1H), 6.88 (s, 1H), 5.85 (s, 1H), 4.47 (dd, *J* = 11.2, 2.0 Hz, 1H), 4.23 (d, *J* = 4.8 Hz, 2H), 4.10-4.05 (m, 1H), 3.99-3.95 (m, 1H), 3.01 (d, *J* = 11.2 Hz, 2H), 2.17 (s, 3H), 2.15-2.13 (m, 2H), 2.12-2.11 (s, 6H), 1.87-1.82 (m, 3H), 1.66-1.62 (m, 2H), 1.43-1.40 (m, 2H), 0.85-0.75 (m, 1H), 0.45-0.42 (m, 2H), 0.05-0.01 (m, 2H).

**Example 53, Compound 91 Synthesis:**

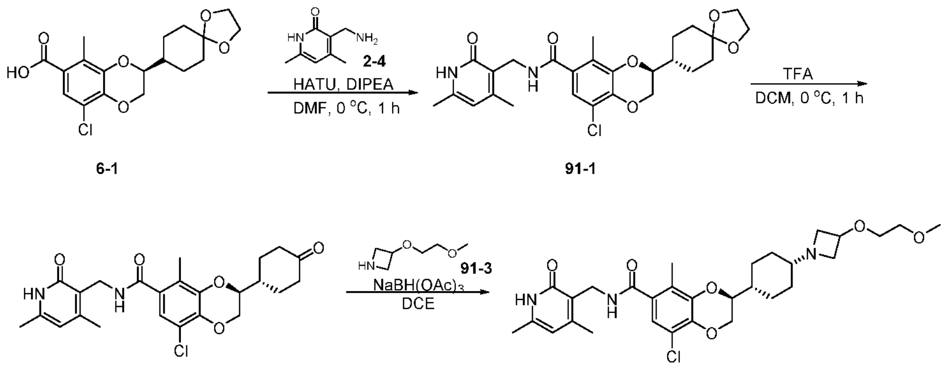


**6-1** **91-1**

**91-2** **91**

**Step 1, Compound 91-1 Synthesis:**

Following the synthesis method of Example **3**, Step **1**, except replacing Step **1, 2-3** with **6-1** (250.0 mg, 0.7 mmol), the same synthesis method was applied, to give Compound **91-1** (300.0 mg, 0.6 mmol), LCMS (ESI$^+$) m/z: 503.2 [M+H]$^+$.

**Step 2, Compound 91-2 Synthesis:**

Following the synthesis method of Example **3**, Step **2**, except replacing Step **2, 3-2** with **91-1** (300.0 mg, 0.6 mmol), the same synthesis method was applied, to give Compound **91-2** (250.0 mg, crude), LCMS (ESI$^+$) m/z: 459.2 [M+H]$^+$.

**Step 3, Compound 91 Synthesis:**

Following the synthesis method of Example **6**, Step **4**, except replacing Step **4, 6-3** with **91-2** (50.0 mg, 0.1 mmol),

**dimethylamine** with **91-3** (43.0 mg, 0.3 mmol), the same synthesis method was applied. The obtained compound was subjected to Prep-MPLC to give Compound **91** (10.0 mg, 17.4 μmol, 17.4% yield), LCMS (ESI+) m/z: 574.2 [M+H]+.

**$^1$H NMR** (400 MHz, DMSO-$d_6$) δ 11.46 (s, 1H), 8.12 (t, *J* = 5.0 Hz, 1H), 6.87 (s, 1H), 5.85 (s, 1H), 4.45 (dd, *J* = 11.4, 2.0 Hz, 1H), 4.22 (d, *J* = 5.0 Hz, 2H), 4.07-4.02 (m, 1H), 3.99-3.97 (m, 1H), 3.93-3.91 (m, 1H), 3.45-3.42 (m, 6H), 3.23 (s, 3H), 2.73-2.70 (m, 2H), 2.17 (s, 3H), 2.11 (s, 6H), 1.98-1.84 (m, 2H), 1.77-1.71 (m, 3H), 1.59-1.48 (m, 1H), 1.19-1.13 (m, 2H), 0.89-0.83 (m, 2H).

**Example 54, Compound 92 Synthesis:**

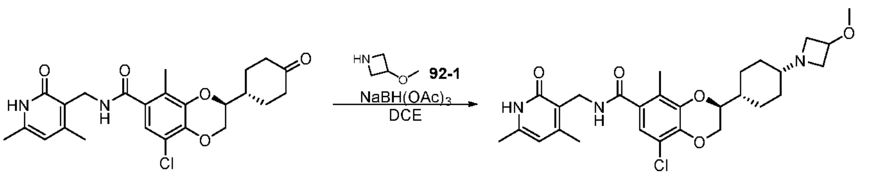


**91-2** **92**

**Step 1, Compound 92 Synthesis:**

Following the synthesis method of Example **6**, Step **4**, except replacing Step **4, 6-3** with **91-2** (50.0 mg, 0.1 mmol), **dimethylamine** with **92-1** (28.0 mg, 0.3 mmol), the same synthesis method was applied. The obtained compound was subjected to Prep-MPLC to give Compound **92** (8.0 mg, 15.1 μmol, 13.8% yield), LCMS (ESI+) m/z: 530.2 [M+H]+.

**$^1$H NMR** (400 MHz, DMSO-$d_6$) δ 11.46 (s, 1H), 8.13 (t, *J* = 5.0 Hz, 1H), 6.88 (s, 1H), 5.85 (s, 1H), 4.46 (dd, *J* = 11.4, 2.2 Hz, 1H), 4.22 (d, *J* = 5.0 Hz, 2H), 4.07-4.02 (m, 1H), 3.92-3.87 (m, 2H), 3.45 (t, *J*=6.6 Hz, 2H), 3.13 (s, 3H), 2.72 (t, *J* = 6.6 Hz, 2H), 2.17 (s, 3H), 2.11 (s, 6H), 1.96-1.88 (m, 2H), 1.78-1.70 (m, 3H), 1.57-1.49 (m, 1H), 1.24-1.10 (m, 2H), 0.94-0.81 (m, 2H).

**Example 55, Compound 93 Synthesis:**

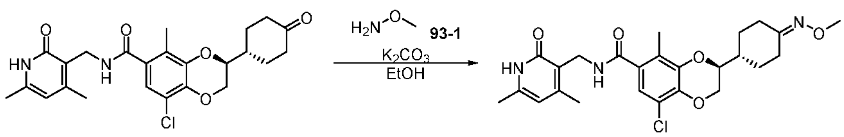


**91-2** **93**

**Step 1, Compound 92 Synthesis:**

A single-necked flask was added with substrate **91-2** (20.0 mg, 44.0 μmol). The mixture was dissolved with EtOH (5 mL) to dissolve, and then added with **93-1** (4.0 mg, 88.0 μmol) and potassium carbonate (18.0 mg, 0.13 mmol), at 80°C reacted for 2 hours, with LC-MS monitoring. After the completion of the reaction, and condensated under reduced pressure, and then subjected to Prep-MPLC preparation to give the product **92** (1.1 mg, 2.3 μmol, 5.1% yield). LCMS (ESI+) m/z: 488.1 [M+H]+.

**$^1$H NMR** (400 MHz, DMSO-$d_6$) δ 11.46 (s, 1H), 8.12 (s, 1H), 6.89 (s, 1H), 5.85 (s, 1H), 4.47 (d, *J*= 10.6 Hz, 1H), 4.23 (d, *J* = 5.0 Hz, 2H), 4.16-3.92 (m, 2H), 3.71 (s, 3H), 3.06 (m, 1H), 2.33 (m, 1H), 2.29 (s, 1H), 2.17 (s, 3H), 2.14-2.09 (m, 6H), 2.08-2.07 (m,1H) 1.93-1.78 (m, 3H), 1.33 (m, 2H).

**Example 56, Compound 94 Synthesis:**

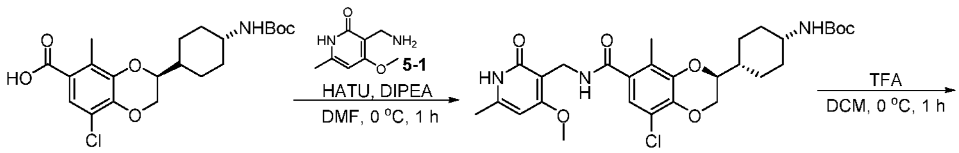


53-1 94-1

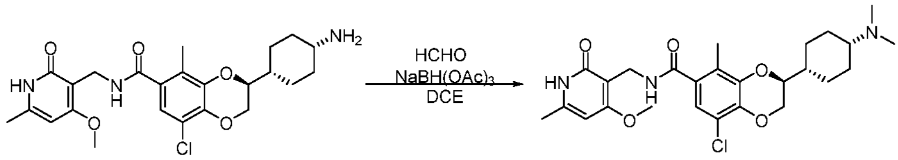


94-2 94

### Step 1, Compound 94-1 Synthesis:

Following the synthesis method of Example **3**, Step **1**, except replacing Step **1, 2-3** with **53-1** (250.0 mg, 0.6 mmol), **2-4** with **5-1** (252.0 mg, 1.5 mmol), the same synthesis method was applied, to give Compound **94-1** (300.0 mg, 0.5 mmol), LCMS (ESI$^+$) m/z: 576.2 [M+H]$^+$.

### Step 2, Compound 94-2 Synthesis:

Following the synthesis method of Example **3**, Step **2**, except replacing Step **2, 3-2** with **94-1** (300.0 mg, 0.5 mmol), the same synthesis method was applied, to give Compound **94-2** (250.0 mg, crude), LCMS (ESI$^+$) m/z: 476.2 [M+H]$^+$.

### Step 3, Compound 94 Synthesis:

Following the synthesis method of Example **6**, Step **4**, except replacing Step **4, 6-3** with **94-2** (50.0 mg, 0.1 mmol), the same synthesis method was applied. The obtained compound was subjected to Prep-MPLC to give Compound **94** (10.0 mg, 0.02 mmol, 19.8% yield), LCMS (ESI$^+$) m/z: 504.3 [M+H]$^+$.

**$^1$H NMR** (600 MHz, Methanol-$d_4$) δ 6.91 (s, 1H), 6.25 (s, 1H), 4.42-4.40 (m, 1H), 4.40 (s, 2H), 4.06-4.03 (m, 1H), 3.92 (s, 3H), 3.89-3.86 (m, 1H), 2.31 (s, 3H), 2.30 (s, 6H), 2.18 (s, 3H), 2.17-2.15 (m, 1H), 2.09 (m, 2H), 1.89-1.88 (m, 1H), 1.63-1.61 (m, 1H), 1.32-1.25 (m, 5H).

### Example 57, Compound 95 and 96 Synthesis:

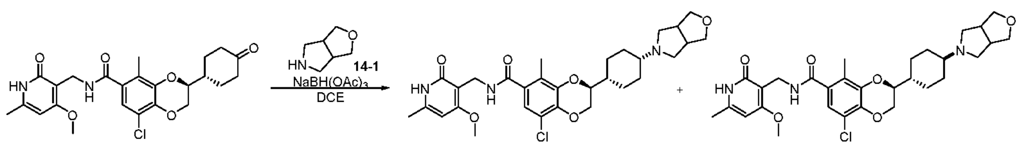


94-2 95 96

### Step 1, Compound 95 and 96 Synthesis:

Following the synthesis method of Example **6**, Step **4**, except replacing Step **4, 6-3** with **94-2** (1.3 g, 2.7 mmol), **dimethylamine** with **14-1** (929.0 mg, 8.0 mmol), the same synthesis method was applied. The obtained compound was subjected to Prep-MPLC to give Compound 95 (64.0 mg, 0.1 mmol, 4.1% yield) and **96** (190.0 mg, 0.3 mmol, 12.3% yield), LCMS (ESI$^+$) m/z: 572.3 [M+H]$^+$.

**Compound 95 NMR: $^1$H NMR (400 MHz, Methanol-$d_4$)** δ 6.92 (s, 1H), 6.25 (s, 1H), 4.42-4.40 (m, 1H), 4.39 (s, 2H), 4.08-4.03 (m, 1H), 3.92 (s, 3H), 3.90-3.86 (m, 1H), 3.70-3.62 (m, 4H), 3.20-3.18 (m, 2H), 2.88-2.84 (m, 2H), 2.31 (s, 3H), 2.29-2.25 (m, 1H), 2.18 (s, 3H), 2.12-2.10 (m, 4H), 1.87-1.83 (m, 1H), 1.66-1.60 (m, 1H), 1.33-1.26 (m, 5H).

**Compound 96 NMR: $^1$H NMR (400 MHz, Methanol-$d_4$)** δ 6.91 (s, 1H), 6.25 (s, 1H), 4.46-4.42 (m, 1H), 4.40 (s, 2H), 4.07-4.02 (m, 2H), 3.92 (s, 3H), 3.83-3.79 (m, 2H), 3.56-3.52 (m, 2H), 2.81-2.77 (m, 2H), 2.72-2.68 (m, 2H), 2.43-2.39 (m, 2H), 2.31 (s, 3H), 2.18 (s, 3H), 2.17-2.13 (m, 1H), 1.87-1.68 (m, 5H), 1.65-1.50 (m, 4H)

**Example 58, Compound 97 and 98 Synthesis:**

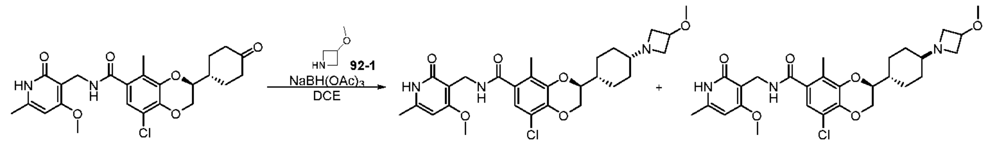


94-2 97 98

**Step 1, Compound 97 and 98 Synthesis:**

Following the synthesis method of Example **6**, Step **4**, except replacing Step **4, 6-3** with **94-2** (1.0 g, 2.1 mmol), **dimethylamine** with **92-1** (220.0 mg, 2.5 mmol), the same synthesis method was applied. The obtained compound was subjected to Prep-MPLC to give Compound **97** (90.0 mg, 0.2 mmol, 7.8% yield) and **98** (180.0 mg, 0.3 mmol, 15.7% yield), LCMS ($ESI^+$) m/z: 546.6 $[M+H]^+$.

**Compound 97 NMR: $^1$H NMR** (400 MHz, DMSO-$d_6$) δ 11.42 (s, 1H), 7.93 (t, *J* = 4.4 Hz, 1H), 6.87 (s, 1H), 6.09 (s, 1H), 4.46 (dd, *J* = 11.6, 2.0 Hz, 1H), 4.17 (d, *J* = 4.4 Hz, 2H), 4.06-4.02 (m, 1H), 3.92-3.87 (m, 2H), 3.79 (s, 3H), 3.45 (t, *J* = 7.6 Hz, 2H), 3.13 (s, 3H), 2.73 (t, *J* = 6.0 Hz, 2H), 2.18 (s, 3H), 2.12 (s, 3H), 1.99-1.83 (m, 2H), 1.78-1.74 (m, 3H), 1.55-1.53 (m, 1H), 1.18-1.15 (m, 2H), 0.89-0.86 (m, 2H).

**Compound 98 NMR: $^1$H NMR** (600 MHz, DMSO-$d_6$) δ 11.42 (s, 1H), 7.93 (t, *J* = 4.2 Hz, 1H), 6.87 (s, 1H), 6.09 (s, 1H), 4.46 (dd, *J* = 12.0, 2.4 Hz, 1H), 4.17 (d, *J* = 4.8 Hz, 2H), 4.06-4.02 (m, 1H), 3.92-3.87 (m, 2H), 3.79 (s, 3H), 3.45 (t, *J* = 6.6 Hz, 2H), 3.31-3.29 (m, 1H), 3.13 (s, 3H), 2.72 (t, *J* = 6.0 Hz, 2H), 2.18 (s, 3H), 2.12 (s, 3H), 1.95-1.93 (m, 1H), 1.88-1.87 (m, 1H), 1.77-1.75 (m, 2H), 1.56-1.51 (m, 1H), 1.20-1.12 (m, 2H), 0.91-0.83 (m, 2H).

**Example 59, Compound 99 and 100 Synthesis:**

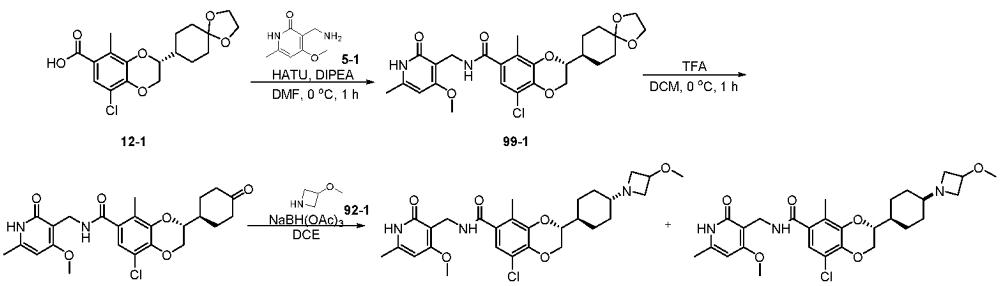


12-1 99-1

99-2 99 100

**Step 1, Compound 99-1 Synthesis:**

Following the synthesis method of Example **3**, Step **1**, except replacing Step **1, 2-3** with **12-1** (250.0 mg, 0.7 mmol), **3-1** with **5-1** (252.0 mg, 1.5 mmol)the same synthesis method was applied, to give Compound **99-1** (300.0 mg, 0.6 mmol), LCMS ($ESI^+$) m/z: 519.1 $[M+H]^+$.

**Step 2, Compound 99-2 Synthesis:**

Following the synthesis method of Example **3**, Step **2**, except replacing Step **2, 3-2** with **99-1** (300.0 mg, 0.6 mmol), the same synthesis method was applied, to give Compound **99-2** (250.0 mg, crude), LCMS ($ESI^+$) m/z: 475.2 $[M+H]^+$.

**Step 3, Compound 99 and 100 Synthesis:**

Following the synthesis method of Example **6**, Step **4**, except replacing Step **4, 6-3** with **99-2** (200.0 mg, 0.4 mmol), **dimethylamine** with **92-1** (174.0 mg, 2.0 mmol), the same synthesis method was applied. The obtained compound was subjected to Prep-MPLC to give Compound **99** (20.0 mg, 0.04 mmol, 9.2% yield) and **100** (22.0 mg, 0.04 mmol, 10.1%

yield), LCMS (ESI+) m/z: 546.6 [M+H]+.

**Compound 99 NMR**: **$^1$H NMR** (600 MHz, DMSO-$d_6$) δ 11.42 (s, 1H), 7.92 (t, *J* = 4.8 Hz, 1H), 6.86 (s, 1H), 6.09 (s, 1H), 4.43 (dd, *J* = 11.4, 2.4 Hz, 1H), 4.17 (d, *J* = 4.8 Hz, 2H), 4.06-4.04 (m, 1H), 3.93-3.91 (m, 2H), 3.79 (s, 3H), 3.47-3.43 (m, 2H), 3.14 (s, 3H), 2.68 (t, *J* = 6.0 Hz, 2H), 2.21-2.20 (m, 1H), 2.18 (s, 3H), 2.11 (s, 3H), 1.60-1.56 (m, 4H), 1.50-1.48 (m, 2H), 1.44-1.42 (m, 1H), 1.38-1.30 (m, 2H).

**Compound 100 NMR**: **$^1$H NMR** (600 MHz, DMSO-$d_6$) δ 11.42 (s, 1H), 7.92 (t, *J* = 4.8 Hz, 1H), 6.86 (s, 1H), 6.09 (s, 1H), 4.43 (dd, *J* = 11.4, 2.2 Hz, 1H), 4.17 (d, *J* = 4.8 Hz, 2H), 4.08-4.04 (m, 1H), 3.93-3.89 (m, 2H), 3.79 (s, 3H), 3.49-3.42 (m, 2H), 3.14 (s, 3H), 2.68 (t, *J* = 6.0 Hz, 2H), 2.21-2.19 (m, 1H), 2.18 (s, 3H), 2.11 (s, 3H), 1.60-1.56 (m, 4H), 1.50-1.48 (m, 2H), 1.44-1.42 (m, 1H), 1.38-1.30 (m, 2H).

**Example 60, Compound 101 and 102 Synthesis:**

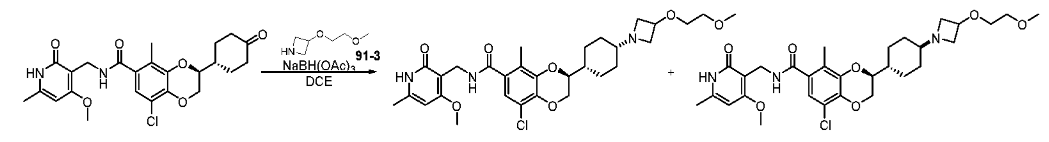


99-2 101 102

**Step 1, Compound 101 and 102 Synthesis:**

Following the synthesis method of Example **6**, Step **4**, except replacing Step **4, 6-3** with **99-2** (20.0 mg, 0.04 mmol), **dimethylamine** with **91-3** (17.0 mg, 0.1 mmol), the same synthesis method was applied. The obtained compound was subjected to Prep-MPLC to give Compound **101** (3.0 mg, 5.0 μmol, 12.1% yield) and **102** (3.0 mg, 5.0 μmol, 12.1% yield), LCMS (ESI+) m/z: 590.2 [M+H]+.

**Compound 101 NMR**: **$^1$H NMR** (400 MHz, DMSO-$d_6$) δ 11.42 (s, 1H), 7.93 (t, *J* = 4.8 Hz, 1H), 6.87 (s, 1H), 6.09 (s, 1H), 4.46 (dd, *J* = 11.4, 2.2 Hz, 1H), 4.18 (d, *J* = 4.6 Hz, 2H), 4.07-4.02 (m, 2H), 4.00-3.91 (m, 1H), 3.79 (s, 3H), 3.46-3.45 (m, 5H), 3.24-3.23 (m, 4H), 2.71-2.73 (m, 2H), 2.18 (s, 3H), 2.12 (s, 3H), 1.92-1.77 (m, 2H), 1.19-1.13 (m, 3H), 1.54-1.50 (m, 1H), 1.21-1.12 (m, 2H), 0.89-0.84 (m, 2H).

**Compound 102 NMR**: **$^1$H NMR** (400 MHz, DMSO-$d_6$) δ 11.42 (s, 1H), 7.93 (t, *J* = 4.4 Hz, 1H), 6.87 (s, 1H), 6.09 (s, 1H), 4.46 (dd, *J* = 11.4, 2.2 Hz, 1H), 4.18 (d, *J* = 4.6 Hz, 2H), 4.07-4.02 (m, 2H), 4.00-3.91 (m, 1H), 3.79 (s, 3H), 3.46-3.45 (m, 5H), 3.24-3.23 (m, 4H), 2.71-2.73 (m, 2H), 2.18 (s, 3H), 2.12 (s, 3H), 1.92-1.77 (m, 2H), 1.19-1.13 (m, 3H), 1.54-1.50 (m, 1H), 1.21-1.12 (m, 2H), 0.89-0.84 (m, 2H).

**Example 61, Compound 103 and 104 Synthesis:**

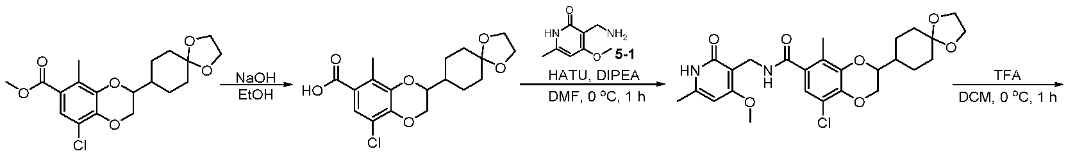


IM-3-6 103-1 103-2

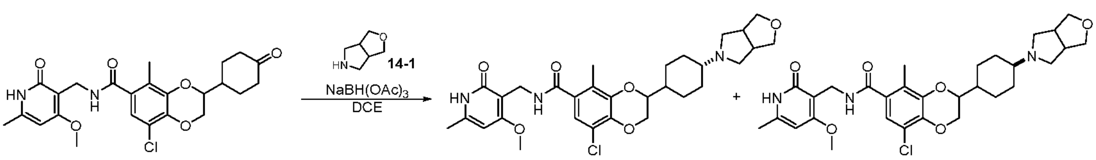


103-3 103 104

**Step 1, Compound 103-1 Synthesis:**

Following the synthesis method of Example **6**, Step **1**, except replacing Step **1**, **IM-3a** with **IM-3-6** (150.0 mg, 0.4 mmol), the same synthesis method was applied, to give Compound **103-1** (100 mg, crude), LCMS (ESI+) m/z: 369.1 [M+H]+.

**Step 2, Compound 103-2 Synthesis:**

Following the synthesis method of Example **3**, Step **1**, except replacing Step **1, 2-3** with **103-1** (100.0 mg, crude), **3-1** with **5-1** (68.5 mg, 0.4 mmol), the same synthesis method was applied, to give Compound **103-2** (85.0 mg, 0.2 mmol, 60.5% yield), LCMS (ESI$^+$) m/z: 519.2 [M+H]$^+$.

**Step 3, Compound 103-3 Synthesis:**

Following the synthesis method of Example **3**, Step **2**, except replacing Step **2, 3-2** with **103-2** (85.0 mg, 0.2 mmol), the same synthesis method was applied, to give Compound **103-3** (50.0 mg, crude), LCMS (ESI$^+$) m/z: 475.2 [M+H]$^+$.

**Step 4, Compound 103 and 104 Synthesis:**

Following the synthesis method of Example **6**, Step **4**, except replacing Step **4, dimethylamine** with **14-1** (17.9 mg, 0.2 mmol), the same synthesis method was applied. The obtained compound was subjected to Prep-MPLC to give Compound **103** (10.0 mg, 17.5 μmol, 17.5% yield) and **104** (10.0 mg, 17.5 μmol, 17.5% yield), LCMS (ESI$^+$) m/z: 572.2 [M+H]$^+$.

**Compound 103 NMR**: **$^1$H NMR** (400 MHz, DMSO-$d_6$) δ 11.42 (s, 1H), 7.93 (t, *J* = 4.2 Hz, 1H), 6.87 (s, 1H), 6.09 (s, 1H), 4.44 (dd, *J* = 11.4, 1.8 Hz, 1H), 4.17 (d, *J* = 4.2 Hz, 2H), 4.08-4.05 (m, 1H), 3.97-3.95 (m, 1H), 3.86-3.85 (m, 2H), 3.79 (s, 3H), 3.30-3.28 (m, 2H), 2.67-2.65 (m, 2H), 2.48-2.46 (m, 2H), 2.32-2.31 (m, 2H), 2.18 (s, 3H), 2.15-2.13 (m, 1H), 2.11 (s, 3H), 1.80-1.78 (m, 2H), 1.67-1.58 (m, 4H), 1.42-1.40 (m, 3H).

**Compound 104 NMR**: **$^1$H NMR** (400 MHz, DMSO-$d_6$) δ 11.42 (s, 1H), 7.93 (t, *J* = 4.2 Hz, 1H), 6.87 (s, 1H), 6.09 (s, 1H), 4.46-4.44 (m, 1H), 4.17 (d, *J* = 4.8 Hz, 2H), 4.05-4.02 (m, 1H), 3.96-3.90 (m, 1H), 3.79 (s, 3H), 3.70-3.69 (m, 2H), 3.30-3.28 (m, 2H), 2.68-2.62 (m, 4H), 2.36-2.34 (m, 2H), 2.18 (s, 3H), 2.12 (s, 3H), 1.96-1.91 (m, 4H), 1.75-1.73 (m, 1H), 1.57-1.55 (m, 1H), 1.22-1.11 (m, 4H).

**Example 62, Compound 105 and 106 Synthesis:**

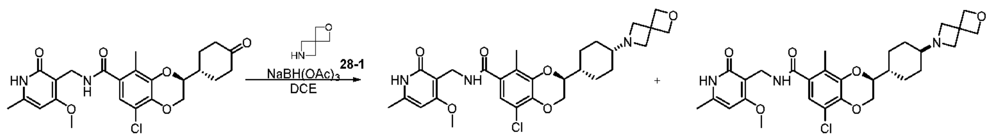


99-2 105 106

**Step 1, Compound 105 and 106 Synthesis:**

Following the synthesis method of Example **6**, Step **4**, except replacing Step **4, 6-3** with **99-2** (30.0 mg, 0.06 mmol), **dimethylamine** with **28-1** (12.5 mg, 0.1 mmol), the same synthesis method was applied. The obtained compound was subjected to Prep-MPLC to give Compound **105** (3.0 mg, 5.0 μmol, 8.5% yield) and **106** (6.0 mg, 0.01 mmol, 17.0% yield), LCMS (ESI$^+$) m/z: 558.1 [M+H]$^+$.

**Compound 105 NMR**: **$^1$H NMR** (600 MHz, DMSO-$d_6$) δ 11.42 (s, 1H), 7.93 (t, *J* = 4.2 Hz, 1H), 6.87 (s, 1H), 6.09 (s, 1H), 4.60 (s, 4H), 4.43 (d, *J* = 11.4 Hz, 1H), 4.17 (d, *J* = 4.8 Hz, 2H), 4.07-4.04 (m, 1H), 3.97-3.95 (m, 1H), 3.79 (s, 3H), 3.25-3.15 (m, 4H), 2.57-2.54 (m, 1H), 2.18 (s, 3H), 2.11 (s, 3H), 1.60-1.49 (m, 5H), 1.46-1.33 (m, 4H).

**Compound 106 NMR**: **$^1$H NMR** (400 MHz, DMSO-$d_6$) δ 11.42 (s, 1H), 7.93 (t, *J* = 4.8 Hz, 1H), 6.87 (s, 1H), 6.09 (s, 1H), 4.57 (s, 4H), 4.45 (d, *J* = 11.6 Hz, 1H), 4.17 (d, *J* = 4.4 Hz, 2H), 4.06-4.01 (m, 1H), 3.92-3.89 (m, 1H), 3.79 (s, 3H), 3.24-3.19 (m, 4H), 2.44-2.41 (m, 1H), 2.18 (s, 3H), 2.11 (s, 3H), 1.95-1.91 (m, 2H), 1.83-1.80 (m, 1H), 1.73-1.71 (m, 4H), 1.50-1.52 (m, 2H).

**Example 63, Compound 107 and 108 Synthesis:**

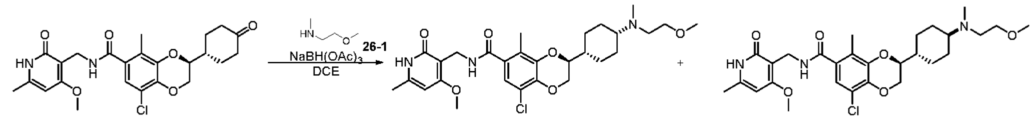


94-2 107 108

**Step 1, Compound 107 and 108 Synthesis:**

Following the synthesis method of Example **6**, Step **4**, except replacing Step **4, 6-3** with **94-2** (50.0 mg, 0.1 mmol), **dimethylamine** with **26-1** (19.0 mg, 0.2 mmol), the same synthesis method was applied. The obtained compound was subjected to Prep-MPLC to give Compound **107** (5.0 mg, 0.01 mmol, 8.7% yield) and **108** (1.0 mg, 2.0 μmol, 1.7% yield), LCMS ($ESI^+$) m/z: 548.3 $[M+H]^+$.

**Compound 107 NMR**: **$^1$H NMR** (400 MHz, $CDCl_3$) δ 11.08 (s, 1H), 7.30-7.26 (m, 1H), 7.00 (s, 1H), 5.95 (s, 1H), 4.53 (d, *J* = 5.2 Hz, 2H), 4.35 (dd, *J* = 11.2, 2.0 Hz, 1H), 4.05-4.02 (m, 1H), 3.89 (s, 3H), 3.87-3.83 (m, 2H), 3.76-3.49 (m, 1H), 3.49 (s, 2H), 3.37 (s, 3H), 3.13-3.11 (m, 3H), 2.69-2.66 (m, 3H), 2.31 (s, 3H), 2.23 (s, 3H), 2.19-2.16 (m, 1H), 1.94-1.93 (m, 1H), 1.88-1.81 (m, 1H), 1.68-1.64 (m, 2H), 1.52-1.42 (m, 2H).

**Compound 108 NMR**: **$^1$H NMR** (400 MHz, CDCl3) δ12.16 (s, 1H), 7.37-7.35 (m, 1H), 7.00 (s, 1H), 5.96 (s, 1H), 4.52-4.50 (m, 2H), 4.43 (d, *J* = 10.8 Hz, 1H), 4.26-4.24 (m, 1H), 4.08-4.06 (m, 1H), 4.05-4.03 (m, 1H), 3.98 (s, 3H), 3.94-3.86 (m, 1H), 3.37 (s, 3H), 3.33-3.32 (m, 2H), 3.20-3.18 (m, 1H), 2.79-2.77 (m, 3H), 2.32 (s, 3H), 2.23 (s, 3H), 2.09-2.04 (m, 1H), 1.94-1.93 (m, 2H), 1.85-1.80 (m, 3H), 1.73-1.68 (m, 2H), 1.56-1.54 (m, 1H).

**Example 64, Compound 109 and 110 Synthesis:**

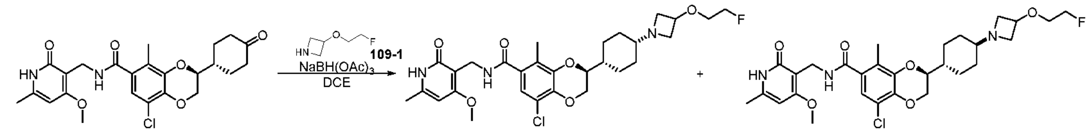


99-2 109 110

**Step 1, Compound 109 and 110 Synthesis:**

Following the synthesis method of Example **6**, Step **4**, except replacing Step **4, 6-3** with **99-2** (50.0 mg, 0.1 mmol), **dimethylamine** with **109-1** (25.0 mg, 0.2 mmol), the same synthesis method was applied. The obtained compound was subjected to Prep-MPLC to give Compound **109** (7.5 mg, 0.01 mmol, 12.3% yield) and **110** (4.6 mg, 8.0 μmol, 7.6% yield), LCMS ($ESI^+$) m/z: 578.3 $[M+H]^+$.

**Compound 109 NMR**: **$^1$H NMR** (400 MHz, $CDCl_3$) δ 11.07 (m, 1H), 7.33-7.32 (m, 1H), 7.00 (s, 1H), 5.95 (s, 1H), 4.59-4.58 (m, 1H), 4.57-4.54 (m, 2H), 4.53-4.47 (m, 1H), 4.46-4.45 (m, 1H), 4.37-4.34 (m, 1H), 4.03-4.01 (m, 1H), 4.00-3.98 (m, 1H), 3.97-3.95 (m, 3H), 3.92-3.89 (m, 1H), 3.87-3.85 (m, 1H), 3.76-3.70 (m, 1H), 3.68-3.66 (m, 1H), 2.30 (s, 3H), 2.24 (s, 3H), 2.13-2.12 (m, 1H), 2.05-2.04 (m, 2H), 1.94-1.89 (m, 1H), 1.83-1.69 (m, 3H), 1.28-1.25 (m, 4H), 0.90-0.84 (m, 1H).

**Compound 110 NMR**: **$^1$H NMR** (400 MHz, $CDCl_3$) δ 12.04 (m, 1H), 7.33-7.32 (m, 1H), 7.00 (s, 1H), 6.09 (s, 1H), 4.61-4.59 (m, 2H), 4.58-4.57 (m, 2H), 4.53-4.47 (m, 2H), 4.46-4.45 (m, 2H), 4.45-4.35 (m, 2H), 4.25-4.21 (m, 1H), 4.17-4.15 (m, 1H), 3.96-3.94 (m, 3H), 3.77-3.76 (m, 2H), 3.70-3.66 (m, 4H), 3.26-3.24 (m, 1H), 2.45-2.39 (m, 3H), 2.25 (s, 3H), 2.22-2.04 (m, 4H), 0.98-0.95 (m, 1H).

**Example 65, Compound 111 and 112 Synthesis:**

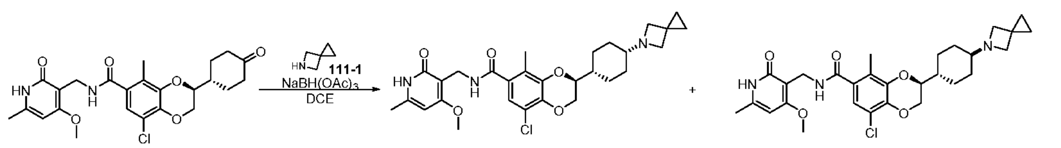


99-2 111 112

**Step 1, Compound 111 and 112 Synthesis:**

Following the synthesis method of Example **6**, Step **4**, except replacing Step **4, 6-3** with **99-2** (40.0 mg, 0.08 mmol), **dimethylamine** with **111-1** (14.0 mg, 0.2 mmol), the same synthesis method was applied. The obtained compound was subjected to Prep-MPLC to give Compound **111** (2.0 mg, 3.7 μmol, 4.4% yield) and **112** (4.0 mg, 7.4 μmol, 8.8% yield), LCMS (ESI$^+$) m/z: 542.4 $[M+H]^+$.

**Compound 111 NMR**: $^1$**H NMR** (400 MHz, DMSO-$d_6$) δ 11.42 (s, 1H), 7.93 (t, *J* = 4.4 Hz, 1H), 6.87 (s, 1H), 6.09 (s, 1H), 4.47-4.44 (m, 1H), 4.18 (d, *J* = 4.8 Hz, 2H), 4.08-4.03 (m, 1H), 3.94-3.91 (m, 1H), 3.79 (s, 3H), 3.24-3.22 (m, 4H), 2.45-2.40 (m, 1H), 2.18 (s, 3H), 2.12 (s, 3H), 1.98-1.95 (m, 1H), 1.78-1.73 (m, 3H), 1.57-1.55 (m, 1H), 1.23-1.13 (m, 2H), 1.01-0.84 (m, 2H), 0.49 (s, 4H).

**Compound 112 NMR**: $^1$H NMR (400 MHz, DMSO-$d_6$) δ 11.42 (s, 1H), 7.93 (t, *J* = 4.4 Hz, 1H), 6.87 (s, 1H), 6.09 (s, 1H), 4.45-4.42 (m, 1H), 4.18 (d, *J* = 4.4 Hz, 2H), 4.09-4.04 (m, 1H), 3.99-3.95 (m, 1H), 3.79 (s, 3H), 3.17 (s, 4H), 2.34-2.32 (m, 1H), 2.18 (s, 3H), 2.12 (s, 3H), 1.66-1.57 (m, 5H), 1.50-1.47 (m, 1H), 1.40-1.33 (m, 3H), 0.48 (s, 4H).

**Example 66, Compound 113 and 114 Synthesis:**

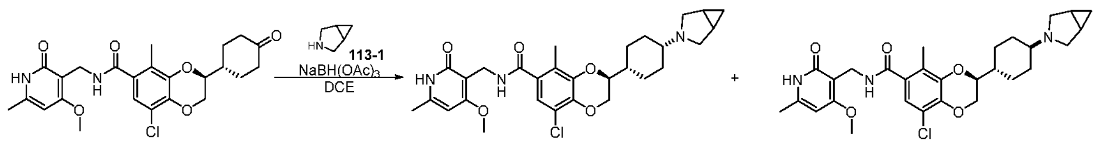


99-2 113 114

**Step 1, Compound 113 and 114 Synthesis:**

Following the synthesis method of Example **6**, Step **4**, except replacing Step **4, 6-3** with **99-2** (40.0 mg, 0.08 mmol), **dimethylamine** with **113-1** (14.0 mg, 0.2 mmol), the same synthesis method was applied. The obtained compound was subjected to Prep-MPLC to give Compound **113** (2.0 mg, 3.7 μmol, 4.3% yield) and **114** (5.0 mg, 9.2 μmol, 10.7% yield), LCMS (ESI$^+$) m/z: 542.3 $[M+H]^+$.

**Compound 113 NMR**: $^1$**H NMR** (400 MHz, Methanol-$d_4$) δ 6.91 (s, 1H), 6.25 (s, 1H), 4.42-4.40 (m, 2H), 4.39-4.38 (m, 1H), 4.07-4.02 (m, 1H), 3.92 (s, 3H), 3.89-3.85 (m, 1H), 3.18-3.16 (m, 1H), 2.68-2.63 (m, 2H), 2.31 (s, 3H), 2.18 (s, 3H), 2.11-2.07 (m, 4H), 1.84-1.81 (m, 1H), 1.65-1.61 (m, 1H), 1.53-1.51 (m, 2H), 1.32-1.23 (m, 5H), 0.63-0.60 (m, 1H), 0.52-0.49 (m, 1H).

**Compound 114 NMR**: $^1$**H NMR** (400 MHz, DMSO-$d_6$) δ 11.42 (s, 1H), 7.93 (t, *J* = 4.8 Hz, 1H), 6.87 (s, 1H), 6.09 (s, 1H), 4.43 (d, *J* = 11.6 Hz, 1H), 4.18 (d, *J* = 4.8 Hz, 2H), 4.08-4.03 (m, 1H), 4.00-3.94 (m, 1H), 3.79 (s, 3H), 2.96 (d, *J* = 8.4 Hz, 2H), 2.23-2.20 (m, 1H), 2.18 (s, 3H), 2.16-2.14 (m, 1H), 2.12 (s, 3H), 1.75-1.74 (m, 2H), 1.73-1.71 (m, 1H), 1.66-1.64 (m, 3H), 1.37-1.34 (m, 5H), 1.24-1.23 (m, 1H), 0.58-0.57 (m, 1H), 0.30-0.28 (m, 1H).

**Example 67, Compound 115 and 116 Synthesis:**

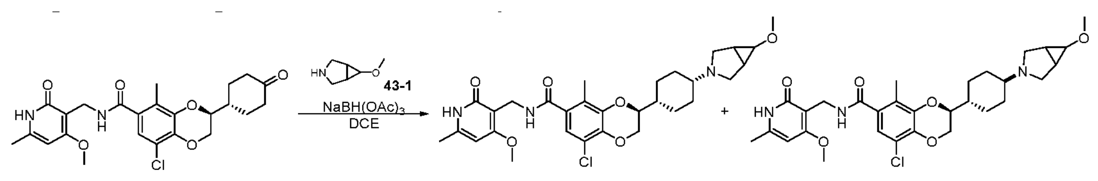


99-2 115 116

**Step 1, Compound 115 and 116 Synthesis:**

Following the synthesis method of Example **6**, Step **4**, except replacing Step **4, 6-3** with **99-2** (50.0 mg, 0.1 mmol), **dimethylamine** with **43-1** (11.6 mg, 0.1 mmol), the same synthesis method was applied. The obtained compound was subjected to Prep-MPLC to give Compound **115** (12.0 mg, 0.02 mmol, 19.9% yield) and **116** (11.0 mg, 19.2 μmol, 18.3% yield), LCMS (ESI$^+$) m/z: 572.3 $[M+H]^+$.

**Compound 115 NMR**: **$^1$H NMR** (600 MHz, $CDCl_3$) δ 6.98 (s, 1H), 5.98 (s, 1H), 4.53-4.51 (m, 2H), 4.35-4.33 (m, 1H), 4.17 (s, 1H), 3.95-3.93 (m, 1H), 3.91 (s, 3H), 3.86-3.84 (m, 3H), 3.35 (s, 3H), 3.13-3.11 (m, 2H), 2.84-2.82 (m, 1H) 2.47 (s, 3H), 2.35 (s, 3H), 2.22-2.03 (m, 5H), 1.88-1.86 (m, 3H), 1.43-1.40 (m, 3H).

**Compound 116 NMR**: **$^1$H NMR** (600 MHz, $CDCl_3$) δ 7.00 (s, 1H), 6.51 (s, 1H), 4.56-4.41 (m, 2H), 4.39-4.37 (m, 1H), 4.35-4.33 (m, 1H), 4.17-4.14 (m, 1H), 4.07 (s, 3H), 3.84-3.82 (m, 1H), 3.37 (s, 3H), 3.16-3.14 (m, 2H), 2.67 (s, 3H), 2.54-2.52 (m, 3H), 2.23 (s, 3H), 2.19-2.18 (m, 2H), 1.88 (s, 3H), 1.87-1.61 (m, 6H).

**Example 68, Compound 117 and 118 Synthesis:**

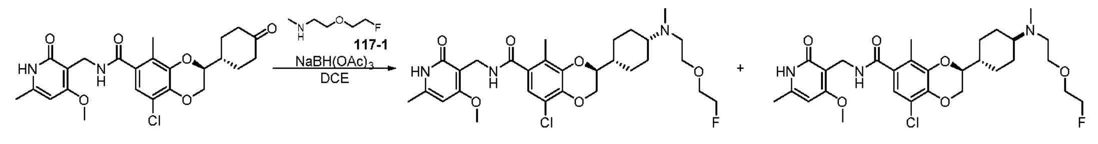


99-2 117 118

**Step 1, Compound 117 and 118 Synthesis:**

Following the synthesis method of Example **6**, Step **4**, except replacing Step **4, 6-3** with **99-2** (30.0 mg, 0.06 mmol), **dimethylamine** with **117-1** (15.0 mg, 0.1 mmol), the same synthesis method was applied. The obtained compound was subjected to Prep-MPLC to give Compound **117** (3.0 mg, 5.0 μmol, 8.2 % yield) and **118** (3.0 mg, 5.0 μmol, 8.2% yield), LCMS (ESI$^+$) m/z: 580.4 $[M+H]^+$.

**Example 69, Compound 119 and 120 Synthesis:**

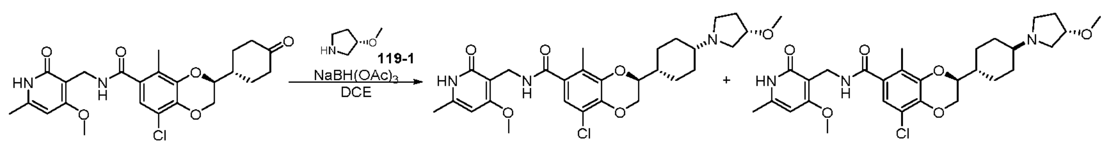


99-2 119 120

**Step 1, Compound 119 and 120 Synthesis:**

Following the synthesis method of Example **6**, Step **4**, except replacing Step **4, 6-3** with **99-2** (30.0 mg, 0.06 mmol), **dimethylamine** with **119-1** (7.7 mg, 7.6 μmol), the same synthesis method was applied. The obtained compound was subjected to Prep-MPLC to give Compound **119** (1.5 mg, 2.7 μmol, 4.2% yield) and **120** (3.5 mg, 6.3 μmol, 9.9% yield), LCMS (ESI$^+$) m/z: 280.8 $[(M+H)/2]^+$.

**Compound 119 NMR**: **$^1$H NMR** (400 MHz, DMSO-$d_6$) δ11.42 (s, 1H), 7.93 (t, *J* = 4.8 Hz, 1H), 6.87 (s, 1H), 6.09 (s, 1H), 4.47-4.44 (m, 1H), 4.18 (d, *J* = 4.4 Hz, 2H), 4.07-4.02 (m, 1H), 3.92-3.82 (m, 1H), 3.82-3.80 (m, 1H), 3.79 (s, 3H), 3.15 (s, 3H), 2.74-2.72 (m, 1H), 2.18 (s, 3H), 2.12 (s, 3H), 1.98-1.97 (m, 6H), 1.76-1.73 (m, 1H), 1.60-1.57 (m, 3H), 1.22-1.07 (m, 5H).

**Compound 120 NMR: $^1$H NMR** (600 MHz, Methanol-$d_4$) δ 11.42 (s, 1H), 7.93 (t, *J* = 4.8 Hz, 1H), 6.87 (s, 1H), 6.09 (s, 1H), 4.49 (s, 2H), 4.46 (dd, *J* = 11.2, 2.0 Hz, 1H), 4.18 (d, *J* = 4.8 Hz, 1H), 4.10-4.05 (m, 1H), 4.00-3.98 (m, 1H), 3.88-3.86 (m, 1H), 3.79 (s, 3H), 3.17 (s, 3H), 2.74-2.72 (m, 1H), 2.44-2.43 (m, 3H), 2.18 (s, 3H), 2.12 (s, 3H), 1.98-1.91 (m, 2H), 1.92-1.90 (m, 2H), 1.62-1.58 (m, 3H), 1.54-1.52 (m, 1H), 1.44-1.42 (m, 3H).

**Example 70, Compound 121, 122 and 123 Synthesis:**

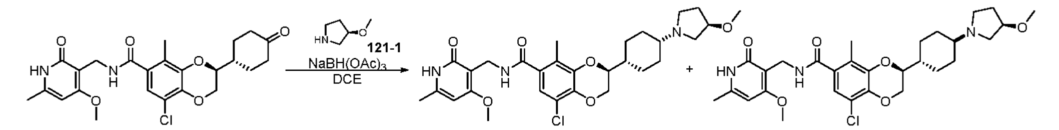


99-2 121 122

**Step 1, Compound 121 and 122 Synthesis:**

Following the synthesis method of Example **6**, Step **4**, except replacing Step **4, 6-3** with **99-2** (30.0 mg, 0.06 mmol), **dimethylamine** with **121-1** (6.3 mg, 6.3 μmol), the same synthesis method was applied. The obtained compound was subjected to Prep-MPLC to give Compound **121** (2.5 mg, 4.5 μmol, 7.1% yield) and **122** (2.4 mg, 4.3 μmol, 6.8% yield), LCMS (ESI$^+$) m/z: 281.0 [(M+H)/2+H]$^+$.

**Compound 121 NMR**: **$^1$H NMR** (400 MHz, Methanol-$d_4$) δ 6.91 (s, 1H), 6.25 (s, 1H), 4.86-4.39 (m, 3H), 4.08-4.03 (m, 1H), 3.96-3.94 (m, 1H), 3.92 (s, 3H), 3.90-3.86 (m, 1H), 3.31 (s, 3H), 2.90-2.86 (m, 4H), 2.80-2.66 (m, 1H), 2.31 (s, 3H), 2.18 (s, 3H), 2.12-2.04 (m, 4H), 1.89-1.84 (m, 2H), 1.66-1.64 (m, 1H), 1.33-1.26 (m, 4H).

**Compound 122 NMR: $^1$H NMR** (400 MHz, Methanol-$d_4$) δ 6.93 (s, 1H), 6.25 (s, 1H), 4.49-4.46 (m, 1H), 4.44 (s, 2H), 4.21-4.15 (m, 1H), 4.06-4.02 (m, 2H), 3.92 (s, 3H), 3.31 (s, 3H), 3.06-3.04 (m, 3H), 2.92-2.88 (m, 1H), 2.65-2.61 (s, 1H), 2.31 (s, 3H), 2.19 (s, 3H), 2.15-2.12 (m, 1H), 2.03-1.99 (m, 2H), 1.88-1.59 (m, 8H).

**Compound 123 Synthesis:**

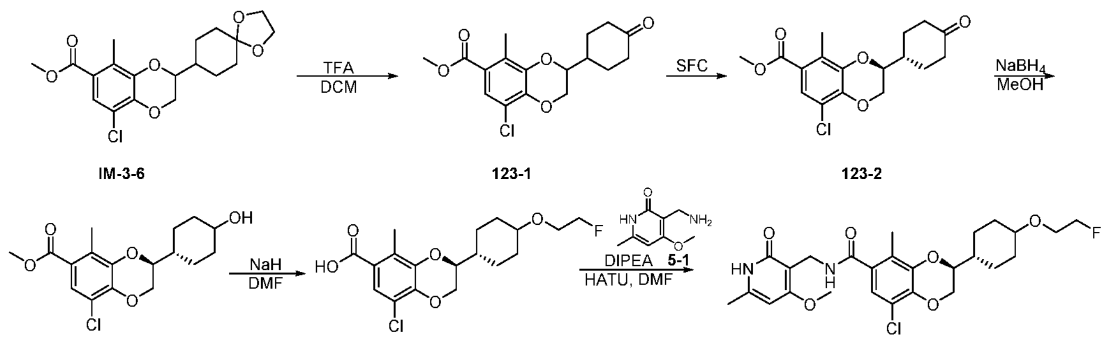


IM-3-6 123-1 123-2

123-3 123-4 123

**Step 1, Compound 123-1 Synthesis:**

Following the synthesis method of Example **3**, Step **2**, except replacing Step **2, 3-2** with **IM-3-6** (100.0 mg, 0.3 mmol), the same synthesis method was applied, to give Compound **123-1** (120.0 mg, crude), LCMS (ESI$^+$) m/z: 338.8 [M+H]$^+$.

**Step 2, Compound 123-2 Synthesis:**

Compound 123-2 can be obtained by SFC resolution from Compound 123-1.

**Step 3, Compound 123-3 Synthesis:**

Following the synthesis method of Example **IM-3**, **Step 3**, except replacing Step **3**, **IM-3-4** with **123-2** (40.0 mg, 0.1 mmol), the same synthesis method was applied, to give Compound **123-3** (36.2 mg, 0.1 mmol, 90.0% yield), LCMS (ESI$^+$) m/z: 340.8 [M+H]$^+$.

**Step 4, Compound 123-4 Synthesis:**

A single-necked flask was added with substrate **123-3** (8.0 mg, 0.02 mmol). The mixture was dissolved with DMF (1 mL) under stirring, and then at 0°C and under nitrogen protection, added with NaH (9.4 mg, 0.2 mmol), and stirred to react for 0.5 hours, and then added with **1-fluoro-2-iodo-ethane** (20.0 mg, 0.1 mmol), reacted at room temerature for 12

hours, and then added with water (5 mL) further stirred for 12 hours, with LC-MS monitoring. After the completion of the reaction, the reaction mixture was adjusted with 4 N HCl solution to pH = 6, added with petroleum ether and ethyl acetate(8: 1) to extract three times. The organic phase was combined, washed with saturated saline solution, and dried with anhydrous sodium sulfate and then condensated under reduced pressure to give the crude product **123-4** (8.0 mg, crude). LCMS ($ESI^+$) m/z: 372.8 $[M+H]^+$

**Step 4, Compound 123 Synthesis:**

Following the synthesis method of Example **3**, Step **1**, except replacing Step **1, 2-3** with **123-4** (8.0 mg, 0.02 mmol), **2-4** with **5-1** (3.6 mg, 0.02 mmol)the same synthesis method was applied, to give Compound **123** (1.2 mg, 2.3 μmol, 9.8% yield), LCMS ($ESI^+$) m/z: 523.4 $[M+H]^+$.

**$^1$H NMR** (400 MHz, $CDCl_3$) δ 10.85 (s, 1H), 7.14-7.11 (m, 1H), 6.93 (s, 1H), 5.89 (s, 1H), 4.55-4.46 (m, 1H), 4.44-4.42 (m, 2H), 4.42-4.41 (m, 1H), 4.32-4.30 (m, 1H), 3.94-3.92 (m, 1H), 3.83 (s, 3H), 3.71-3.70 (m, 1H), 3.69-3.63 (m, 1H), 3.62-3.60 (m, 1H), 3.28-3.21 (m, 1H), 2.25 (s, 3H), 2.18 (s, 3H), 2.09-2.06 (m, 3H), 1.75-1.72 (m, 1H), 1.18-1.15 (m, 2H), 0.80-0.77 (m, 3H).

**Example 71, Compound 124 Synthesis:**

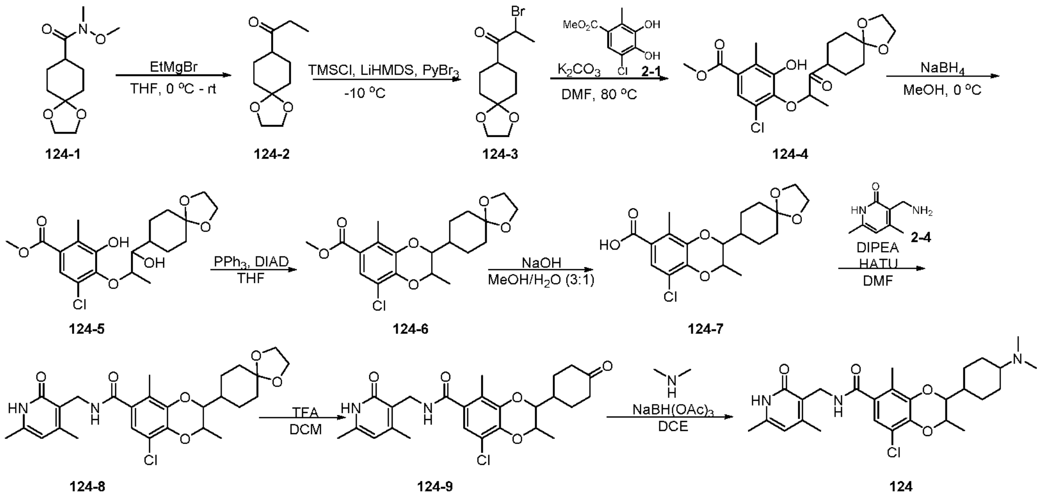


**Step 1, Compound 124-2 Synthesis:**

A single-necked flask was added with substrate **124-1** (1.0 g, 4.4 mmol). The mixture was dissolved with THF (20 mL) under stirring, and then at 0°C added with **EtMgBr** (754.0 mg, 5.7 mmol), stirred to react for 16 h, with TLC monitoring. After the completion of the reaction, the mixture was added with saturated ammounium chloride solution and ethyl acetate to extract three times. The organic phase was combined, washed with saturated saline solution, and dried with anhydrous sodium sulfate, and then purified by silica gel column chromatography (PE: EA = 5: 1), to give the product **124-2** (500 mg, 2.5 mmol, 57.8% yield).

**Step 2, Compound 124-3 Synthesis:**

A single-necked flask was added with substrate **124-2** (500.0 mg, 2.5 mmol). The mixture was dissolved with THF (20 mL) under stirring, and then at 0°C added with **LiHMDS** (842.0 mg, 5.0 mmol)stirred for 0.5 h, and then added with $PyHBr_3$ (1.2 g, 3.8 mmol), further stirred to react for 2 hours, with TLC monitoring. After the completion of the reaction, the mixture was added with saturated sodium bicarbonate solution and ethyl acetate to extract three times. The organic phase was combined, washed with saturated saline solution, and dried with anhydrous sodium sulfate, and then purified by silica gel column chromatography (PE: EA = 5: 1), to give the product **124-3** (240.0 mg, 0.9 mmol, 34.3% yield).

**Step 3, Compound 124-4 Synthesis:**

Following the synthesis method of Example **2**, Step **1**, except replacing Step **1**, **IM-2** with **123-3** (240.0 mg, 0.9

mmol), the same synthesis method was applied, to give Compound **124-4** (220.0 mg, 0.5 mmol, 61.5% yield).

**Step 4, Compound 124-5 Synthesis:**

Following the synthesis method of Example **IM-3**, Step **3**, except replacing Step **3**, **IM-3-4** with **123-4** (220.0 mg, 0.5 mmol), the same synthesis method was applied, to give Compound **124-5** (180.0 mg, 0.4 mmol, 81.4% yield).

**Step 5, Compound 124-6 Synthesis:**

Following the synthesis method of Example **IM-3**, Step **4**, except replacing Step **4**, **IM-3-5** with **123-5** (180.0 mg, 0.4 mmol), the same synthesis method was applied, to give Compound **124-6** (130.0 mg, 0.3 mmol, 75.5% yield).

**Step 6, Compound 124-7 Synthesis:**

A single-necked flask was added with substrate **124-6** (130.0 mg, 0.3 mmol). The mixture was dissolved with MeOH/$H_2O$ (20 mL) to dissolve, and then added with **NaOH** (131.0 mg, 3.0 mmol), 80°C to react for 1 hour, with LC-MS monitoring. After the completion of the reaction, the mixture was added with 1 N HCl to adjust to pH = 6, and then added with EA solution to extract three times. The organic phase was combined, washed with saturated saline solution, and dried with anhydrous sodium sulfate and then condensated under reduced pressure, to give the crude product **124-7** (130.0 mg, crude). LCMS ($ESI^+$) m/z: 382.8 $[M+H]^+$

**Step 7, Compound 124-8 Synthesis:**

Following the synthesis method of Example **1**, Step **3**, except replacing Step **3, 1-3** with **124-7** (130.0 mg, crude), the same synthesis method was applied, to give Compound **124-8** (150.0 mg, crude), LCMS ($ESI^+$) m/z: 517.0 $[M+H]^+$.

**Step 8, Compound 124-9 Synthesis:**

Following the synthesis method of Example **3**, Step **2**, except replacing Step **2, 3-2** with **124-8** (150.0 mg, crude), the same synthesis method was applied, to give Compound **124-9** (120.0 mg, crude), LCMS ($ESI^+$) m/z: 472.9 $[M+H]^+$.

**Step 9, Compound 124 Synthesis:**

Following the synthesis method of Example **6**, Step **4**, except replacing Step **4, 6-3** with **124-9** (80.0 mg, 0.2 mmol), the same synthesis method was applied, to give Compound **124** (21.0 mg, 0.04 mmol, 24.7% yield), LCMS ($ESI^+$) m/z: 502.6 $[M+H]^+$.

**$^1$H NMR** (600 MHz, $CDCl_3$) $\delta$ 10.11 (s, 1H), 7.10-7.08 (m, 1H), 6.98 (s, 1H), 5.93 (s, 1H), 4.64-4.49 (m, 3H), 4.07-4.05 (m, 1H), 2.99-2.97 (m, 1H), 2.72 (s, 6H), 2.68-2.65 (m, 2H), 2.38 (s, 3H), 2.24 (s, 3H), 2.21 (s, 3H), 1.98-1.95 (m, 3H), 1.87-1.85 (m, 2H), 1.70-1.65 (m, 5H).

**Example 72, Compound 125 Synthesis:**

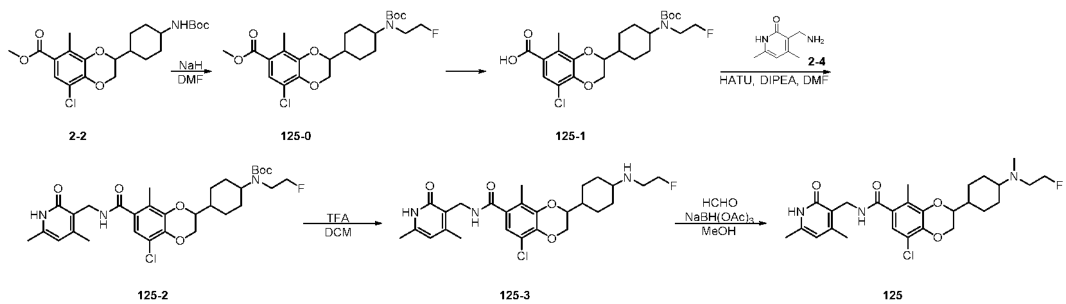


2-2 125-0 125-1 125-2 125-3 125

**Step 1, Compound 125-1 Synthesis:**

Following the synthesis method of Example **70**, Step **4**, except replacing Step **4, 123-3** with **2-2** (220.0 mg, 0.5

mmol), **1-fluoro-2-iodo-ethane** with **1-bromo-2-fluoro-ethane** (127.0 mg, 1.0 mmol), the same synthesis method was applied. After the completion of the reaction, the system was added with water, stirred at room temperature for 0.5 hours, with LC-MS monitoring. After the completion of the reaction, the mixture was added with 1 N HCl to adjust to pH = 6, and then added with EA solution to extract three times. The organic phase was combined, washed with saturated saline solution, and dried with anhydrous sodium sulfate and then condensated under reduced pressure, to give Compound **125-1** (35.0 mg, 0.07 mmol, 14.8% yield), LCMS ($ESI^+$) m/z: 471.9 $[M+H]^+$.

**Step 2, Compound 125-2 Synthesis:**

Following the synthesis method of Example **1**, Step **3**, except replacing Step **3, 1-3** with **125-1** (35.0 mg, 0.07 mmol), the same synthesis method was applied, to give Compound **125-2** (20.0 mg, crude), LCMS ($ESI^+$) m/z: 606.1 $[M+H]^+$.

**Step 3, Compound 125-3 Synthesis:**

Following the synthesis method of Example **3**, Step **2**, except replacing Step **2, 3-2** with **125-2** (20.0 mg, crude), the same synthesis method was applied, to give Compound **125-3** (16.5 mg, crude), LCMS ($ESI^+$) m/z: 506.1 $[M+H]^+$.

**Step 4, Compound 125 Synthesis:**

Following the synthesis method of Example **1**, Step **5**, except replacing Step **5, 1-6** with **125-3** (16.5 mg, crude), the same synthesis method was applied, to give Compound **125** (8.0 mg, 15.4 μmol, 47.1% yield), LCMS ($ESI^+$) m/z: 520.2 $[M+H]^+$.

**$^1$H NMR** (600 MHz, Methanol-$d_4$) δ 6.92 (s, 1H), 6.10 (s, 1H), 4.62-4.56 (m, 3H), 4.51 (t, $J$ = 4.8 Hz, 1H), 4.42 (m, 2H), 4.08-4.03 (m, 1H), 3.91-3.83 (m, 1H), 2.92-2.88 (m, 1H), 2.88-2.84 (m, 1H), 2.61-2.55 (s, 1H), 2.38 (s, 3H), 2.35 (s, 3H), 2.24 (s, 3H), 2.98 (s, 3H), 2.01-1.96 (m, 2H), 1.91-1.87 (m, 1H), 0.93-0.84 (m, 5H).

**Example 73, Compound 126 Synthesis:**

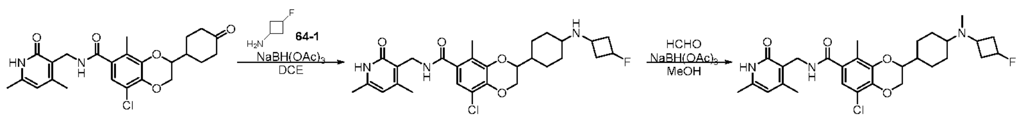


**57-3** **126-1** **126**

**Step 1, Compound 126-1 Synthesis:**

Following the synthesis method of Example **6**, Step **4**, except replacing Step **4, 6-3** with **57-3** (46.0 mg, 0.1 mmol), **dimethylamine** with **64-1** (13.0 mg, 0.2 mmol), the same synthesis method was applied, to give Compound **126-1** (167.0 mg, crude), LCMS ($ESI^+$) m/z: 532.3 $[M+H]^+$.

**Step 2, Compound 126 Synthesis:**

Following the synthesis method of Example **1**, Step **5**, except replacing Step **5, 1-6** with **126-1** (167.0 mg, crude), the same synthesis method was applied, to give Compound **126** (18.0 mg, 0.03 mmol, 10.5% yield), LCMS ($ESI^+$) m/z: 546.3 $[M+H]^+$.

**$^1$H NMR** (600 MHz, DMSO-$d_6$) δ 11.47 (s, 1H), 8.12 (s, 1H), 6.89 (s, 1H), 5.85 (s, 1H), 5.15-5.05 (m, 1H), 4.51-4.33 (m, 2H), 4.22 (s, 2H), 4.09-3.85 (m, 2H), 3.58-3.56 (m, 1H), 2.23-2.09 (m, 12H), 2.04-1.96 (m, 2H), 1.83-1.65 (m, 3H), 1.62-1.38 (m, 5H), 1.32-1.13 (m, 3H).

**Example 74, Compound 127 Synthesis:**

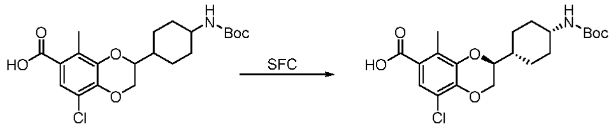


2-3 127-1

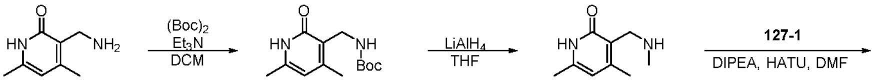


127-2 127-3 127-4

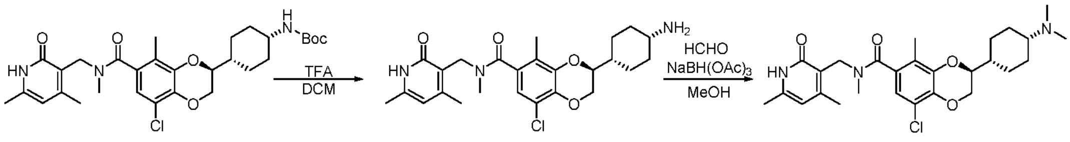


127-5 127-6 127

**Step 1, Compound 127-1 Synthesis:**

Compound 127-1 can be obtained by SFC resolution from Compound 2-3.

**Step 2, Compound 127-3 Synthesis:**

A single-necked flask was added with substrate **127-2** (636.7 mg, 2.9 mmol). The mixture was dissolved with DCM (5 mL) to dissolve, and then added with **$Et_3N$** (883.0 mg, 7.3 mmol), and reacted at room temperature for 1 hour, with LC-MS monitoring. After the completion of the reaction, and condensated under reduced pressure, and then the reaction mixture was purified by medium pressure liquid chromatography to give the product **127-3** (500.0 mg, 2.0 mmol, 47.4% yield). LCMS ($ESI^+$) m/z: 252.3 $[M+H]^+$

**Step 3, Compound 127-4 Synthesis:**

A single-necked flask was added with substrate **127-3** (500.0 mg, 2.0 mmol). The mixture was dissolved with THF (20 mL) to dissolve. Then at 0°C and under nitrogen protection, the mixture was added with **$LiAlH_4$** (75.2 mg, 2.0 mmol), 7stirred at 0°C to react for 2 hours, with LC-MS monitoring. After the completion of the reaction, the reaction mixture was extracted with water and EA solution three times. The organic phase was combined, washed with saturated saline solution, and dried with anhydrous sodium sulfate and then condensated under reduced pressure, to give the crude product **127-4** (500.0 mg, crude). LCMS ($ESI^+$) m/z: 166.2 $[M+H]^+$

**Step 4, Compound 127-5 Synthesis:**

Following the synthesis method of Example **1**, Step **3**, except replacing Step **3, 1-3** with **127-1** (190.0 mg, 0.5 mmol), **1-4** with **127-4** (148.0 mg, 0.9 mmol), the same synthesis method was applied, to give Compound **127-5** (110.0 mg, 0.2 mmol, 43.0% yield), LCMS ($ESI^+$) m/z: 574.1 $[M+H]^+$.

**Step 5, Compound 127-6 Synthesis:**

Following the synthesis method of Example **3**, Step **2**, except replacing Step **2, 3-2** with **127-5** (110.0 mg, 0.2 mmol), the same synthesis method was applied, to give Compound **127-6** (98.0 mg, crude), LCMS ($ESI^+$) m/z: 474.0 $[M+H]^+$.

**Step 6, Compound 127 Synthesis:**

Following the synthesis method of Example **6**, Step **4**, except replacing Step **4, 6-3** with **127-6** (47.0 mg, 0.1 mmol), added with formaldehydewater solution (30.0 mg, 1.0 mmol), DCE with MeOH, the same synthesis method was applied, to give Compound **127** (35.0 mg, 0.07 mmol, 69.7% yield). LCMS ($ESI^+$) m/z: 502.3 $[M+H]^+$

**$^1$H NMR** (600 MHz, DMSO-$d_6$) δ 11.53-11.48 (m, 1H), 7.00 (s, 0.3H), 6.75 (s, 0.7H), 5.90 (s, 0.7H), 5.83 (s, 0.3H), 4.56-4.54 (m, 1H), 4.47-4.46 (m, 1H), 4.07-4.06 (m, 1H), 3.95-3.93 (m, 1H), 2.73 (s, 1H), 2.58 (s, 2H), 2.18 (s, 9H), 2.13 (s,

3H), 2.11 (s, 1H), 2.09 (s, 2H), 1.97 (s, 3H), 1.87-1.85 (m, 2H), 1.80-1.79 (m, 1H), 1.57-1.55 (m, 1H), 1.20-1.16 (m, 4H).

**Example 75, Compound 128 Synthesis:**

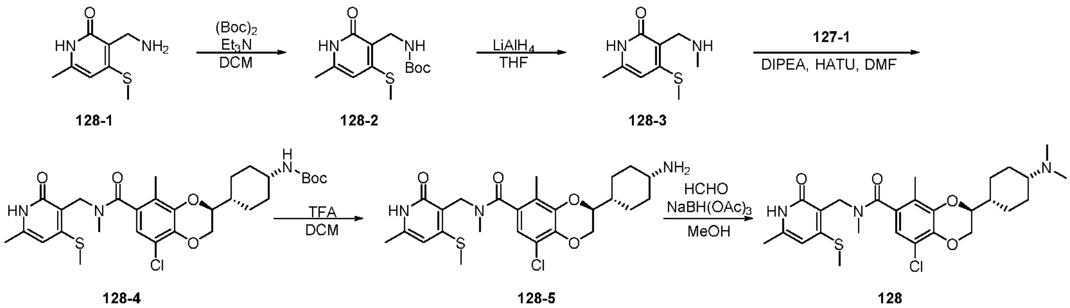


**Step 1, Compound 128-2 Synthesis:**

Following the synthesis method of Example **74**, Step **2**, except replacing Step **2, 127-2** with **128-1** (628.0 mg, 3.4 mmol), the same synthesis method was applied, to give Compound **128-2** (284.0 mg, crude), LCMS (ESI$^+$) m/z: 284.4 $[M+H]^+$.

**Step 2, Compound 128-3 Synthesis:**

Following the synthesis method of Example **74**, Step **3**, except replacing Step **3, 127-3** with **128-2** (284.0 mg, 1.0 mmol), the same synthesis method was applied, to give Compound **128-3** (100.0 mg, crude), LCMS (ESI$^+$) m/z: 198.3 $[M+H]^+$.

**Step 3, Compound 128-4 Synthesis:**

Following the synthesis method of Example **1**, Step **3**, except replacing Step **3, 1-3** with **127-1** (78.0 mg, 0.2 mmol), **1-4** with **128-3** (73.0 mg, 0.4 mmol), the same synthesis method was applied, to give Compound **128-4** (110.0 mg, crude), LCMS (ESI$^+$) m/z: 606.2 $[M+H]^+$.

**Step 4, Compound 128-5 Synthesis:**

Following the synthesis method of Example **3**, Step **2**, except replacing Step **2, 3-2** with **128-4** (30.0 mg, 0.05 mmol), the same synthesis method was applied, to give Compound **128-5** (50.0 mg, crude), LCMS (ESI$^+$) m/z: 506.1 $[M+H]^+$.

**Step 5, Compound 128 Synthesis:**

Following the synthesis method of Example **6**, Step **4**, except replacing Step **4, 6-3** with **128-5** (50.0 mg, crude), added with formaldehydewater solution (60.0 mg, 2.0 mmol), DCE with MeOH, the same synthesis method was applied, to give Compound **128** (10.0 mg, 18.7 μmol, 18.9% yield). LCMS (ESI$^+$) m/z: 534.3 $[M+H]^+$

$^1$**H NMR** (600 MHz, DMSO-$d_6$) δ 11.49 (m, 1H), 6.98-6.77 (m, 1H), 6.18-6.09 (m, 1H), 4.63-4.58 (m, 1H), 4.47-4.42 (m, 1H), 4.24-3.96 (m, 3H), 3.14-3.09 (m, 1H), 2.75-2.71 (m, 6H), 2.54-2.53 (m, 4H), 2.48-2.47 (m, 1H), 2.43 (s, 1H), 2.22 (s, 2H), 2.19 (m, 1H), 2.08-2.04 (m, 5H), 1.91-1.88 (m, 1H), 1.67-1.63 (m, 1H), 1.48-1.39 (m, 2H), 1.35-1.27 (m, 2H).

**Example 76, Compound 129 Synthesis:**

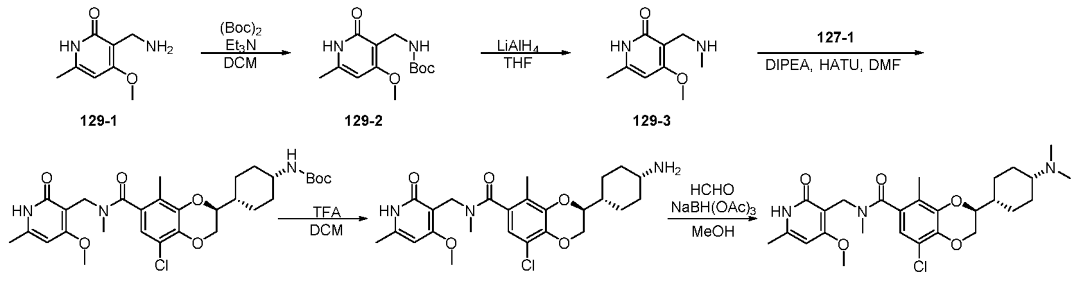


129-1 129-2 129-3

129-4 129-5 129

**Step 1, Compound 129-2 Synthesis:**

Following the synthesis method of Example **74**, Step **2**, except replacing Step **2, 127-2** with **129-1** (422.0 mg, 2.5 mmol), the same synthesis method was applied, to give Compound **129-2** (282.0 mg, crude), LCMS (ESI$^+$) m/z: 268.3 [M+H]$^+$.

**Step 2, Compound 129-3 Synthesis:**

Following the synthesis method of Example **74**, Step **3**, except replacing Step **3, 127-3** with **129-2** (282.0 mg, 1.1 mmol), the same synthesis method was applied, to give Compound **129-3** (100.0 mg, crude), LCMS (ESI$^+$) m/z: 182.2 [M+H]$^+$.

**Step 3, Compound 129-4 Synthesis:**

Following the synthesis method of Example **1**, Step **3**, except replacing Step **3, 1-3** with **127-1** (37.0 mg, 0.09 mmol), **1-4** with **129-3** (48.0 mg, 0.3 mmol), the same synthesis method was applied, to give Compound **129-4** (100.0 mg, crude), LCMS (ESI$^+$) m/z: 590.1 [M+H]$^+$.

**Step 4, Compound 129-5 Synthesis:**

Following the synthesis method of Example **3**, Step **2**, except replacing Step **2, 3-2** with **129-4** (60.0 mg, 0.1 mmol), the same synthesis method was applied, to give Compound **129-5** (50.0 mg, crude), LCMS (ESI$^+$) m/z: 490.0 [M+H]$^+$.

**Step 5, Compound 129 Synthesis:**

Following the synthesis method of Example **6**, Step **4**, except replacing Step **4, 6-3** with **129-5** (50.0 mg, crude), added with formaldehydewater solution (60.0 mg, 2.0 mmol), DCE with MeOH, the same synthesis method was applied, to give Compound **129** (12.0 mg, 23.2 μmol, 22.7% yield). LCMS (ESI$^+$) m/z: 518.3 [M+H]$^+$

**$^1$H NMR** (600 MHz, DMSO-$d_6$) δ 11.45 (s, 1H), 6.89-6.73 (m, 1H), 6.12-6.04 (m, 1H), 4.50-4.45 (m, 1H), 4.14-3.94 (m, 4H), 3.80 (s, 1H), 3.70 (s, 2H), 2.78 (s, 2H), 2.47 (s, 1H), 2.23-2.16 (m, 11H), 2.02-1.98 (m, 4H), 1.87-1.81 (m, 3H), 1.21-1.18 (m, 4H).

**Example 77, Compound 130 Synthesis:**

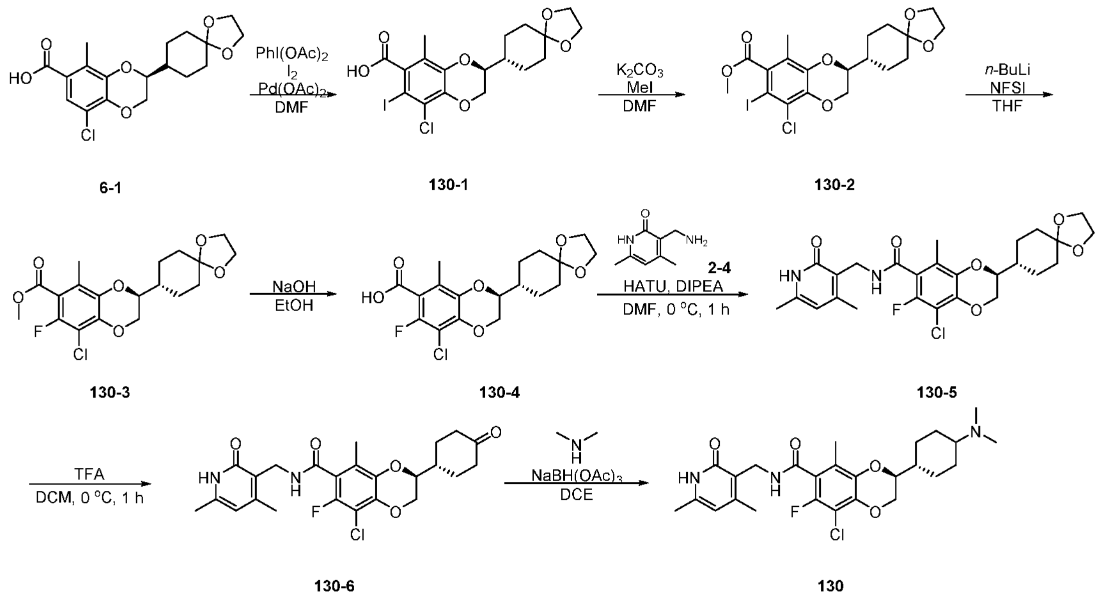


6-1 130-1 130-2

130-3 130-4 130-5

130-6 130

**Step 1, Compound 130-1 Synthesis:**

A single-necked flask was added with substrate **6-1** (520.0 mg, 1.4 mmol). The mixture was dissolved with DMF (5 mL) to dissolve, and then added with **PhI(OAc)$_2$** (450.0 mg, 1.4 mmol), $I_2$ (358.0 mg, 1.4 mmol) and Pd(OAc)$_2$ (317.0 mg, 1.4 mmol), Under nitrogen protection10stirred at 0°C to react for 24 hours, with LC-MS monitoring. After the completion of the reaction, the reaction mixture was cooled to room temperature, the reaction mixture was condensated under reduced pressure, and then extracted with water and EA solution three times. The organic phase was combined, washed with saturated saline solution, and dried with anhydrous sodium sulfate and then condensated under reduced pressure, to give the crude product **130-1** (480.0 mg, crude). LCMS (ESI$^+$) m/z: 494.7 [M+H]$^+$

**Step 2, Compound 130-2 Synthesis:**

A single-necked flask was added with substrate **130-1** (480.0 mg, crude). The mixture was dissolved with DMF (2 mL) to dissolve, and then added with **$K_2CO_3$** (270.0 mg, 2.0 mmol) and MeI (275.0 mg, 2.0 mmol), stirred at 50°C to react for 1 hour, with LC-MS monitoring. After the completion of the reaction, the reaction mixture was cooled to room temperature, the reaction mixture was condensated under reduced pressure, and then extracted with water and EA solution three times. The organic phase was combined, washed with saturated saline solution, and dried with anhydrous sodium sulfate and then condensated under reduced pressure, to give the crude product **130-2** (465.0 mg, crude). LCMS (ESI$^+$) m/z: 508.7 [M+H]$^+$

**Step 3, Compound 130-3 Synthesis:**

A single-necked flask was added with substrate **130-2** (152.0 mg, 0.3 mmol). The mixture was dissolved with THF (3 mL) to dissolve, and then under nitrogen protection and at -78°C slowly added dropwise with ***n*-BuLi** (2.5 M, 240 μL), at -78°C stirred to react for 5 mins, and then added with **NFSI** (189.0 mg, 0.6 mmol), with LC-MS monitoring. After the completion of the reaction, the reaction mixture was cooled to room temperature, the reaction mixture was condensated under reduced pressure, and then extracted with water and EA solution three times. The organic phase was combined, washed with saturated saline solution, and dried with anhydrous sodium sulfate and then condensated under reduced pressure, and then purified by medium pressure liquid chromatography to give the product **130-3** (46.0 mg, 0.1 mmol, 38.4% yield). LCMS (ESI$^+$) m/z: 400.8 [M+H]$^+$

**Step 4, Compound 130-4 Synthesis:**

Following the synthesis method of Example **6**, Step **1**, except replacing Step **1**, **IM-3a** with **130-3** (45.0 mg, 0.1 mmol), the same synthesis method was applied, to give Compound **130-4** (45.0 mg, crude), LCMS (ESI$^+$) m/z: 386.8 [M+H]$^+$.

**Step 5, Compound 130-5 Synthesis:**

Following the synthesis method of Example **1**, Step **3**, except replacing Step **3, 1-3** with **130-4** (45.0 mg, crude), **1-4** with **2-4** (21.0 mg, 0.2 mmol), the same synthesis method was applied, to give Compound **130-5** (70.0 mg, crude), LCMS (ESI$^+$) m/z: 521.0 [M+H]$^+$.

**Step 6, Compound 130-6 Synthesis:**

Following the synthesis method of Example **3**, Step **2**, except replacing Step **2, 3-2** with **130-5** (70.0 mg, crude), the same synthesis method was applied, to give Compound **130-6** (65.0 mg, crude), LCMS (ESI$^+$) m/z: 477.0 [M+H]$^+$.

**Step 7, Compound 130 Synthesis:**

Following the synthesis method of Example **6**, Step **4**, except replacing Step **4, 6-3** with **130-6** (65.0 mg, crude), the same synthesis method was applied, to give Compound **130** (20.0 mg, 39.5 μmol, 29.0% yield), LCMS (ESI$^+$) m/z: 506.1 [M+H]$^+$.

**$^1$H NMR** (400 MHz, DMSO-$d_6$) δ 11.46 (s, 1H), 8.40 (t, *J* = 4.8 Hz, 1H), 5.85 (s, 1H), 4.49 (dd, *J* = 11.2, 1.6 Hz, 1H), 4.26 (d, *J* = 5.2 Hz, 2H), 4.12-4.00 (m, 2H), 2.17-2.14 (m, 9H), 2.11 (s, 3H), 2.02 (s, 3H), 1.85-1.15 (m, 10H).

**$^{19}$F NMR** (376 MHz, DMSO-$d_6$) δ -128.42.

**Example 78, Compound 131 and 132 Synthesis:**

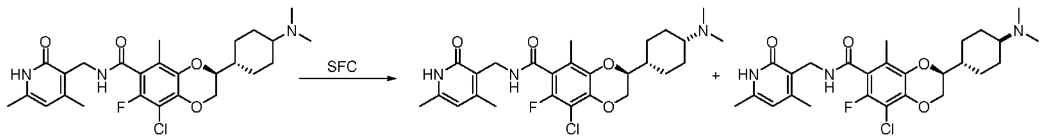


**130** **131** **132**

**Step 1, Compound 131 and 132 Synthesis:**

Compound 131 and 132 can be obtained by SFC resolution from Compound 130.

**Compound 131 NMR: $^1$H NMR** (600 MHz, DMSO-$d_6$) δ 11.46 (s, 1H), 8.40 (t, *J* = 4.8 Hz, 1H), 5.85 (s, 1H), 4.49-4.47 (m, 1H), 4.26 (d, *J* = 4.8 Hz, 2H), 4.11-4.08 (m, 1H), 4.03-4.01 (m, 1H), 2.17-2.13 (m, 9H), 2.11 (s, 3H), 2.08 (s, 3H), 1.76-1.41 (m, 10H).

$^{19}$F **NMR** (564 MHz, DMSO-$d_6$) δ -128.42.

**Compound 132 NMR: $^1$H NMR** (600 MHz, DMSO-$d_6$) δ 11.47 (s, 1H), 8.39 (t, *J* = 4.8 Hz, 1H), 5.85 (s, 1H), 4.48 (d, *J* = 10.2 Hz, 1H), 4.26 (d, *J* = 4.8 Hz, 2H), 4.10-4.06 (m, 1H), 3.89-3.86 (m, 1H), 2.16-2.15 (m, 9H), 2.10 (s, 3H), 2.03 (s, 3H), 1.88-1.77 (m, 3H), 1.30-1.23 (m, 4H), 1.19-1.11 (m, 3H).

$^{19}$F **NMR** (564 MHz, DMSO-$d_6$) δ -128.42.

**Example 79, Compound 133 Synthesis:**

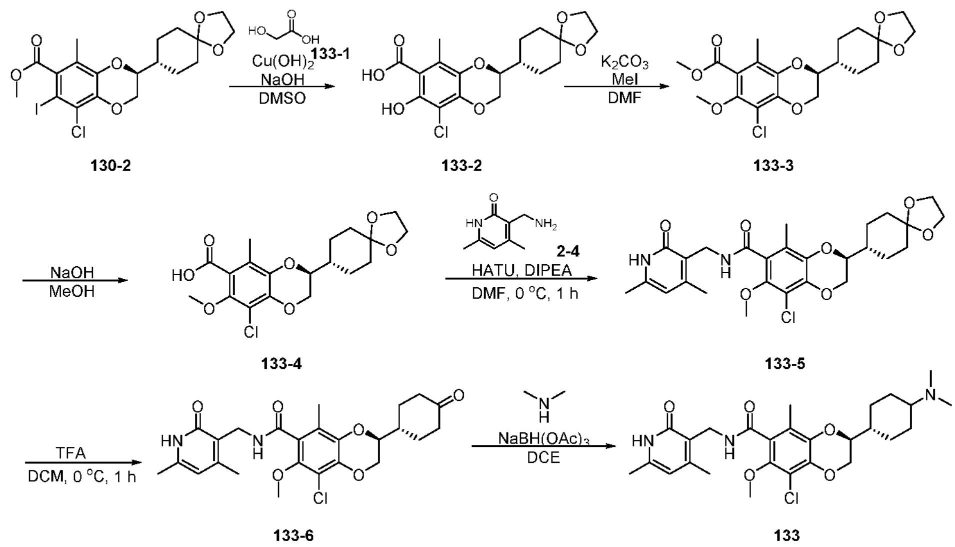


130-2 133-2 133-3 133-4 133-5 133-6 133

**Step 1, Compound 133-2 Synthesis:**

A single-necked flask was added with substrate **130-2** (110.0 mg, 0.2 mmol). The mixture was dissolved with DMSO (0.5 mL) and water (0.5 mL) to dissolve, and then added with **133-1** (5.0 mg, 65.8 μmol), **NaOH** (52.0 mg, 1.3 mmol) and $Cu(OH)_2$ (1.0 mg, 10.0 μmol), and at stirred at 120°C to react for 6 hours, with LC-MS monitoring. After the completion of the reaction, the reaction mixture was cooled to room temperature, the reaction mixture was condensated under reduced pressure, and then extracted with water and EA solution three times. The organic phase was combined, washed with saturated saline solution, and dried with anhydrous sodium sulfate and then condensated under reduced pressure, and then purified by medium pressure liquid chromatography to give the product **133-2** (50.0 mg, crude). LCMS (ESI$^+$) m/z: 384.8 $[M+H]^+$

**Step 2, Compound 133-3 Synthesis:**

Following the synthesis method of Example **77**, Step **2**, except replacing Step **2**, **130-1** with **133-2** (50.0 mg, crude), the same synthesis method was applied, to give Compound **133-3** (50.0 mg, crude), LCMS (ESI$^+$) m/z: 412.9 $[M+H]^+$.

**Step 3, Compound 133-4 Synthesis:**

Following the synthesis method of Example **77**, Step **2**, except replacing Step **2**, **130-1** with **133-3** (50.0 mg, crude), the same synthesis method was applied, to give Compound **133-4** (50.0 mg, crude), LCMS (ESI$^+$) m/z: 399.2 $[M+H]^+$.

**Step 4, Compound 133-5 Synthesis:**

Following the synthesis method of Example **1**, Step **3**, except replacing Step **3, 1-3** with **133-4** (25.0 mg, 0.06 mmol), **1-4** with **2-4** (19.0 mg, 0.1 mmol), the same synthesis method was applied, to give Compound **133-5** (35.0 mg, crude), LCMS (ESI$^+$) m/z: 533.0 $[M+H]^+$.

**Step 5, Compound 133-6 Synthesis:**

Following the synthesis method of Example **3**, Step **2**, except replacing Step **2, 3-2** with **133-5** (35.0 mg, crude), the same synthesis method was applied, to give Compound **133-6** (30.0 mg, crude), LCMS (ESI$^+$) m/z: 489.0 $[M+H]^+$.

**Step 6, Compound 133 Synthesis:**

Following the synthesis method of Example **6**, Step **4**, except replacing Step **4, 6-3** with **133-6** (30.0 mg, crude), the same synthesis method was applied, to give Compound **133** (7.6 mg, 14.7 μmol, 23.9% yield), LCMS (ESI$^+$) m/z: 518.3 $[M+H]^+$.

**Example 80, Compound 134 and 135 Synthesis:**

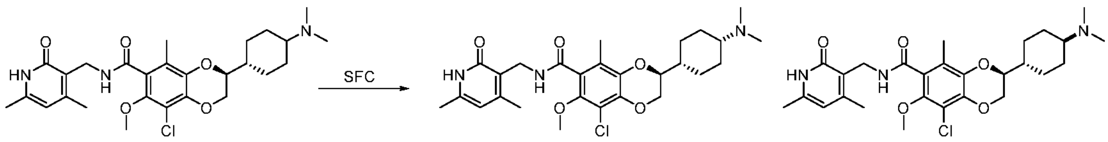


133 134 135

**Step 1, Compound 134 and 135 Synthesis:**

Compound 134 and 135 can be obtained by SFC resolution from Compound 133.

**Compound 134 NMR: $^1$H NMR** (400 MHz, DMSO-$d_6$) δ 11.44 (s, 1H), 8.13 (t, *J* = 5.2 Hz, 1H), 5.85 (s, 1H), 4.44 (dd, *J* = 10.8, 1.6 Hz, 1H), 4.27 (d, *J* = 5.2 Hz, 2H), 4.07-3.97 (m, 2H), 3.64 (s, 3H), 2.19 (s, 3H), 2.15 (s, 6H), 2.10 (s, 3H), 2.08-2.07 (m, 1H), 1.97 (s, 3H), 1.76-1.75 (m, 2H), 1.69-1.67 (m, 2H), 1.65-1.58 (m, 2H), 1.44-1.41 (m, 3H).

**Compound 135 NMR: $^1$H NMR** (400 MHz, DMSO-$d_6$) δ 11.44 (s, 1H), 8.13 (t, *J* = 4.8 Hz, 1H), 5.85 (s, 1H), 4.44 (dd, *J* = 11.6, 2.0 Hz, 1H), 4.27 (d, *J* = 4.8 Hz, 2H), 4.07-4.02 (m, 1H), 3.87-3.85 (m, 1H), 3.64 (s, 3H), 2.23 (s, 6H), 2.20 (s, 3H), 2.10 (s, 3H), 2.06-2.02 (m, 1H), 1.99 (s, 3H), 1.90-1.78 (m, 4H), 1.54-1.50 (m, 2H), 1.19-1.15 (m, 3H).

**Example 81, Compound 136 Synthesis:**

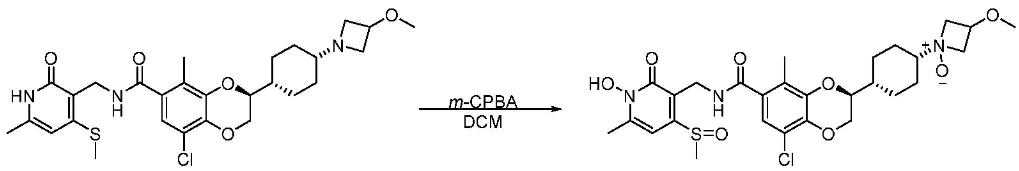


9 136

**Step 1, Compound 136 Synthesis:**

A single-necked flask was added with substrate **9** (20.0 mg, 0.04 mmol). The mixture was dissolved with DCM (5 mL) to dissolve, and then at 0°C added with ***m*-CPBA** (18.0 mg, 0.1 mmol)stirred at 0°C to react for 4 hours, with LC-MS monitoring. After the completion of the reaction, the reaction mixture was condensated under reduced pressure, and then purified by medium pressure liquid chromatography to give the product **136** (6.0 mg, 9.8 μmol, 27.6% yield). LCMS (ESI$^+$) m/z: 610.5 [M+H]$^+$

$^1$H NMR (600 MHz, DMSO-$d_6$) δ 12.56 (s, 1H), 8.19 (t, *J* = 4.8 Hz, 1H), 6.93 (s, 1H), 6.44 (s, 1H), 4.59 (d, *J* = 3.6 Hz, 2H), 4.48-4.45 (m, 2H), 4.27-4.25 (m, 2H), 4.08-4.04 (m, 1H), 3.94-3.91 (m, 3H), 3.36 (s, 3H), 3.21 (s, 3H), 3.10-3.06 (m, 1H), 2.26 (s, 3H), 2.14 (s, 3H), 2.05-2.03 (m, 1H), 1.88-1.82 (m, 3H), 1.56-1.51 (m, 3H), 1.30-1.19 (m, 2H).

**Example 82, Compound 137 and 138 Synthesis:**

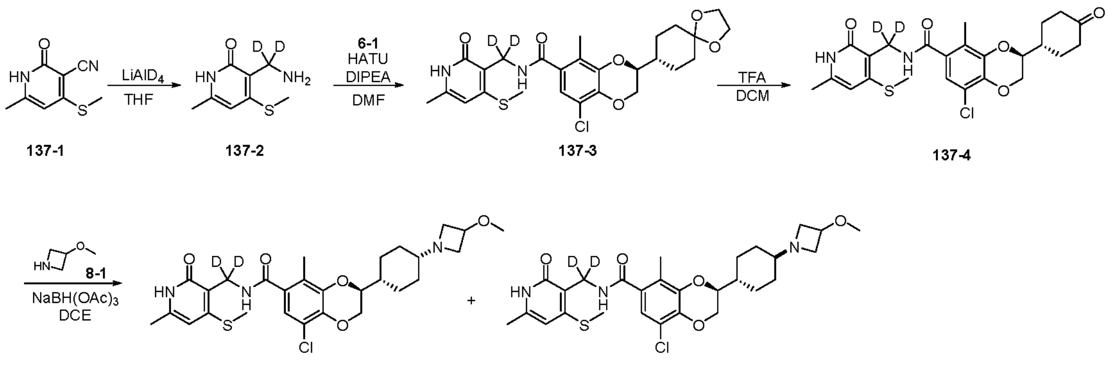


137-1 137-2 137-3 137-4

137 138

**Step 1, Compound 137-2 Synthesis:**

A single-necked flask was added with substrate **137-1** (36.0 mg, 0.2 mmol). The mixture was dissolved with THF (1 mL) to dissolve, and then at 0°C added with **LiAlD4** (8.4 mg, 0.2 mmol)stirred at 0°C to react for 1 hour, with LC-MS monitoring. After the completion of the reaction, the reaction mixture was condensated under reduced pressure, and then extracted with water and EA solution three times. The organic phase was combined, washed with saturated saline solution, and dried with anhydrous sodium sulfate and then condensated under reduced pressure to give the crude product **137-2** (36.0 mg, crude). LCMS ($ESI^+$) m/z: 187.2 $[M+H]^+$

**Step 2, Compound 137-3 Synthesis:**

Following the synthesis method of Example **1**, Step **3**, except replacing Step **3, 1-3** with **6-1** (36.0 mg, 0.1 mmol), **1-4** with **137-2** (36.0 mg, 0.2 mmol), the same synthesis method was applied, to give Compound **137-3** (14.0 mg, 26.1 μmol, 26.7% yield), LCMS ($ESI^+$) m/z: 537.0 $[M+H]^+$.

**Step 3, Compound 137-4 Synthesis:**

Following the synthesis method of Example **3**, Step **2**, except replacing Step **2, 3-2** with **137-3** (14.0 mg, 0.03 mmol), the same synthesis method was applied, to give Compound **137-4** (14.0 mg, crude), LCMS ($ESI^+$) m/z: 493.0 $[M+H]^+$.

**Step 4, Compound 137 and 138 Synthesis:**

Following the synthesis method of Example **6**, Step **4**, except replacing Step **4, 6-3** with **137-4** (14.0 mg, crude), **dimethylamine** with **8-1** (12.0 mg, 0.09 mmol)the same synthesis method was applied. The obtained compound was subjected to Prep-MPLC to give **137** (1.0 mg, 1.8 μmol, 6.3% yield) and **138** (2.5 mg, 4.4 μmol, 15.6% yield), LCMS ($ESI^+$) m/z: 564.3 $[M+H]^+$.

**Compound 137 NMR: $^1$H NMR** (400 MHz, DMSO-$d_6$) δ 11.51 (s, 1H), 7.99 (s, 1H), 6.89 (s, 1H), 6.08 (s, 1H), 4.46-4.43 (m, 1H), 4.07-4.02 (m, 1H), 3.93-3.87 (m, 2H), 3.45-3.42 (m, 2H), 3.13 (s, 3H), 2.73-2.70 (m, 2H), 2.45 (s, 3H), 2.16 (s, 3H), 2.14 (s, 3H), 1.98-1.87 (m, 2H), 1.78-1.70 (m, 3H), 1.54-1.51 (m, 1H), 1.19-1.13 (m, 2H), 0.89-0.84 (m, 2H).

**Compound 138 NMR: $^1$H NMR** (400 MHz, DMSO-$d_6$) δ 11.51 (s, 1H), 7.99 (s, 1H), 6.89 (s, 1H), 6.08 (s, 1H), 4.44-4.40 (m, 1H), 4.08-4.03 (m, 1H), 3.94-3.90 (m, 2H), 3.47-3.43 (m, 2H), 3.14 (s, 3H), 2.68-2.65 (m, 2H), 2.45 (s, 3H), 2.22-2.20 (m, 1H), 2.16 (s, 3H), 2.13 (s, 3H), 1.63-1.31 (m, 9H).

**Example 83, Compound 139 and 140 Synthesis:**

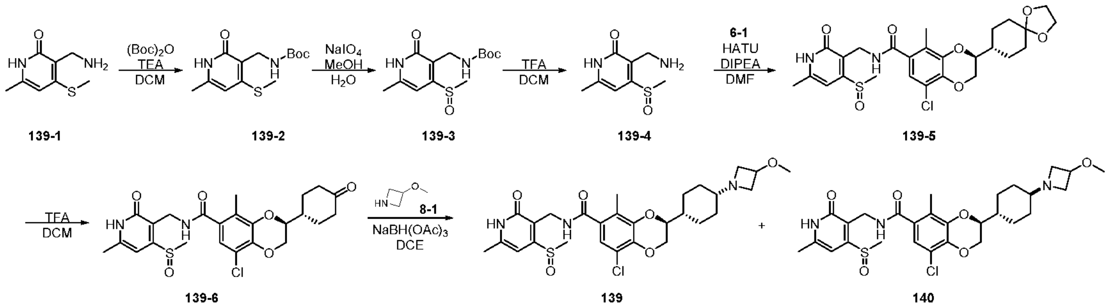


139-1 139-2 139-3 139-4 139-5

139-6 139 + 140

**Step 1, Compound 139-2 Synthesis:**

A single-necked flask was added with substrate **139-1** (184.0 mg, 1.0 mmol). The mixture was dissolved with DCM (3 mL) to dissolve, and then added with **$(Boc)_2O$** (327.0 mg, 1.5 mmol) and TEA (202.0 mg, 2.0 mmol, 279.0 μL), at 25°C stirred to react for 1 hour, with LC-MS monitoring. After the completion of the reaction, the reaction mixture was condensated under reduced pressure, and then extracted with water and EA solution three times. The organic phase was combined, washed with saturated saline solution, and dried with anhydrous sodium sulfate and then condensated under reduced pressure to give the crude product **139-2** (280.0 mg, crude). LCMS ($ESI^+$) m/z: 284.4 $[M+H]^+$

**Step 2, Compound 139-3 Synthesis:**

A single-necked flask was added with substrate **139-2** (100.0 mg, 0.4 mmol). The mixture was dissolved with MeOH (3 mL) and $H_2O$ (1 mL) to dissolve, and then added with **$NaIO_4$** (150.0 mg, 0.7 mmol), at 25°C stirred to react for 36 hours, with LC-MS monitoring. After the completion of the reaction, the reaction mixture was condensated under reduced pressure, and then extracted with water and EA solution three times. The organic phase was combined, washed with saturated saline solution, and dried with anhydrous sodium sulfate and then condensated under reduced pressure to give the crude product **139-3** (63.0 mg, crude). LCMS ($ESI^+$) m/z: 300.4 $[M+H]^+$

**Step 3, Compound 139-4 Synthesis:**

Following the synthesis method of Example **3**, Step **2**, except replacing Step **2, 3-2** with **139-3** (63.0 mg, crude), the same synthesis method was applied, to give Compound **139-4** (40.0 mg, crude), LCMS ($ESI^+$) m/z: 200.3 $[M+H]^+$.

**Step 4, Compound 139-5 Synthesis:**

Following the synthesis method of Example **1**, Step **3**, except replacing Step **3, 1-3** with **6-1** (74.0 mg, 0.2 mmol), **1-4** with **139-4** (40.0 mg, crude), the same synthesis method was applied, to give Compound **139-5** (120.0 mg, crude), LCMS ($ESI^+$) m/z: 551.1 $[M+H]^+$.

**Step 5, Compound 139-6 Synthesis:**

Following the synthesis method of Example **3**, Step **2**, except replacing Step **2, 3-2** with **139-5** (120.0 mg, crude), the same synthesis method was applied, to give Compound **139-6** (120.0 mg, crude), LCMS ($ESI^+$) m/z: 507.0 $[M+H]^+$.

**Step 6, Compound 139 and 140 Synthesis:**

Following the synthesis method of Example **6**, Step **4**, except replacing Step **4, 6-3** with **139-6** (120.0 mg, crude), **dimethylamine** with **8-1** (41.0 mg, 0.5 mmol)the same synthesis method was applied. The obtained compound was subjected to Prep-MPLC to give **139** (25.0 mg, 43.3 μmol, 18.3% yield) and **140** (20.0 mg, 34.6 μmol, 14.6% yield), LCMS ($ESI^+$) m/z: 578.1 $[M+H]^+$.

**Compound 139 NMR: $^1$H NMR** (600 MHz, DMSO-$d_6$) δ 12.04 (s, 1H), 8.37 (t, *J* = 4.8 Hz, 1H), 6.91 (s, 1H), 6.46 (s, 1H), 4.45-4.42 (m, 1H), 4.34-4.31 (m, 1H), 4.15-4.12 (m, 1H), 4.07-4.04 (m, 1H), 3.95-3.90 (m, 2H), 3.46 (s, 2H), 3.14 (s, 3H), 2.74 (s, 3H), 2.69-2.68 (m, 2H), 2.26 (s, 3H), 2.23-2.22 (m, 1H), 2.11 (s, 3H), 1.62-1.24 (m, 9H).

**Compound 140 NMR: $^1$H NMR** (600 MHz, DMSO-$d_6$) δ 12.04 (s, 1H), 8.37 (t, *J* = 4.8 Hz, 1H), 6.91 (s, 1H), 6.46 (s, 1H), 4.44-4.42 (m, 1H), 4.33-4.30 (m, 1H), 4.16-4.13 (m, 1H), 4.08-4.05 (m, 1H), 3.95-3.91 (m, 2H), 3.49-3.47 (m, 2H), 3.14 (s, 3H), 2.73-2.71 (m, 5H), 2.27-2.25 (m, 4H), 2.11 (s, 3H), 1.62-1.24 (m, 9H).

**Example 84, Compound 141 and 142 Synthesis:**

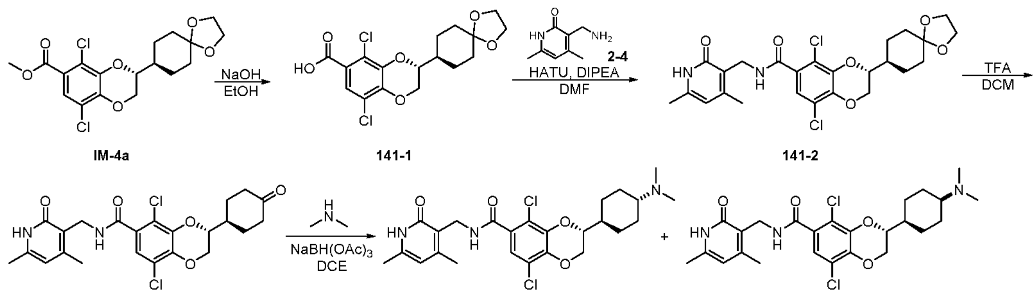


IM-4a 141-1 141-2

141-3 141 142

**Step 1, Compound 141-1 Synthesis:**

Following the synthesis method of Example **6**, Step **1**, except replacing Step **1**, **IM-3a** with **IM-4a** (166.0 mg, 0.4 mmol), the same synthesis method was applied, to give Compound **141-1** (160.0 mg, crude), LCMS (ESI$^+$) m/z: 389.3 [M+H]$^+$.

**Step 2, Compound 141-2 Synthesis:**

Following the synthesis method of Example **1**, Step **3**, except replacing Step **3, 1-3** with **141-1** (160.0 mg, crude), **1-4** with **2-4** (125.0 mg, 0.8 mmol), the same synthesis method was applied, to give Compound **141-2** (200.0 mg, crude), LCMS (ESI$^+$) m/z: 523.4 [M+H]$^+$.

**Step 3, Compound 141-3 Synthesis:**

Following the synthesis method of Example **3**, Step **2**, except replacing Step **2, 3-2** with **141-2** (200.0 mg, 0.4 mmol), the same synthesis method was applied, to give Compound **141-3** (180.0 mg, crude), LCMS (ESI$^+$) m/z: 479.4 [M+H]$^+$.

**Step 4, Compound 141 and 142 Synthesis:**

Following the synthesis method of Example **6**, Step **4**, except replacing Step **4, 6-3** with **141-3** (180.0 mg, crude), the same synthesis method was applied. The obtained compound was subjected to Prep-MPLC to give **141** (55.0 mg, 0.1 mmol, 28.8% yield) and **142** (28.5 mg, 56.1 μmol, 14.9% yield), LCMS (ESI$^+$) m/z: 508.1 [M+H]$^+$.

**Compound 141 NMR: $^1$H NMR** (600 MHz, DMSO-$d_6$) δ 11.48 (s, 1H), 8.30 (t, *J* = 4.8 Hz, 1H), 6.99 (s, 1H), 5.86 (s, 1H), 4.52-4.89 (m, 1H), 4.24 (d, *J* = 4.8 Hz, 2H), 4.19-4.14 (m, 2H), 3.40-3.38 (m, 1H), 2.18 (s, 3H), 2.15 (s, 6H), 2.11 (s, 3H), 2.06-2.03 (m, 1H), 1.89-1.74 (m, 2H), 1.73-1.64 (m, 2H), 1.63-1.53 (m, 2H), 1.44-1.40 (m, 2H).

**Compound 142 NMR: $^1$H NMR** (600 MHz, DMSO-$d_6$) δ 11.48 (s, 1H), 8.30 (t, *J* = 4.8 Hz, 1H), 7.02 (s, 1H), 5.86 (s, 1H), 4.51 (dd, *J* = 11.4, 1.8 Hz, 1H), 4.24 (d, *J* = 5.4 Hz, 2H), 4.17-4.14 (m, 1H), 4.05-4.03 (m, 1H), 3.41-3.39 (m, 1H), 2.28 (s, 6H), 2.22 (s, 3H), 2.18 (s, 3H), 2.10-2.06 (m, 1H), 1.88-1.82 (m, 2H), 1.81-1.78 (m, 1H), 1.59-1.56 (m, 1H), 1.29-1.16 (m, 4H).

**Example 85, Compound 143 and 144 Synthesis:**

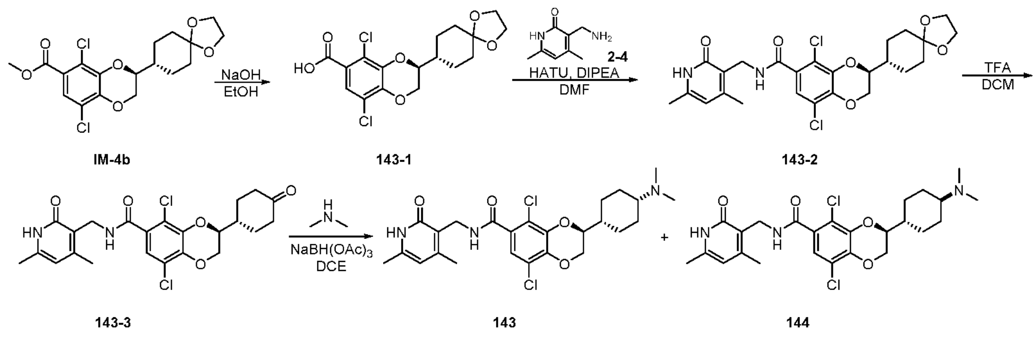


IM-4b, 143-1, 143-2, 143-3, 143, 144

**Step 1, Compound 143-1 Synthesis:**

Following the synthesis method of Example **6**, Step **1**, except replacing Step **1**, **IM-3a** with **IM-4b** (170.0 mg, 0.4 mmol), the same synthesis method was applied, to give Compound **143-1** (160.0 mg, crude), LCMS (ESI$^+$) m/z: 389.3 [M+H]$^+$.

**Step 2, Compound 143-2 Synthesis:**

Following the synthesis method of Example **1**, Step **3**, except replacing Step **3, 1-3** with **143-1** (160.0 mg, crude), **1-4** with **2-4** (125.0 mg, 0.8 mmol), the same synthesis method was applied, to give Compound **143-2** (200.0 mg, crude), LCMS (ESI$^+$) m/z: 523.4 [M+H]$^+$.

**Step 3, Compound 143-3 Synthesis:**

Following the synthesis method of Example **3**, Step **2**, except replacing Step **2, 3-2** with **143-2** (200.0 mg, crude), the same synthesis method was applied, to give Compound **143-3** (180.0 mg, crude), LCMS (ESI$^+$) m/z: 479.4 [M+H]$^+$.

**Step 4, Compound 143 and 144 Synthesis:**

Following the synthesis method of Example **6**, Step **4**, except replacing Step **4, 6-3** with **143-3** (180.0 mg, crude), the same synthesis method was applied. The obtained compound was subjected to Prep-MPLC to give **143** (17.3 mg, 34.0 μmol, 9.1% yield) and **144** (37.0 mg, 72.8 μmol, 19.4% yield), LCMS (ESI$^+$) m/z: 508.1 [M+H]$^+$.

**Compound 143 NMR: $^1$H NMR** (600 MHz, DMSO-$d_6$) δ 11.48 (s, 1H), 8.30 (t, *J* = 4.8 Hz, 1H), 6.99 (s, 1H), 5.86 (s, 1H), 4.52 (dd, *J* = 11.8, 2.2 Hz, 1H), 4.46-4.44 (m, 1H), 4.24 (d, *J* = 4.8 Hz, 2H), 4.19-4.14 (m, 1H), 3.42-3.39 (m, 1H), 2.18 (s, 3H), 2.15 (s, 6H), 2.11 (s, 3H), 2.06-2.04 (m, 1H), 1.87-1.81 (m, 2H), 1.81-1.76 (m, 2H), 1.55 (m, 2H), 1.20-1.11 (m, 2H).

**Compound 144 NMR: $^1$H NMR** (600 MHz, DMSO-$d_6$) δ 11.48 (s, 1H), 8.30 (t, *J* = 4.8 Hz, 1H), 6.99 (s, 1H), 5.86 (s, 1H), 4.52 (d, *J* = 9.8 Hz, 1H), 4.46-4.44 (m, 1H), 4.24 (d, *J* = 4.8 Hz, 2H), 4.19-4.14 (m, 1H), 3.42-3.39 (m, 1H), 2.18 (s, 3H), 2.15 (s, 6H), 2.11 (s, 3H), 2.06-2.03 (m, 1H), 1.82-1.74 (m, 2H), 1.73-1.64 (m, 2H), 1.61-1.52 (m, 2H), 1.43-1.40 (m, 2H).

**Example 86, Compound 145 and 146 Synthesis:**

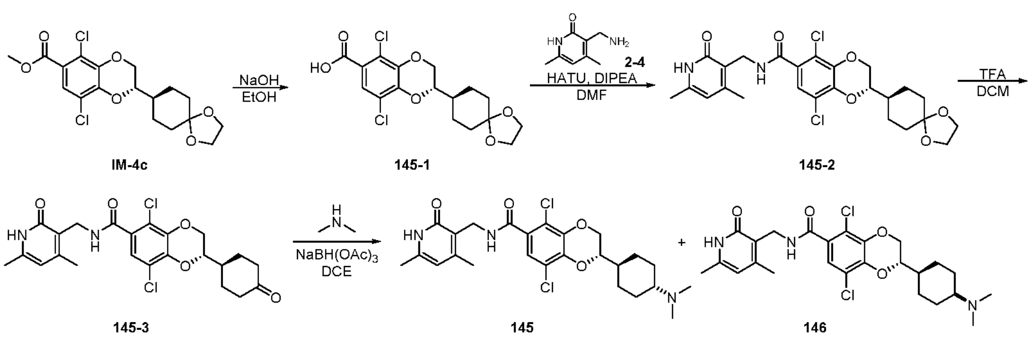


IM-4c, 145-1, 145-2, 145-3, 145, 146

**Step 1, Compound 145-1 Synthesis:**

Following the synthesis method of Example **6**, Step **1**, except replacing Step **1**, **IM-3a** with **IM-4c** (74.0 mg, 0.2 mmol), the same synthesis method was applied, to give Compound **145-1** (70.0 mg, crude), LCMS ($ESI^+$) m/z: 389.3 $[M+H]^+$.

**Step 2, Compound 145-2 Synthesis:**

Following the synthesis method of Example **1**, Step **3**, except replacing Step **3, 1-3** with **145-1** (70.0 mg, crude), **1-4** with **2-4** (33.0 mg, 0.2 mmol), the same synthesis method was applied, to give Compound **145-2** (80.0 mg, crude), LCMS ($ESI^+$) m/z: 523.4 $[M+H]^+$.

**Step 3, Compound 145-3 Synthesis:**

Following the synthesis method of Example **3**, Step **2**, except replacing Step **2, 3-2** with **145-2** (80.0 mg, crude), the same synthesis method was applied, to give Compound **145-3** (65.0 mg, crude), LCMS ($ESI^+$) m/z: 479.4 $[M+H]^+$.

**Step 4, Compound 145 and 146 Synthesis:**

Following the synthesis method of Example **6**, Step **4**, except replacing Step **4, 6-3** with **145-3** (65.0 mg, crude), the same synthesis method was applied. The obtained compound was subjected to Prep-MPLC to give **145** (17.3 mg, 34.0 μmol, 25.1% yield) and **146** (37.0 mg, 72.8 μmol, 53.7% yield), LCMS ($ESI^+$) m/z: 508.1 $[M+H]^+$.

**Compound 145 NMR: $^1$H NMR** (600 MHz, DMSO-$d_6$) δ 11.47 (s, 1H), 8.30 (t, *J* = 4.8 Hz, 1H), 7.01 (s, 1H), 5.86 (s, 1H), 4.52 (dd, *J* = 11.4, 1.8 Hz, 1H), 4.24 (d, *J* = 5.4 Hz, 2H), 4.16-4.13 (m, 1H), 4.06-4.04 (m, 1H), 3.46-3.32 (m, 1H), 2.17 (s, 3H), 2.15 (s, 6H), 2.11 (s, 3H), 2.03-2.01 (m, 1H), 1.85-1.78 (m, 3H), 1.53-1.48 (m, 1H), 1.23-1.14 (m, 4H).

**Compound 146 NMR: $^1$H NMR** (600 MHz, DMSO-$d_6$) δ 11.47 (s, 1H), 8.30 (t, *J* = 4.8 Hz, 1H), 7.01 (s, 1H), 5.86 (s, 1H), 4.51 (dd, *J* = 10.2, 2.2 Hz, 1H), 4.24 (d, *J* = 5.4 Hz, 2H), 4.19-4.16 (m, 2H), 3.46-3.32 (m, 1H), 2.17 (s, 3H), 2.15 (s, 6H), 2.11 (s, 3H), 2.03-1.99 (m, 1H), 1.87-1.76 (m, 2H), 1.60-1.50 (m, 2H), 1.42-1.40 (m, 2H), 1.21-1.09 (m, 2H).

**Example 87, Compound 147 and 148 Synthesis:**

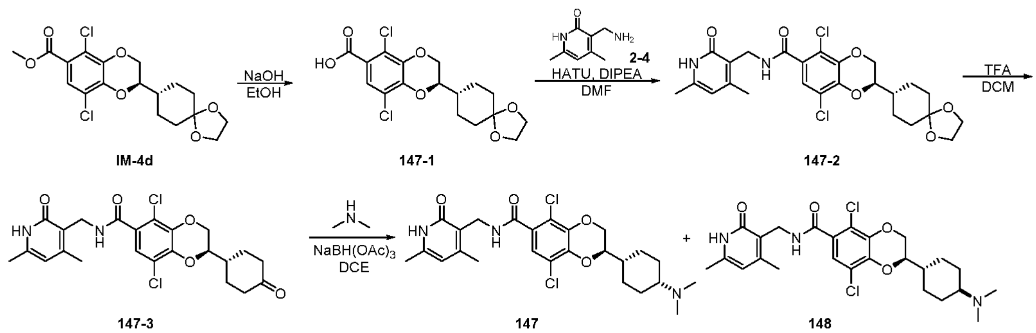


IM-4d 147-1 147-2 147-3 147 148

**Step 1, Compound 147-1 Synthesis:**

Following the synthesis method of Example **6**, Step **1**, except replacing Step **1**, **IM-3a** with **IM-4d** (72.0 mg, 0.2 mmol), the same synthesis method was applied, to give Compound **147-1** (65.0 mg, crude), LCMS ($ESI^+$) m/z: 389.3 $[M+H]^+$.

**Step 2, Compound 147-2 Synthesis:**

Following the synthesis method of Example **1**, Step **3**, except replacing Step **3, 1-3** with **147-1** (65.0 mg, crude), **1-4** with **2-4** (30.0 mg, 0.2 mmol), the same synthesis method was applied, to give Compound **147-2** (80.0 mg, crude), LCMS ($ESI^+$) m/z: 523.4 $[M+H]^+$.

**Step 3, Compound 147-3 Synthesis:**

Following the synthesis method of Example **3**, Step **2**, except replacing Step **2, 3-2** with **147-2** (80.0 mg, crude), the same synthesis method was applied, to give Compound **147-3** (60.0 mg, crude), LCMS (ESI$^+$) m/z: 479.4 [M+H]$^+$.

**Step 4, Compound 147 and 148 Synthesis:**

Following the synthesis method of Example **6,** Step **4,** except replacing Step **4, 6-3** with **147-3** (60.0 mg, crude), the same synthesis method was applied. The obtained compound was subjected to Prep-MPLC to give **147** (17.3 mg, 34.0 μmol, 27.2% yield) and **148** (37.0 mg, 72.8 μmol, 58.2% yield), LCMS (ESI$^+$) m/z: 508.1 [M+H]$^+$.

**Compound 147 NMR: $^1$H NMR** (600 MHz, DMSO-$d_6$) δ 11.48 (s, 1H), 8.30 (t, *J* = 4.8 Hz, 1H), 7.03 (s, 1H), 5.87 (s, 1H), 4.52 (dd, *J* = 12.0, 2.4 Hz, 1H), 4.24 (d, *J* = 4.8 Hz, 2H), 4.16-4.13 (m, 1H), 4.06-4.04 (m, 1H), 3.40-3.38 (m, 1H), 2.18 (s, 3H), 2.15 (s, 6H), 2.11 (s, 3H), 2.04-2.00 (m, 1H), 1.89-1.87 (m, 3H), 1.58-1.55 (m, 1H), 1.28-1.15 (m, 4H).

**Compound 148 NMR: $^1$H NMR** (600 MHz, DMSO-$d_6$) δ 11.48 (s, 1H), 8.29 (t, *J* = 4.8 Hz, 1H), 7.01 (s, 1H), 5.87 (s, 1H), 4.52 (dd, *J* = 11.6, 2.2 Hz, 1H), 4.25 (d, *J* = 4.8 Hz, 2H), 4.19-4.17 (m, 2H), 3.40-3.38 (m, 1H), 2.18 (s, 3H), 2.15 (s, 6H), 2.11 (s, 3H), 2.04-2.00 (m, 1H), 1.89-1.87 (m, 2H), 1.86-1.77 (m, 2H), 1.58-1.55 (m, 2H), 1.48-1.45 (m, 2H).

**Example 88, Compound 149 and 150 Synthesis:**

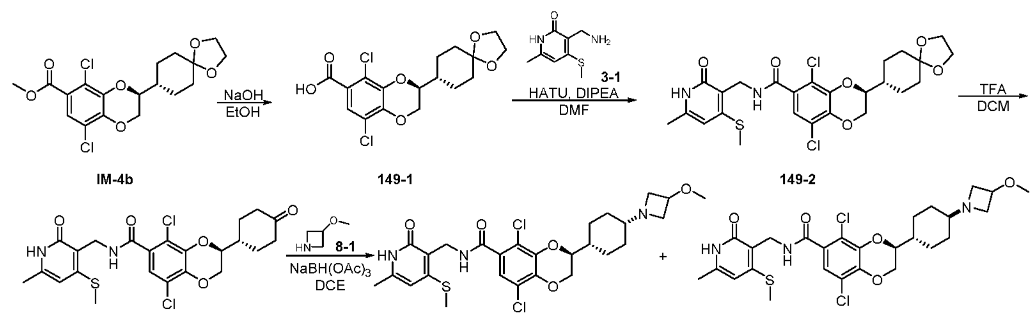


IM-4b 149-1 149-2 149-3 149 150

**Step 1, Compound 149-1 Synthesis:**

Following the synthesis method of Example **6,** Step **1,** except replacing Step **1, IM-3a** with **IM-4b** (200.0 mg, 0.5 mmol), the same synthesis method was applied, to give Compound **149-1** (200.0 mg, crude), LCMS (ESI$^+$) m/z: 389.3 [M+H]$^+$.

**Step 2, Compound 149-2 Synthesis:**

Following the synthesis method of Example **1,** Step **3,** except replacing Step **3, 1-3** with **149-1** (200.0 mg, crude), **1-4** with **3-1** (113.0 mg, 0.6 mmol), the same synthesis method was applied, to give Compound **149-2** (200.0 mg, crude), LCMS (ESI$^+$) m/z: 555.5 [M+H]$^+$.

**Step 3, Compound 149-3 Synthesis:**

Following the synthesis method of Example **3,** Step **2,** except replacing Step **2, 3-2** with **149-2** (200.0 mg, crude), the same synthesis method was applied, to give Compound **149-3** (200.0 mg, crude), LCMS (ESI$^+$) m/z: 511.4 [M+H]$^+$.

**Step 4, Compound 149 and 150 Synthesis:**

Following the synthesis method of Example **6,** Step **4,** except replacing Step **4, 6-3** with **149-3** (200.0 mg, crude), **dimethylamine** with **8-1** (103.0 mg, 1.2 mmol), the same synthesis method was applied. The obtained compound was subjected to Prep-MPLC to give **149** (27.3 mg, 46.9 μmol, 12.0% yield) and **150** (61.0 mg, 0.1 mmol, 26.8% yield), LCMS (ESI$^+$) m/z: 582.2 [M+H]$^+$.

**Compound 149 NMR: $^1$H NMR** (600 MHz, DMSO-$d_6$) δ 11.54 (s, 1H), 8.19 (t, *J* = 4.8 Hz, 1H), 6.98 (s, 1H), 6.08 (s, 1H), 4.50-4.48 (m, 1H), 4.28 (d, *J* = 4.2 Hz, 2H), 4.16-4.13 (m, 1H), 4.07-4.05 (m, 1H), 3.93-3.90 (m, 1H), 3.47 (q, *J* = 6.0 Hz,

2H), 3.14 (s, 3H), 2.67 (s, 2H), 2.45 (s, 3H), 2.24-2.20 (m, 1H), 2.17 (s, 3H), 1.68-1.52 (m, 4H), 1.51-1.28 (m, 5H).

**Compound 150 NMR:** δ 11.54 (s, 1H), 8.19 (t, *J* = 4.2 Hz, 1H), 7.00 (s, 1H), 6.09 (s, 1H), 4.52 (d, *J* = 9.6 Hz, 1H), 4.28 (d, *J* = 4.2 Hz, 2H), 4.15-4.12 (m, 1H), 4.05-4.02 (m, 1H), 3.91-3.87 (m, 1H), 3.47-3.43 (m, 2H), 3.14 (s, 3H), 2.76-2.73 (m, 2H), 2.46 (s, 3H), 2.17 (s, 3H), 1.98-1.94 (m, 1H), 1.80-1.70 (m, 3H), 1.57-1.53 (m, 1H), 1.31-1.09 (m, 3H), 0.94-0.83 (m, 2H).

**Example 89, Compound 151 and 152 Synthesis:**

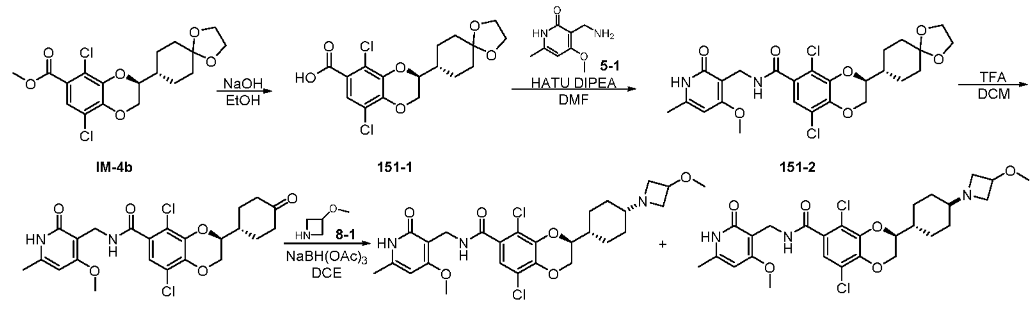


IM-4b 151-1 151-2

151-3 151 152

**Step 1, Compound 151-1 Synthesis:**

Following the synthesis method of Example **6,** Step **1,** except replacing Step **1, IM-3a** with **IM-4b** (200.0 mg, 0.5 mmol), the same synthesis method was applied, to give Compound **151-1** (200.0 mg, crude), LCMS (ESI$^+$) m/z: 389.3 [M+H]$^+$.

**Step 2, Compound 151-2 Synthesis:**

Following the synthesis method of Example **1,** Step **3,** except replacing Step **3, 1-3** with **151-1** (200.0 mg, crude), **1-4** with **5-1** (100.0 mg, 0.6 mmol), the same synthesis method was applied, to give Compound **151-2** (200.0 mg, crude), LCMS (ESI$^+$) m/z: 539.1 [M+H]$^+$.

**Step 3, Compound 151-3 Synthesis:**

Following the synthesis method of Example **3,** Step **2,** except replacing Step **2, 3-2** with **151-2** (200.0 mg, crude), the same synthesis method was applied, to give Compound **151-3** (200.0 mg, crude), LCMS (ESI$^+$) m/z: 495.1 [M+H]$^+$.

**Step 4, Compound 151 and 152 Synthesis:**

Following the synthesis method of Example **6,** Step **4,** except replacing Step **4, 6-3** with **151-3** (200.0 mg, crude), **dimethylamine** with **8-1** (103.0 mg, 1.2 mmol), the same synthesis method was applied. The obtained compound was subjected to Prep-MPLC to give **151** (4.0 mg, 7.1 μmol, 1.7% yield) and **152** (11.0 mg, 19.4 μmol, 4.8% yield), LCMS (ESI$^+$) m/z: 566.2 [M+H]$^+$.

**Compound 151 NMR: $^1$H NMR** (600 MHz, DMSO-$d_6$) δ 11.43 (s, 1H), 8.09 (t, *J* = 4.8 Hz, 1H), 6.99 (s, 1H), 6.09 (s, 1H), 4.52-4.51 (m, 1H), 4.19 (d, *J* = 4.8 Hz, 2H), 4.15-4.02 (m, 2H), 3.94-3.90 (m, 1H), 3.80 (s, 3H), 3.52-3.50 (m, 2H), 3.15 (s, 3H), 2.81-2.79 (m, 2H), 2.18 (s, 3H), 1.99-1.96 (m, 2H), 1.80-1.72 (m, 3H), 1.57-1.56 (m, 1H), 1.26-1.14 (m, 3H), 0.96-0.84 (m, 2H).

**Compound 152 NMR: $^1$H NMR** (600 MHz, DMSO-$d_6$) δ 11.43 (s, 1H), 8.09 (t, *J* = 4.2 Hz, 1H), 6.98 (s, 1H), 6.09 (s, 1H), 4.50 (d, *J* = 10.8 Hz, 1H), 4.19 (d, *J* = 4.2 Hz, 2H), 4.16-4.12 (m, 1H), 4.07-4.04 (m, 1H), 3.94-3.90 (m, 1H), 3.80 (s, 3H), 3.46-3.44 (m, 2H), 3.14 (s, 3H), 2.68-2.66 (m, 2H), 2.23-2.21 (m, 1H), 2.18 (s, 3H), 1.65-1.57 (m, 4H), 1.51-1.39 (m, 5H).

**Example 90, Compound 153 and 154 Synthesis:**

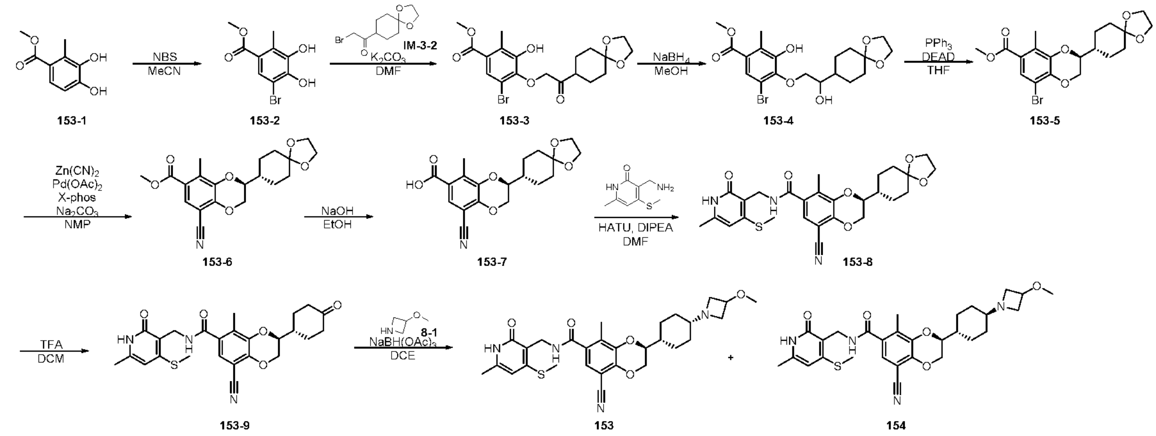


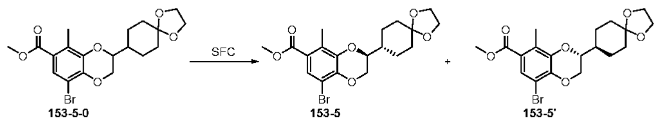


**Step 1, Compound 153-2 Synthesis:**

A single-necked flask was added with substrate **153-1** (5.4 g, 29.6 mmol). The mixture was dissolved with ACN (50 mL) to dissolve, and then added with **NBS** (5.8 g, 32.0 mmol), at 25°C stirred to react for 3 hours, with LC-MS monitoring. After the completion of the reaction, the reaction mixture was condensated under reduced pressure, and then extracted with water and EA solution three times. The organic phase was combined, washed with saturated saline solution, and dried with anhydrous sodium sulfate and then condensated under reduced pressure to give the crude product **153-2** (9.8 g, crude). LCMS (ESI$^+$) m/z: 261.0 $[M+H]^+$

**Step 2, Compound 153-3 Synthesis:**

Following the synthesis method of Example **2,** Step **1,** except replacing Step **1, IM-2** with **IM-3-2** (8.5 g, 32.3 mmol), **2-1** with **153-2** (7.7 g, 29.5 mmol), the same synthesis method was applied, to give Compound **153-3** (12.6 g, crude). LCMS (ESI$^+$) m/z: 443.3 $[M+H]^+$

**Step 3, Compound 153-4 Synthesis:**

Following the synthesis method of Example **IM-3,** Step **3,** except replacing Step **3, IM-3-4** with **153-3** (12.6 g, crude), the same synthesis method was applied, to give Compound **153-4** (7.0 g, 15.7 mmol, 55.1% yield). LCMS (ESI$^+$) m/z: 445.3 $[M+H]^+$

**Step 4, Compound 153-5 Synthesis:**

Following the synthesis method of Example **IM-3,** Step **4,** except replacing Step **4, IM-3-5** with **153-4** (7.0 g, 15.7 mmol), the same synthesis method was applied, to give Compound **153-5-0** which was separated by SFC to give Compound **153-5** (2.4 g, 5.6 mmol, 35.8% yield) and Compound **153-5'**(2.4 g, 5.5 mmol, 35.8% yield). LCMS (ESI$^+$) m/z: 427.3 $[M+H]^+$

**Step 5, Compound 153-6 Synthesis:**

A single-necked flask was added with substrate **153-5** (213.7 mg, 0.5 mmol). The mixture was dissolved with NMP (5 mL) to dissolve, and then added with $Zn(CN)_2$ (117.7 mg, 1.0 mmol), $Pd(OAc)_2$ (22.5 mg, 0.1 mmol), X-phos (95.5 mg,0.2 mmol) and $Na_2CO_3$ (53.0 mg, 0.5 mmol), and under nitrogen protection and at 100°C stirred to react for 2 hours, with LC-MS monitoring. After the completion of the reaction, the reaction mixture was cooled to room temperature, and condensated under reduced pressure, and then purified by medium pressure liquid chromatography to give the product **153-6** (165.0 mg, 0.4 mmol, 88.4% yield). LCMS (ESI$^+$) m/z: 373.4 $[M+H]^+$

**Step 6, Compound 153-7 Synthesis:**

Following the synthesis method of Example **6,** Step **1,** except replacing Step **1, IM-3a** with **153-6** (165.0 mg, 0.4 mmol), the same synthesis method was applied, to give Compound **153-7** (159.0 mg, crude), LCMS (ESI$^+$) m/z: 359.3 $[M+H]^+$.

**Step 7, Compound 153-8 Synthesis:**

Following the synthesis method of Example **1,** Step **3,** except replacing Step **3, 1-3** with **153-7** (159.0 mg, crude), **1-4** with **3-1** (150.0 mg, 0.9 mmol), the same synthesis method was applied, to give Compound **153-8** (255.0 mg, crude), LCMS (ESI$^+$) m/z: 525.6 $[M+H]^+$.

**Step 8, Compound 153-9 Synthesis:**

Following the synthesis method of Example **3,** Step **2,** except replacing Step **2, 3-2** with **153-8** (255.0 mg, crude), the same synthesis method was applied, to give Compound **153-9** (122.0 mg, crude), LCMS (ESI$^+$) m/z: 481.5 $[M+H]^+$.

**Step 9, Compound 153 and 154 Synthesis:**

Following the synthesis method of Example **6,** Step **4,** except replacing Step **4, 6-3** with **153-9** (48.0 mg, 0.1 mmol), **dimethylamine** with **8-1** (17.0 mg, 0.2 mmol), the same synthesis method was applied. The obtained compound was subjected to Prep-MPLC to give **153** (8.0 mg, 14.5 μmol, 14.5% yield) and **154** (14.0 mg, 25.3 μmol, 25.4% yield), LCMS (ESI$^+$) m/z: 553.4 $[M+H]^+$.

**Compound 153 NMR: $^1$H NMR** (600 MHz, DMSO-$d_6$) δ 11.53 (s, 1H), 8.10 (t, *J* = 4.2 Hz, 1H), 7.17 (s, 1H), 6.08 (s, 1H), 4.54-4.52 (m, 1H), 4.28 (d, *J* = 4.2 Hz, 2H), 4.15-4.12 (m, 1H), 3.98-3.96 (m, 1H), 3.89-3.87 (m, 1H), 3.45-3.42 (m, 2H), 3.13 (s, 3H), 2.72-2.70 (m, 2H), 2.45 (s, 3H), 2.21 (s, 3H), 2.17 (s, 3H), 1.95-1.88 (m, 2H), 1.77-1.71 (m, 3H), 1.56-1.55 (m, 1H), 1.19-1.14 (m, 2H), 0.89-0.85 (m, 2H).

**Compound 154 NMR: $^1$H NMR** (600 MHz, DMSO-$d_6$) δ 11.53 (s, 1H), 8.10 (t, *J* = 4.2 Hz, 1H), 7.16 (s, 1H), 6.08 (s, 1H), 4.51-4.50 (m, 1H), 4.28 (d, *J* = 4.2 Hz, 2H), 4.16-4.13 (m, 1H), 4.01-3.99 (m, 1H), 3.92-3.89 (m, 1H), 3.45-3.44 (m, 2H), 3.14 (s, 3H), 2.68-2.66 (m, 2H), 2.45 (s, 3H), 2.21 (s, 3H), 2.17 (s, 3H), 1.66-1.20 (m, 10H).

**Example 91, Compound 155 and 156 Synthesis:**

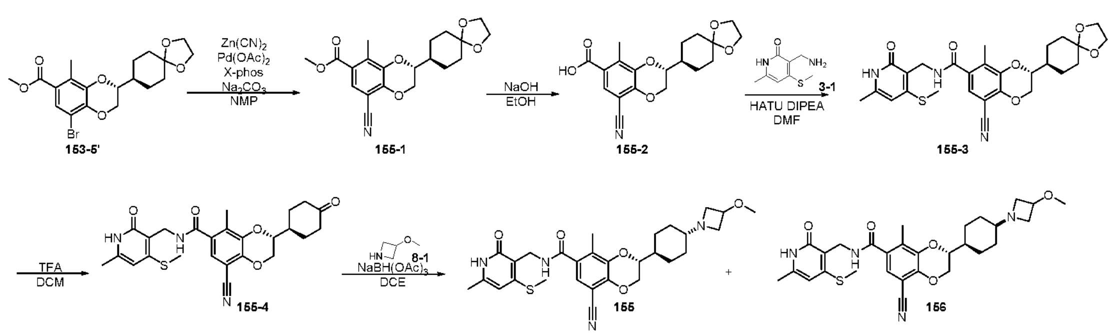


**Step 1, Compound 155-1 Synthesis:**

A single-necked flask was added with substrate **153-5'** (214.0 mg, 0.5 mmol). The mixture was dissolved with NMP (5 mL) to dissolve, and then added with $Zn(CN)_2$ (117.7 mg, 1.0 mmol), $Pd(OAc)_2$ (22.5 mg, 0.1 mmol), X-phos (95.5 mg,0.2 mmol) and $Na_2CO_3$ (53.0 mg, 0.5 mmol), and under nitrogen protection and at 100°C stirred to react for 2 hours, with LC-MS monitoring. After the completion of the reaction, the reaction mixture was cooled to room temperature, and condensated under reduced pressure, and then purified by medium pressure liquid chromatography to give the product **155-1** (165.0 mg, 0.4 mmol, 88.4%). LCMS (ESI$^+$) m/z: 373.4 $[M+H]^+$

**Step 2, Compound 155-2 Synthesis:**

Following the synthesis method of Example **6,** Step **1,** except replacing Step **1, IM-3a** with **155-1** (165.0 mg, 0.4 mmol), the same synthesis method was applied, to give Compound **155-2** (159.0 mg, crude), LCMS (ESI$^+$) m/z: 359.3 $[M+H]^+$.

**Step 3, Compound 155-3 Synthesis:**

Following the synthesis method of Example **1,** Step **3,** except replacing Step **3, 1-3** with **155-2** (159.0 mg, crude), **1-4** with **3-1** (150.0 mg, 0.9 mmol), the same synthesis method was applied, to give Compound **155-3** (255.0 mg, crude), LCMS (ESI$^+$) m/z: 525.6 $[M+H]^+$.

**Step 4, Compound 155-4 Synthesis:**

Following the synthesis method of Example **3,** Step **2,** except replacing Step **2, 3-2** with **155-3** (255.0 mg, crude), the same synthesis method was applied, to give Compound **155-4** (122.0 mg, crude), LCMS (ESI$^+$) m/z: 481.5 $[M+H]^+$.

**Step 5, Compound 155 and 156 Synthesis:**

Following the synthesis method of Example **6,** Step **4,** except replacing Step **4, 6-3** with **155-4** (48.0 mg, 0.1 mmol), **dimethylamine** with **8-1** (17.0 mg, 0.2 mmol), the same synthesis method was applied. The obtained compound was subjected to Prep-MPLC to give **155** (8.0 mg, 14.5 μmol, 14.5% yield) and **156** (14.0 mg, 25.3 μmol, 25.4% yield), LCMS (ESI$^+$) m/z: 553.3 $[M+H]^+$.

**Compound 155 NMR: $^1$H NMR** (600 MHz, DMSO-$d_6$) δ 11.53 (s, 1H), 8.12 (t, $J$ = 4.2 Hz, 1H), 7.16 (s, 1H), 6.08 (s, 1H), 4.55-4.50 (m, 1H), 4.28 (d, $J$ = 4.2 Hz, 2H), 4.17-4.11 (m, 1H), 4.03-3.97 (m, 1H), 3.94-3.89 (m, 1H), 3.47-3.41 (m, 2H), 3.13 (s, 3H), 2.70-2.67 (m, 2H), 2.45 (s, 3H), 2.21 (s, 3H), 2.17 (s, 3H), 1.57-1.38 (m, 10H).

**Compound 156 NMR: $^1$H NMR** (600 MHz, DMSO-$d_6$) δ 11.53 (s, 1H), 8.14 (t, $J$ = 4.2 Hz 1H), 7.16 (s, 1H), 6.08 (s, 1H), 4.54-4.50 (m, 1H), 4.29 (d, $J$ = 4.6 Hz, 2H), 4.18-4.03 (m, 1H), 3.98-3.96 (m, 1H), 3.90-3.88 (m, 1H), 3.46-3.42 (m, 2H), 3.13 (s, 3H), 2.72-2.68 (m, 2H), 2.45 (s, 3H), 2.21 (s, 3H), 2.17 (s, 3H), 1.95-1.84 (m, 2H), 1.79-1.70 (m, 3H), 1.58-1.52 (m, 1H), 1.19-1.12 (m, 2H), 0.91-0.82 (m, 2H).

**Example 92, Compound 157 and 158 Synthesis:**

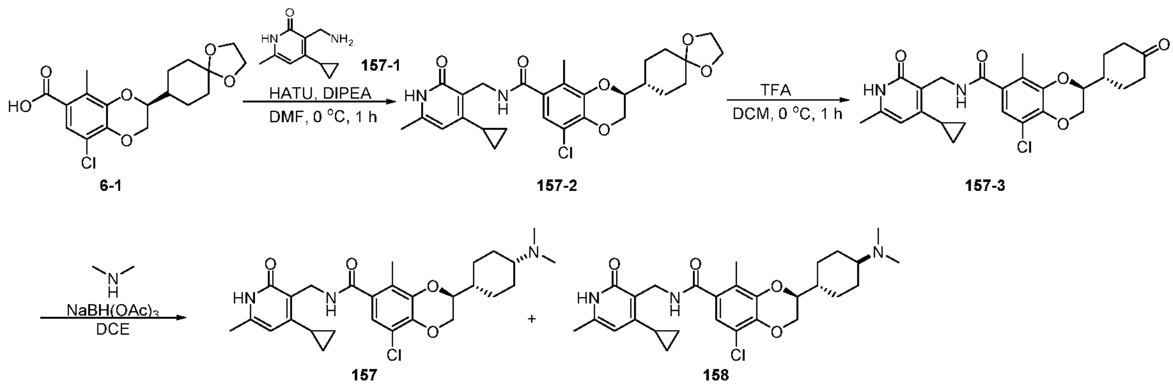


6-1, 157-2, 157-3

157 + 158

**Step 1, Compound 157-2 Synthesis:**

Following the synthesis method of Example **1,** Step **3,** except replacing Step **3, 1-3** with **6-1** (73.0 mg, 0.2 mmol), **1-4** with **157-1** (107.0 mg, 0.6 mmol), the same synthesis method was applied, to give Compound **157-2** (248.0 mg, crude), LCMS (ESI$^+$) m/z: 543.1 $[M+H]^+$.

**Step 2, Compound 157-3 Synthesis:**

Following the synthesis method of Example **3,** Step **2,** except replacing Step **2, 3-2** with **157-2** (248.0 mg, crude),

the same synthesis method was applied, to give Compound **157-3** (150.0 mg, crude), LCMS (ESI+) m/z: 499.0 [M+H]+.

**Step 3, Compound 157 and 158 Synthesis:**

Following the synthesis method of Example **6,** Step **4,** except replacing Step **4, 6-3** with **157-3** (150.0 mg, crude), the same synthesis method was applied. The obtained compound was subjected to Prep-MPLC to give **157** (22.0 mg, 42.8 μmol, 13.8% yield) and **158** (28.0 mg, 54.5 μmol, 17.6% yield), LCMS (ESI+) m/z: 514.2 [M+H]+.

**Compound 157 NMR: $^1$H NMR** (600 MHz, DMSO-$d_6$) δ 11.48 (s, 1H), 8.10 (t, *J* = 4.8 Hz, 1H), 6.88 (s, 1H), 5.69 (s, 1H), 4.48-4.43 (m, 1H), 4.21 (d, *J* = 4.8 Hz, 2H), 4.09-4.03 (m, 1H), 4.02-3.88 (m, 1H), 2.19-2.13 (m, 9H), 2.11 (s, 3H), 2.03-1.98 (m, 1H), 1.85-1.81 (m, 2H), 1.80-1.74 (m, 2H), 1.59-1.50 (m, 1H), 1.35-1.10 (m, 5H), 0.95-0.90 (m, 2H), 0.79-0.74 (m, 2H).

**Compound 158 NMR: $^1$H NMR** (600 MHz, DMSO-$d_6$) δ 11.49 (s, 1H), 8.11 (t, *J* = 4.8 Hz, 1H), 6.88 (s, 1H), 5.69 (s, 1H), 4.44 (d, *J* = 10.4 Hz, 1H), 4.21 (d, *J* = 4.8 Hz, 2H), 4.10-4.03 (m, 2H), 2.18-2.12 (m, 9H), 2.11 (s, 3H), 2.05-2.01 (m, 1H), 1.82-1.74 (m, 3H), 1.72-1.63 (m, 2H), 1.60-1.52 (m, 2H), 1.46-1.36 (m, 3H), 0.96-0.90 (m, 2H), 0.79-0.74 (m, 2H).

**Example 93, Compound 159 and 160 Synthesis:**

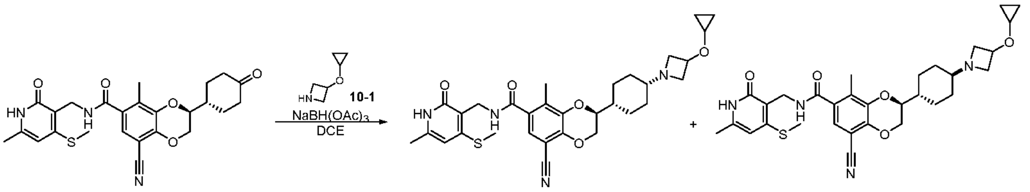


153-9 159 160

**Step 1, Compound 159 and 160 Synthesis:**

Following the synthesis method of Example **6,** Step **4,** except replacing Step **4, 6-3** with **153-9** (67.0 mg, 0.1 mmol), **dimethylamine** with **10-1** (32.0 mg, 0.3 mmol), the same synthesis method was applied. The obtained compound was subjected to Prep-MPLC to give **159** (8.0 mg, 13.8 μmol, 9.9% yield) and 160 (16.0 mg, 27.6 μmol, 19.9% yield), LCMS (ESI+) m/z: 579.3 [M+H]+.

**Compound 159 NMR: $^1$H NMR** (600 MHz, DMSO-$d_6$) δ 11.53 (s, 1H), 8.09 (t, *J* = 4.8 Hz,, 1H), 7.17 (s, 1H), 6.08 (s, 1H), 4.57-4.48 (m, 1H), 4.28 (d, *J* = 4.2 Hz, 2H), 4.17-4.11 (m, 1H), 4.09-4.03 (m, 1H), 3.99-3.94 (m, 1H), 3.50-3.42 (m, 2H), 3.21-3.17 (m, 1H), 2.77-2.71 (m, 2H), 2.45 (s, 3H), 2.21 (s, 3H), 2.17 (s, 3H), 1.96-1.84 (m, 2H), 1.79-1.68 (m, 3H), 1.60-1.51 (m, 1H), 1.21-1.11 (m, 2H), 0.92-0.81 (m, 2H), 0.47-0.42 (m, 2H), 0.41-0.36 (m, 2H).

**Compound 160 NMR: $^1$H NMR** (600 MHz, DMSO-$d_6$) δ 11.53 (s, 1H), 8.12 (t, *J* = 4.2 Hz, 1H), 7.16 (s, 1H), 6.08 (s, 1H), 4.53-4.47 (m, 1H), 4.28 (d, *J* = 4.8 Hz, 2H), 4.16-4.12 (m, 1H), 4.11-4.07 (m, 1H), 4.04-3.97 (m, 1H), 3.50-3.44 (m, 2H), 3.24-3.18 (m, 1H), 2.73-2.66 (m, 2H), 2.45 (s, 3H), 2.21 (s, 3H), 2.17 (s, 3H), 1.65-1.24 (m, 10H), 0.48-0.43 (m, 2H), 0.41-0.37 (m, 2H).

**Example 94, Compound 161 and 162 Synthesis:**

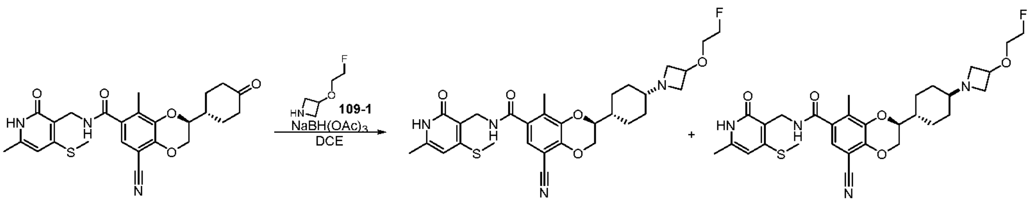


153-9 161 162

**Step 1, Compound 161 and 162 Synthesis:**

Following the synthesis method of Example **6,** Step **4,** except replacing Step **4, 6-3** with **153-9** (96.0 mg, 0.2

mmol), **dimethylamine** with **109-1** (48.0 mg, 0.4 mmol), the same synthesis method was applied. The obtained compound was subjected to Prep-MPLC to give **161** (8.0 mg, 13.7 umol, 6.9% yield) and **162** (16.0 mg, 27.4 μmol, 13.8% yield), LCMS ($ESI^+$) m/z: 585.4 $[M+H]^+$.

**Compound 161 NMR: $^1$H NMR** (600 MHz, DMSO-$d_6$) δ 11.53 (s, 1H), 8.15 (t, *J* = 4.2 Hz,, 1H), 7.17 (s, 1H), 6.08 (s, 1H), 4.57-4.50 (m, 2H), 4.47-4.41 (m, 1H), 4.28 (d, *J* = 4.2 Hz, 2H), 4.18-4.11 (m, 1H), 4.08-4.00 (m, 1H), 4.00-3.94 (m, 1H), 3.60-3.55 (m, 1H), 3.55-3.50 (m, 1H), 3.50-3.44 (m, 2H), 2.80-2.71 (m, 2H), 2.45 (s, 3H), 2.21 (s, 3H), 2.17 (s, 3H), 1.98-1.86 (m, 2H), 1.81-1.69 (m, 3H), 1.60-1.50 (m, 1H), 1.22-1.10 (m, 2H), 0.96-0.80 (m, 2H).

**Compound 162 NMR: $^1$H NMR** (600 MHz, DMSO-$d_6$) δ 11.53 (s, 1H), 8.14 (t, *J* = 4.2 Hz,, 1H ), 7.17 (s, 1H), 6.08 (s, 1H), 4.59-4.49 (m, 2H), 4.47-4.43 (m, 1H), 4.28 (d, *J* = 4.4 Hz, 2H), 4.20-4.12 (m, 1H), 4.11-4.00 (m, 2H), 3.59 (m, 1H), 3.54 (m, 1H), 3.48 (m, 2H), 2.70 (m, 2H), 2.45 (s, 3H), 2.21 (s, 3H), 2.17 (s, 3H), 1.68-1.23 (m, 10H).

**Example 95, Compound 163 and 164 Synthesis:**

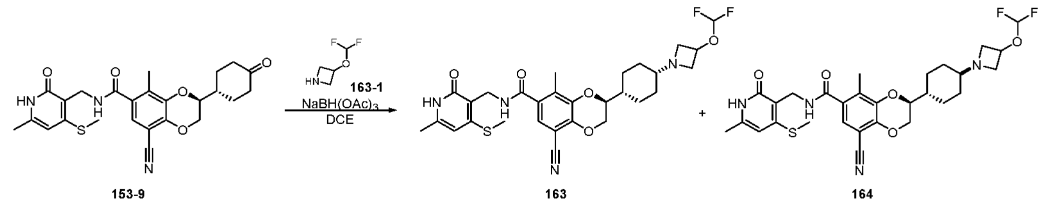


**Step 1, Compound 163 and 164 Synthesis:**

Following the synthesis method of Example **6,** Step **4,** except replacing Step **4, 6-3** with **153-9** (72.0 mg, 0.15 mmol), **dimethylamine** with **163-1** (37.0 mg, 0.3 mmol), the same synthesis method was applied. The obtained compound was subjected to Prep-MPLC to give **163** (6.0 mg, 10.2 μmol, 6.8% yield) and **164** (23.0 mg, 39.1 μmol, 26.1% yield), LCMS ($ESI^+$) m/z: 589.2 $[M+H]^+$.

**Compound 163 NMR: $^1$H NMR** (600 MHz, DMSO-$d_6$) δ 11.53 (s, 1H), 8.14 (t, *J* = 4.2 Hz, 1H), 7.17 (s, 1H), 6.80-6.78 (m, 1H), 6.08 (s, 1H), 4.68-4.59 (m, 1H), 4.56-4.49 (m, 1H), 4.28 (d, *J* = 4.4 Hz, 2H), 4.19-4.11 (m, 1H), 4.01-3.95 (m, 1H), 3.56-3.48 (m, 2H), 2.93-2.89 (m, 2H), 2.45 (s, 3H), 2.21 (s, 3H), 2.17 (s, 3H), 1.97-1.92 (m, 2H), 1.80-1.69 (m, 3H), 1.27-1.11 (m, 3H), 0.94-0.82 (m, 2H).

**Compound 164 NMR: $^1$H NMR** (600 MHz, DMSO-$d_6$) δ 11.53 (s, 1H), 8.10 (t, *J* = 4.2 Hz, 1H), 7.17 (s, 1H), 6.79-6.54 (m, 1H), 6.08 (s, 1H), 4.70-4.62 (m, 1H), 4.52-4.49 (m, 1H), 4.28 (d, *J* = 4.2 Hz, 2H), 4.18-4.12 (m, 1H), 4.03-3.96 (m, 1H), 3.58-3.48 (m, 2H), 2.91-2.82 (m, 2H), 2.45 (s, 3H), 2.21 (s, 3H), 2.17 (s, 3H), 1.68-1.30 (m, 10H).

**Example 96, Compound 165 and 166 Synthesis:**

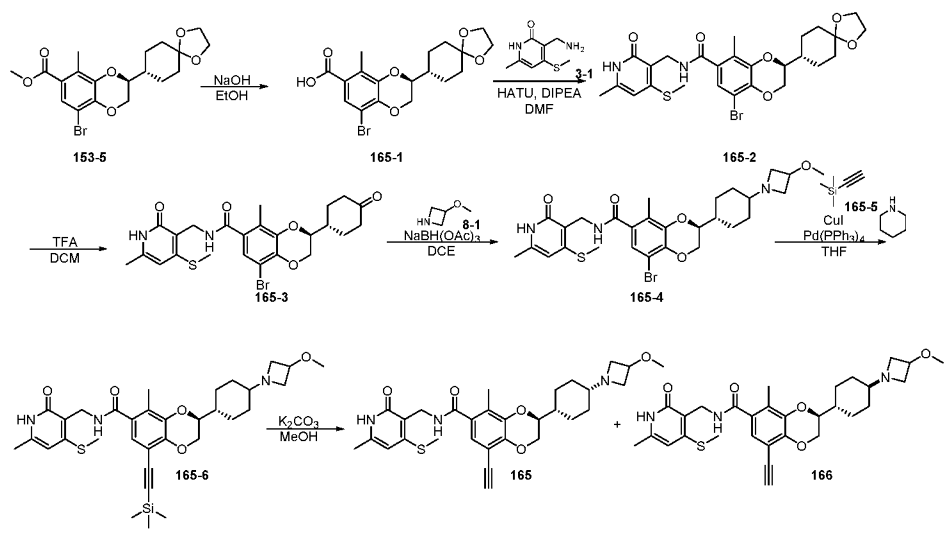

**Step 1, Compound 165-1 Synthesis:**

Following the synthesis method of Example **6,** Step **1,** except replacing Step **1, IM-3a** with **153-5** (214.0 mg, 0.5 mmol), the same synthesis method was applied, to give Compound **165-1** (210.0 mg, crude), LCMS ($ESI^+$) m/z: 413.6 $[M+H]^+$.

**Step 2, Compound 165-2 Synthesis:**

Following the synthesis method of Example **1,** Step **3,** except replacing Step **3, 1-3** with **165-1** (210.0 mg, crude), **1-4** with **3-1** (184.0 mg, 1.0 mmol), the same synthesis method was applied, to give Compound **165-2** (352.0 mg, crude), LCMS ($ESI^+$) m/z: 579.5 $[M+H]^+$.

**Step 3, Compound 165-3 Synthesis:**

Following the synthesis method of Example **3,** Step **2,** except replacing Step **2, 3-2** with **165-2** (352.0 mg, crude), the same synthesis method was applied, to give Compound **165-3** (453.0 mg, crude), LCMS ($ESI^+$) m/z: 535.5 $[M+H]^+$.

**Step 4, Compound 165-4 Synthesis:**

Following the synthesis method of Example **6,** Step **4,** except replacing Step **4, 6-3** with **165-3** (453.0 mg, crude), **dimethylamine** with **8-1** (147.0 mg, 1.7 mmol), the same synthesis method was applied, to give Compound **165-4** (283.0 mg, 0.5 mmol, 55.1% yield), LCMS ($ESI^+$) m/z: 606.6 $[M+H]^+$

**Step 5, Compound 165-6 Synthesis:**

A single-necked flask was added with substrate **165-4** (283.0 mg, 0.5 mmol). The mixture was dissolved with THF (2 mL) and piperidine (2.3 mL) to dissolve, and then added with **165-5** (98.2 mg, 1.0 mmol), CuI (44.0 mg, 0.2 mmol) and $Pd(PPh_3)_4$ (270.0 mg, 0.2 mmol), under nitrogen protection heated to stirred at 50°C to react for 48 hours, with LC-MS monitoring. After the completion of the reaction, the reaction mixture was cooled to room temperature, the reaction mixture was condensated under reduced pressure, and then extracted with water and EA solution three times. The organic phase was combined, washed with saturated saline solution, and dried with anhydrous sodium sulfate and then condensated under reduced pressure, and then purified by medium pressure liquid chromatography to give the product **165-6** (150.0 mg, 0.2 mmol, 51.5% yield). LCMS ($ESI^+$) m/z: 623.9 $[M+H]^+$

**Step 6, Compound 165 and 166 Synthesis:**

A single-necked flask was added with substrate **165-6** (150.0 mg, 0.2 mmol). The mixture was dissolved with MeOH (2 mL) to dissolve, and then added with $K_2CO_3$ (69.0 mg, 0.5 mmol), at 25°C stirred to react for 1 hour, with LC-MS monitoring. After the completion of the reaction, the reaction mixture was condensated under reduced pressure, and then purified by medium pressure liquid chromatography to give the product **165** (18.0 mg, 0.03 mmol, 13.6% yield) and **166** (26.0 mg, 0.05 mmol, 19.6% yield). LCMS ($ESI^+$) m/z: 552.3 $[M+H]^+$

**Compound 165 NMR: $^1$H NMR** (600 MHz, DMSO-$d_6$) δ 11.51 (s, 1H), 7.99 (t, *J* = 4.2 Hz, 1H), 6.90 (s, 1H), 6.07 (s, 1H), 4.46-4.40 (m, 1H), 4.26 (d, *J* = 4.6 Hz, 2H), 4.21 (s, 1H), 4.05-3.99 (m, 1H), 3.93-3.83 (m, 2H), 3.45-3.42 (m, 2H), 3.13 (s, 3H), 2.72-2.69 (m, 2H), 2.45 (s, 3H), 2.16 (m, 6H), 1.96-1.91 (m, 1H), 1.90-1.84 (m, 1H), 1.78-1.69 (m, 3H), 1.56-1.48 (m, 1H), 1.21-1.10 (m, 2H), 0.94-0.80 (m, 2H).

**Compound 166 NMR: $^1$H NMR** (400 MHz, DMSO-$d_6$) δ 11.51 (s, 1H), 8.02 (t, *J* = 4.4 Hz, 1H), 6.89 (s, 1H), 6.13-6.05 (m, 1H), 4.42-4.35 (m, 1H), 4.27 (d, *J* = 4.4 Hz, 2H), 4.21 (s, 1H), 4.08-3.97 (m, 1H), 3.95-3.86 (m, 2H), 3.49-3.40 (m, 2H), 3.14 (s, 3H), 2.70-2.63 (m, 2H), 2.45 (s, 3H), 2.18-2.13 (m, 6H), 1.67-1.20 (m, 10H).

**Example 97, Compound 167 and 168 Synthesis:**

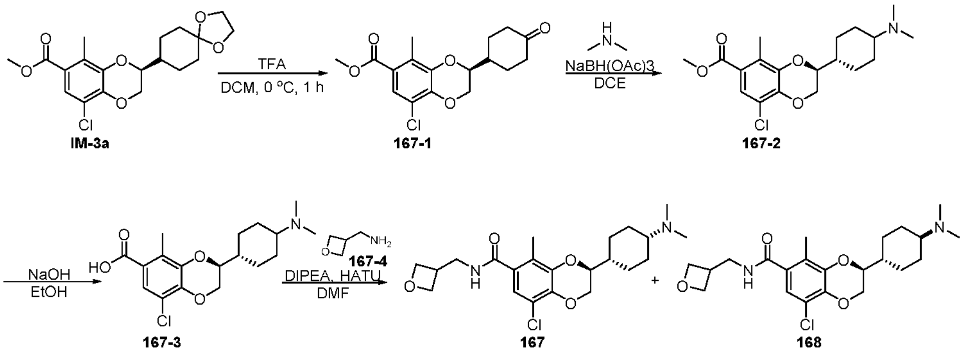


**Step 1, Compound 167-1 Synthesis:**

Following the synthesis method of Example **3,** Step **2,** except replacing Step **2, 3-2** with **IM-3a** (250.0 mg, 0.7 mmol), the same synthesis method was applied, to give Compound **167-1** (170.0 mg, crude), LCMS ($ESI^+$) m/z: 338.8 $[M+H]^+$.

**Step 2, Compound 167-2 Synthesis:**

Following the synthesis method of Example **6,** Step **4,** except replacing Step **4, 6-3** with **167-1** (170.0 mg, crude), the same synthesis method was applied, to give Compound **167-2** (116.0 mg, crude), LCMS ($ESI^+$) m/z: 367.9 $[M+H]^+$.

**Step 3, Compound 167-3 Synthesis:**

Following the synthesis method of Example **6,** Step **1,** except replacing Step **1, IM-3a** with **167-2** (116.0 mg, crude), the same synthesis method was applied, to give Compound **167-3** (100.0 mg, crude), LCMS ($ESI^+$) m/z: 353.8 $[M+H]^+$.

**Step 4, Compound 167 and 168 Synthesis:**

Following the synthesis method of Example **1,** Step **3,** except replacing Step **3, 1-3** with **167-3** (100.0 mg, crude), **1-4** with **167-4** (49.0 mg, 0.6 mmol), the same synthesis method was applied. The obtained compound was subjected to Prep-MPLC to give Compound **167** (13.0 mg, 30.7 μmol, 10.9% yield) and Compound **168** (7.5 mg, 17.7 μmol, 6.3% yield), LCMS ($ESI^+$) m/z: 423.1 $[M+H]^+$.

**Compound 167 NMR: $^1$H NMR** (400 MHz, DMSO-$d_6$) 8.38 (t, *J* = 6.0 Hz, 1H), 6.89 (s, 1H), 4.64-4.60 (m, 2H), 4.46-4.42 (m, 1H), 4.32 (t, *J* = 6.0 Hz, 2H), 4.11-4.04 (m, 2H), 3.48-3.44 (m, 2H), 3.16-3.09 (m, 1H), 2.17-2.15 (m, 6H), 2.12 (s, 3H), 1.82-1.39 (m, 10H).

**Compound 168 NMR: $^1$H NMR** (400 MHz, DMSO-$d_6$) 8.38 (t, *J* = 6.0 Hz, 1H), 6.89 (s, 1H), 4.64-4.60 (m, 2H), 4.50-4.46 (m, 1H), 4.32 (t, *J* = 6.0 Hz, 2H), 4.11-4.06 (m, 1H), 3.94-3.90 (m, 1H), 3.48-3.44 (m, 2H), 3.16-3.09 (m, 1H), 2.17-2.15 (m, 6H), 2.13 (s, 3H), 2.12-1.93 (m, 1H), 1.88-1.77 (m, 3H), 1.58-1.52 (m, 1H), 1.26-1.12 (m, 5H).

**Example 98, Compound 169 and 170 Synthesis:**

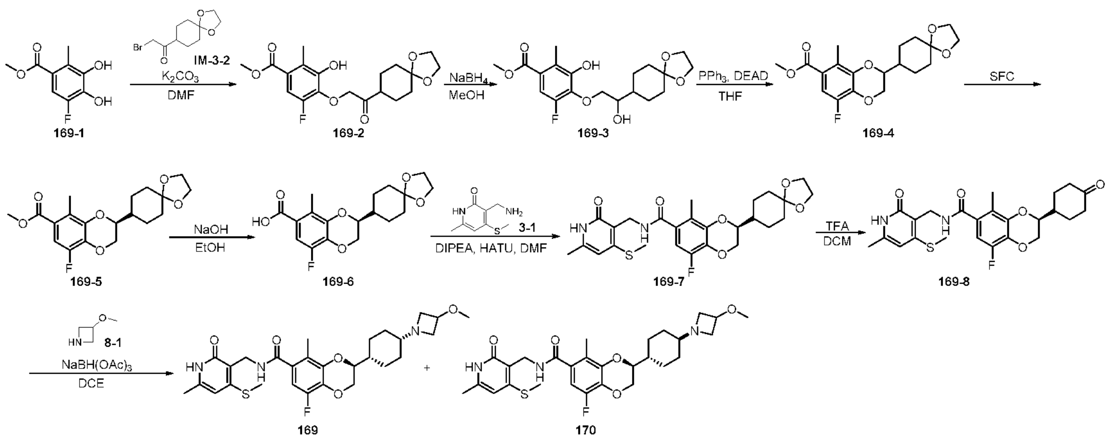


**Step 1, Compound 169-2 Synthesis:**

Following the synthesis method of Example **2,** Step **1,** except replacing Step **1, IM-2** with **IM-3-2** (2.0 g, 7.6 mmol), **2-1** with **169-1** (1.0 g, 5.0 mmol), the same synthesis method was applied, to give Compound **169-2** (875.0 mg, crude). LCMS ($ESI^+$) m/z: 383.1 $[M+H]^+$

**Step 2, Compound 169-3 Synthesis:**

Following the synthesis method of Example **IM-3,** Step **3,** except replacing Step **3, IM-3-4** with **169-2** (875.0 mg, crude), the same synthesis method was applied, to give Compound **169-3** (840.0 mg, crude). LCMS ($ESI^+$) m/z: 385.2 $[M+H]^+$

**Step 3, Compound 169-4 Synthesis:**

Following the synthesis method of Example **IM-3,** Step **4,** except replacing Step **4, IM-3-5** with **169-3** (840.0 mg, crude), the same synthesis method was applied, to give Compound **169-4** (200.0 mg, 0.6 mmol, 25.0% yield). LCMS ($ESI^+$) m/z: 367.2 $[M+H]^+$

**Step 4, Compound 169-5 Synthesis:**

Compound 169-5 can be obtained by SFC resolution from Compound 169-4.

**Step 5, Compound 169-6 Synthesis:**

Following the synthesis method of Example **6,** Step **1,** except replacing Step **1, IM-3a** with **169-5** (86.0 mg, 0.2 mmol), the same synthesis method was applied, to give Compound **169-6** (101.0 mg, crude), LCMS ($ESI^+$) m/z: 353.1 $[M+H]^+$.

**Step 6, Compound 169-7 Synthesis:**

Following the synthesis method of Example **1,** Step **3,** except replacing Step **3, 1-3** with **169-6** (101.0 mg, crude), **1-4** with **3-1** (105.0 mg, 0.6 mmol), the same synthesis method was applied, to give Compound **169-7** (158.0 mg, crude), LCMS ($ESI^+$) m/z: 519.9 $[M+H]^+$.

**Step 7, Compound 169-8 Synthesis:**

Following the synthesis method of Example **3,** Step **2,** except replacing Step **2, 3-2** with **169-7** (158.0 mg, crude), the same synthesis method was applied, to give Compound **169-8** (132.0 mg, crude), LCMS ($ESI^+$) m/z: 475.2 $[M+H]^+$.

**Step 8, Compound 169 and 170 Synthesis:**

Following the synthesis method of Example **6,** Step **4,** except replacing Step **4, 6-3** with **169-8** (132.0 mg, crude), **dimethylamine** with **8-1** (48.0 mg, 0.6 mmol), the same synthesis method was applied. The obtained compound was

subjected to Prep-MPLC to give **169** (26.0 mg, 0.05 mmol, 17.1% yield) and **170** (60.0 mg, 0.1 mmol, 39.5% yield), LCMS ($ESI^+$) m/z: 546.6 $[M+H]^+$.

**Compound 169 NMR: $^1$H NMR** (600 MHz, DMSO-$d_6$) δ 11.51 (s, 1H), 7.95 (t, *J* = 4.8 Hz, 1H), 6.73-6.71 (m, 1H), 6.08 (s, 1H), 4.41-4.39 (m, 1H), 4.27 (d, *J* = 4.8 Hz, 2H), 4.03-4.00 (m, 1H), 3.95-3.88 (m, 2H), 3.43-3.41 (m, 2H), 3.13 (s, 3H), 2.75-2.70 (m, 2H), 2.45 (s, 3H), 2.16 (s, 3H), 2.11(s, 3H) 1.96-1.85 (m, 2H), 1.80-1.69 (m, 3H), 1.58-1.52 (m, 1H), 1.19-1.15 (m, 2H), 0.91-0.84 (m, 2H).

**$^{19}$F NMR** (564 MHz, DMSO-$d_6$) δ -140.00.

**Compound 170 NMR: $^1$H NMR** (600 MHz, DMSO-$d_6$) δ 11.51 (s, 1H), 7.95 (t, *J* = 4.2 Hz, 1H), 6.71 (d, *J* = 10.8 Hz, 1H), 6.08 (s, 1H), 4.39-4.37 (m, 1H), 4.27 (d, *J* = 4.2 Hz, 2H), 4.06-4.01 (m, 1H), 3.98-3.91 (m, 2H), 3.47-3.45 (m, 1H), 3.14 (s, 3H), 2.68-2.66 (m, 2H), 2.45 (s, 3H), 2.16 (s, 3H), 2.10 (s, 3H), 1.65-1.28 (m, 10H).

**$^{19}$F NMR** (564 MHz, DMSO-$d_6$) δ -140.03.

**Example 99, Compound 171 and 172 Synthesis:**

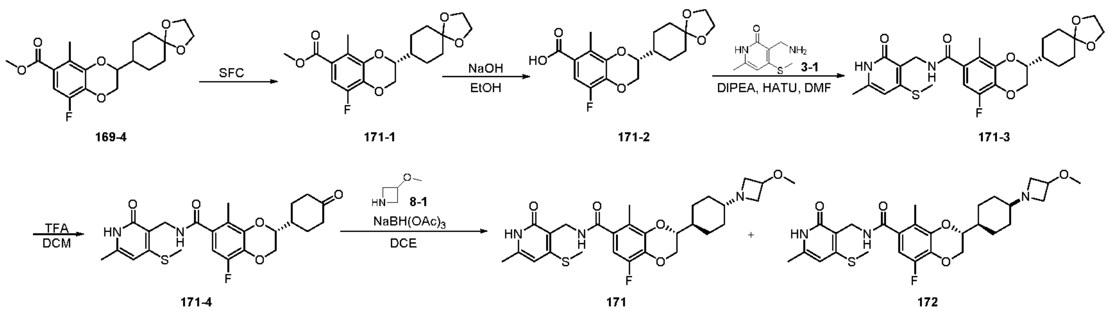

169-4 171-1 171-2 171-3 171-4 171 172

**Step 1, Compound 171-1 Synthesis:**

Compound 171-1 can be obtained by SFC resolution from Compound 169-4.

**Step 2, Compound 171-2 Synthesis:**

Following the synthesis method of Example **6,** Step **1,** except replacing Step **1, IM-3a** with **171-1** (86.0 mg, 0.2 mmol), the same synthesis method was applied, to give Compound **171-2** (87.6 mg, crude), LCMS ($ESI^+$) m/z: 353.1 $[M+H]^+$.

**Step 3, Compound 171-3 Synthesis:**

Following the synthesis method of Example **1,** Step **3,** except replacing Step **3, 1-3** with **171-2** (87.6 mg, crude), **1-4** with **3-1** (92.0 mg, 0.5 mmol), the same synthesis method was applied, to give Compound **171-3** (155.0 mg, crude), LCMS ($ESI^+$) m/z: 519.9 $[M+H]^+$.

**Step 4, Compound 171-4 Synthesis:**

Following the synthesis method of Example **3,** Step **2,** except replacing Step **2, 3-2** with **171-3** (155.0 mg, crude), the same synthesis method was applied, to give Compound **171-4** (130.0 mg, crude), LCMS ($ESI^+$) m/z: 475.2 $[M+H]^+$.

**Step 5, Compound 171 and 172 Synthesis:**

Following the synthesis method of Example **6,** Step **4,** except replacing Step **4, 6-3** with **171-4** (130.0 mg, crude), **dimethylamine** with **8-1** (48.0 mg, 0.6 mmol), the same synthesis method was applied. The obtained compound was subjected to Prep-MPLC to give **171** (14.0 mg, 25.7 μmol, 9.4% yield) and **172** (45.0 mg, 82.5 μmol, 30.1% yield), LCMS ($ESI^+$) m/z: 546.4 $[M+H]^+$.

**Compound 171 NMR: $^1$H NMR** (600 MHz, DMSO-$d_6$) δ 11.51 (s, 1H), 7.95 (t, *J* = 4.2 Hz, 1H), 6.71 (d, *J* = 10.8 Hz, 1H), 6.08 (s, 1H), 4.41-4.39 (m, 1H), 4.27 (d, *J* = 4.2 Hz, 2H), 4.06-4.01 (m, 1H), 3.95-3.91 (m, 1H), 3.91-3.86 (m, 1H), 3.44-3.42 (m, 2H), 2.46-2.44 (m, 3H), 2.75-2.70 (m, 2H), 2.45 (s, 3H), 2.16 (s, 3H), 2.11 (s, 3H), 1.98-1.95 (m, 2H), 1.78-1.70 (m, 3H), 1.58-1.52 (m, 1H), 1.20-1.12 (m, 2H), 0.91-0.82 (m, 2H).

**$^{19}$F NMR** (564 MHz, DMSO-$d_6$) δ -139.76.

**Compound 172 NMR: $^1$H NMR** (600 MHz, DMSO-$d_6$) δ 11.51 (s, 1H), 7.95 (t, *J* = 4.2 Hz, 1H), 6.71 (d, *J* =11.4 Hz, 1H), 6.08 (s, 1H), 4.39-4.34 (m, 1H), 4.27 (d, *J* = 4.2 Hz, 2H), 4.06-4.01 (m, 1H), 3.99-3.90 (m, 2H), 3.50-3.42 (m, 2H), 3.14 (s, 3H), 2.70-2.64 (m, 2H), 2.45 (s, 3H), 2.16 (s, 3H), 2.11 (s, 3H), 1.65-1.26 (m, 10H).

**$^{19}$F NMR** (564 MHz, DMSO-$d_6$) δ -140.03.

**Example 100, Compound 173 and 174 Synthesis:**

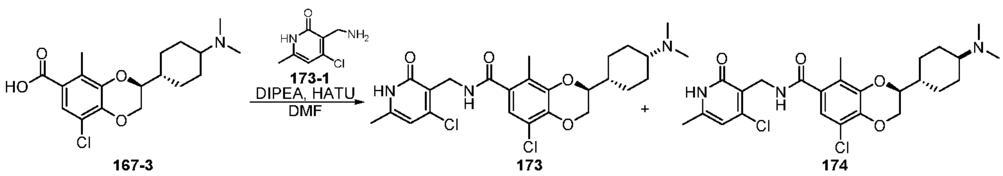


167-3 173 + 174

**Step 1, Compound 173 and 174 Synthesis:**

Following the synthesis method of Example **1,** Step **3,** except replacing Step **3, 1-3** with **167-3** (20.0 mg, 0.06 mmol), **1-4** with **173-1** (34.0 mg, 0.2 mmol), the same synthesis method was applied. The obtained compound was subjected to Prep-MPLC to give Compound **173** (1.0 mg, 2.0 μmol, 3.5%) and Compound **174** (1.0 mg, 2.0 μmol, 3.5%), LCMS (ESI$^+$) m/z: 508.2 [M+H]$^+$.

**Compound 173 NMR: $^1$H NMR** (600 MHz, DMSO-$d_6$) δ 11.91 (s, 1H), 8.17 (t, *J* = 4.8 Hz, 1H), 6.90 (s, 1H), 6.17 (s, 1H), 4.49 (d, *J* = 9.6 Hz, 1H), 4.29 (d, *J* = 4.8 Hz, 2H), 4.10-4.03 (m, 2H), 2.17-2.12 (m, 12H), 2.07-2.04 (m, 1H), 1.81-1.73 (m, 2H), 1.72-1.64 (m, 2H), 1.62-1.52 (m, 2H), 1.45-1.38 (m, 2H), 1.24-1.21 (m, 1H).

**Compound 174 NMR: $^1$H NMR** (600 MHz, DMSO-$d_6$) δ 11.91 (s, 1H), 8.17 (t, *J* = 4.8 Hz, 1H), 6.90 (s, 1H), 6.17 (s, 1H), 4.47 (d, *J* = 10.2 Hz, 1H), 4.29 (d, *J* = 4.2 Hz, 2H), 4.08-4.04 (m, 1H), 3.93-3.89 (m, 1H), 2.20-2.10 (m, 12H), 2.04-2.00 (m, 1H), 1.89-1.83 (m, 2H), 1.81-1.77 (m, 2H), 1.56-1.50 (m, 1H), 1.26-1.22 (m, 3H), 1.28-1.26 (m, 1H).

**Example 101, Compound 175 and 176 Synthesis:**

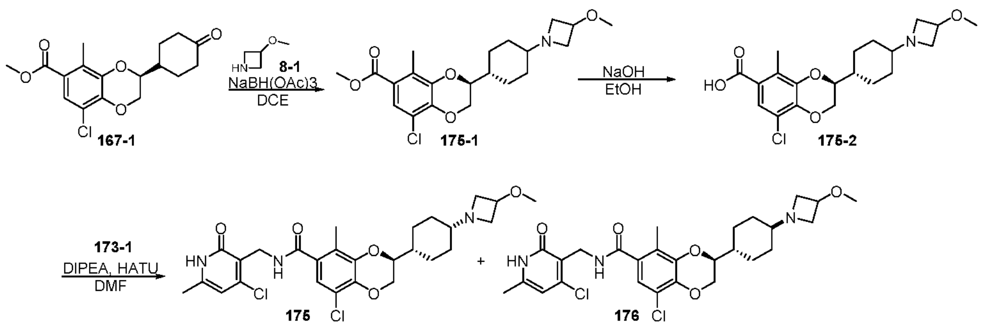


167-1 175-1 175-2 175 + 176

**Step 1, Compound 175-1 Synthesis:**

Following the synthesis method of Example **6,** Step **4,** except replacing Step **4, 6-3** with **167-1** (320.0 mg, 1.0 mmol), **dimethylamine** with **8-1** (85.0 mg, 1.9 mmol), the same synthesis method was applied, to give Compound **175-1** (275.0 mg, crude), LCMS (ESI$^+$) m/z: 410.2 [M+H]$^+$.

**Step 2, Compound 175-2 Synthesis:**

Following the synthesis method of Example **6,** Step **1,** except replacing Step **1, IM-3a** with **175-1** (275.0 mg, crude), the same synthesis method was applied, to give Compound 175-2 (340.0 mg, crude), LCMS (ESI$^+$) m/z: 396.2 [M+H]$^+$.

**Step 3, Compound 175 and 176 Synthesis:**

Following the synthesis method of Example **1,** Step **3,** except replacing Step **3, 1-3** with **175-2** (20 mg, 0.05 mmol), **1-4** with **173-1** (17.0 mg, 0.1 mmol), the same synthesis method was applied. The obtained compound was subjected to Prep-MPLC to give Compound **175** (3.5 mg, 6.4 μmol, 12.6% yield) and Compound **176** (3.5 mg, 6.4 μmol, 12.6% yield), LCMS (ESI$^+$) m/z: 550.2 $[M+H]^+$.

**Compound 175 NMR: $^1$H NMR** (600 MHz, DMSO-$d_6$) δ 11.91 (s, 1H), 8.17 (t, *J* = 4.8 Hz, 1H), 6.90 (s, 1H), 6.17 (s, 1H), 4.49 (d, *J*= 9.6 Hz, 1H), 4.29 (d, *J* = 4.8 Hz, 2H), 4.08-4.02 (m, 1H), 3.95-3.88 (m, 2H), 3.46-3.44 (m, 2H), 3.14 (s, 3H), 2.74-2.72 (m, 2H), 2.16 (s, 3H), 2.12 (s, 3H), 1.98-1.81 (m, 2H), 1.80-1.70 (m, 3H), 1.60-1.50 (m, 1H), 1.20-1.10 (m, 2H), 0.91-0.80 (m, 2H).

**Compound 176 NMR: $^1$H NMR** (600 MHz, DMSO-$d_6$) δ 11.91 (s, 1H), 8.17 (t, *J* = 4.8 Hz, 1H), 6.90 (s, 1H), 6.17 (s, 1H), 4.47 (dd, *J* = 10.2, 2.0 Hz, 1H), 4.29 (d, *J* = 4.8 Hz, 2H), 4.08-4.04 (m, 1H), 3.97-3.89 (m, 2H), 3.46-3.44 (m, 2H), 3.14 (s, 3H), 2.67 (t, *J* = 6.0 Hz, 2H), 2.15 (s, 3H), 2.12 (s, 3H), 1.65-1.20 (m, 10H).

**Example 102, Compound 177 and 178 Synthesis:**

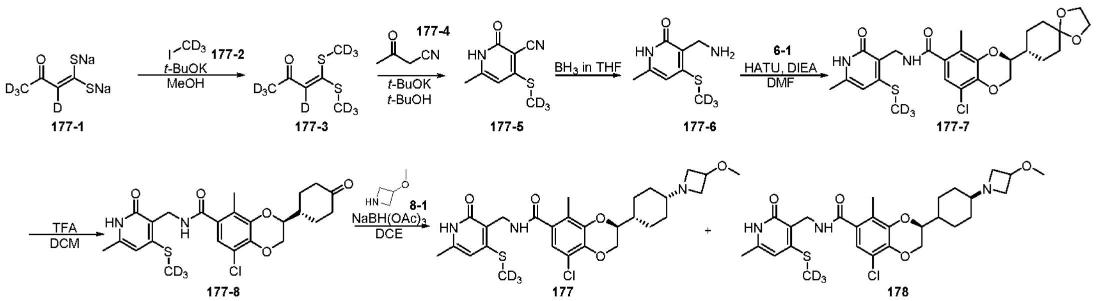


**Step 1, Compound 177-3 Synthesis:**

A single-necked flask was added with substrate **177-1** (100.0 mg, 0.5 mmol). The mixture was dissolved with methanol solution (5 mL) under stirring, and then added with **177-2** (80.0 mg, 0.5 mmol) and potassium t-butanolate (100.0 mg, 0.9 mmol), heated to 80 °C to react for 16 hours, with LC-MS monitoring. After the completion of the reaction, the reaction mixture was cooled to room temperature, added with water and ethyl acetate to extract three times. The organic phase was combined, washed with saturated saline solution, and dried with anhydrous sodium sulfate and then condensated under reduced pressure to give the crude product **177-3** (100.0 mg, crude), LCMS (ESI$^+$) m/z: 172.3 $[M+H]^+$.

**Step 2, Compound 177-5 Synthesis:**

A single-necked flask was added with substrate **177-3** (100.0 mg, crude). The mixture was dissolved with t-butanol solution (1 mL) under stirring, and then added with **177-4** (50.0 mg, 0.6 mmol) and potassium t-butanolate (78.0 mg, 0.7 mmol), heated to 80 °C to react for 1 hour, with LC-MS monitoring. After the completion of the reaction, the reaction mixture was cooled to room temperature, added with water and ethyl acetate to extract three times. The organic phase was combined, washed with saturated saline solution, and dried with anhydrous sodium sulfate and then condensated under reduced pressure to give the crude product **177-5** (90.0 mg, crude), LCMS (ESI$^+$) m/z: 183.3 $[M+H]^+$.

**Step 3, Compound 177-6 Synthesis:**

Under nitrogen protection, a three-necked flask was added with substrate **177-5** (90.0 mg, crude). The mixture was dissolved with borane solution in tetrahydrofuran (1 mL) under stirring. Then, heated to 80 °C to react for 5 hours, with LC-MS monitoring. After the completion of the reaction, the reaction mixture was cooled to room temperature, added with water and ethyl acetate to extract three times. The organic phase was combined, washed with saturated saline solution, and dried with anhydrous sodium sulfate and then condensated under reduced pressure to give the crude product **177-6** (50.0 mg, crude), LCMS (ESI$^+$) m/z: 187.3 $[M+H]^+$.

**Step 4, Compound 177-7 Synthesis:**

Following the synthesis method of Example **1,** Step **3,** except replacing Step **3, 1-3** with **6-1** (50.0 mg, 0.1 mmol), **1-4** with **177-6** (50.0 mg, 0.3 mmol), the same synthesis method was applied, to give Compound **177-7** (34.0 mg, crude), LCMS ($ESI^+$) m/z: 537.2 $[M+H]^+$.

**Step 5, Compound 177-8 Synthesis:**

Following the synthesis method of Example **3,** Step **2,** except replacing Step **2, 3-2** with **177-7** (34.0 mg, crude), the same synthesis method was applied, to give Compound **177-8** (34.0 mg, crude), LCMS ($ESI^+$) m/z: 493.3 $[M+H]^+$.

**Step 6, Compound 177 and 178 Synthesis:**

Following the synthesis method of Example **6,** Step **4,** except replacing Step **4, 6-3** with **177-8** (34.0 mg, crude), **dimethylamine** with **8-1** (19.0 mg, 0.2 mmol), the same synthesis method was applied. The obtained compound was subjected to Prep-MPLC to give **177** (1.5 mg, 2.7 μmol, 3.9% yield) and **178** (1.5 mg, 2.7 μmol, 3.9% yield), LCMS ($ESI^+$) m/z: 565.2 $[M+H]^+$.

**Compound 177 NMR: $^1$H NMR** (600 MHz, DMSO-$d_6$) δ 11.51 (s, 1H), 8.01 (t, *J* = 4.2 Hz, 1H), 6.90 (s, 1H), 6.07 (s, 1H), 4.42 (d, *J* = 9.6 Hz, 1H), 4.27 (d, *J* = 4.2 Hz, 2H), 4.08-4.03 (m, 1H), 3.96-3.89 (m, 2H), 3.47-3.43 (m, 3H), 3.14 (s, 3H), 2.66 (t, *J* = 4.0 Hz, 2H), 2.21 (m, 1H), 2.16 (s, 3H), 2.13 (s, 3H), 1.64-1.53 (m, 4H), 1.42-1.25 (m, 4H).

**Compound 178 NMR: $^1$H NMR** (600 MHz, DMSO-$d_6$) δ 11.51 (s, 1H), 8.01 (t, *J* = 4.2 Hz, 1H), 6.90 (s, 1H), 6.07 (s, 1H), 4.42 (d, *J* = 9.6 Hz, 1H), 4.27 (d, *J* = 4.2 Hz, 2H), 4.08-4.03 (m, 2H), 3.93-3.86 (m, 2H), 3.13 (s, 3H), 2.71 (t, *J* = 6.6 Hz, 2H), 2.16 (s, 3H), 2.15 (s, 3H), 1.98-1.85 (m, 2H), 1.80-1.70 (m, 3H), 1.57-1.50 (m, 1H), 1.36-1.10 (m, 3H), 0.92-0.80 (m, 2H).

**Example 103, Compound 179 and 180 Synthesis:**

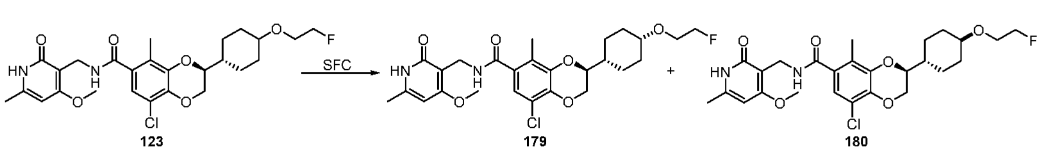


**Step 1, Compound 179 and 180 Synthesis:**

Compound 179 and 180 can be obtained by SFC resolution from Compound 123. LCMS ($ESI^+$) m/z: 523.2 $[M+H]^+$.

**Compound 179 NMR: $^1$H NMR** (600 MHz, DMSO-$d_6$) δ 11.42 (s, 1H), 7.93 (t, *J* = 4.2 Hz, 1H), 6.87 (s, 1H), 6.09 (s, 1H), 4.80-4.50 (m, 2H), 4.48-4.43 (m, 2H), 4.18 (d, *J* = 4.8 Hz, 2H), 4.09-4.06 (m, 1H), 3.96-3.94 (m, 1H), 3.80 (s, 3H), 3.64-3.62 (m, 1H), 3.58-3.56 (m, 1H), 2.18 (s, 3H), 2.12 (s, 3H), 1.85-1.84 (m, 2H), 1.70-1.66 (m, 2H), 1.47-1.39 (m, 1H), 1.23-1.11 (m, 4H).

**Compound 180 NMR: $^1$H NMR** (600 MHz, DMSO-$d_6$) δ 11.42 (s, 1H), 7.93 (t, *J* = 4.8 Hz, 1H), 6.87 (s, 1H), 6.09 (s, 1H), 4.53-4.52 (m, 1H), 4.47-4.44 (m, 2H), 4.18 (d, *J* = 4.8 Hz, 1H), 4.06-4.03 (m, 1H), 3.93-3.91 (m, 1H), 3.80 (s, 3H), 3.68-3.67 (m, 1H), 3.63-3.62 (m, 1H), 3.27-3.24 (m, 1H), 2.18 (s, 3H), 2.12 (s, 3H), 2.05-1.98 (m, 3H), 1.78-1.76 (m, 1H), 1.59-1.57 (m, 1H), 1.23-1.11 (m, 4H).

**Example 104, Compound 181 Synthesis:**

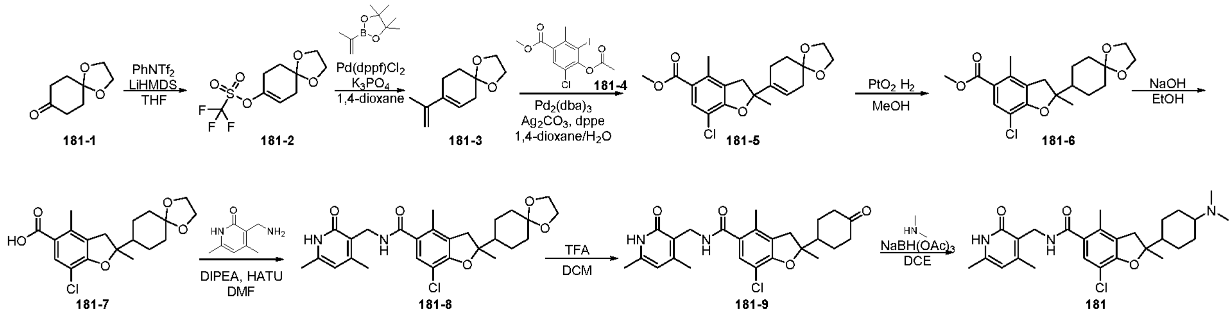


**Step 1, Compound 181-2 Synthesis:**

A single-necked flask was added with substrate **181-1** (3.0 g, 19.2 mmol). The mixture was dissolved with THF solution (50 mL) under stirring, and then at 0°C added dropwise with **LiHMDS** (4.1 g, 25.0 mmol), at room temperature stirred for 0.5 hours, added with **$PhNTf_2$** (8.9 g, 25.0 mmol), with LC-MS monitoring. After the completion of the reaction, the mixture was added with water and ethyl acetate to extract three times. The organic phase was combined, washed with saturated saline solution, and dried with anhydrous sodium sulfate, and then purified by silica gel column chromatography to give the product **181-2** (5.4 g, 18.7 mmol, 97.5% yield), LCMS ($ESI^+$) m/z: 288.2 $[M+H]^+$.

**Step 2, Compound 181-3 Synthesis:**

A single-necked flask was added with substrate **181-2** (2.9 g, 10.1 mmol). The mixture was dissolved with 1,4-dioxane solution (40 mL) under stirring, and then added with **2-isopropenyl-4,4,5,5-tetramethyl-1,3,2-dioxaborolane** (2.0 g, 12.0 mmol), Pd(dppf)$Cl_2$ (817.0 mg, 1.0 mmol) and $K_3PO_4$ (3.2 g, 15.0 mmol), stirred at 80°C for 18 hours, with LC-MS monitoring. After the completion of the reaction, the mixture was added with water and ethyl acetate to extract three times. The organic phase was combined, washed with saturated saline solution, and dried with anhydrous sodium sulfate, and then purified by silica gel column chromatography to give the product **181-3** (350.0 mg, 1.9 mmol, 19.2% yield), LCMS ($ESI^+$) m/z: 180.2 $[M+H]^+$.

**Step 3, Compound 181-5 Synthesis:**

A single-necked flask was added with substrate **181-3** (180.0 mg, 1.0 mmol). The mixture was dissolved with 1,4-dioxane solution (4 mL) and water (1 mL) under stirring, and then added with **181-4** (92.0 mg, 0.3 mmol), $Pd_2(dba)_3$ (11.4 mg, 0.01 mmol) and $Ag_2CO_3$ (138.0 mg, 0.5 mmol) and dppe (5.0 mg, 0.01 mmol), stirred at 100°C for 24 hours, with LC-MS monitoring. After the completion of the reaction, the mixture was added with water and ethyl acetate to extract three times. The organic phase was combined, washed with saturated saline solution, and dried with anhydrous sodium sulfate, and then purified by silica gel column chromatography to give the product **181-5** (46.0 mg, 0.1 mmol, 12.1% yield), LCMS ($ESI^+$) m/z: 378.2 $[M+H]^+$.

**Step 4, Compound 181-6 Synthesis:**

A single-necked flask was added with substrate **181-5** (46.0 mg, 0.1 mmol), **$PtO_2$** (5.0 mg, 0.02 mmol), and **MeOH** (3 mL). The system was reacted under 1 atm hydrogen atmosphere for 3hours, to give Compound **181-6** (13.0 mg, 0.03 mmol, 28.1% yield), LCMS ($ESI^+$) m/z: 380.9 $[M+H]^+$.

**Step 5, Compound 181-7 Synthesis:**

Following the synthesis method of Example **6,** Step **1,** except replacing Step **1, IM-3a** with **181-6** (13.0 mg, 0.03 mmol), the same synthesis method was applied, to give Compound **181-7** (12.0 mg, crude), LCMS ($ESI^+$) m/z: 366.8 $[M+H]^+$.

**Step 6, Compound 181-8 Synthesis:**

Following the synthesis method of Example **1,** Step **3,** except replacing Step **3, 1-3** with **181-7** (10.0 mg, 0.03 mmol), **1-4** with **2-4** (8.0 mg, 0.05 mmol), the same synthesis method was applied, to give Compound **181-8** (12.0 mg, 0.02 mmol, 87.6% yield), LCMS ($ESI^+$) m/z: 501.0 $[M+H]^+$.

**Step 7, Compound 181-9 Synthesis:**

Following the synthesis method of Example **3,** Step **2,** except replacing Step **2, 3-2** with **181-8** (12.0 mg, 0.02 mmol), the same synthesis method was applied, to give Compound **181-9** (8.0 mg, crude), LCMS ($ESI^+$) m/z: 456.9 $[M+H]^+$.

**Step 8, Compound 181 Synthesis:**

Following the synthesis method of Example **6,** Step **4,** except replacing Step **4, 6-3** with **181-9** (8.0 mg, 0.02 mmol), the same synthesis method was applied, to give Compound **181** (1.3 mg, 2.7 μmol, 15.3% yield), LCMS ($ESI^+$) m/z: 486.0 $[M+H]^+$.

**Biological evaluation assay:**

**Test Example 1: Evaluation of the inhibitory effect of compounds on EZH2 and EZH1 proteins by the MTase-Glo method**

The compound was dissolved in DMSO, and the compound was serially diluted using ECHO665 and transferred to a 384-well reaction plate (Coming, 3824). A sample containing only DMSO was added as a positive control (maximum signal control) and a negative control (minimum signal control), while ensuring that the final DMSO content in each reaction well was 0.5%. The protein was configured in reaction buffer (50mM Bicine, 0.01% Triton X-100, 1mM DTT, 0.01% BSA, pH 7.6), such that the final reaction concentrations of EZH2/SUZ/EED (BPS, 51003) protein solution and EZH1/SUZ/EED (BPS, 51006) were 6.25 nM and 25 nM, respectively. The negative control (minimum signal control) used an equal amount of buffer instead of protein solution. The prepared protein solution was added to the 384-well reaction plate and pre-incubated at room temperature for 15 minutes. The mixture of the substrate H3 peptide fragment (custom-synthesized by GenScript, biotinylated Histone H3(21-44) peptide (ATKAARKSAPATGGVKKPHRYRP-GG-K(Biotin)-OH)) prepared in the reaction buffer and SAM (Promega, V7601) was added into the 384-well plate, such that the final reaction concentration of H3 peptide fragment was 10 μM, and the final concentration of SAM was 3 μM. The mixture was centrifuged at 1000 rpm for 1 min. The 384-well reaction plate was placed in a constant temperature shaker and incubate at 30 °C for 3 h at a speed of 300 rpm.. Then the detection reagent was added according to the experimental process of the kit MTase-Glo (Promega, V7601) and incubated.

After incubation, read with BMG PHERAStar. The luminescence signals obtained by positive control (maximum signal control) and negative control (minimum signal control) were normalized to obtain the inhibition rate of different concentrations of compounds. Then, the $IC_{50}$ of the compound on the enzyme activity inhibition was calculated by GraphPad Prism 6 with log(inhibitor) vs. response-Variable slope model fitting. The fitting equation is: Y=Bottom+(Top-Bottom)/(1+10^((LogIC $_{50}$ -X)*HillSlope)), wherein Y represents the known percentage remaining enzyme activity, and X represents the known activity after Log compound concentration.

The results of Test Example 1 is shown in Table 1.

Table 1

| Compound | Test Example 1 WT EZH2 $IC_{50}$(nM) | Test Example 1 WT EZH1 $IC_{50}$(nM) | Compound | Test Example 1 WT EZH2 $IC_{50}$(nM) | Test Example 1 WT EZH1 $IC_{50}$ (nM) |
|---|---|---|---|---|---|
| **1** | 27.5 | 1296 | **93** | 6.0 | 132.4 |
| **2** | 3.9 | 88.5 | **94** | 3.0 (mean, n=2) | 8.1 (mean, n=3) |
| **3** | 3.3 | 4.4 | **95** | 11.6 | 227 |
| **4** | 7208 | ND | **96** | 7.0 | 167 |
| **5** | 2.3 | 26 | **97** | 3.2455 (mean, n=2) | 15.22 |
| **6** | 2.5 (mean, n=3) | 4.0 (mean, n=3) | **98** | 5.7 | ND |
| **6'** | ND | ND | **99** | 93.4 | ND |
| **7** | 130 | 1080 | **100** | 127.7 | ND |
| **8** | 5.6 (mean, n=2) | 6.2 | **101** | 3.1 | 5.5 |
| **9** | 3.1 | ND | **102** | 2.6 | 655 |

(continued)

| Compound | Test Example 1 WT EZH2 $IC_{50}$(nM) | Test Example 1 WT EZH1 $IC_{50}$(nM) | Compound | Test Example 1 WT EZH2 $IC_{50}$(nM) | Test Example 1 WT EZH1 $IC_{50}$ (nM) |
|---|---|---|---|---|---|
| **10** | 158 | ND | **103** | ND | ND |
| **11** | 8.1 (mean, n=2) | 4.8 | **104** | ND | ND |
| **12** | 2.6 | ND | **105** | ND | ND |
| **13** | 17.7 | ND | **106** | ND | ND |
| **14** | 3.7(mean, n=2) | 3.7 | **107** | 3.2 | 17.8 |
| **15** | 2.1 | ND | **108** | 5.0 | 69.3 |
| **16** | 5.2 | 29.6 | **109** | 2.0 | 3.8 |
| **17** | 4.6 | 16.5 | **110** | 2.7 | 15.4 |
| **18** | 3.5 | 7.4 | **111** | 13.4 | 27.3 |
| **19** | 3.4 | 26.3 | **112** | ND | 49.9 |
| **20** | 3.1 | 3.3 | **113** | 9.4 | 91.2 |
| **21** | 6.4 | 18.9 | **114** | ND | 267 |
| **22** | 2.1 | 2.3 | **115** | 4.9 | 74.8 |
| **23** | 2.4 | 8.1 | **116** | ND | 156 |
| **24** | 4.5 | 9.2 | **117** | 6.2 | ND |
| **25** | 1.6 | 8.3 | **118** | ND | ND |
| **26** | 2.0 | 5.4 | **119** | ND | ND |
| **27** | 2.3 | 14.4 | **120** | ND | ND |
| **28** | 3.7 | 9.4 | **121** | ND | ND |
| **29** | 3.3 | 5.8 | **122** | ND | ND |
| **30** | 3.3 | 17.9 | **123** | ND | 84 |
| **31** | 66.3 | 491 | **124** | 16.1 | 326 |
| **32** | 5.1 | 5.7 | **125** | 33.9 | 307 |
| **33** | 3.6 | 9.4 | **126** | 20.3 (mean, n=2) | 218 |
| **34** | 4.1 | 6.0 | **127** | 25.5 | ND |
| **35** | 3.4 | 7.4 | **128** | 9.5 | ND |
| **36** | 2.4 | 9.7 | **129** | 3.9 | ND |
| **37** | 3.1 | 5.9 | 130 | 12.6 | 201 |
| **38** | 2.3 | 4.7 | 131 | 3.1 | 46.8 |
| **39** | 4.0 | 7.3 | 132 | 7.3 | 722 |
| **40** | ND | 7.3 | 133 | 16.6 | 584 |
| **41** | 4.3 | 3.9 | 134 | 6.6 | 125 |
| **42** | ND | 8.6 | 135 | 80.1 | 5605 |
| **43** | 3.6 | 4.1 | 136 | 42.5 | 192 |
| **44** | ND | 13.5 | 137 | ND | ND |
| **45** | 9.5 | 3.0 | 138 | ND | ND |
| **46** | ND | 7.7 | 139 | 1256 | 8490 |
| **47** | 1.2 | 9.5 | 140 | 897 | 8914 |

(continued)

| Compound | Test Example 1 WT EZH2 $IC_{50}$(nM) | Test Example 1 WT EZH1 $IC_{50}$(nM) | Compound | Test Example 1 WT EZH2 $IC_{50}$(nM) | Test Example 1 WT EZH1 $IC_{50}$ (nM) |
|---|---|---|---|---|---|
| **48** | ND | 6.0 | 141 | 179 | >10000 |
| **49** | ND | 2.2 | 142 | 1163 | 7611 |
| **50** | ND | 11.8 | 143 | 5.5 | 39.9 |
| **51** | ND | ND | 144 | 15.6 | 769 |
| **52** | ND | ND | 145 | ND | ND |
| **53** | 3.0 (mean, n=3) | 26.8 (mean, n=3) | 146 | ND | ND |
| **54** | 250 | 400 | 147 | 6.8 | 92.0 |
| **55** | 4.5 | 150 | 148 | 67.1 | 2825 |
| **56** | 340 | 810 | 149 | 2.3 | 3.3 |
| **57** | 21.7 | 260 | 150 | 11.6 | 22.2 |
| **58** | 35.8 | 2297 | 151 | ND | ND |
| **59** | 9.6 | 7.0 | 152 | ND | 126 |
| **60** | 16.4 | 94.9 | 153 | 3.2 | 6.9 |
| **61** | 8.8 | 162 | 154 | 4.2 | 30.0 |
| **62** | 22.1 | 1441 | 155 | 21.4 | 585 |
| **63** | 11.5 | 375 | 156 | 67.0 | 366 |
| **64** | 16.6 | 254 | 157 | ND | 190 |
| **65** | 29.7 | 641 | 158 | ND | 3435 |
| **66** | 24.4 | 687 | 159 | 1.1 | 7.0 |
| **67** | 47.6 | 367 | 160 | ND | 31.6 |
| **68** | 59.3 | >10000 | 161 | 3.7 | 5.5 |
| **69** | 84.6 | 879 | 162 | ND | 29.3 |
| **70** | 22.1 | 177 | 163 | 7.1 | ND |
| **71** | 41.8 | 564 | 164 | ND | ND |
| **72** | 1254 | >10000 | 165 | 3.6 | 5.7 |
| **73** | 51.3 | 196 | 166 | ND | 17.13 |
| **74** | 998 | >10000 | 167 | ND | ND |
| **75** | 48.1 | 199 | 168 | ND | ND |
| **76** | 7.6 | 270 | 169 | 6.0 | 5.8 |
| **77** | 27.3 | 142 | 170 | ND | ND |
| **78** | 11.9 | ND | 171 | ND | ND |
| **79** | 2.0 | ND | **172** | ND | ND |
| **80** | 49.3 | ND | **173** | ND | ND |
| **81** | 31.0 | ND | **174** | 3.1 | 7.2 |
| **82** | 85.1 | ND | **175** | 3.4 | 16.6 |
| **83** | 8.2 | ND | **176** | ND | ND |
| **84** | 11.0 | ND | **177** | 1.5 | 2.3 |
| **85** | 10.5 | ND | **178** | ND | 17.5 |

(continued)

| Compound | Test Example 1 WT EZH2 $IC_{50}$(nM) | Test Example 1 WT EZH1 $IC_{50}$(nM) | Compound | Test Example 1 WT EZH2 $IC_{50}$(nM) | Test Example 1 WT EZH1 $IC_{50}$ (nM) |
|---|---|---|---|---|---|
| **86** | 20.1 | ND | **179** | ND | ND |
| **87** | 780 | ND | **180** | ND | ND |
| **88** | 29.5 | ND | **181** | 30.4 | 1347 |
| **89** | 1844 | ND | | | |
| **90** | 18.6 (mean, n=2) | 945 (mean, n=2) | | | |
| **91** | 3.8 | 27.3 | | | |
| **92** | 2.5 | 33.1 | | | |

**Test Example 2: Evaluatation of the effect of compounds on Karpas-422 cells in inhibiting $H_3K_{27}Me_3$ methylation by $H_3K_{27}Me_3$ cell method**

Karpas-422 (Nanjing Kebai, CBP60629) cells were seeded in a 96-well plate (Corning, 3894) at 15,000 cells per well in culture medium (80% 1640 (Hyclone, SH30809.01) + 20% FBS (Corning, 35-081-CV)), and then added with different concentrations of compounds for treatment. The cell growth group without adding compounds is used as a positive control (maximum signal control), and the medium is used as a negative control (minimum signal control), while ensuring that in each reaction well, the final concentration of DMSO was 0.2%. Compounds and cells were incubated for 3 days in a cell culture incubator (37°C, 5% $CO_2$). The 96-well plate was taken out from the cell culture incubator and equilibrated to room temperature, and 25ul of cell suspension was taken out into a 384-well plate. The CCK8 (Beiyuntian, C0040) detection reagent was added, incubated at 37°C for 1h, and OD405 nm was detected. The rest of the cells followed the procedure of the kit EPIgeneous $H_3K_{27}Me_3$ cellular kit (Cisbio, 62KC3PAE) to carry out experiment, read HTRF signal with BMG PHERAStar. The inhibition rate was calculated according to HTRF signal: first calculating positive control (maximum signal control) and negative control (minimum signal control) mean value, with inhibition rate = [1-(compound signal value -negative control signal value)/(positive control signal value-negative control signal value)] × 100% to calculate the inhibition rate of different concentrations of compound cells. By GraphPad Prism 6 with log(inhibitor) vs.response-Variable slope model simulation, the $IC_{50}$ of the compound on cell activity inhibition was calculated. The fitting equation is: Y=Bottom+(Top-Bottom)/(1+10^((LogIC $_{50}$ -X)*HillSlope)), wherein Y represents the inhibition rate, and X represents the known concentration of the compound after Log.

**Test Example 3: Evaluation of the anti-proliferation effect of compounds on Karpas-422 cells by Cell Titer-Glo method**

Karpas-422 (Nanjing Kebai, CBP60629) cells were seeded in a 96-well plate (Corning, 3894) at 1,000 cells per well in growth medium (80% 1640 (Hyclone, SH30809.01) + 20% FBS (Corning, 35-081-CV)), in a total of 100 μL, and then was added with 25 μL of 5-fold different concentrations of compounds diluted in growth medium. The cell growth group without compound addition was used as a positive control (maximum signal control), and the medium was used as a negative control (minimum signal control), while ensuring that the final contration of DMSO in each reaction well was 0.2%. Then the 96-well cell plate was placed in a cell culture incubator (37°C, 5% $CO_2$) and incubated for 3 days. On the fourth day, the cell culture plate was taken out of the incubator, using a row gun to gently blow the cells up and down to mix, removing 62.5 μL of the cell suspension, adding 50 μL of growth medium and 12.5 μL of compounds of 5 times different concentrations, and then putting them in the cell incubator to incubate for 4 days. On the eighth day, the same method was used as the fourth day to treat the cells, then incubating in the cell culture incubator for 3 days. On the eleventh day, the Cell-Titer Glo (Promega, G7573) was used to detect cell viability, and a microplate reader was used to detect the signal value of luminescence.

The inhibition rate of different concentrations of compounds on cells was calculated as inhibition rate=[1-(compound signal value-negative control signal value)/(positive control signal value-negative control signal value)] × 100%. The $IC_{50}$ of the compound on cell activity inhibition was calculated by GraphPad Prism 6 with log(inhibitor) vs. response-Variable slope model fitting. The fitting equation is: Y=Bottom+(Top-Bottom)/(1+10^((LogIC $_{50}$ -X)*HillSlope)), wherein Y represents the inhibition rate, and X represents the known concentration of the compound after Log.

The results of Test Example 2 and 3 are shown in Table 2

Table 2

| Compound | Test Example 2 $H_3K_{27}Me_3$ inhibition (Karpas-422) $IC_{50}$ (nM) | Test Example 3 Karpas-422 cell anti-proliferation $IC_{50}$(nM) | Compound | Test Example 2 $H_3K_{27}Me_3$ inhibition (Karpas-422) $IC_{50}$ (nM) | Test Example 3 Karpas-422 cell anti-proliferation $IC_{50}$(nM) |
|---|---|---|---|---|---|
| **1** | ND | ND | **93** | ND | ND |
| **2** | 31.2 (mean, n=3) | 42.4 (mean, n=2) | **94** | 2.8 (mean, n=2) | 5.1 |
| **3** | 2.1 | 7.6 (mean, n=2) | **95** | ND | ND |
| **4** | ND | ND | **96** | ND | ND |
| **5** | 7.9 | 23.2 | **97** | 8.2 (mean, n=2) | 5.3 (mean, n=2) |
| **6** | 0.59 (mean, n=2) | 1.3 (mean, n=2) | **98** | 462 | ND |
| **6'** | ND | ND | **99** | ND | ND |
| **7** | >1000 | >1000 | **100** | ND | ND |
| **8** | 2.4 (mean, n=2) | 1.8 | **101** | 2.2 | 3.8 |
| **9** | 14.2 | ND | **102** | ND | ND |
| **10** | ND | ND | **103** | ND | ND |
| **11** | 1.7 | 1.5 | **104** | ND | ND |
| **12** | 9.4 | ND | **105** | ND | ND |
| **13** | ND | ND | **106** | ND | ND |
| **14** | 6.8 | 3.3 | **107** | 56.9 | ND |
| **15** | 101 | ND | **108** | ND | ND |
| **16** | 85.9 | 19.8 | **109** | 1.8 | 2.5 |
| **17** | 12.0 | ND | **110** | 21.3 | ND |
| **18** | 22.8 | 17.7 | **111** | ND | ND |
| **19** | 284 | 89.5 | **112** | ND | ND |
| **20** | 3.2 | 1.6 | **113** | ND | ND |
| **21** | ND | ND | **114** | ND | ND |
| **22** | 1.8 | ND | **115** | ND | ND |
| **23** | 22.8 | ND | **116** | ND | ND |
| **24** | 9.8 | ND | **117** | 39.4 | 26.1 (mean, n=2) |
| **25** | 17.3 | ND | **118** | ND | 209 |
| **26** | 3.4 | ND | **119** | ND | 12.9 |
| **27** | 11.4 | ND | **120** | ND | 31.5 |
| **28** | 8.2 | ND | **121** | 92.3 | 35.8 |
| **29** | 34.2 | ND | **122** | ND | 472 |
| **30** | 23.3 | ND | **123** | ND | ND |
| **31** | ND | ND | **124** | 133 | ND |
| **32** | 1.7 | 2.0 | **125** | 402 | ND |
| **33** | 7.7 | ND | **126** | 818 | ND |
| **34** | 6.2 | 5.8 | **127** | ND | ND |
| **35** | 60.5 | ND | **128** | 43.2 | ND |

(continued)

| Compound | Test Example 2 $H_3K_{27}Me_3$ inhibition (Karpas-422) $IC_{50}$ (nM) | Test Example 3 Karpas-422 cell anti-proliferation $IC_{50}$(nM) | Compound | Test Example 2 $H_3K_{27}Me_3$ inhibition (Karpas-422) $IC_{50}$ (nM) | Test Example 3 Karpas-422 cell anti-proliferation $IC_{50}$(nM) |
|---|---|---|---|---|---|
| **36** | 11.0 | ND | **129** | ND | ND |
| **37** | 7.0 | ND | **130** | 209 | 115 |
| **38** | 11.5 | ND | **131** | 43.3 | ND |
| **39** | ND | ND | **132** | ND | ND |
| **40** | 8.3 | ND | **133** | ND | ND |
| **41** | ND | ND | **134** | ND | ND |
| **42** | 47.1 | ND | **135** | ND | ND |
| **43** | 4.1 | ND | **136** | ND | ND |
| **44** | ND | ND | **137** | ND | ND |
| **45** | 6.6 | ND | **138** | ND | ND |
| **46** | 65.8 | ND | **139** | ND | ND |
| **47** | 8.9 | ND | **140** | ND | ND |
| **48** | 27.9 | ND | **141** | ND | ND |
| **49** | 2.8 | ND | **142** | ND | ND |
| **50** | 23.8 | ND | **143** | 22.0 | 10.7 |
| **51** | ND | ND | **144** | ND | ND |
| **52** | ND | ND | **145** | ND | ND |
| **53** | 5.0 (mean, n=2) | 14.1 (mean, n=2) | **146** | ND | ND |
| **54** | >1000 | >1000 | **147** | 83.0 | ND |
| **55** | 19.4 | 47.3 | **148** | ND | ND |
| **56** | 944 | >1000 | **149** | 3.6 | 2.7 |
| **57** | >1000 | ND | **150** | ND | ND |
| **58** | >1000 | ND | **151** | 132 | ND |
| **59** | 233 | ND | **152** | ND | ND |
| **60** | >1000 | ND | **153** | 6.2 | 3.7 |
| **61** | 73.8 (mean, n=2) | 93.8 | **154** | ND | ND |
| **62** | 504 | ND | **155** | ND | ND |
| **63** | 616 | 341 | **156** | ND | ND |
| **64** | 896 | ND | **157** | ND | ND |
| **65** | ND | ND | **158** | ND | ND |
| **66** | ND | ND | **159** | 1.4 | 2.3 |
| **67** | ND | ND | **160** | ND | ND |
| **68** | ND | ND | **161** | 2.2 | 2.4 |
| **69** | ND | ND | **162** | ND | ND |
| **70** | 201 | ND | **163** | 1.9 | 1.7 (mean, n=2) |
| **71** | ND | ND | **164** | ND | ND |

(continued)

| Compound | Test Example 2 $H_3K_{27}Me_3$ inhibition (Karpas-422) $IC_{50}$ (nM) | Test Example 3 Karpas-422 cell anti-proliferation $IC_{50}$(nM) | Compound | Test Example 2 $H_3K_{27}Me_3$ inhibition (Karpas-422) $IC_{50}$ (nM) | Test Example 3 Karpas-422 cell anti-proliferation $IC_{50}$(nM) |
|---|---|---|---|---|---|
| **72** | ND | ND | **165** | 1.4 | 1.2 (mean, n=2) |
| **73** | >1000 | ND | **166** | ND | ND |
| **74** | ND | ND | **167** | ND | >1000 |
| **75** | 245 | ND | **168** | ND | >1000 |
| **76** | 7.6 | ND | **169** | 4.2 | 1.8 |
| **77** | 260 | ND | **170** | ND | 46.8 |
| **78** | 370 | ND | **171** | >1000 | >1000 |
| **79** | ND | ND | **172** | ND | 373 |
| **80** | ND | ND | **173** | ND | 124 |
| **81** | >1000 | ND | **174** | ND | 5.6 |
| **82** | ND | ND | **175** | 16.1 | 13.6 |
| **83** | ND | ND | **176** | ND | 197 |
| **84** | ND | ND | **177** | ND | ND |
| **85** | 95.5 | ND | **178** | ND | ND |
| **86** | >1000 | ND | **179** | ND | 36.6 |
| **87** | ND | ND | **180** | ND | 10.8 |
| **88** | 310 | ND | **181** | 410 | ND |
| **89** | ND | ND | | | |
| **90** | ND | ND | | | |
| **91** | 11.7 | ND | | | |
| **92** | 22.0 | ND | | | |

**Test Example 4: Karpas-422 Subcutaneous Transplantation Model Evaluation of Antitumor Activity Against EZH2 Y641N Mutant Human Diffuse Large B-Cell Lymphoma**

1. Experimental materials

Karpas-422 are human diffuse large B-cell lymphoma cells. NCG mice, female, 7 weeks old, weighing 19-22 grams, were purchased from Chengdu Yaokang Biotechnology Co., Ltd.

2. Experimental method

Karpas-422 cells in logarithmic growth period were collected, counted, and the final concentration of cells was adjusted. The matrix glue of Matrigel, which was completely liquefied, was mixed at a ratio of 1:1. $1 \times 10^7$ cells/mouse were inoculated under the right armpit, and the inoculation volume was 100 μL/mouse. When the average tumor volume reached about 188 $mm^3$, they were randomly divided into groups (n = 7). The solvent used in Compound (DS-3201, Compound 8 and Compound 97) was DMSO: Solutol: HPBCD (20%, w/v) = 5: 20: 75. From the day of grouping, Compound of Example A, Example B or Example C were administered orally, the dose was set at 72 or 144 mg/kg/day, and the dosage regimen was 2 times a day for 28 consecutive days (BID×28). During the experiment, animal activities were observed once a day, each animal was weighed once before administration, and the long diameter and short diameter of the tumor were measured with a vernier caliper twice a week. After the experiment was over, all surviving experimental animals were sacrificed.

3. Data analysis

The calculation formula of the tumor volume is: $V=0.5(a\times b^2)$, where a and b represent the long diameter and short diameter of the tumor respectively;

$$\text{Tumor growth inhibition degree TGI (\%)}=(1-TVCt/TVCc)\times 100;$$

TVC=(Tumor volume per individual on the end of test day) - (Tumor volume per individual on grouping day);

TVCt: the average TVC of the administration group; TVCc: the average TVC of the non-administration group.
The results are shown in Figures 1-3 of the drawings in the description.
The compounds of the present invention are significantly superior to other compounds in their inhibitory activity against EZH2 and EZH1 kinases, Karpas 422 cell inhibitory activity and antiproliferative activity against Karpas 422 cells.

**Test Example 5: X1LNCaP Subcutaneous Transplantation Model Evaluation of Prostate Cancer Antitumor Activity**

1. Experimental materials

LNCaP is a human prostate cancer cell line. The X1LNCaP cell line is derived from the LNCaP cell line: LNCaP cells were inoculated into mice, xenograft tumors of about 300-500 $mm^3$ were removed from the mice, and tumor cells were isolated and cultured. These recultured cells were inoculated into mice, and the model was named X1LNCaP xenograft model. BALB/c nude mice, male, 6-8 weeks old, weighing 18-20 g, were purchased from LC.

2. Experimental method

A 0.2 mL cell suspension containing $1\times 10^7$ cells (cell suspension in RPMI-1640: Matrigel = 100 uL: 100 uL) was subcutaneously inoculated on the right back of the mouse. When the tumor volume reached about 100-150 $mm^3$, they were randomized (n=3). The solvent used for the compounds (DS-3201, Compound 97 and Compound 169) was DMSO:Solutol:HPBCD (20%, W/V)=5:20:75. From the day of grouping, DS-3201, Compound 97 or Compound 169 were administered orally, the dose was set at 60 mg/kg/day, and the dosage regimen was 2 times a day for 28 consecutive days (BID×28). During the experiment, animal activities were observed once a day, each animal was weighed once before administration, and the long diameter and short diameter of the tumor were measured with a vernier caliper twice a week. After the experiment was over, all surviving experimental animals were sacrificed.

3. Data analysis

The calculation formula of the tumor volume is: $V=0.5(a\times b^2)$, where a and b represent the long diameter and short diameter of the tumor respectively;
Tumor growth inhibition degree TGI (%) = $(1-(TV_{Treatment/Dx}-TV_{Treatment/D1})/(TV_{Control/Dx}-TV_{Control/D1})\times 100\%$; the results are shown in Figure 4.

## Claims

1. A compound represented by Formula I or Formula II, or a deuterated compound thereof, or a stereoisomer thereof, or a pharmaceutically acceptable salt thereof:

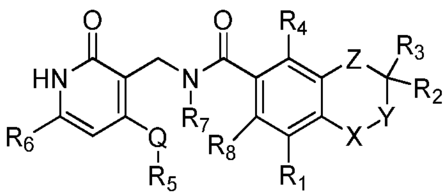

Formula I

or

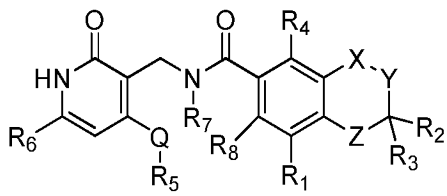

Formula II

wherein,

X is selected from a group consisting of single bond, O, $NR^{X1}$ or $CHR^{X2}$,
Y is selected from a group consisting of O, S, $NR^{Y1}$ or $CHR^{Y2}$;
Z is selected from a group consisting of C=O, O, S, $NR^{Z1}$ or $CHR^{Z2}$;
Q is selected from a group consisting of single bond, O or S;
when X is single bond, Y and Z are not both O;
$R^{X1}$ is selected from a group consisting of the following groups: hydrogen, $-C_{1\sim6}$alkyl, halo-$C_{1\sim6}$alkyl, $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring);
$R^{X2}$ is selected from a group consisting of the following groups: hydrogen, halogen, cyano, hydroxyl, $-C_{1\sim6}$alkyl, halo-$C_{1\sim6}$alkyl, -O($C_{1\sim6}$alkyl), -O(halo-$C_{1\sim6}$alkyl), $-NH_2$, -NH($C_{1\sim6}$alkyl), -N($C_{1\sim6}$alkyl) ($C_{1\sim6}$alkyl), $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring);
$R^{Y1}$ is selected from a group consisting of the following groups: hydrogen, $-C_{1\sim6}$alkyl, halo-$C_{1\sim6}$alkyl, $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring);
$R^{Y2}$ is selected from a group consisting of the following groups: hydrogen, halogen, cyano, hydroxyl, $-C_{1\sim6}$alkyl, halo-$C_{1\sim6}$alkyl, -O($C_{1\sim6}$alkyl), -O(halo-$C_{1\sim6}$alkyl), $-NH_2$, -NH($C_{1\sim6}$alkyl), -N($C_{1\sim6}$alkyl) ($C_{1\sim6}$alkyl), $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring);
$R^{Z1}$ is selected from a group consisting of the following groups: hydrogen, $C_{1\sim6}$alkyl, halo-$C_{1\sim6}$alkyl, $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring);
$R^{Z2}$ is selected from a group consisting of the following groups: hydrogen, halogen, cyano, hydroxyl, $-C_{1\sim6}$alkyl, halo-$C_{1\sim6}$alkyl, -O($C_{1\sim6}$alkyl), -O(halo-$C_{1\sim6}$alkyl), $-NH_2$, -NH($C_{1\sim6}$alkyl), -N($C_{1\sim6}$alkyl) ($C_{1\sim6}$alkyl), $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring);
Ri is selected from a group consisting of the following groups: hydrogen, halogen, cyano, nitro, amino, hydroxyl, $-C_{1\sim6}$alkyl, -O($C_{1\sim6}$alkyl), halo-$C_{1\sim6}$alkyl, $-C_{0\sim2}$alkylene-$OR^{1b}$, $-C_{0\sim2}$alkylene-C(O)$R^{1b}$, $-C_{0\sim2}$alkylene-C(O)$NR^{1b}R^{1c}$, $-C_{0\sim2}$alkylene-$NR^{1b}R^{1c}$, $-C_{0\sim2}$alkylene-$NR^{1b}$C(O)$R^{1c}$, $-C_{0\sim4}$alkylene-$S(O)_2R^{1b}$, $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring); wherein said alkyl, alkylene, cycloalkyl, heterocycloalkyl, aryl ring, heteroaryl ring is optionally further substituted by one, two or three independent $R^{1b}$;
$R^{1b}$, $R^{1c}$ is each independently selected from a group consisting of the following groups: hydrogen, -OH, $C_{1\sim6}$alkyl, halo-$C_{1\sim6}$alkyl, -O($C_{1\sim6}$alkyl), -O(halo-$C_{1\sim6}$alkyl), $-NH_2$, -NH($C_{1\sim6}$alkyl), -N($C_{1\sim6}$alkyl) ($C_{1\sim6}$alkyl), $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring);
$R_2$ is selected from a group consisting of the following groups: $-C_{0\sim2}$alkylene-(3~10 membered cycloalkyl), $-C_{0\sim2}$alkylene-(3~10 membered heterocycloalkyl); wherein said alkylene, cycloalkyl, heterocycloalkyl is optionally further substituted by one, two or three independent $R^{2b}$;
$R^{2b}$ is each independently selected from a group consisting of the following groups: hydrogen, halogen, cyano, nitro, amino, hydroxyl, $-C_{1\sim6}$alkyl, -O($C_{1\sim6}$alkyl), halo-$C_{1\sim6}$alkyl, $-C_{0\sim2}$alkylene-$OR^{2c}$, $-C_{0\sim2}$alkylene-C(O)$R^{2c}$, $-C_{0\sim2}$alkylene-C(O)$NR^{2c}R^{2d}$, $-C_{0\sim2}$alkylene-$NR^{2c}R^{2d}$, $-C_{0\sim2}$alkylene-$NR^{2c}$C(O)$R^{2d}$, $-C_{0\sim4}$alkylene-$S(O)_2R^{2c}$, $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring); wherein alkyl, alkylene, cycloalkyl, heterocycloalkyl, aryl ring, heteroaryl ring is optionally further substituted by one, two or three independent $R^{2c}$;
$R^{2c}$, $R^{2d}$ is each independently selected from a group consisting of hydrogen, cyano, nitro, -OH, halogen, $-C_{1\sim6}$alkyl, halo-$C_{1\sim6}$alkyl, $-C_{0\sim2}$alkylene-O($C_{1\sim6}$alkyl), -O(halo-$C_{1\sim6}$alkyl), -O(3~10 membered cycloalkyl), -O(3~10 membered heterocycloalkyl), $-NH_2$, -NH($C_{1\sim6}$alkyl), -N($C_{1\sim6}$alkyl) ($C_{1\sim6}$alkyl), $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring); wherein said alkyl, alkylene, cycloalkyl, heterocycloalkyl, aryl ring, heteroaryl ring is optionally further substituted by one, two or three independent $R^{2e}$;
$R^{2e}$ is each independently selected from a group consisting of hydrogen, cyano, nitro, -OH, halogen, $-C_{1\sim6}$alkyl, halo-$C_{1\sim6}$alkyl, $-C_{0\sim2}$alkylene-O($C_{1\sim6}$alkyl), -O(halo-$C_{1\sim6}$alkyl), -O(3~10 membered cycloalkyl), -O(3~10 membered heterocycloalkyl), $-NH_2$, -NH($C_{1-6}$alkyl), -N($C_{1\sim6}$alkyl) ($C_{1\sim6}$alkyl), $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$al-

kylene-(5~10 membered heteroaryl ring);

$R_3$ is selected from a group consisting of the following groups: hydrogen, halogen, cyano, nitro, amino, -OH, $-C_{1\sim6}$alkyl, $-O(C_{1\sim6}$alkyl), halo-$C_{1\sim6}$alkyl, $-C_{0\sim2}$alkylene-$OR^{3b}$, $-C_{0\sim2}$alkylene-$C(O)R^{3b}$, $-C_{0\sim2}$alkylene-C(O) $NR^{3b}R^{3c}$, $-C_{0\sim2}$alkylene-$NR^{3b}R^{3c}$, $-C_{0\sim2}$alkylene-$NR^{3b}C(O)R^{3c}$, $-C_{0\sim4}$alkylene-$S(O)_2R^{3b}$, $-C_{0\sim2}$alkylene-(3~10 membered cycloalkyl), $-C_{0\sim2}$alkylene-(3~10 membered heterocycloalkyl);

$R^{3b}$, $R^{3c}$ is each independently selected from a group consisting of the following groups: hydrogen, halogen, cyano, nitro, -OH, $C_{1\sim6}$alkyl, halo-$C_{1\sim6}$alkyl, $-O(C_{1\sim6}$alkyl), -O(halo-$C_{1\sim6}$alkyl), $-NH_2$, $-NH(C_{1\sim6}$alkyl), $-N(C_{1\sim6}$alkyl) ($C_{1\sim6}$alkyl), $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring);

$R_4$ is selected from a group consisting of the following groups: hydrogen, halogen, cyano, nitro, hydroxyl, $-C_{1\sim6}$alkyl, halo-$C_{1\sim6}$alkyl, $-O(C_{1\sim6}$alkyl), -O(halo-$C_{1\sim6}$alkyl), $-NH_2$, $-NH(C_{1\sim6}$alkyl), $-N(C_{1\sim6}$alkyl) ($C_{1\sim6}$alkyl), $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring); wherein said alkyl, alkylene, cycloalkyl, heterocycloalkyl, aryl ring, heteroaryl ring is optionally further substituted by one, two or three independent $R^{4b}$;

$R^{4b}$ is each independently selected from a group consisting of the following groups: hydrogen, halogen, cyano, nitro, -OH, $C_{1\sim6}$alkyl, halo-$C_{1\sim6}$alkyl, $-O(C_{1\sim6}$alkyl), -O(halo-$C_{1\sim6}$alkyl), $-NH_2$, $-NH(C_{1\sim6}$alkyl), $-N(C_{1\sim6}$alkyl) ($C_{1\sim6}$alkyl), $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring);

$R_5$ is selected from a group consisting of the following groups: hydrogen, halogen, cyano, nitro, hydroxyl, $-C_{1\sim6}$alkyl, halo-$C_{1\sim6}$alkyl, $-O(C_{1\sim6}$alkyl), -O(halo-$C_{1\sim6}$alkyl), $-NH_2$, $-NH(C_{1\sim6}$alkyl), $-N(C_{1\sim6}$alkyl) ($C_{1\sim6}$alkyl), $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring);

$R_6$ is selected from a group consisting of the following groups: hydrogen, halogen, cyano, nitro, hydroxyl, $-C_{1\sim6}$alkyl, halo-$C_{1\sim6}$alkyl, $-O(C_{1\sim6}$alkyl), -O(halo-$C_{1\sim6}$alkyl), $-NH_2$, $-NH(C_{1\sim6}$alkyl), $-N(C_{1\sim6}$alkyl) ($C_{1\sim6}$alkyl), $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring);

$R_7$ is selected from a group consisting of the following groups: hydrogen, $-C_{1\sim6}$alkyl, halo-$C_{1\sim6}$alkyl, $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring);

Rs is selected from a group consisting of the following groups: hydrogen, halogen, cyano, nitro, -OH, Ciealkyl, halo-$C_{1\sim6}$alkyl, $-O(C_{1\sim6}$alkyl), -O(halo-$C_{1\sim6}$alkyl), $-NH_2$, $-NH(C_{1\sim6}$alkyl), $-N(C_{1\sim6}$alkyl) ($C_{1\sim6}$alkyl), $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring);

or, $R^7$, $R^8$ and the atom directly bound thereto form a 4~10 membered heterocyclic ring; wherein said heterocyclic ring is optionally further substituted by one, two or three independent $R^{71}$;

each $R^{71}$ is each independently selected from a group consisting of the following groups: hydrogen, halogen, cyano, nitro, -OH, $C_{1\sim6}$alkyl, halo-$C_{1\sim6}$alkyl, $-O(C_{1\sim6}$alkyl), -O(halo-$C_{1\sim6}$alkyl), $-NH_2$, $-NH(C_{1\sim6}$alkyl), $-N(C_{1\sim6}$alkyl) ($C_{1\sim6}$alkyl), $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring).

**2.** The compound, or a deuterated compound thereof, or a stereoisomer thereof, or a pharmaceutically acceptable salt thereof according to claim 1, wherein said $R_1$, $R_3$, $R_4$, $R_5$, $R_6$, $R_7$, $R_8$ is each independently selected from a group consisting of hydrogen, halogen, $-C_{1\sim3}$alkyl, halo-$C_{1\sim3}$alkyl.

**3.** The compound, or a deuterated compound thereof, or a stereoisomer thereof, or a pharmaceutically acceptable salt thereof according to claim 2, wherein said $R_1$, $R_3$, $R_4$, $R_5$, $R_6$, $R_7$, $R_8$ is each independently selected from a group consisting of hydrogen, Cl, methyl, ethyl, $-CF_3$.

**4.** The compound, or a deuterated compound thereof, or a stereoisomer thereof, or a pharmaceutically acceptable salt thereof according to claim 1, wherein:

$R_2$ is selected from a group consisting of -(3~10 membered cycloalkyl), -(3~10 membered heterocycloalkyl); wherein said cycloalkyl, heterocycloalkyl is optionally further substituted by one, two or three independent $R^{2b}$;

$R^{2b}$ is selected from a group consisting of hydrogen, halogen, $-C_{1\sim6}$alkyl, $-NR^{2c}R^{2d}$, $-NR^{2c}C(O)R^{2d}$, $-C_{0\sim2}$alkylene-(3~10 membered cycloalkyl), $-C_{0\sim2}$alkylene-(3~10 membered heterocycloalkyl); wherein said alkyl, cycloalkyl, heterocycloalkyl is optionally further substituted by one, two or three independent $R^{2c}$;

$R^{2c}$, $R^{2d}$ is each independently selected from a group consisting of hydrogen, halogen, $-C_{1\sim6}$alkyl, halo-$C_{1\sim6}$alkyl,

-O($C_{1\sim6}$alkyl), -O(halo-$C_{1\sim6}$alkyl), -O(3~10 membered cycloalkyl), -(3~10 membered cycloalkyl), -(3~10 membered heterocycloalkyl); wherein said alkyl, cycloalkyl, heterocycloalkyl is optionally further substituted by one, two or three independent $R^{2e}$;

$R^{2e}$ is each independently selected from a group consisting of hydrogen, halogen, -O($C_{1\sim6}$alkyl);

$R_2$ is selected from a group consisting of

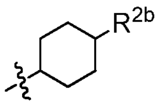 or 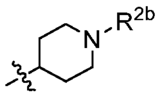

**5.** The compound, or a deuterated compound thereof, or a stereoisomer thereof, or a pharmaceutically acceptable salt thereof according to claim 4, wherein: $R_2$ is specifically selected from a group consisting of

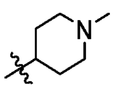, 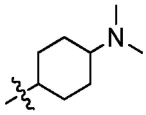, 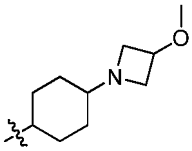,

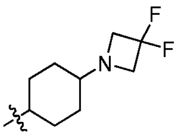, 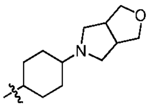, 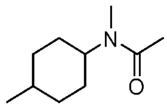, 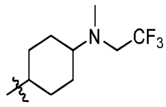, 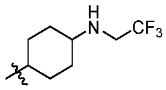, 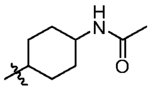,

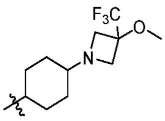, 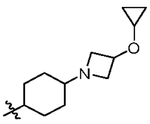, 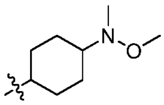, 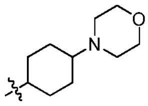, 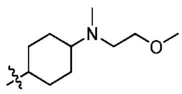, 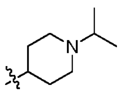, 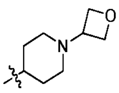,

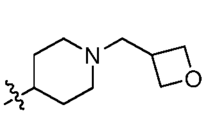, 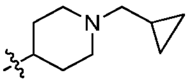, 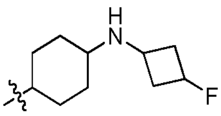

**6.** The compound, or a deuterated compound thereof, or a stereoisomer thereof, or a pharmaceutically acceptable salt thereof according to any one of claims 1 to 5, wherein: $R_2$ is specifically selected from a group consisting of

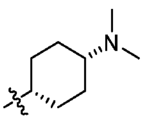, 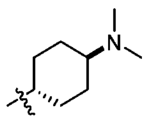, 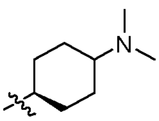,

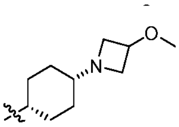, 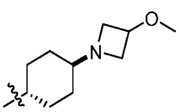, 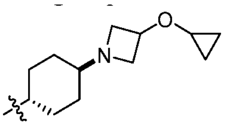, 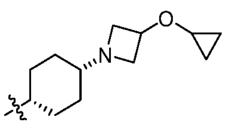, 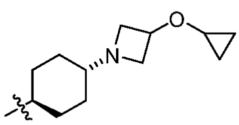,

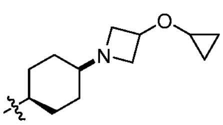, 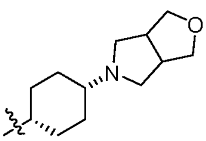, 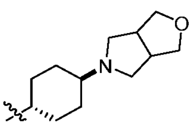, 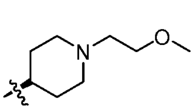, 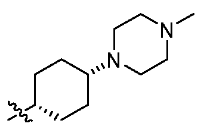,

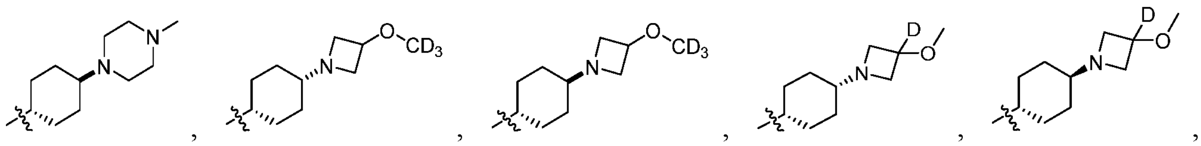

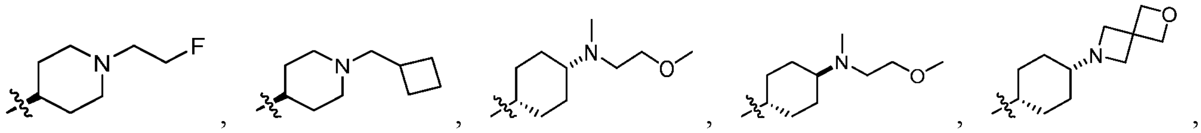

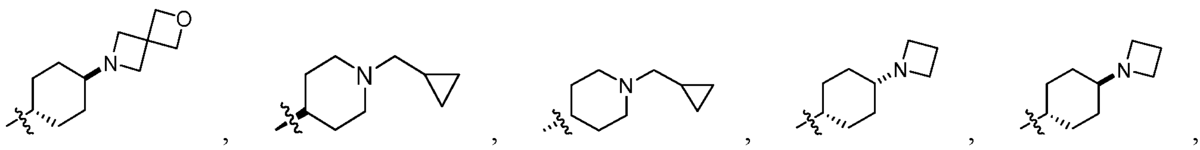

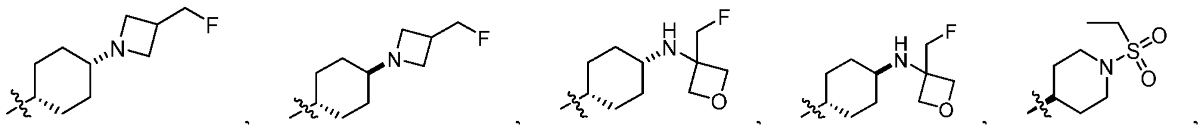

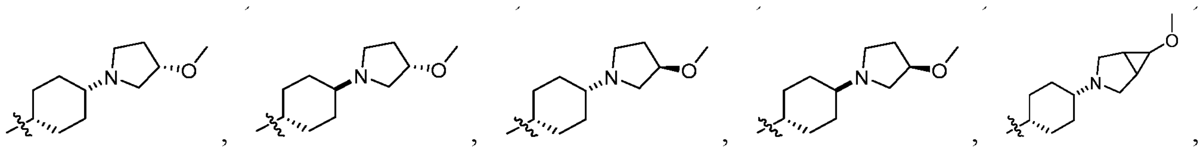

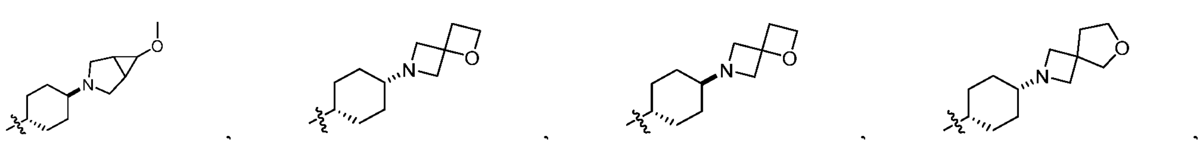

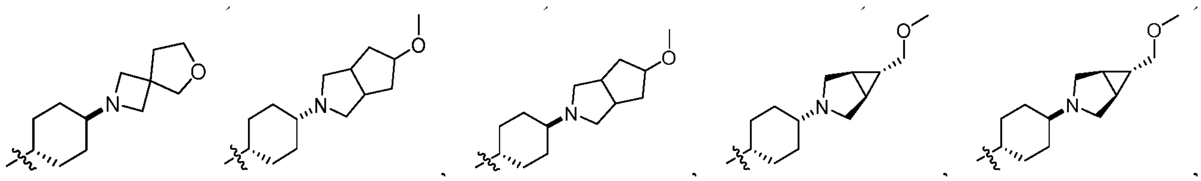

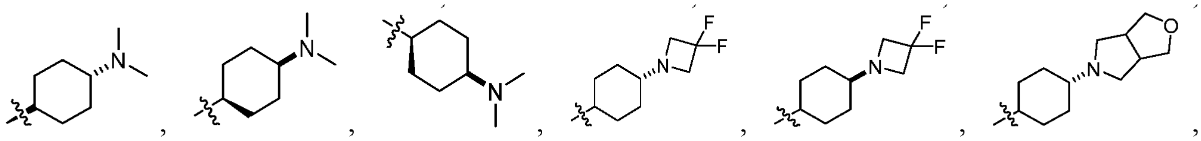

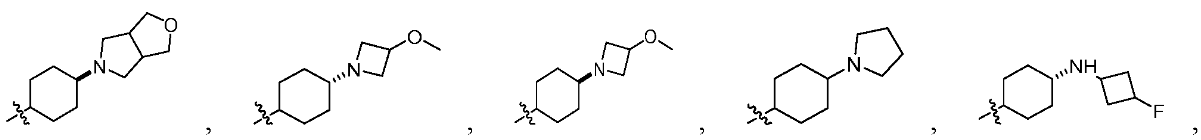

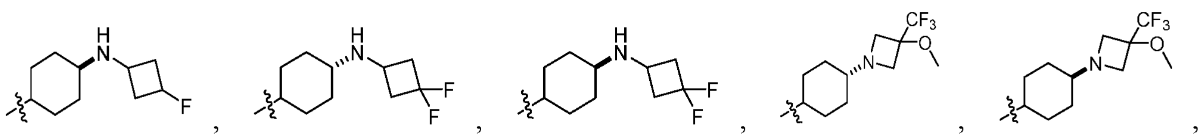

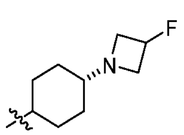, 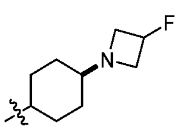, 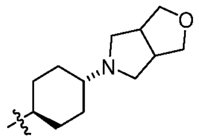, 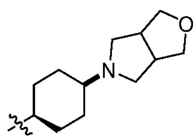, 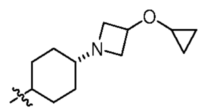,

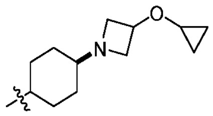, 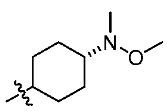, 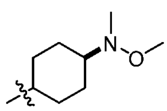, 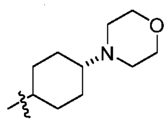, 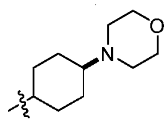, 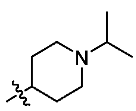,

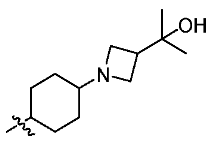, 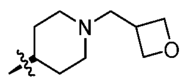, 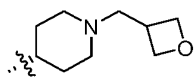, 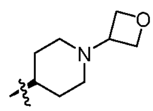, 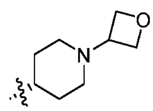,

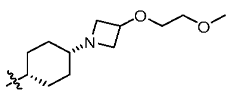, 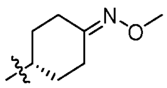, 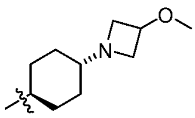, 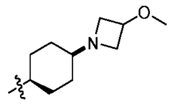, 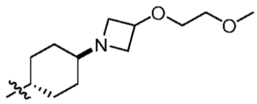,

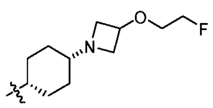, 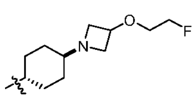, 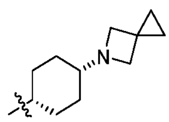, 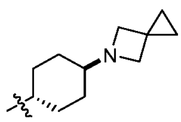, 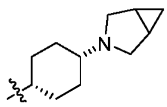, 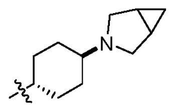,

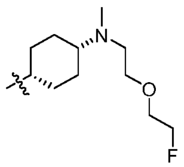, 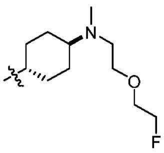, 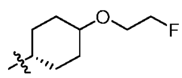, 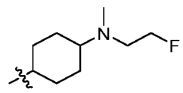, 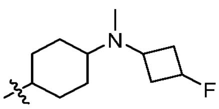, 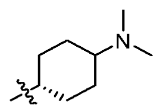,

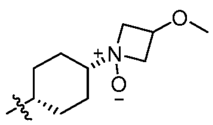, 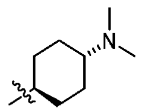, 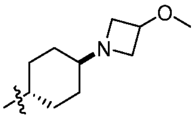, 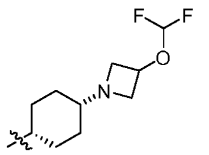, 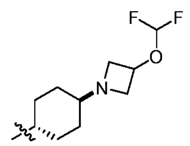,

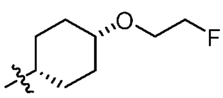, 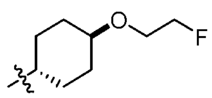

**7.** The compound, or a deuterated compound thereof, or a stereoisomer thereof, or a pharmaceutically acceptable salt thereof according to claim 1, wherein:

X is selected from a group consisting of single bond, O, $CHR^{X2}$; Y is selected from a group consisting of O, $NR^{Y1}$ or $CHR^{Y2}$; Z is selected from a group consisting of -C=O, O, $NR^{Z1}$ or $CHR^{Z2}$; Q is selected from a group consisting of single bond, O or S;
$R^{X2}$ is selected from a group consisting of hydrogen, $-C_{1\sim3}$alkyl;
$R^{Y1}$ is selected from a group consisting of hydrogen, $-C_{1\sim3}$alkyl, halo-$C_{1\sim3}$alkyl;
$R^{Y2}$ is selected from a group consisting of hydrogen, $-C_{1\sim3}$alkyl, halo-$C_{1\sim3}$alkyl;
$R^{Z1}$ is selected from a group consisting of hydrogen, $-C_{1\sim3}$alkyl, -(3~6 membered cycloalkyl), halo-$C_{1\sim3}$alkyl;
$R^{Z2}$ is selected from a group consisting of hydrogen, $-C_{1\sim3}$alkyl, halo-$C_{1\sim3}$alkyl.

**8.** The compound, or a deuterated compound thereof, or a stereoisomer thereof, or a pharmaceutically acceptable salt thereof according to claim 7, wherein:
$R^{Y1}$ is selected from a group consisting of methyl; $R^{Y2}$ is selected from a group consisting of hydrogen, methyl; $R^{Z1}$ is selected from a group consisting of hydrogen, methyl, ethyl, cyclopropyl.

**9.** The compound, or a deuterated compound thereof, or a stereoisomer thereof, or a pharmaceutically acceptable salt thereof according to claim 1, including the following compounds represented by Formula IIa:

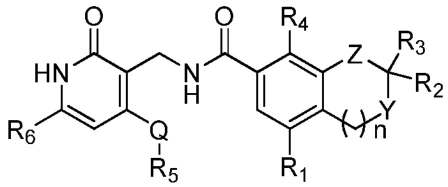

Formula IIa

Q is selected from a group consisting of single bond, O or S;
Z is selected from a group consisting of C=O, O, $NR^{Z1}$ or $CHR^{Z2}$;
Y is selected from a group consisting of O, $NR^{Y1}$ or $CHR^{Y2}$; n is 0 or 1;
when n is 0, Y and Z are not both O;
$R^{Y1}$ is selected from a group consisting of hydrogen, $-C_{1\sim6}$alkyl, halo-$C_{1\sim6}$alkyl, $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring);
$R^{Y2}$ is selected from a group consisting of hydrogen, halogen, cyano, hydroxyl, $-C_{1\sim6}$alkyl, halo-$C_{1\sim6}$alkyl, -O($C_{1\sim6}$alkyl), -O(halo-$C_{1\sim6}$alkyl), $-NH_2$, -NH($C_{1\sim6}$alkyl), -N($C_{1\sim6}$alkyl) ($C_{1\sim6}$alkyl), $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring);
$R^{Z1}$ is selected from a group consisting of hydrogen, $-C_{1\sim6}$alkyl, halo-$C_{1\sim6}$alkyl, $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring);
$R^{Z2}$ is selected from a group consisting of hydrogen, halogen, cyano, hydroxyl, $-C_{1\sim6}$alkyl, halo-$C_{1\sim6}$alkyl, -O($C_{1\sim6}$alkyl), -O(halo-$C_{1\sim6}$alkyl), $-NH_2$, -NH($C_{1\sim6}$alkyl), -N($C_{1\sim6}$alkyl) ($C_{1\sim6}$alkyl), $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring);
$R_1$ is selected from a group consisting of hydrogen, halogen, cyano, nitro, amino, hydroxyl, $-C_{1\sim6}$alkyl, halo-$C_{1\sim6}$alkyl, $-C_{0\sim2}$alkylene-$OR^{1b}$, $-C_{0\sim2}$alkylene-C(O)$R^{1b}$, $-C_{0\sim2}$alkylene-C(O)$NR^{1b}R^{1c}$, $-C_{0\sim2}$alkylene-$NR^{1b}R^{1c}$, $-C_{0\sim2}$alkylene-$NR^{1b}C(O)R^{1c}$, $-C_{0\sim4}$alkylene-$S(O)_2R^{1b}$, $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring); wherein said alkyl, alkylene, cycloalkyl, heterocycloalkyl, aryl ring, heteroaryl ring is optionally further substituted by one, two or three independent $R^{1b}$;
$R^{1b}$, $R^{1c}$ is each independently selected from a group consisting of hydrogen, -OH, $C_{1\sim6}$alkyl, halo-$C_{1\sim6}$alkyl, -O($C_{1\sim6}$alkyl), -O(halo-$C_{1\sim6}$alkyl), $-NH_2$, -NH($C_{1\sim6}$alkyl), -N($C_{1\sim6}$alkyl) ($C_{1\sim6}$alkyl), $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring);
$R_2$ is selected from a group consisting of $-C_{0\sim2}$alkylene-(3~10 membered cycloalkyl), $-C_{0\sim2}$alkylene-(3~10 membered heterocycloalkyl); wherein said alkylene, cycloalkyl, heterocycloalkyl is optionally further substituted by one, two or three independent $R^{2b}$;
$R^{2b}$ is each independently selected from a group consisting of hydrogen, halogen, cyano, nitro, amino, hydroxyl, $-C_{1\sim6}$alkyl, -O($C_{1\sim6}$alkyl), halo-$C_{1\sim6}$alkyl, $-C_{0\sim2}$alkylene-$OR^{2c}$, $-C_{0\sim2}$alkylene-C(O)$R^{2c}$, $-C_{0\sim2}$alkylene-C(O)$NR^{2c}R^{2a}$, $-C_{0\sim2}$alkylene-$NR^{2c}R^{2d}$, $-C_{0\sim2}$alkylene-$NR^{2c}C(O)R^{2d}$, $-C_{0\sim4}$alkylene-$S(O)_2R^{2c}$, $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring); wherein alkyl, alkylene, cycloalkyl, heterocycloalkyl, aryl ring, heteroaryl ring is optionally further substituted by one, two or three independent $R^{2c}$;
$R^{2c}$, $R^{2d}$ is each independently selected from a group consisting of hydrogen, cyano, nitro, -OH, halogen, $-C_{1\sim6}$alkyl, halo-$C_{1\sim6}$alkyl, $-C_{0\sim2}$alkylene-O($C_{1\sim6}$alkyl), -O(halo-$C_{1\sim6}$alkyl), -O(3~10 membered cycloalkyl), -O(3~10 membered heterocycloalkyl), $-NH_2$, -NH($C_{1\sim6}$alkyl), -N($C_{1\sim6}$alkyl) ($C_{1\sim6}$alkyl), $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl

ring), $-C_{0\sim2}$alkylene-(5~10 membered heteroaryl ring); wherein said alkyl, alkylene, cycloalkyl, heterocycloalkyl, aryl ring, heteroaryl ring is optionally further substituted by one, two or three independent $R^{2e}$;

$R^{2e}$ is each independently selected from a group consisting of hydrogen, cyano, nitro, -OH, halogen, $-C_{1\sim6}$alkyl, halo-$C_{1\sim6}$alkyl, $-C_{0\sim2}$alkylene-O($C_{1\sim6}$alkyl), -O(halo-$C_{1\sim6}$alkyl), -O(3~10 membered cycloalkyl), -O(3~10 membered heterocycloalkyl), $-NH_2$, -NH($C_{1\sim6}$alkyl), -N($C_{1\sim6}$alkyl) ($C_{1\sim6}$alkyl), $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring);

$R_3$ is selected from a group consisting of hydrogen, halogen, cyano, nitro, amino, -OH, $-C_{1\sim6}$alkyl, -O($C_{1\sim6}$alkyl), halo-$C_{1\sim6}$alkyl, $-C_{0\sim2}$alkylene-$OR^{3b}$, $-C_{0\sim2}$alkylene-C(O)$R^{3b}$, $-C_{0\sim2}$alkylene-C(O)$NR^{3b}R^{3c}$, $-C_{0\sim2}$alkylene-$NR^{3b}R^{3c}$, $-C_{0\sim2}$alkylene-$NR^{3b}$C(O)$R^{3c}$, $-C_{0\sim4}$alkylene-$S(O)_2R^{3b}$, $-C_{0\sim2}$alkylene-(3~10 membered cycloalkyl), $-C_{0\sim2}$alkylene-(3~10 membered heterocycloalkyl);

$R^{3b}$, $R^{3c}$ is each independently selected from a group consisting of hydrogen, halogen, cyano, nitro, -OH, $C_{1\sim6}$alkyl, halo-$C_{1\sim6}$alkyl, -O($C_{1\sim6}$alkyl), -O(halo-$C_{1\sim6}$alkyl), $-NH_2$, -NH($C_{1\sim6}$alkyl), -N($C_{1\sim6}$alkyl) ($C_{1\sim6}$alkyl), $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring);

$R_4$ is selected from a group consisting of hydrogen, halogen, cyano, nitro, hydroxyl, $-C_{1\sim6}$alkyl, halo-$C_{1\sim6}$alkyl, -O($C_{1\sim6}$alkyl), -O(halo-$C_{1\sim6}$alkyl), $-NH_2$, -NH($C_{1\sim6}$alkyl), -N($C_{1\sim6}$alkyl) ($C_{1\sim6}$alkyl), $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring); wherein said alkyl, alkylene, cycloalkyl, heterocycloalkyl, aryl ring, heteroaryl ring is optionally further substituted by one, two or three independent $R^{4b}$;

$R^{4b}$ is each independently selected from a group consisting of hydrogen, halogen, cyano, nitro, -OH, Ciealkyl, halo-$C_{1\sim6}$alkyl, -O($C_{1\sim6}$alkyl), -O(halo-$C_{1\sim6}$alkyl), $-NH_2$, -NH($C_{1\sim6}$alkyl), -N($C_{1\sim6}$alkyl) ($C_{1\sim6}$alkyl), $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring);

$R_5$ is selected from a group consisting of hydrogen, halogen, cyano, nitro, hydroxyl, $-C_{1\sim6}$alkyl, halo-$C_{1\sim6}$alkyl, -O($C_{1\sim6}$alkyl), -O(halo-Ciealkyl), $-NH_2$, -NH($C_{1\sim6}$alkyl), -N($C_{1\sim6}$alkyl) ($C_{1\sim6}$alkyl), $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring);

$R_6$ is selected from a group consisting of hydrogen, halogen, cyano, nitro, hydroxyl, $-C_{1\sim6}$alkyl, halo-$C_{1\sim6}$alkyl, -O($C_{1\sim6}$alkyl), -O(halo-Ciealkyl), $-NH_2$, -NH($C_{1\sim6}$alkyl), -N($C_{1\sim6}$alkyl) ($C_{1\sim6}$alkyl), $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring).

**10.** The compound, or a deuterated compound thereof, or a stereoisomer thereof, or a pharmaceutically acceptable salt thereof according to claim 9, wherein said $R_1$, $R_3$, $R_4$, $R_5$, $R_6$ is each independently selected from a group consisting of hydrogen, halogen, $-C_{1\sim3}$alkyl;

Q is selected from a group consisting of single bond, O or S; Z is selected from a group consisting of C=O, O, $NR^{Z1}$ or $CHR^{Z2}$; Y is selected from a group consisting of O or $NR^{Y1}$;

$R^{Z1}$ is selected from a group consisting of hydrogen, $-C_{1\sim3}$alkyl, -(3~6 membered cycloalkyl), halo-$C_{1\sim3}$alkyl;

$R^{Z2}$ is selected from a group consisting of hydrogen, $-C_{1\sim3}$alkyl, halo-$C_{1\sim3}$alkyl;

$R^{Y1}$ is selected from a group consisting of hydrogen, $-C_{1\sim3}$alkyl, halo-$C_{1\sim3}$alkyl.

**11.** The compound, or a deuterated compound thereof, or a stereoisomer thereof, or a pharmaceutically acceptable salt thereof according to claim 9, wherein: said $R_2$ is specifically selected from a group consisting of

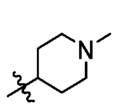 , 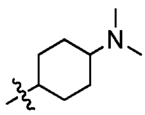 , 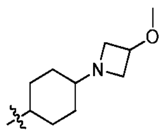 ,

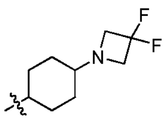 , 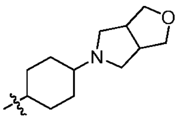 , 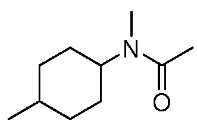 , 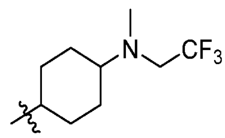 , 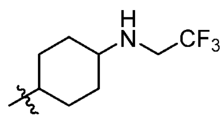 ,

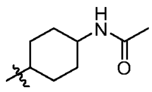, 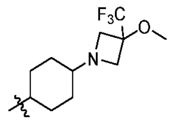, 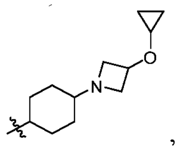, 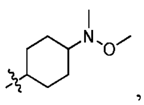, 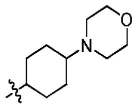, 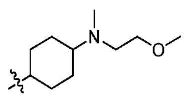, 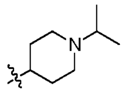,

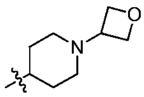, 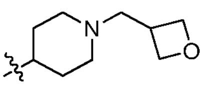, 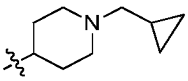, 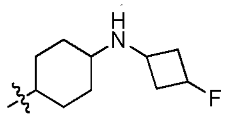;

preferably, said $R_2$ is specifically selected from a group consisting of

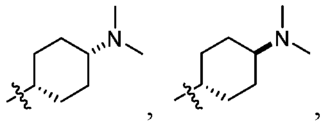

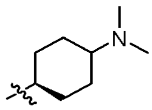, 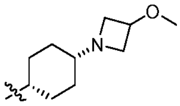, 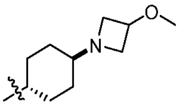, 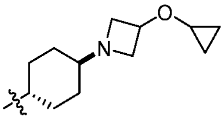, 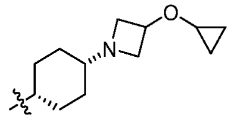,

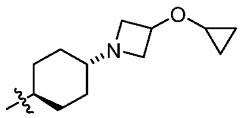, 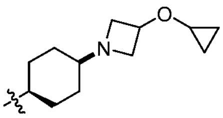, 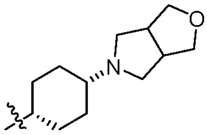, 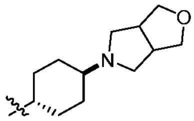, 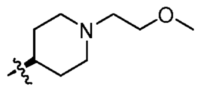,

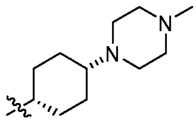, 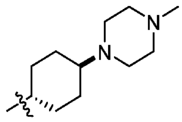, 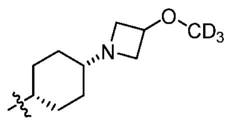, 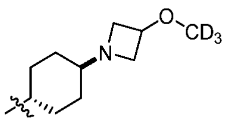, 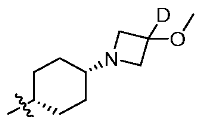,

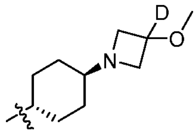, 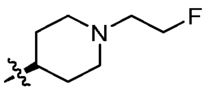, 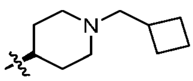, 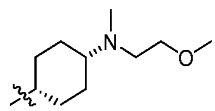, 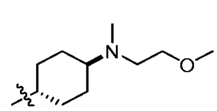,

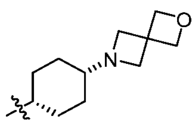, 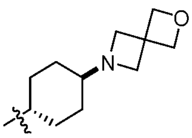, 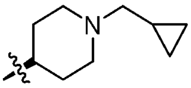, 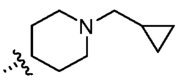, 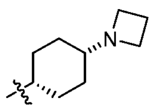, 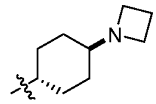,

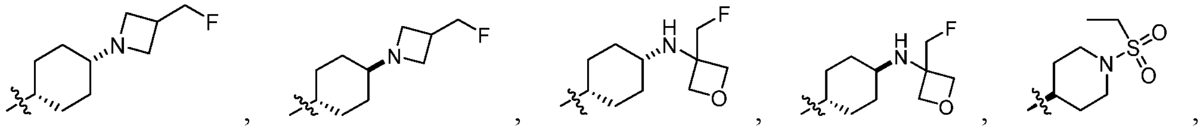

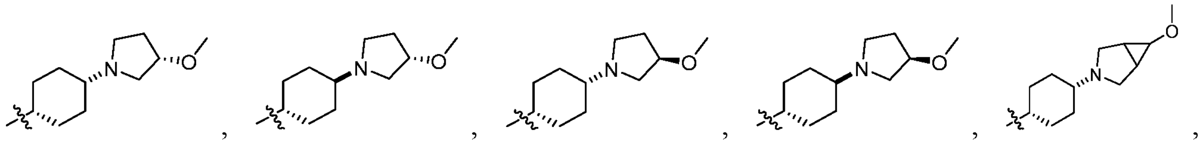

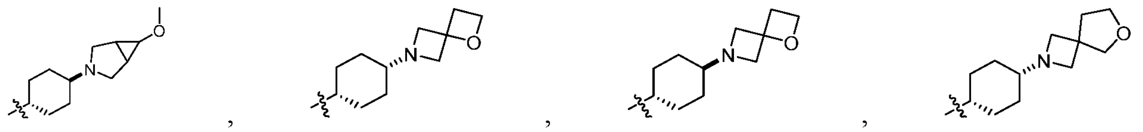 , , , ,

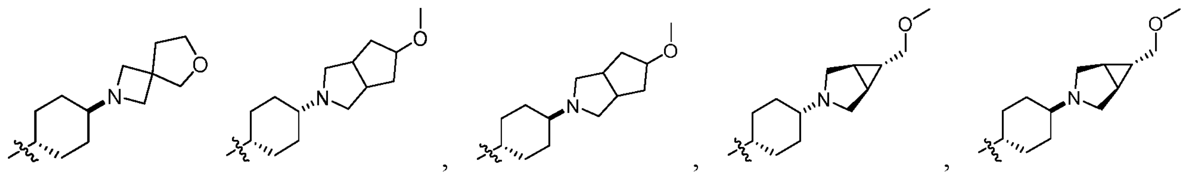 , , , ,

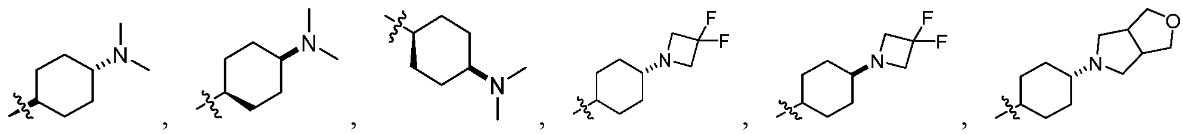 , , , , , ,

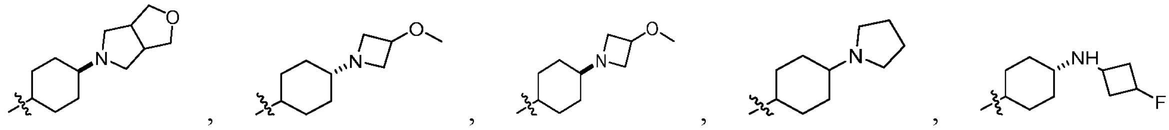 , , , , ,

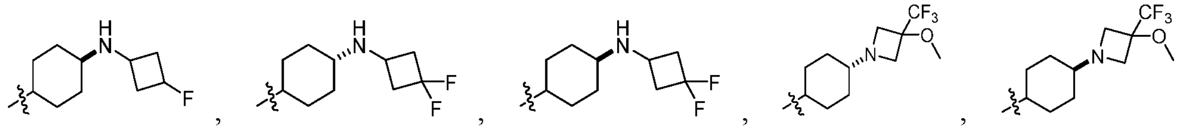 , , , , ,

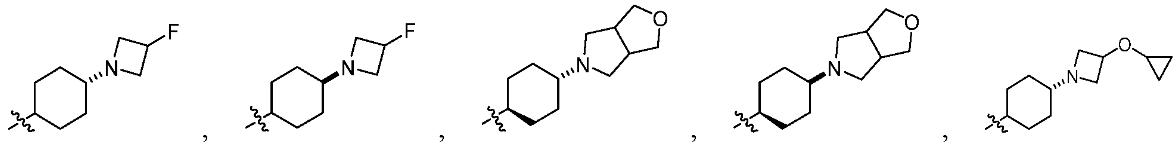 , , , , ,

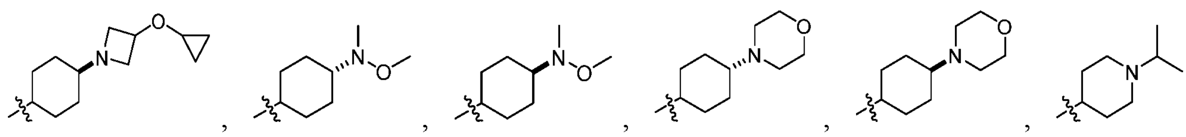 , , , , , ,

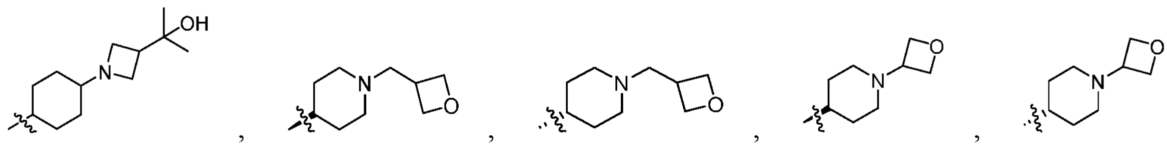 , , , , ,

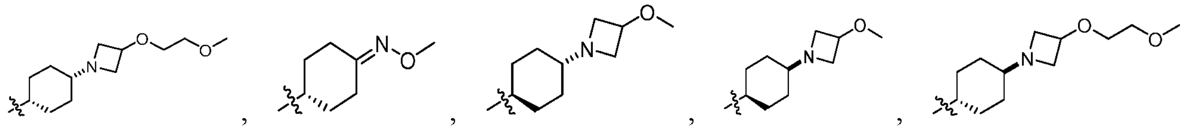 , , , , ,

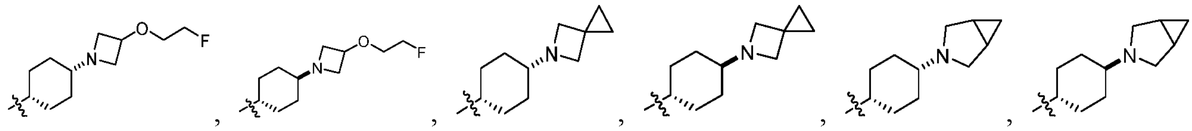 , , , , , ,

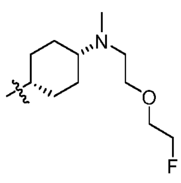, 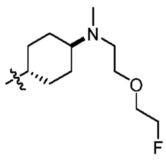, 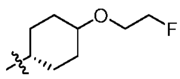, 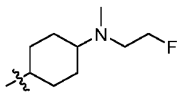, 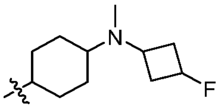, 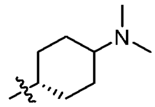,

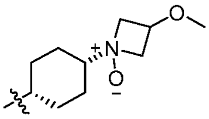, 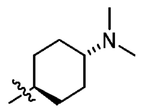, 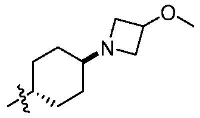, 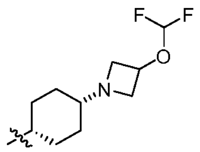, 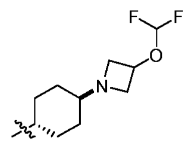,

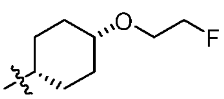, 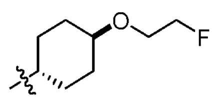

**12.** The compound, or a deuterated compound thereof, or a stereoisomer thereof, or a pharmaceutically acceptable salt thereof according to claim 1, including the following compounds represented by Formula IIb:

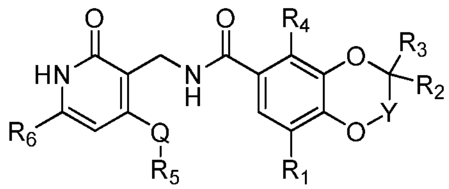

Formula IIb

Q is selected from a group consisting of single bond, O or S; Y is selected from a group consisting of O, $NR^{Y1}$ or $CHR^{Y2}$;

$R^{Y1}$ is selected from a group consisting of hydrogen, $-C_{1\sim6}$alkyl, halo-$C_{1\sim6}$alkyl, $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring);

$R^{Y2}$ is selected from a group consisting of hydrogen, halogen, cyano, hydroxyl, $-C_{1\sim6}$alkyl, halo-$C_{1\sim6}$alkyl, $-O(C_{1\sim6}$alkyl), -O(halo-$C_{1\sim6}$alkyl), $-NH_2$, $-NH(C_{1\sim6}$alkyl), $-N(C_{1\sim6}$alkyl) ($C_{1\sim6}$alkyl), $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring);

Ri is selected from a group consisting of hydrogen, halogen, cyano, nitro, amino, hydroxyl, $-C_{1\sim6}$alkyl, $-O(C_{1\sim6}$alkyl), halo-$C_{1\sim6}$alkyl, $-C_{0\sim2}$alkylene-$OR^{1b}$, $-C_{0\sim2}$alkylene-$C(O)R^{1b}$, $-C_{0\sim2}$alkylene-$C(O)NR^{1b}R^{1c}$, $-C_{0\sim2}$alkylene-$NR^{1b}R^{1c}$, $-C_{0\sim2}$alkylene-$NR^{1b}C(O)R^{1c}$, $-C_{0\sim4}$alkylene-$S(O)_2R^{1b}$, $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring); wherein said alkyl, alkylene, cycloalkyl, heterocycloalkyl, aryl ring, heteroaryl ring is optionally further substituted by one, two or three independent $R^{1b}$;

$R^{1b}$, $R^{1c}$ is each independently selected from a group consisting of hydrogen, -OH, $C_{1\sim6}$alkyl, halo-$C_{1\sim6}$alkyl, $-O(C_{1\sim6}$alkyl), -O(halo-$C_{1\sim6}$alkyl), $-NH_2$, $-NH(C_{1\sim6}$alkyl), $-N(C_{1\sim6}$alkyl) ($C_{1\sim6}$alkyl), $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring);

$R_2$ is selected from a group consisting of $-C_{0\sim2}$alkylene-(3~10 membered cycloalkyl), $-C_{0\sim2}$alkylene-(3~10 membered heterocycloalkyl); wherein said alkylene, cycloalkyl, heterocycloalkyl is optionally further substituted by one, two or three independent $R^{2b}$;

$R^{2b}$ is each independently selected from a group consisting of hydrogen, halogen, cyano, nitro, amino, hydroxyl, $-C_{1\sim6}$alkyl, $-O(C_{1\sim6}$alkyl), halo-$C_{1\sim6}$alkyl, $-C_{0\sim2}$alkylene-$OR^{2c}$, $-C_{0\sim2}$alkylene-$C(O)R^{2c}$, $-C_{0\sim2}$alkylene-$C(O)NR^{2c}R^{2d}$, $-C_{0\sim2}$alkylene-$NR^{2c}R^{2d}$, $-C_{0\sim2}$alkylene-$NR^{2c}C(O)R^{2d}$, $-C_{0\sim4}$alkylene-$S(O)_2R^{2c}$, $-C_{0\sim2}$alkylene-(3~10 membered cycloalkyl), $-C_{0\sim2}$alkylene-(3~10 membered heterocycloalkyl), $-C_{0\sim2}$alkylene-(5~10 membered aryl ring), $-C_{0\sim2}$alkylene-(5~10 membered heteroaryl ring); wherein alkyl, alkylene, cycloalkyl, heterocycloalkyl, aryl ring, heteroaryl ring is optionally further substituted by one, two or three independent $R^{2c}$;

$R^{2c}$, $R^{2d}$ is each independently selected from a group consisting of hydrogen, cyano, nitro, -OH, halogen, -$C_{1\sim6}$alkyl, halo-$C_{1\sim6}$alkyl, -$C_{0\sim2}$alkylene-O($C_{1\sim6}$alkyl), -O(halo-$C_{1\sim6}$alkyl), -O(3~10 membered cycloalkyl), -O(3~10 membered heterocycloalkyl), -$NH_2$, -NH($C_{1\sim6}$alkyl), -N($C_{1\sim6}$alkyl) ($C_{1\sim6}$alkyl), -$C_{0\sim2}$alkylene-(3~10 membered cycloalkyl), -$C_{0\sim2}$alkylene-(3~10 membered heterocycloalkyl), -$C_{0\sim2}$alkylene-(5~10 membered aryl ring), -$C_{0\sim2}$alkylene-(5~10 membered heteroaryl ring); wherein said alkyl, alkylene, cycloalkyl, heterocycloalkyl, aryl ring, heteroaryl ring is optionally further substituted by one, two or three independent $R^{2e}$;

$R^{2e}$ is each independently selected from a group consisting of hydrogen, cyano, nitro, -OH, halogen, -$C_{1\sim6}$alkyl, halo-$C_{1\sim6}$alkyl, -$C_{0\sim2}$alkylene-O($C_{1\sim6}$alkyl), -O(halo-$C_{1\sim6}$alkyl), -O(3~10 membered cycloalkyl), -O(3~10 membered heterocycloalkyl), -$NH_2$, -NH($C_{1\sim6}$alkyl), -N($C_{1\sim6}$alkyl) ($C_{1\sim6}$alkyl), -$C_{0\sim2}$alkylene-(3~10 membered cycloalkyl), -$C_{0\sim2}$alkylene-(3~10 membered heterocycloalkyl), -$C_{0\sim2}$alkylene-(5~10 membered aryl ring), -$C_{0\sim2}$alkylene-(5~10 membered heteroaryl ring);

$R_3$ is selected from a group consisting of hydrogen, halogen, cyano, nitro, amino, -OH, -$C_{1\sim6}$alkyl, -O($C_{1\sim6}$alkyl), halo-$C_{1\sim6}$alkyl, -$C_{0\sim2}$alkylene-$OR^{3b}$, -$C_{0\sim2}$alkylene-C(O)$R^{3b}$, -$C_{0\sim2}$alkylene-C(O)$NR^{3b}R^{3c}$, -$C_{0\sim2}$alkylene-$NR^{3b}R^{3c}$, -$C_{0\sim2}$alkylene-$NR^{3b}$C(O)$R^{3c}$, -$C_{0\sim4}$alkylene-$S(O)_2R^{3b}$, -$C_{0\sim2}$alkylene-(3~10 membered cycloalkyl), -$C_{0\sim2}$alkylene-(3~10 membered heterocycloalkyl);

$R^{3b}$, $R^{3c}$ is each independently selected from a group consisting of hydrogen, halogen, cyano, nitro, -OH, $C_{1\sim6}$alkyl, halo-$C_{1\sim6}$alkyl, -O($C_{1\sim6}$alkyl), -O(halo-$C_{1\sim6}$alkyl), -$NH_2$, -NH($C_{1\sim6}$alkyl), -N($C_{1\sim6}$alkyl) ($C_{1\sim6}$alkyl), -$C_{0\sim2}$alkylene-(3~10 membered cycloalkyl), -$C_{0\sim2}$alkylene-(3~10 membered heterocycloalkyl), -$C_{0\sim2}$alkylene-(5~10 membered aryl ring), -$C_{0\sim2}$alkylene-(5~10 membered heteroaryl ring);

$R_4$ is selected from a group consisting of hydrogen, halogen, cyano, nitro, hydroxyl, -$C_{1\sim6}$alkyl, halo-$C_{1\sim6}$alkyl, -O($C_{1\sim6}$alkyl), -O(halo-$C_{1\sim6}$alkyl), -$NH_2$, -NH($C_{1\sim6}$alkyl), -N($C_{1\sim6}$alkyl) ($C_{1\sim6}$alkyl), -$C_{0\sim2}$alkylene-(3~10 membered cycloalkyl), -$C_{0\sim2}$alkylene-(3~10 membered heterocycloalkyl), -$C_{0\sim2}$alkylene-(5~10 membered aryl ring), -$C_{0\sim2}$alkylene-(5~10 membered heteroaryl ring); wherein said alkyl, alkylene, cycloalkyl, heterocycloalkyl, aryl ring, heteroaryl ring is optionally further substituted by one, two or three independent $R^{4b}$;

$R^{4b}$ is each independently selected from a group consisting of hydrogen, halogen, cyano, nitro, -OH, Ciealkyl, halo-$C_{1\sim6}$alkyl, -O($C_{1\sim6}$alkyl), -O(halo-$C_{1\sim6}$alkyl), -$NH_2$, -NH($C_{1\sim6}$alkyl), -N($C_{1\sim6}$alkyl) ($C_{1\sim6}$alkyl), -$C_{0\sim2}$alkylene-(3~10 membered cycloalkyl), -$C_{0\sim2}$alkylene-(3~10 membered heterocycloalkyl), -$C_{0\sim2}$alkylene-(5~10 membered aryl ring), -$C_{0\sim2}$alkylene-(5~10 membered heteroaryl ring);

$R_5$ is selected from a group consisting of hydrogen, halogen, cyano, nitro, hydroxyl, -$C_{1\sim6}$alkyl, halo-$C_{1\sim6}$alkyl, -O($C_{1\sim6}$alkyl), -O(halo-Ciealkyl), -$NH_2$, -NH($C_{1\sim6}$alkyl), -N($C_{1\sim6}$alkyl) ($C_{1\sim6}$alkyl), -$C_{0\sim2}$alkylene-(3~10 membered cycloalkyl), -$C_{0\sim2}$alkylene-(3~10 membered heterocycloalkyl), -$C_{0\sim2}$alkylene-(5~10 membered aryl ring), -$C_{0\sim2}$alkylene-(5~10 membered heteroaryl ring);

$R_6$ is selected from a group consisting of hydrogen, halogen, cyano, nitro, hydroxyl, -$C_{1\sim6}$alkyl, halo-$C_{1\sim6}$alkyl, -O($C_{1\sim6}$alkyl), -O(halo-Ciealkyl), -$NH_2$, -NH($C_{1\sim6}$alkyl), -N($C_{1\sim6}$alkyl) ($C_{1\sim6}$alkyl), -$C_{0\sim2}$alkylene-(3~10 membered cycloalkyl), -$C_{0\sim2}$alkylene-(3~10 membered heterocycloalkyl), -$C_{0\sim2}$alkylene-(5~10 membered aryl ring), -$C_{0\sim2}$alkylene-(5~10 membered heteroaryl ring).

**13.** The compound, or a deuterated compound thereof, or a stereoisomer thereof, or a pharmaceutically acceptable salt thereof according to claim 12, wherein said $R_1$, $R_3$, $R_4$, $R_5$, $R_6$ is each independently selected from a group consisting of hydrogen, halogen, -$C_{1\sim3}$alkyl;
Q is selected from a group consisting of single bond; Y is selected from a group consisting of $CHR^{Y2}$; $R^{Y2}$ is selected from a group consisting of hydrogen, -$C_{1\sim3}$alkyl.

**14.** The compound, or a deuterated compound thereof, or a stereoisomer thereof, or a pharmaceutically acceptable salt thereof according to claim 12, wherein said $R_2$ is specifically selected from a group consisting of

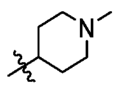 , 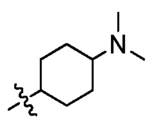 , 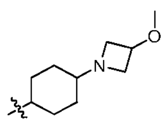 , 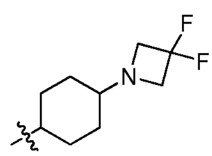 ,

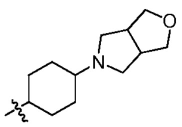 , 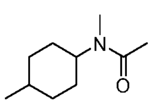 , 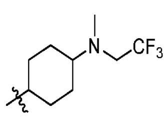 , 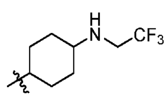 , 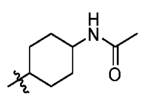 , 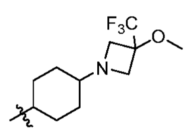 ,

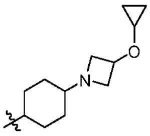, 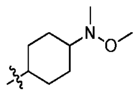, 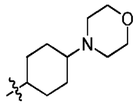, 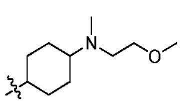, 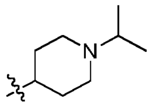, 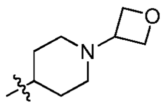,

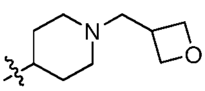, 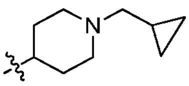, 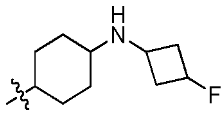;

preferably, said $R_2$ is specifically selected from a group consisting of

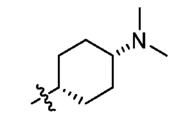, 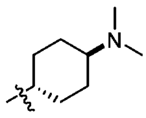,

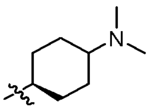, 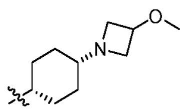, 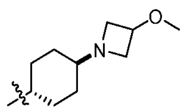, 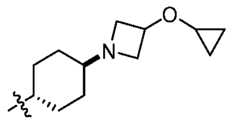, 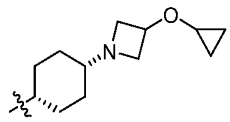,

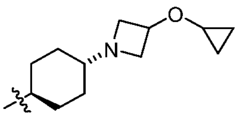, 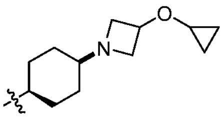, 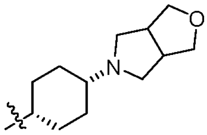, 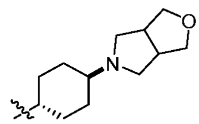, 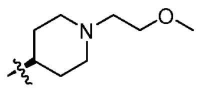,

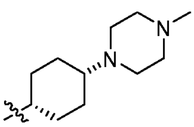, 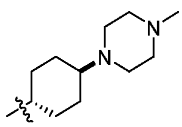, 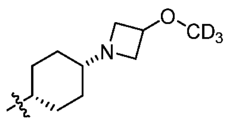, 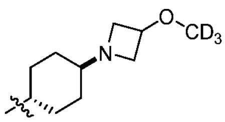, 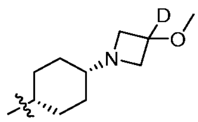,

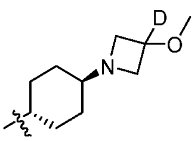, 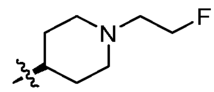, 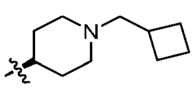, 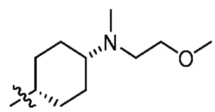, 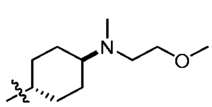,

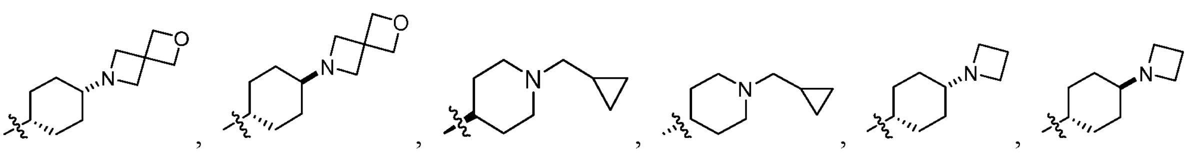
, , , , , ,

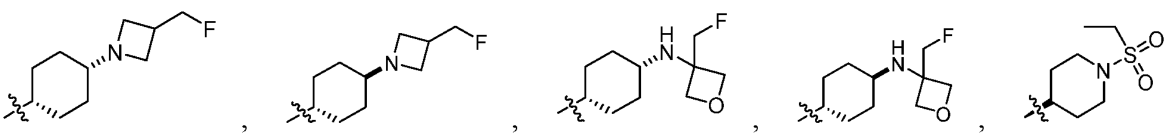
, , , , ,

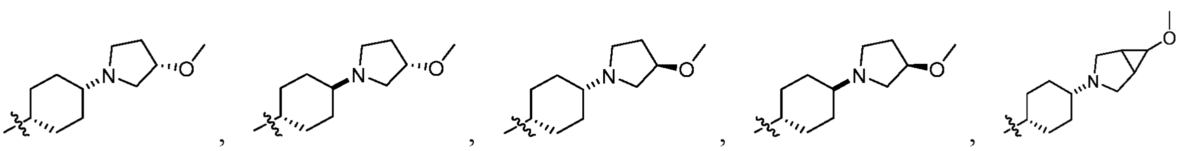
, , , , ,

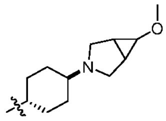, 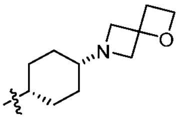, 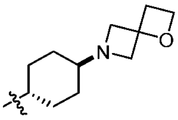, 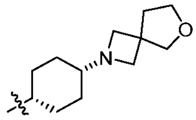,

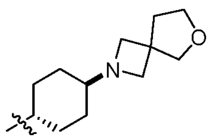 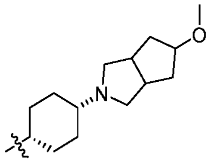, 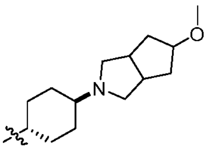, 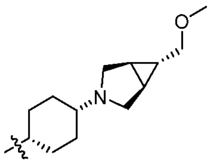, 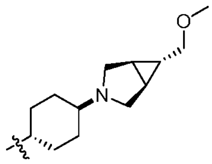,

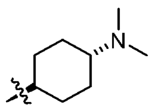, 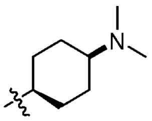, 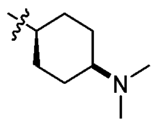, 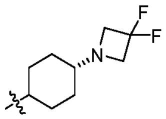, 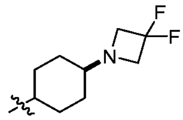, 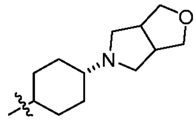,

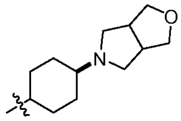, 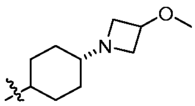, 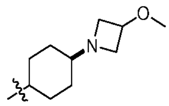, 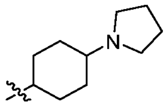, 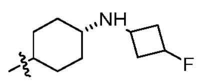,

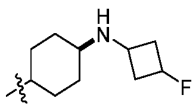, 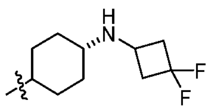, 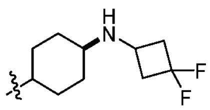, 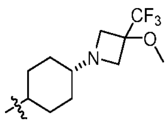, 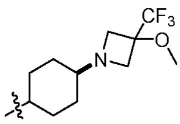,

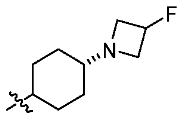, 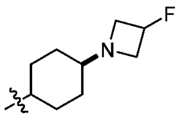, 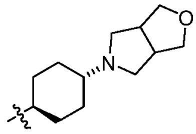, 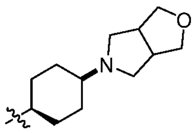, 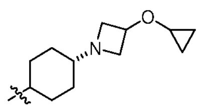,

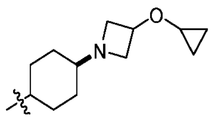, 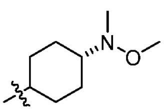, 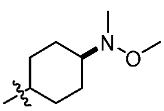, 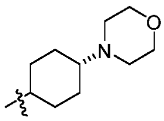, 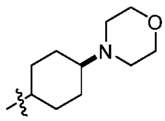, 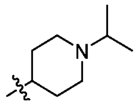,

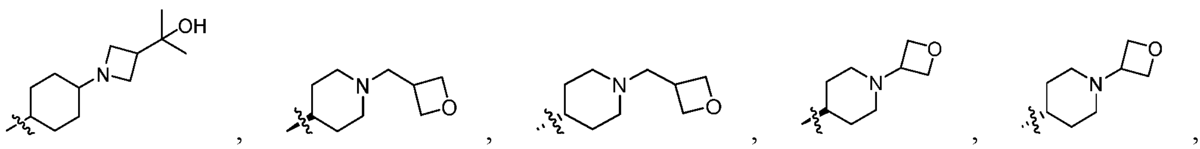, , , , ,

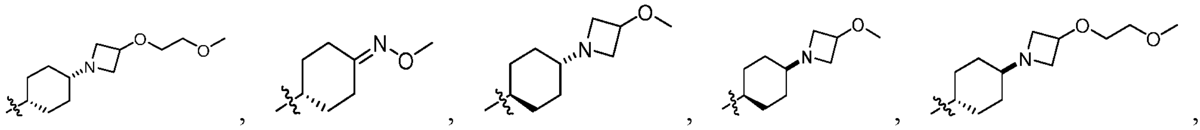, , , , ,

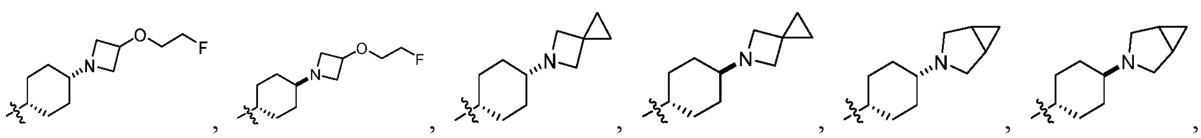, , , , , ,

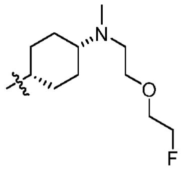, 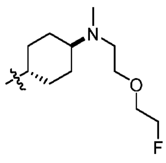, 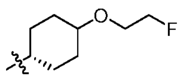, 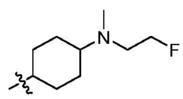, 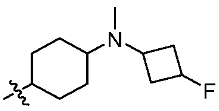, 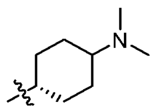,

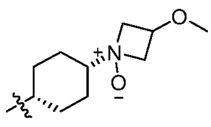, 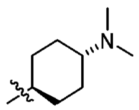, 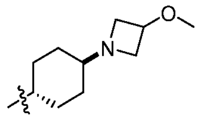, 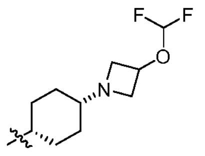, 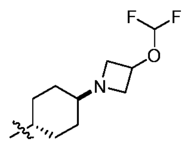,

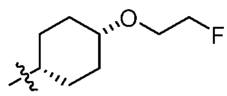, 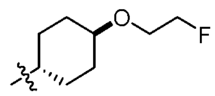

**15.** The compound, or a deuterated compound thereof, or a stereoisomer thereof, or a pharmaceutically acceptable salt thereof according to claim 1, including the following compounds represented by Formula IIc:

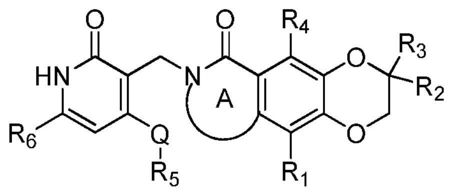

Formula IIc

Q is selected from a group consisting of single bond, O or S;

A-ring is selected from a group consisting of 4~10 membered heterocyclic ring; wherein said heterocyclic ring is optionally further substituted by one, two or three independent $R^{71}$;

each $R^{71}$ is each independently selected from a group consisting of hydrogen, halogen, cyano, nitro, -OH, $C_{1\sim6}$alkyl, halo-$C_{1\sim6}$alkyl, -O($C_{1\sim6}$alkyl), -O(halo-$C_{1\sim6}$alkyl), -$NH_2$, -NH($C_{1\sim6}$alkyl), -N($C_{1\sim6}$alkyl) ($C_{1\sim6}$alkyl), -$C_{0\sim2}$alkylene-(3~10 membered cycloalkyl), -$C_{0\sim2}$alkylene-(3~10 membered heterocycloalkyl), -$C_{0\sim2}$alkylene-(5~10 membered aryl ring), -$C_{0\sim2}$alkylene-(5~10 membered heteroaryl ring);

$R_1$ is selected from a group consisting of hydrogen, halogen, cyano, nitro, amino, hydroxyl, -$C_{1\sim6}$alkyl, -O($C_{1\sim6}$alkyl), halo-$C_{1\sim6}$alkyl, -$C_{0\sim2}$alkylene-O$R^{1b}$, -$C_{0\sim2}$alkylene-C(O)$R^{1b}$, -$C_{0\sim2}$alkylene-C(O)N$R^{1b}R^{1c}$, -$C_{0\sim2}$alkylene-N$R^{1b}R^{1c}$, -$C_{0\sim2}$alkylene-N$R^{1b}$C(O)$R^{1c}$, -$C_{0\sim4}$alkylene-S(O)$_2R^{1b}R^{1c}$, -$C_{0\sim2}$alkylene-(3~10 membered cycloalkyl), -$C_{0\sim2}$alkylene-(3~10 membered heterocycloalkyl), -$C_{0\sim2}$alkylene-(5~10 membered aryl ring), -$C_{0\sim2}$alkylene-(5~10 membered heteroaryl ring); wherein said alkyl, alkylene, cycloalkyl, heterocycloalkyl, aryl ring, heteroaryl ring is optionally further substituted by one, two or three independent $R^{1b}$;

$R^{1b}$, $R^{1c}$ is each independently selected from a group consisting of hydrogen, -OH, $C_{1\sim6}$alkyl, halo-$C_{1\sim6}$alkyl, -O($C_{1\sim6}$alkyl), -O(halo-Ciealkyl), -$NH_2$, -NH($C_{1\sim6}$alkyl), -N($C_{1\sim6}$alkyl) ($C_{1\sim6}$alkyl), -$C_{0\sim2}$alkylene-(3~10 membered cycloalkyl), -$C_{0\sim2}$alkylene-(3~10 membered heterocycloalkyl), -$C_{0\sim2}$alkylene-(5~10 membered aryl ring), -$C_{0\sim2}$alkylene-(5~10 membered heteroaryl ring);

$R_2$ is selected from a group consisting of -$C_{0\sim2}$alkylene-(3~10 membered cycloalkyl), -$C_{0\sim2}$alkylene-(3~10 membered heterocycloalkyl); wherein said alkylene, cycloalkyl, heterocycloalkyl is optionally further substituted by one, two or three independent $R^{2b}$;

$R^{2b}$ is each independently selected from a group consisting of hydrogen, halogen, cyano, nitro, amino, hydroxyl, -$C_{1\sim6}$alkyl, -O($C_{1\sim6}$alkyl), halo-$C_{1\sim6}$alkyl, -$C_{0\sim2}$alkylene-O$R^{2c}$, -$C_{0\sim2}$alkylene-C(O)$R^{2c}$, -$C_{0\sim2}$alkylene-C(O)N$R^{2c}R^{2d}$, -$C_{0\sim2}$alkylene-N$R^{2c}R^{2d}$, -$C_{0-2}$alkylene-N$R^{2c}$C(O)$R^{2d}$, -$C_{0\sim4}$alkylene-S(O)$_2R^{2c}$, -$C_{0\sim2}$alkylene-(3~10 membered cycloalkyl), -$C_{0\sim2}$alkylene-(3~10 membered heterocycloalkyl), -$C_{0\sim2}$alkylene-(5~10 membered aryl ring), -$C_{0\sim2}$alkylene-(5~10 membered heteroaryl ring); wherein alkyl, alkylene, cycloalkyl, heterocycloalkyl, aryl ring, heteroaryl ring is optionally further substituted by one, two or three independent $R^{2c}$;

$R^{2c}$, $R^{2d}$ is each independently selected from a group consisting of hydrogen, cyano, nitro, -OH, halogen, -$C_{1\sim6}$alkyl, halo-$C_{1\sim6}$alkyl, -$C_{0\sim2}$alkylene-O($C_{1\sim6}$alkyl), -O(halo-$C_{1\sim6}$alkyl), -O(3~10 membered cycloalkyl),

-O(3~10 membered heterocycloalkyl), $-NH_2$, -NH($C_{1\sim6}$alkyl), -N($C_{1\sim6}$alkyl) ($C_{1\sim6}$alkyl), -$C_{0\sim2}$alkylene-(3~10 membered cycloalkyl), -$C_{0\sim2}$alkylene-(3~10 membered heterocycloalkyl), -$C_{0\sim2}$alkylene-(5~10 membered aryl ring), -$C_{0\sim2}$alkylene-(5~10 membered heteroaryl ring); wherein said alkyl, alkylene, cycloalkyl, heterocycloalkyl, aryl ring, heteroaryl ring is optionally further substituted by one, two or three independent $R^{2e}$;

$R^{2e}$ is each independently selected from a group consisting of hydrogen, cyano, nitro, -OH, halogen, -$C_{1\sim6}$alkyl, halo-$C_{1\sim6}$alkyl, -$C_{0\sim2}$alkylene-O($C_{1\sim6}$alkyl), -O(halo-$C_{1\sim6}$alkyl), -O(3~10 membered cycloalkyl), -O(3~10 membered heterocycloalkyl), $-NH_2$, -NH($C_{1\sim6}$alkyl), -N($C_{1\sim6}$alkyl) ($C_{1\sim6}$alkyl), -$C_{0\sim2}$alkylene-(3~10 membered cycloalkyl), -$C_{0\sim2}$alkylene-(3~10 membered heterocycloalkyl), -$C_{0\sim2}$alkylene-(5~10 membered aryl ring), -$C_{0\sim2}$alkylene-(5~10 membered heteroaryl ring);

$R_3$ is selected from a group consisting of hydrogen, halogen, cyano, nitro, amino, -OH, -$C_{1\sim6}$alkyl, -O($C_{1\sim6}$alkyl), halo-$C_{1\sim6}$alkyl, -$C_{0\sim2}$alkylene-$OR^{3b}$, -$C_{0\sim2}$alkylene-C(O)$R^{3b}$, -$C_{0\sim2}$alkylene-C(O)$NR^{3b}R^{3c}$, -$C_{0\sim2}$alkylene-$NR^{3b}R^{3c}$, -$C_{0\sim2}$alkylene-$NR^{3b}$C(O)$R^{3c}$, -$C_{0\sim4}$alkylene-$S(O)_2R^{3b}$, -$C_{0\sim2}$alkylene-(3~10 membered cycloalkyl), -$C_{0\sim2}$alkylene-(3~10 membered heterocycloalkyl);

$R^{3b}$, $R^{3c}$ is each independently selected from a group consisting of hydrogen, halogen, cyano, nitro, -OH, $C_{1\sim6}$alkyl, halo-$C_{1\sim6}$alkyl, -O($C_{1\sim6}$alkyl), -O(halo-$C_{1\sim6}$alkyl), $-NH_2$, -NH($C_{1\sim6}$alkyl), -N($C_{1\sim6}$alkyl) ($C_{1\sim6}$alkyl), -$C_{0\sim2}$alkylene-(3~10 membered cycloalkyl), -$C_{0\sim2}$alkylene-(3~10 membered heterocycloalkyl), -$C_{0\sim2}$alkylene-(5~10 membered aryl ring), -$C_{0\sim2}$alkylene-(5~10 membered heteroaryl ring);

$R_4$ is selected from a group consisting of hydrogen, halogen, cyano, nitro, hydroxyl, -$C_{1\sim6}$alkyl, halo-$C_{1\sim6}$alkyl, -O($C_{1\sim6}$alkyl), -O(halo-$C_{1\sim6}$alkyl), $-NH_2$, -NH($C_{1\sim6}$alkyl), -N($C_{1\sim6}$alkyl) ($C_{1\sim6}$alkyl), -$C_{0\sim2}$alkylene-(3~10 membered cycloalkyl), -$C_{0\sim2}$alkylene-(3~10 membered heterocycloalkyl), -$C_{0\sim2}$alkylene-(5~10 membered aryl ring), -$C_{0\sim2}$alkylene-(5~10 membered heteroaryl ring); wherein said alkyl, alkylene, cycloalkyl, heterocycloalkyl, aryl ring, heteroaryl ring is optionally further substituted by one, two or three independent $R^{4b}$;

$R^{4b}$ is each independently selected from a group consisting of hydrogen, halogen, cyano, nitro, -OH, Ciealkyl, halo-$C_{1\sim6}$alkyl, -O($C_{1\sim6}$alkyl), -O(halo-$C_{1\sim6}$alkyl), $-NH_2$, -NH($C_{1\sim6}$alkyl), -N($C_{1\sim6}$alkyl) ($C_{1\sim6}$alkyl), -$C_{0\sim2}$alkylene-(3~10 membered cycloalkyl), -$C_{0\sim2}$alkylene-(3~10 membered heterocycloalkyl), -$C_{0\sim2}$alkylene-(5~10 membered aryl ring), -$C_{0\sim2}$alkylene-(5~10 membered heteroaryl ring);

$R_5$ is selected from a group consisting of hydrogen, halogen, cyano, nitro, hydroxyl, -$C_{1\sim6}$alkyl, halo-$C_{1\sim6}$alkyl, -O($C_{1\sim6}$alkyl), -O(halo-Ciealkyl), $-NH_2$, -NH($C_{1\sim6}$alkyl), -N($C_{1\sim6}$alkyl) ($C_{1\sim6}$alkyl), -$C_{0\sim2}$alkylene-(3~10 membered cycloalkyl), -$C_{0\sim2}$alkylene-(3~10 membered heterocycloalkyl), -$C_{0\sim2}$alkylene-(5~10 membered aryl ring), -$C_{0\sim2}$alkylene-(5~10 membered heteroaryl ring);

$R_6$ is selected from a group consisting of hydrogen, halogen, cyano, nitro, hydroxyl, -$C_{1\sim6}$alkyl, halo-$C_{1\sim6}$alkyl, -O($C_{1\sim6}$alkyl), -O(halo-Ciealkyl), $-NH_2$, -NH($C_{1\sim6}$alkyl), -N($C_{1\sim6}$alkyl) ($C_{1\sim6}$alkyl), -$C_{0\sim2}$alkylene-(3~10 membered cycloalkyl), -$C_{0\sim2}$alkylene-(3~10 membered heterocycloalkyl), -$C_{0\sim2}$alkylene-(5~10 membered aryl ring), -$C_{0\sim2}$alkylene-(5~10 membered heteroaryl ring).

**16.** The compound, or a deuterated compound thereof, or a stereoisomer thereof, or a pharmaceutically acceptable salt thereof according to claim 15, wherein said $R_1$, $R_3$, $R_4$, $R_5$, $R_6$ is each independently selected from a group consisting of hydrogen, halogen, -$C_{1\sim3}$alkyl; Q is selected from a group consisting of single bond.

**17.** The compound, or a deuterated compound thereof, or a stereoisomer thereof, or a pharmaceutically acceptable salt thereof according to claim 15 wherein said $R_2$ is specifically selected from a group consisting of

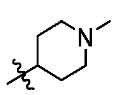 , 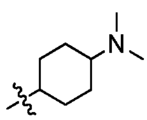 , 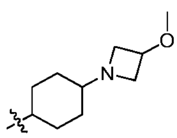 , 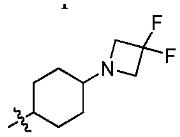 ,

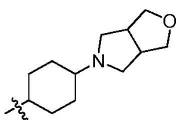 , 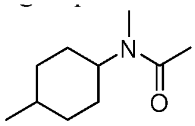 , 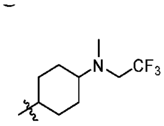 , 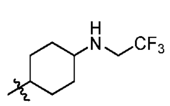 , 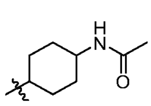 , 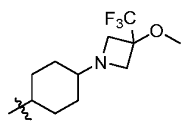 ,

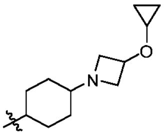, 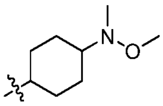, 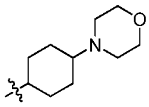, 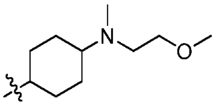, 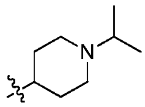, 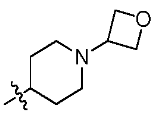,

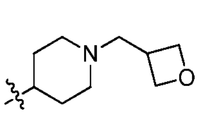, 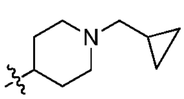, 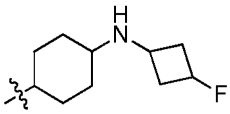;

preferably, said $R_2$ is specifically selected from a group consisting of

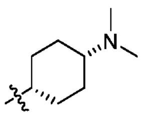, 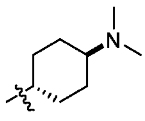,

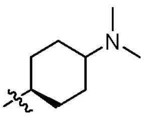, 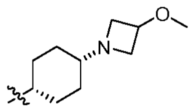, 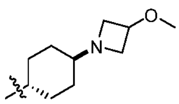, 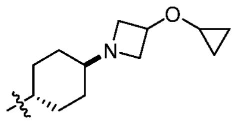, 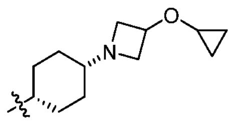,

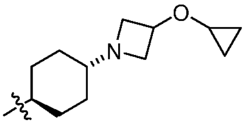, 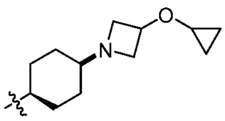, 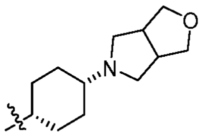, 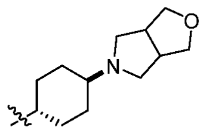, 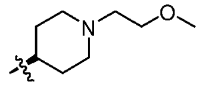,

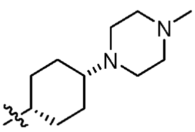, 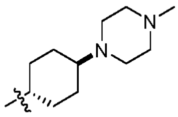, 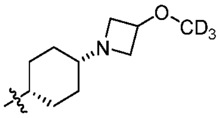, 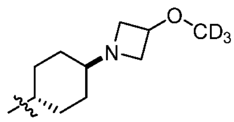, 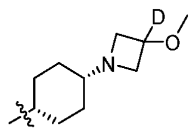,

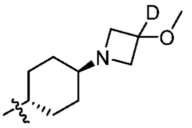, 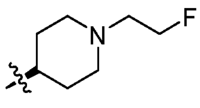, 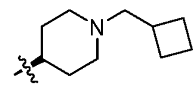, 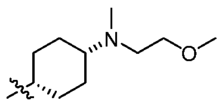, 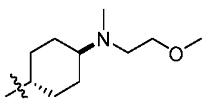,

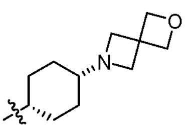, 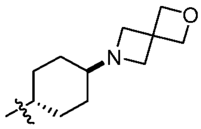, 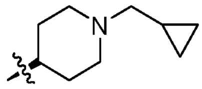, 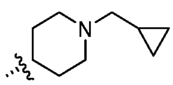, 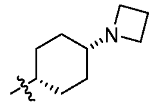, 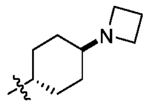,

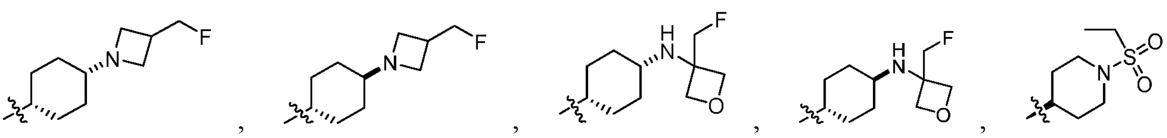, , , , ,

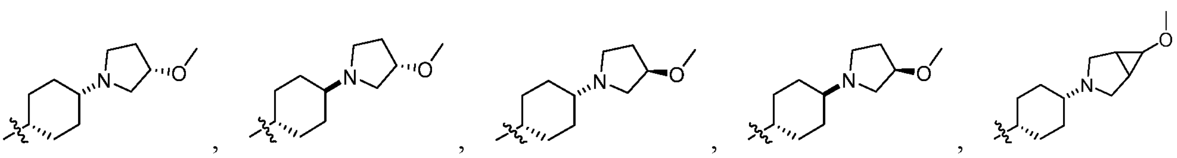, , , , ,

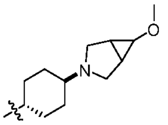, 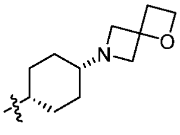, 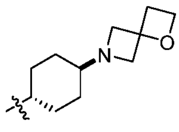, 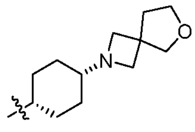,

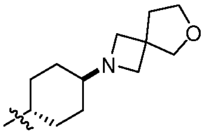 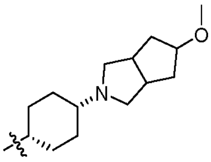, 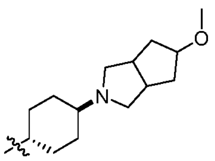, 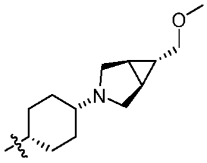, 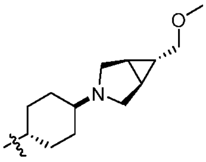,

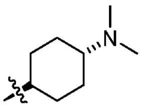, 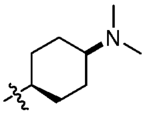, 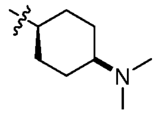, 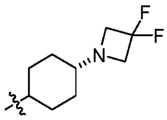, 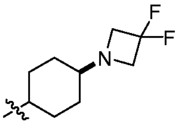, 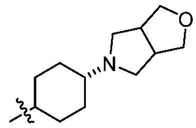,

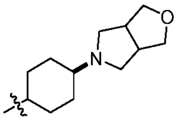, 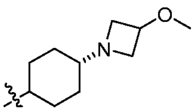, 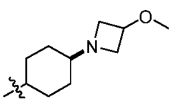, 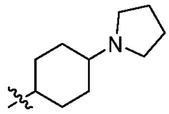, 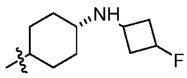,

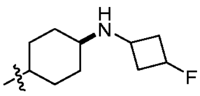, 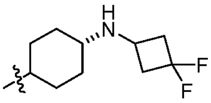, 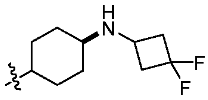, 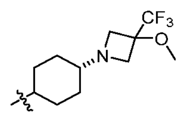, 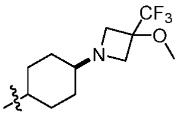,

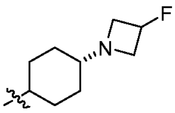, 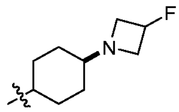, 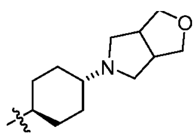, 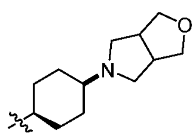, 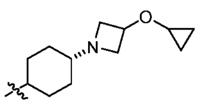,

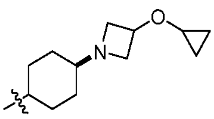, 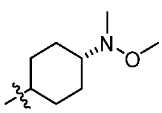, 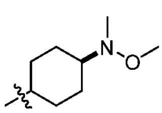, 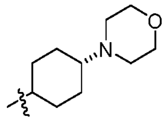, 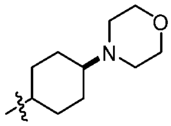, 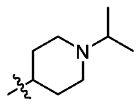,

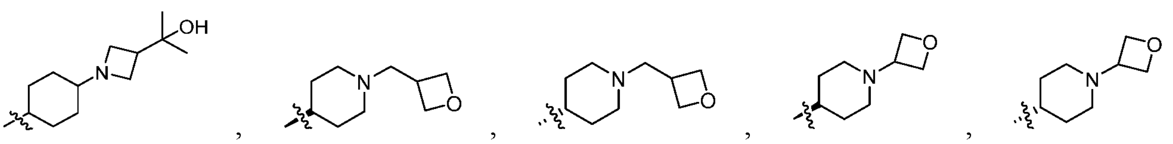
, , , , ,

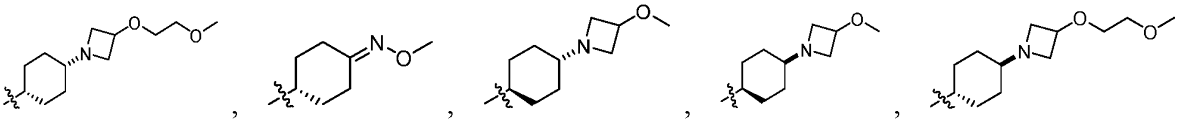
, , , , ,

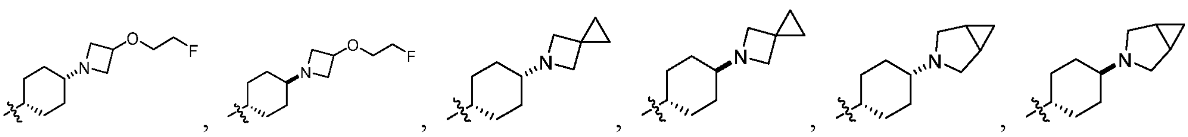
, , , , , ,

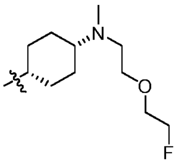, 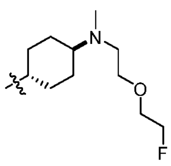, 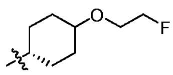, 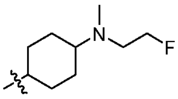, 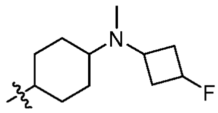, 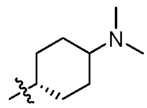,

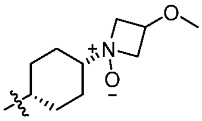, 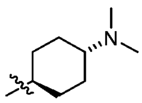, 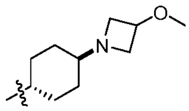, 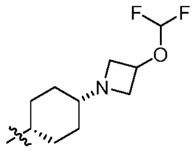, 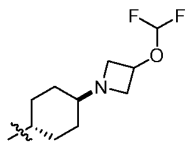,

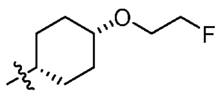, 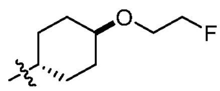

**18.** The compound, or a deuterated compound thereof, or a stereoisomer thereof, or a pharmaceutically acceptable salt thereof according to any one of claims 1 to 5, wherein:
the compound represented by Formula I or Formula II is specifically:

| | | |
|---|---|---|
| 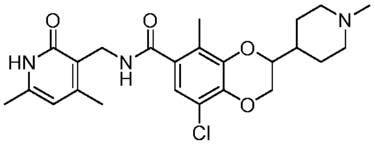 1 | 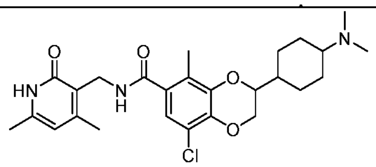 2 | 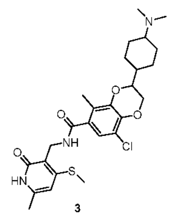 3 |
| 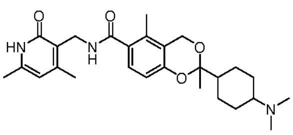 4 | 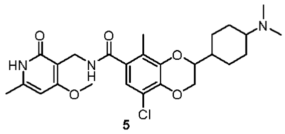 5 | 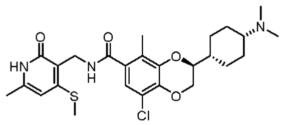 6 |
| 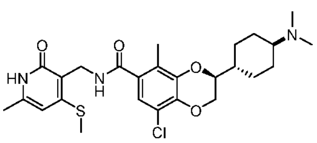 6' | 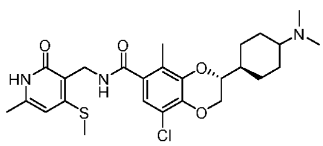 7 | 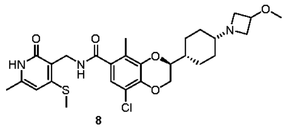 8 |
| 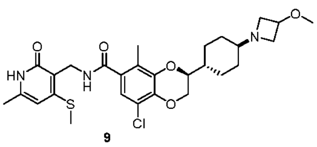 9 | 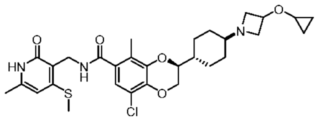 10 | 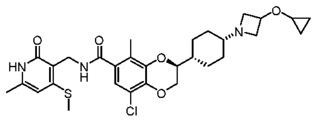 11 |
| 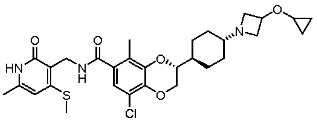 12 | 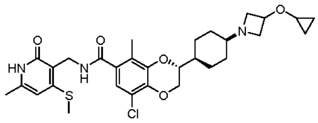 13 | 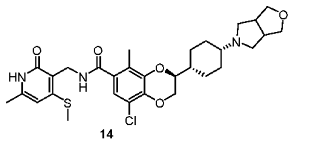 14 |

| | | |
|---|---|---|
| 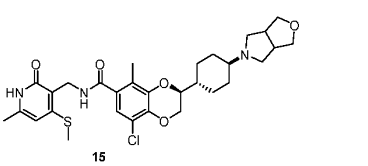<br>15 | 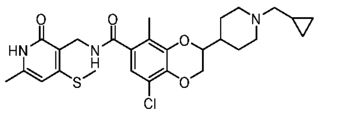<br>16 | 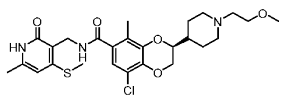<br>17 |
| 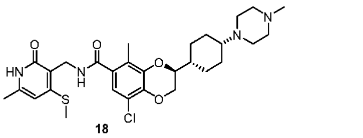<br>18 | 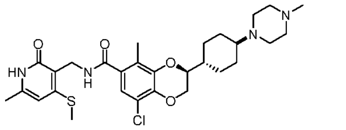<br>19 | 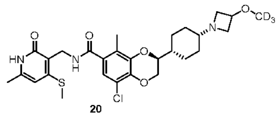<br>20 |
| 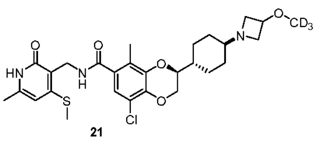<br>21 | 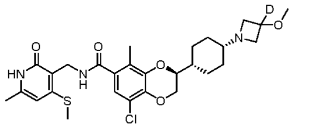<br>22 | 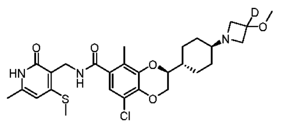<br>23 |
| 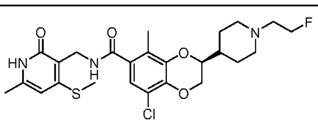<br>24 | 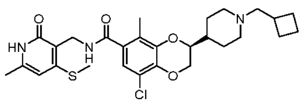<br>25 | 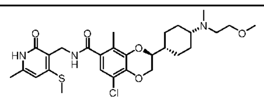<br>26 |
| 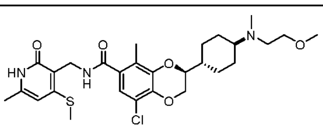<br>27 | 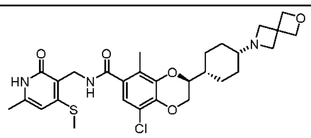<br>28 | 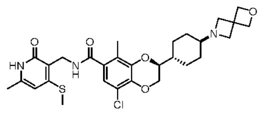<br>29 |
| 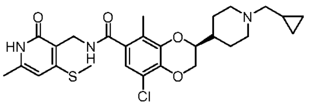<br>30 | 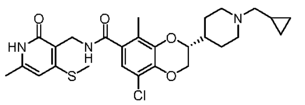<br>31 | 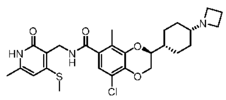<br>32 |
| 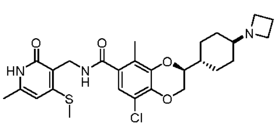<br>33 | 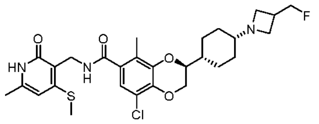<br>34 | 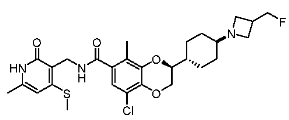<br>35 |
| 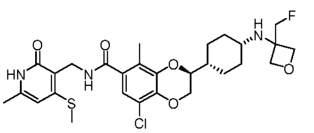<br>36 | 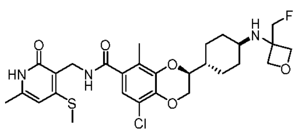<br>37 | 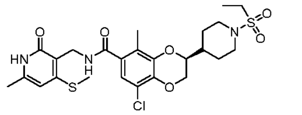<br>38 |
| 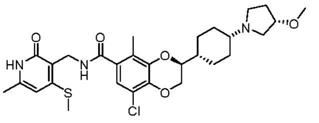<br>39 | 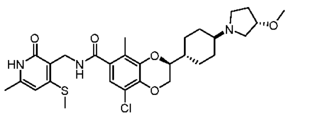<br>40 | 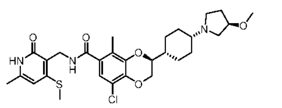<br>41 |
| 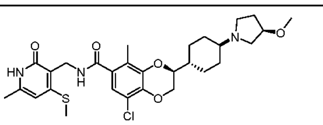<br>42 | 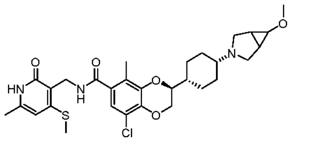<br>43 | 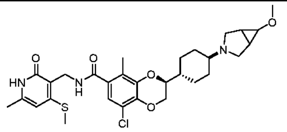<br>44 |

| | | |
|---|---|---|
| 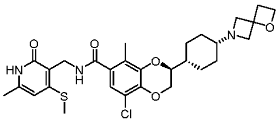 45 | 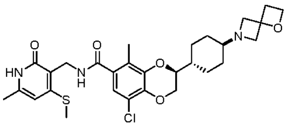 46 | 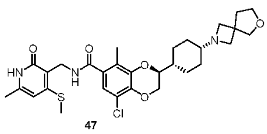 47 |
| 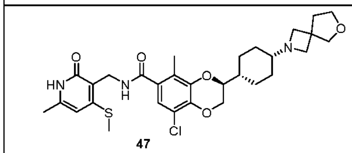 47 | 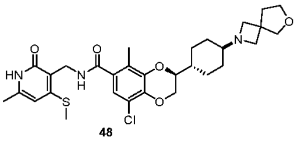 48 | 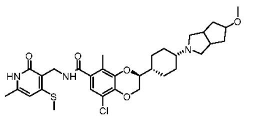 49 |
| 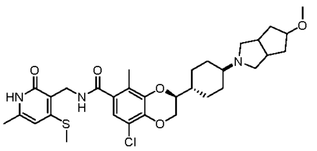 50 | 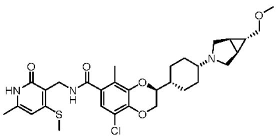 51 | 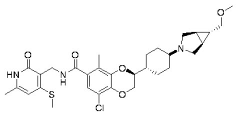 52 |
| 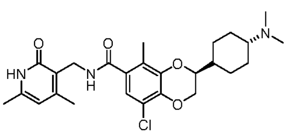 53 | 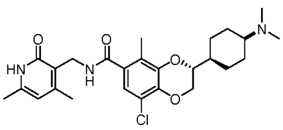 54 | 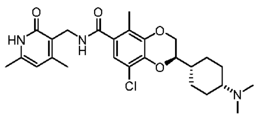 55 |
| 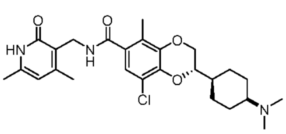 56 | 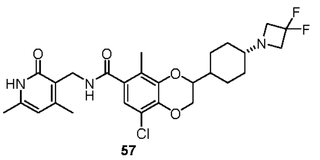 57 | 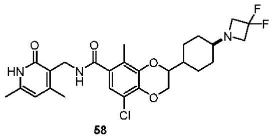 58 |
| 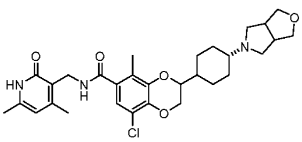 59 | 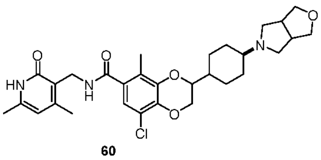 60 | 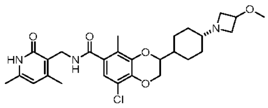 61 |
| 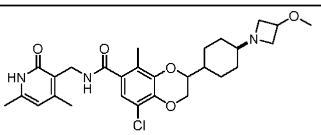 62 | 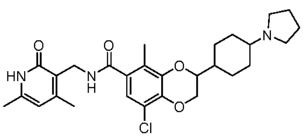 63 | 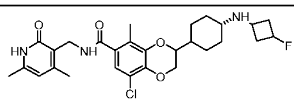 64 |
| 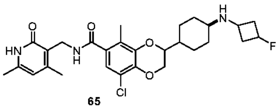 65 | 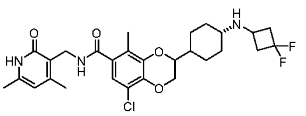 66 | 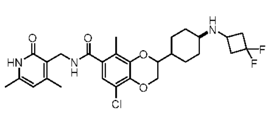 67 |
| 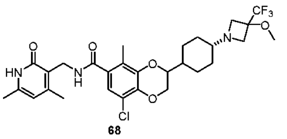 68 | 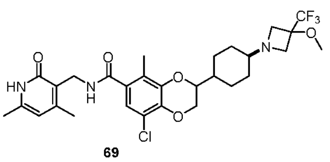 69 | 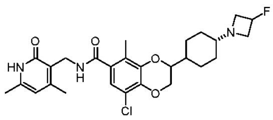 70 |

| | | |
|---|---|---|
| 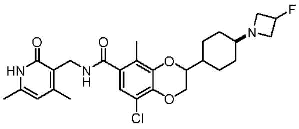 71 | 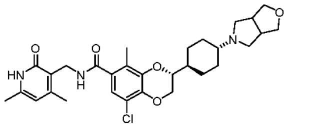 72 | 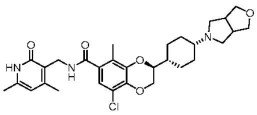 73 |
| 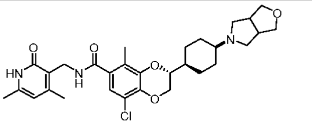 74 | 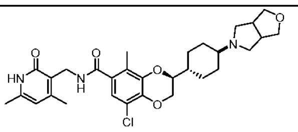 75 | 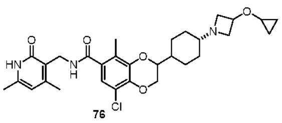 76 |
| 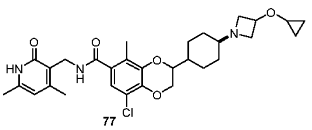 77 | 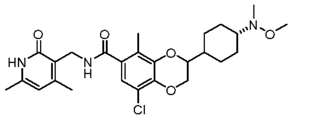 78 | 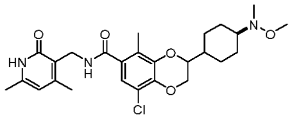 79 |
| 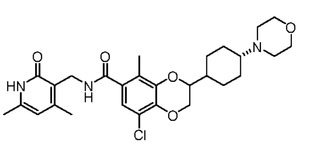 80 | 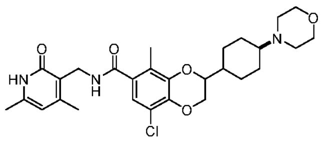 81 | 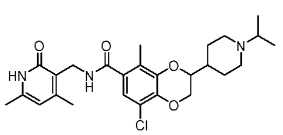 82 |
| 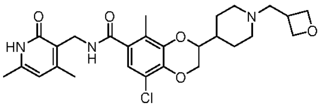 83 | 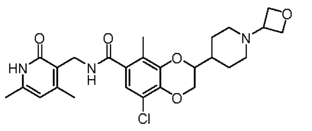 84 | 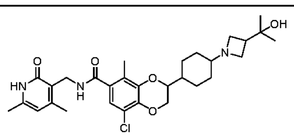 85 |
| 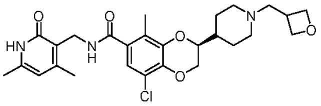 86 | 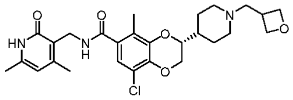 87 | 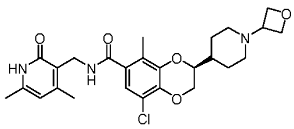 88 |
| 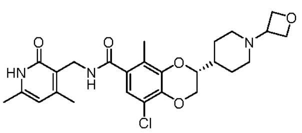 89 | 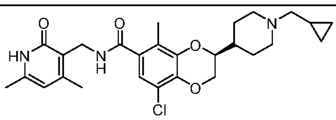 90 | 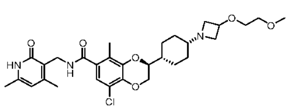 91 |
| 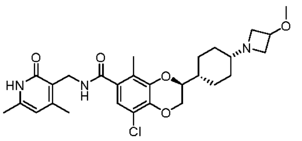 92 | 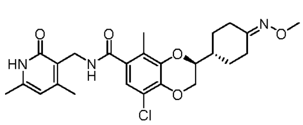 93 | 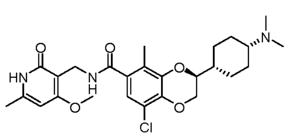 94 |
| 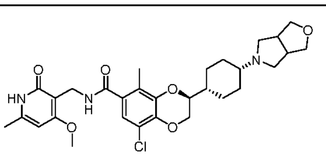 95 | 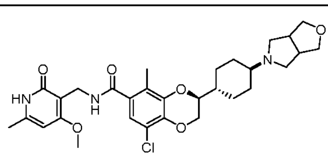 96 | 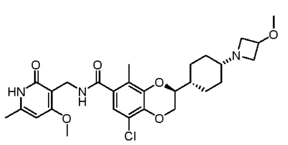 97 |

| | | |
|---|---|---|
| 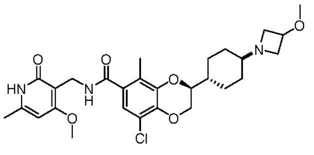<br>98 | 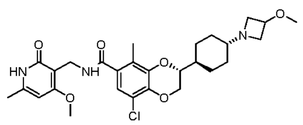<br>99 | 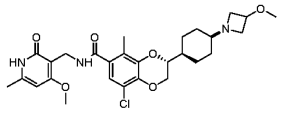<br>100 |
| 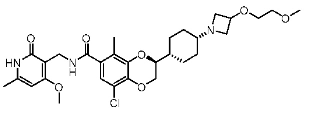<br>101 | 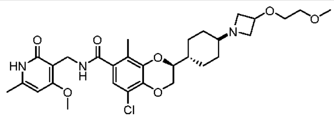<br>102 | 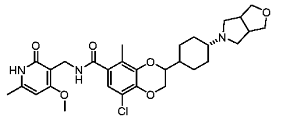<br>103 |
| 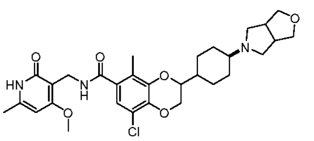<br>104 | 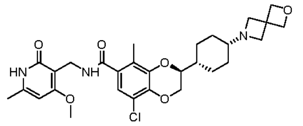<br>105 | 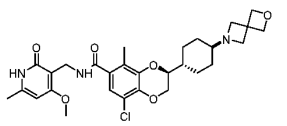<br>106 |
| 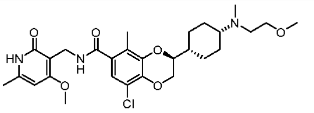<br>107 | 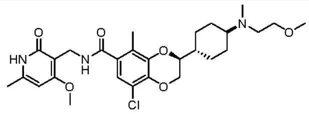<br>108 | 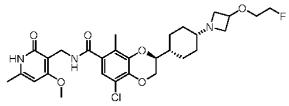<br>109 |
| 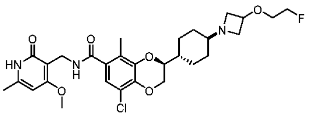<br>110 | 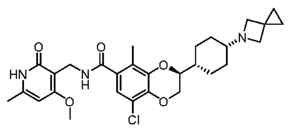<br>111 | 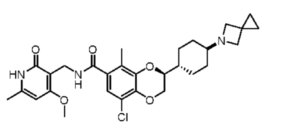<br>112 |
| 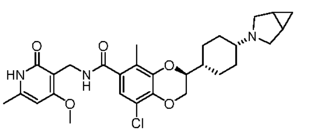<br>113 | 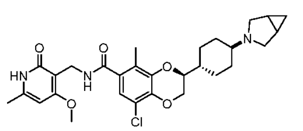<br>114 | 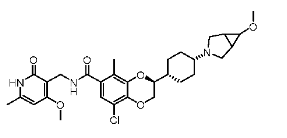<br>115 |
| 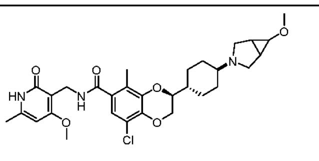<br>116 | 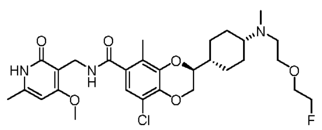<br>117 | 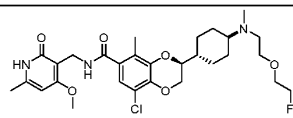<br>118 |
| 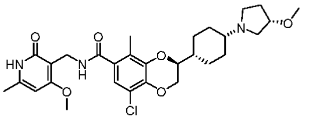<br>119 | 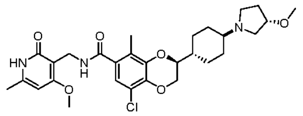<br>120 | 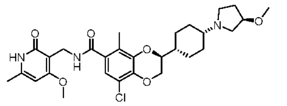<br>121 |
| 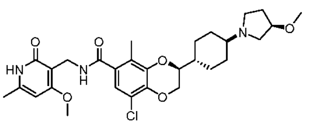<br>122 | 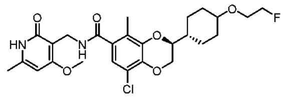<br>123 | 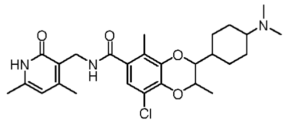<br>124 |

| | | |
|---|---|---|
| 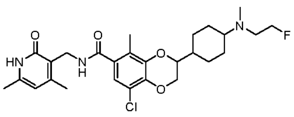<br>125 | 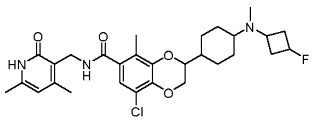<br>126 | 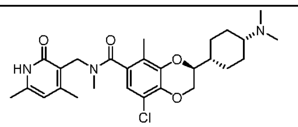<br>127 |
| 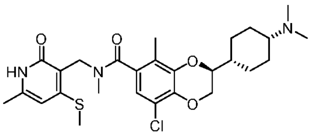<br>128 | 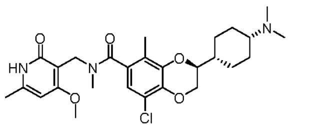<br>129 | 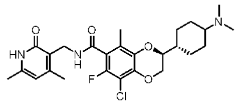<br>130 |
| 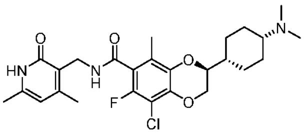<br>131 | 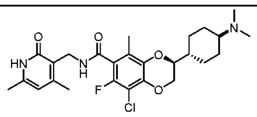<br>132 | 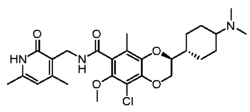<br>133 |
| 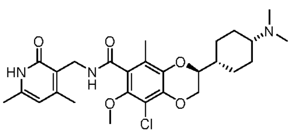<br>134 | 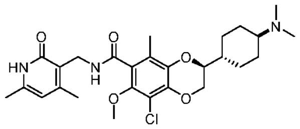<br>135 | 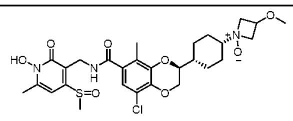<br>136 |
| 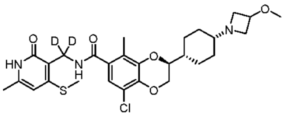<br>137 | 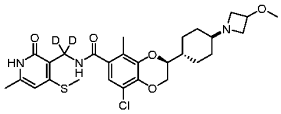<br>138 | 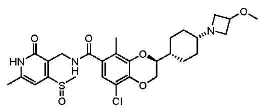<br>139 |
| 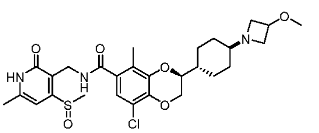<br>140 | 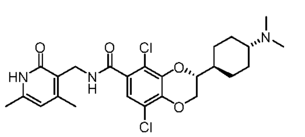<br>141 | 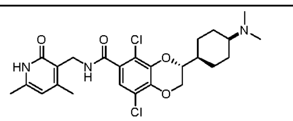<br>142 |
| 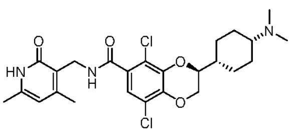<br>143 | 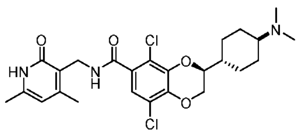<br>144 | 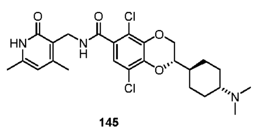<br>145 |
| 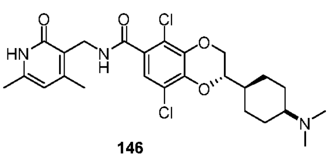<br>146 | 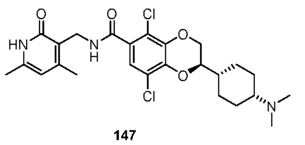<br>147 | 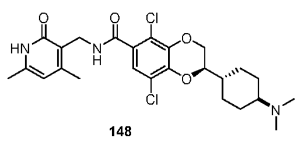<br>148 |
| 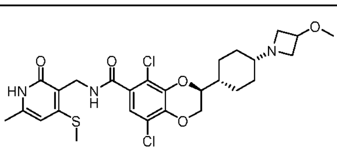<br>149 | 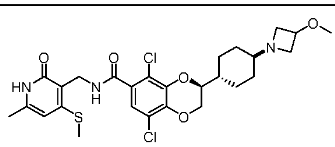<br>150 | 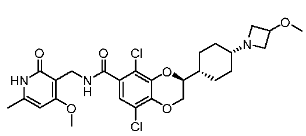<br>151 |

| | | |
|---|---|---|
| 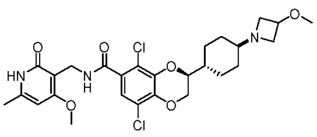 152 | 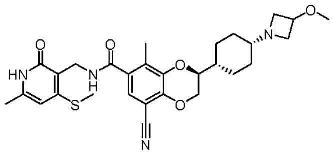 153 | 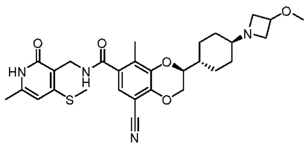 154 |
| 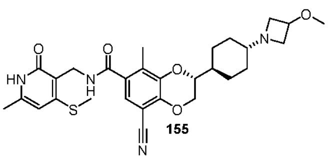 155 | 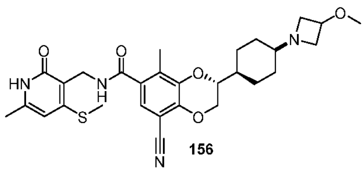 156 | 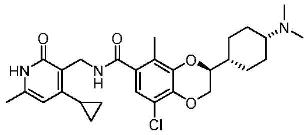 157 |
| 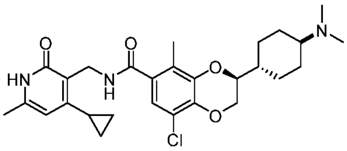 158 | 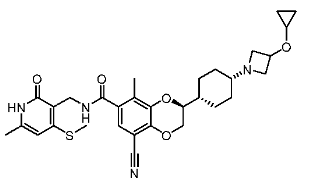 159 | 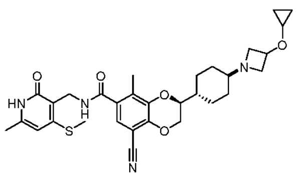 160 |
| 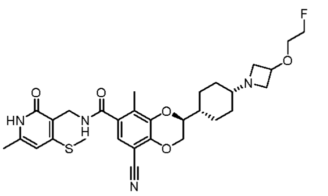 161 | 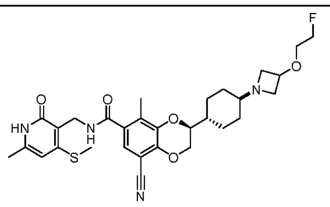 162 | 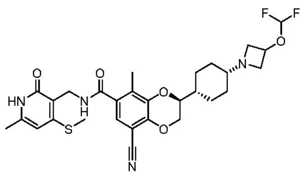 163 |
| 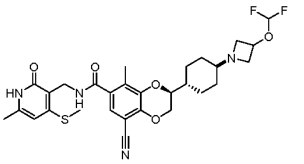 164 | 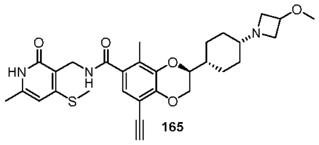 165 | 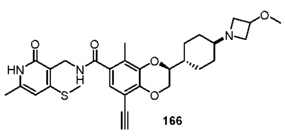 166 |
| 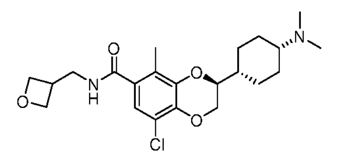 167 | 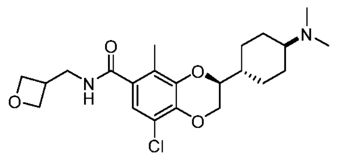 168 | 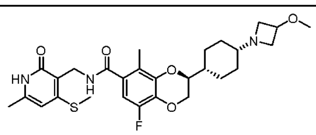 169 |
| 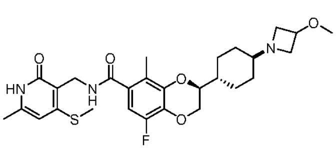 170 | 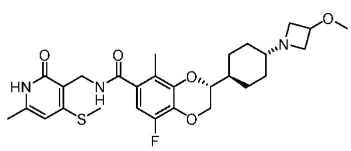 171 | 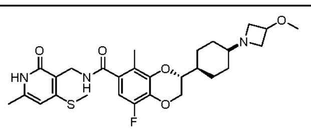 172 |
| 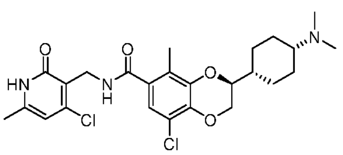 173 | 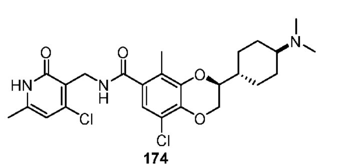 174 | 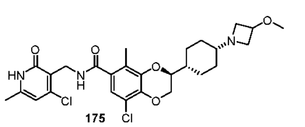 175 |

| | | |
|---|---|---|
| 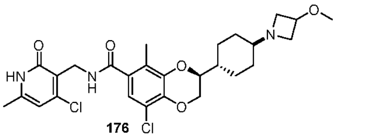 176 | 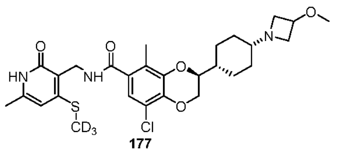 177 | 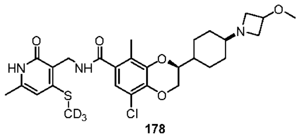 178 |
| 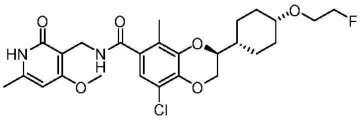 179 | 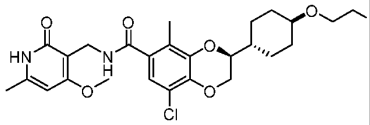 180 | 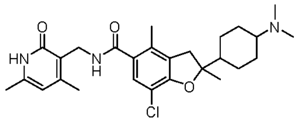 181 |

**19.** The compound, or a deuterated compound thereof, or a stereoisomer thereof, or a pharmaceutically acceptable salt thereof according to any one of claims 1 to 5, wherein the compound represented by Formula I or Formula II is specifically:

| | | |
|---|---|---|
| 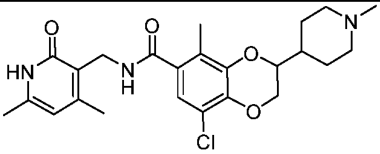 182 | 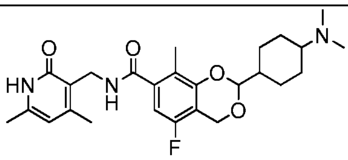 183 | 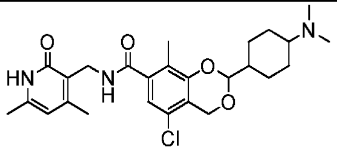 184 |
| 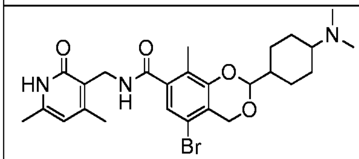 185 | 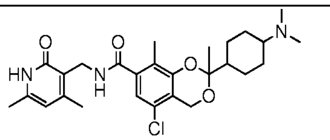 186 | 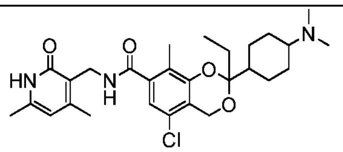 187 |
| 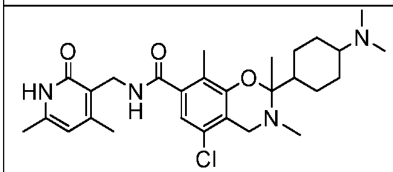 188 | 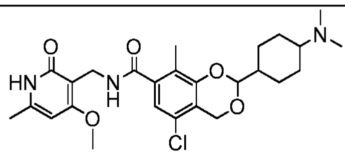 189 | 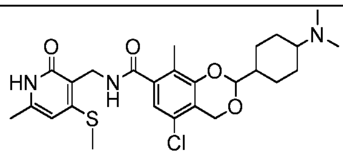 190 |
| 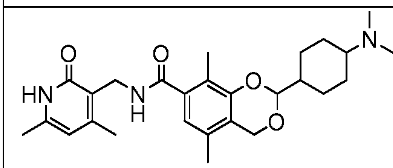 191 | 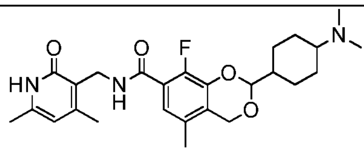 192 | 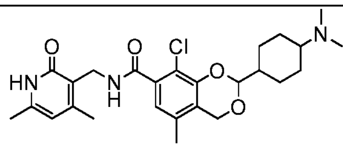 193 |
| 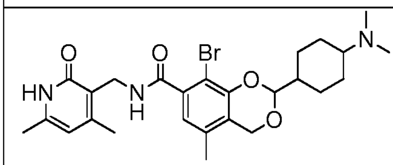 194 | 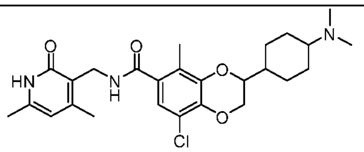 195 | 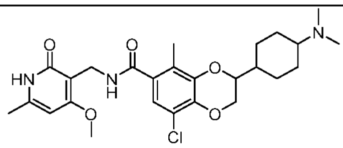 196 |

| | | |
|---|---|---|
| 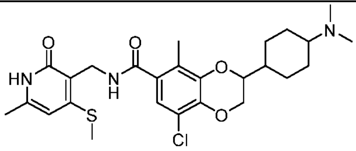 197 | 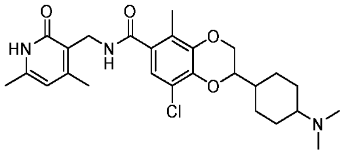 198 | 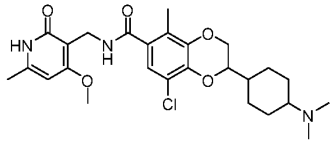 199 |
| 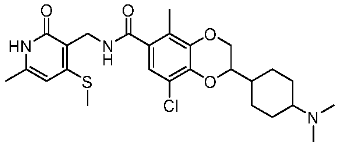 200 | 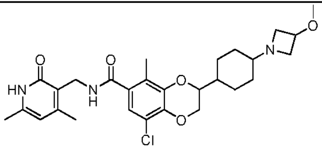 201 | 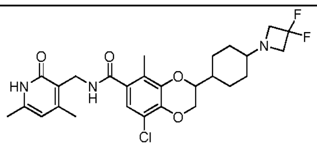 202 |
| 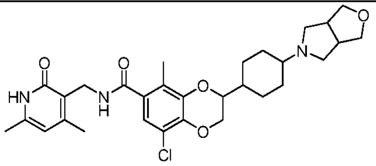 203 | 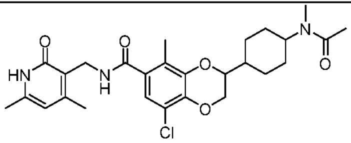 204 | 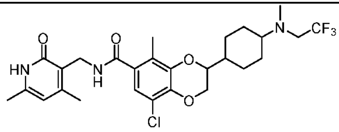 205 |
| 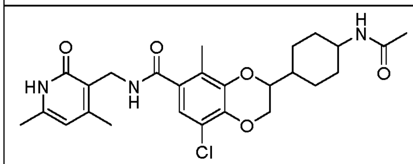 206 | 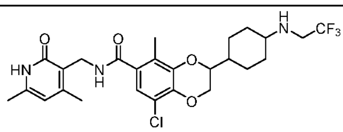 207 | 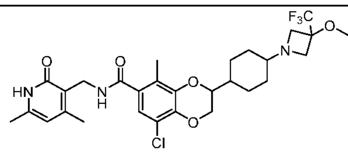 208 |
| 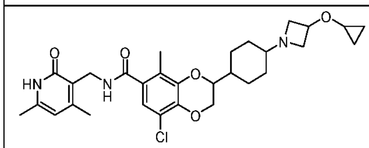 209 | 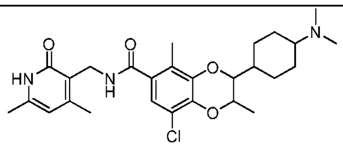 210 | 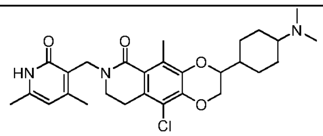 211 |
| 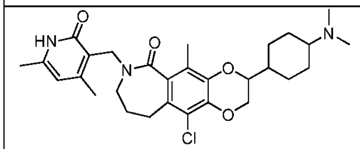 212 | 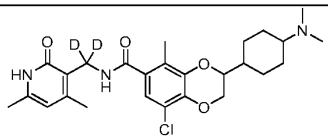 213 | 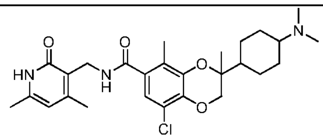 214 |
| 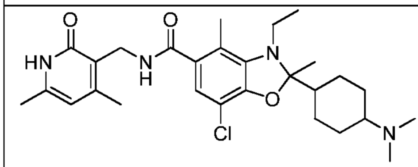 215 | 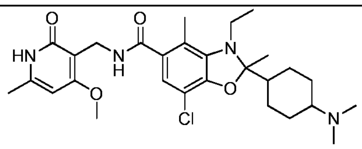 216 | 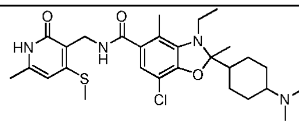 217 |

| | | |
|---|---|---|
| 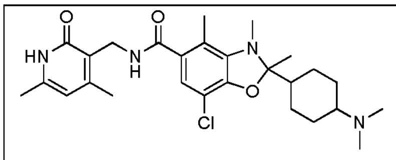 218 | 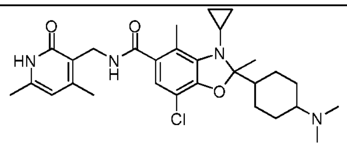 219 | 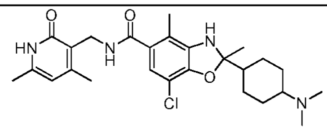 220 |
| 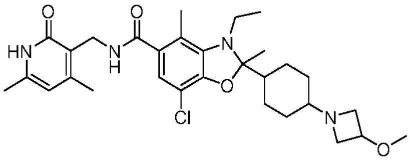 221 | 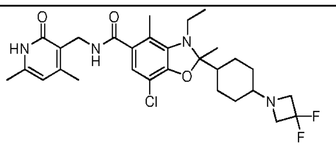 222 | 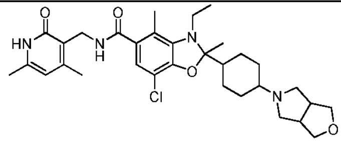 223 |
| 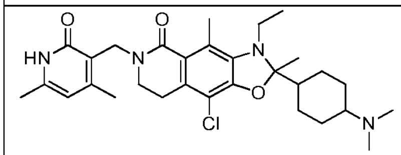 224 | 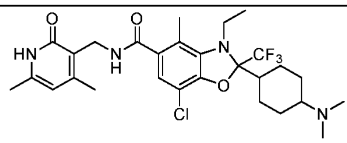 225 | 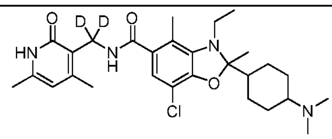 226 |
| 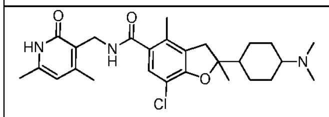 227 | 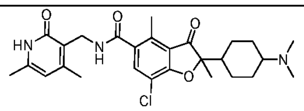 228 | 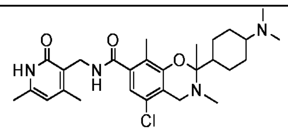 229 |
| 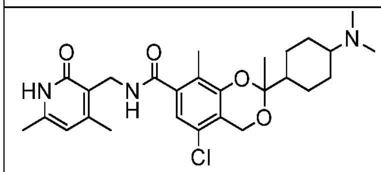 230 | 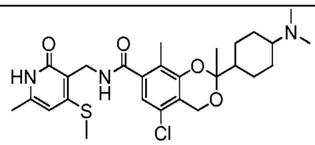 231 | 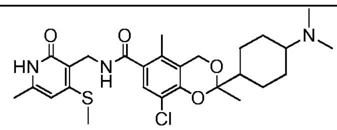 232 |
| 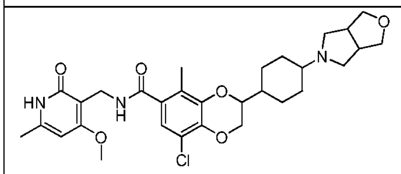 233 | 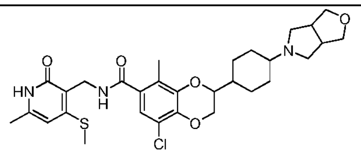 234 | 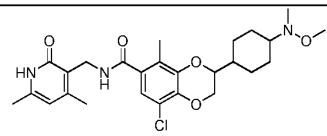 235 |
| 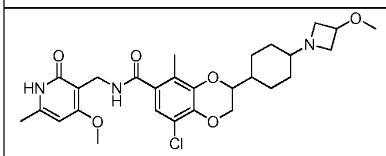 236 | 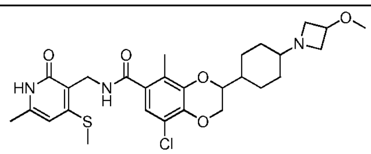 237 | 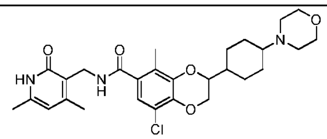 238 |

| | | |
|---|---|---|
| 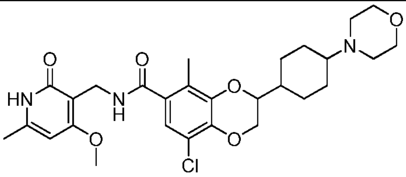 239 | 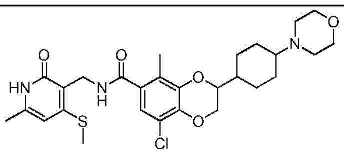 240 | 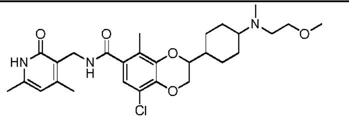 241 |
| 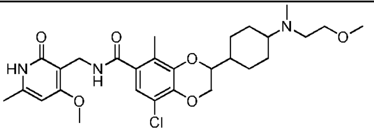 242 | 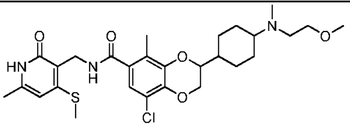 243 | 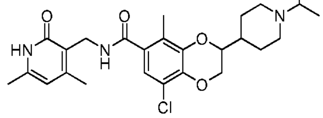 244 |
| 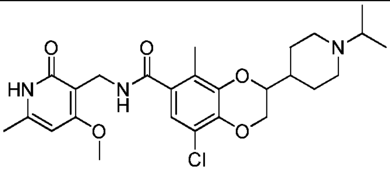 245 | 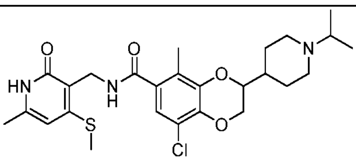 246 | 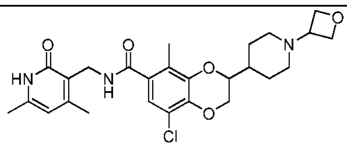 247 |
| 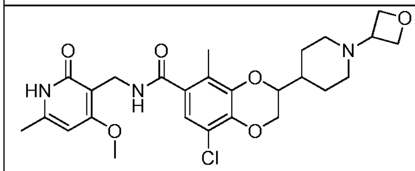 248 | 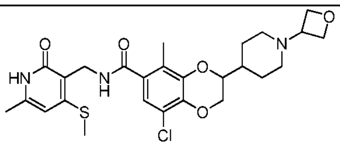 249 | 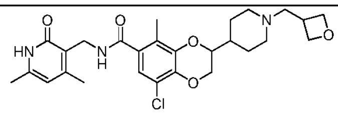 250 |
| 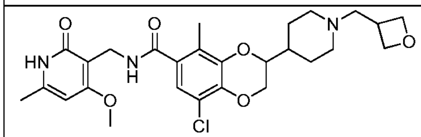 251 | 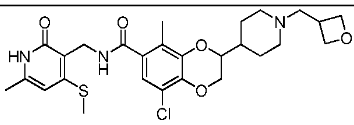 252 | 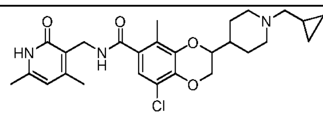 253 |
| 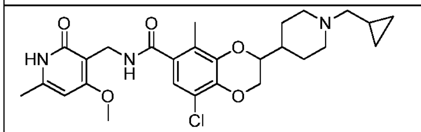 254 | 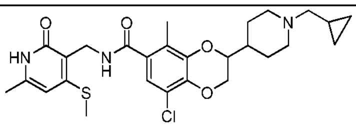 255 | 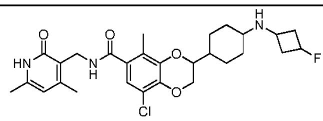 256 |
| 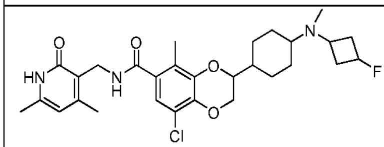 257 | 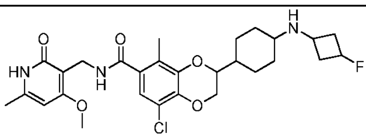 258 | 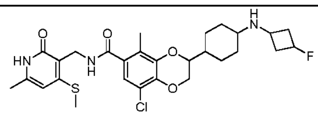 259 |
| 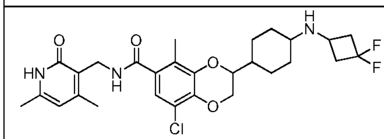 | 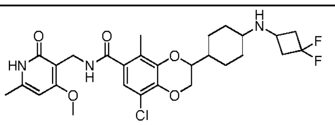 261 | 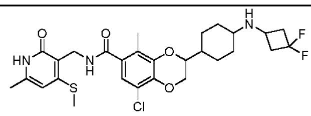 262 |

| 260 | | |
|---|---|---|
| 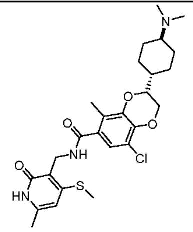 263 | 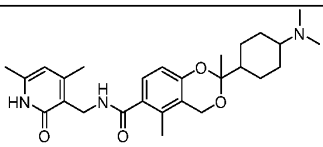 264 | |

20. An intermediate compound shown as follows:

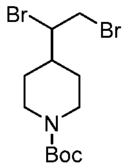
IM-1 ,
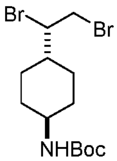
IM-2 ,
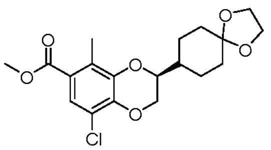
IM-3a ,

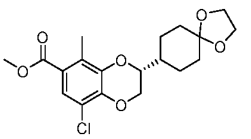
IM-3b ,
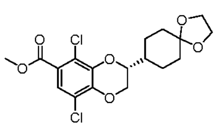
IM-4a ,
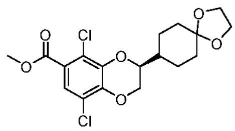
IM-4b ,
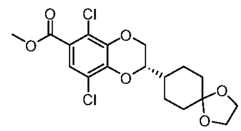
IM-4c ,

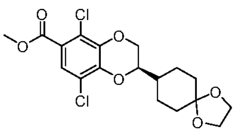
IM-4d ,
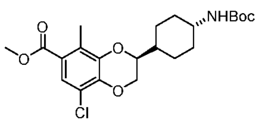
IM-5a ,
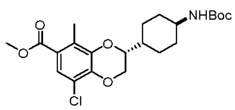
IM-5b ,
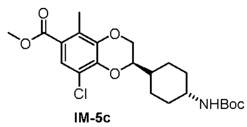
IM-5c ,

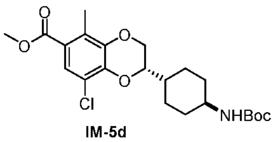
IM-5d .

21. Use of the compound according to any one of claims 1 to 19, or a deuterated compound thereof, or a stereoisomer thereof, or a pharmaceutically acceptable salt thereof, in the preparation of a medicament for treating an EZH1/2-mediated disease.

22. The use according to claim 21, wherein the EZH1/2-mediated disease is one or more selected from diseases related to inflammation, autoimmune disease, infectious disease, cancer, and precancerous syndrome.

23. A pharmaceutical composition, **characterized in that** it is a formulation prepared with the compound of any one of claims 1 to 19, or a deuterated compound thereof, or a stereoisomer thereof, or a stereoisomer thereof, or a pharmaceutically acceptable salt thereof, as the pharmaceutically active ingredient, together with pharmaceutically acceptable excipients.

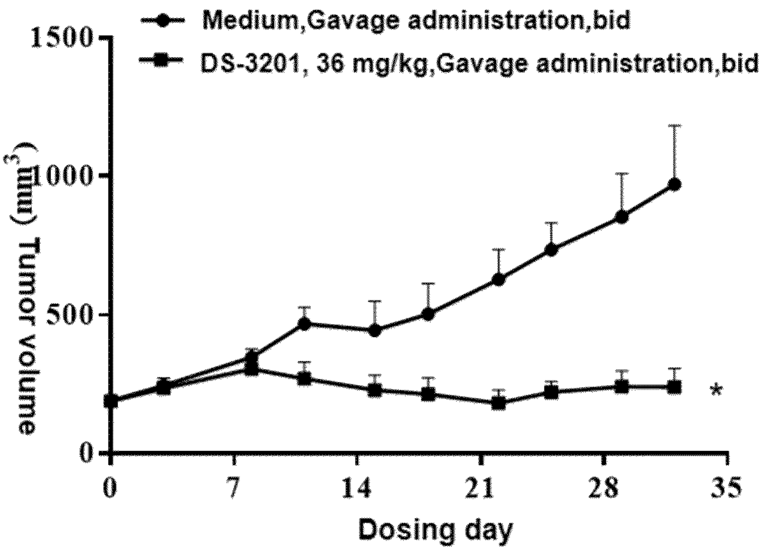


Figure 1

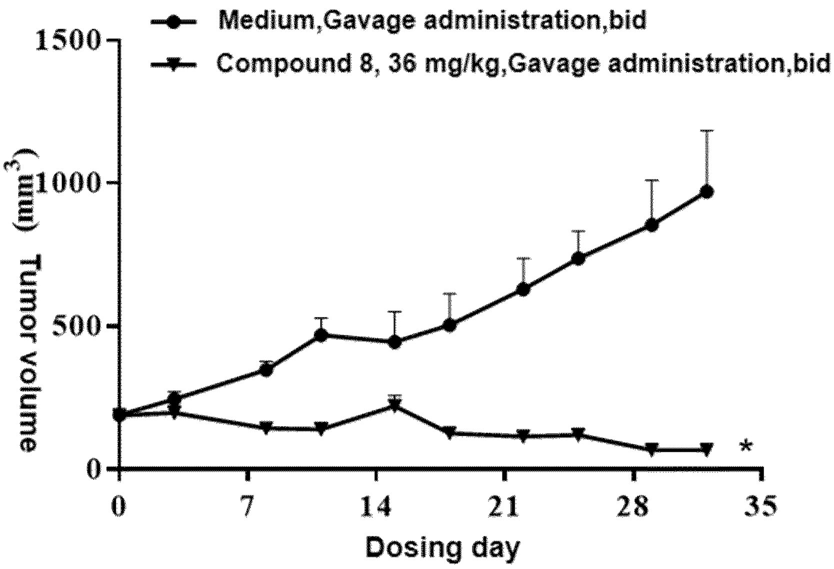


Figure 2

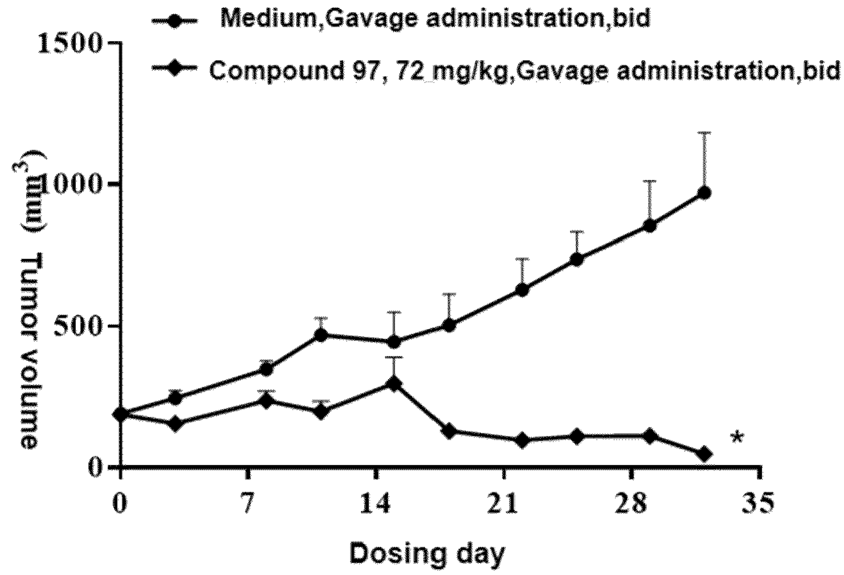


Figure 3

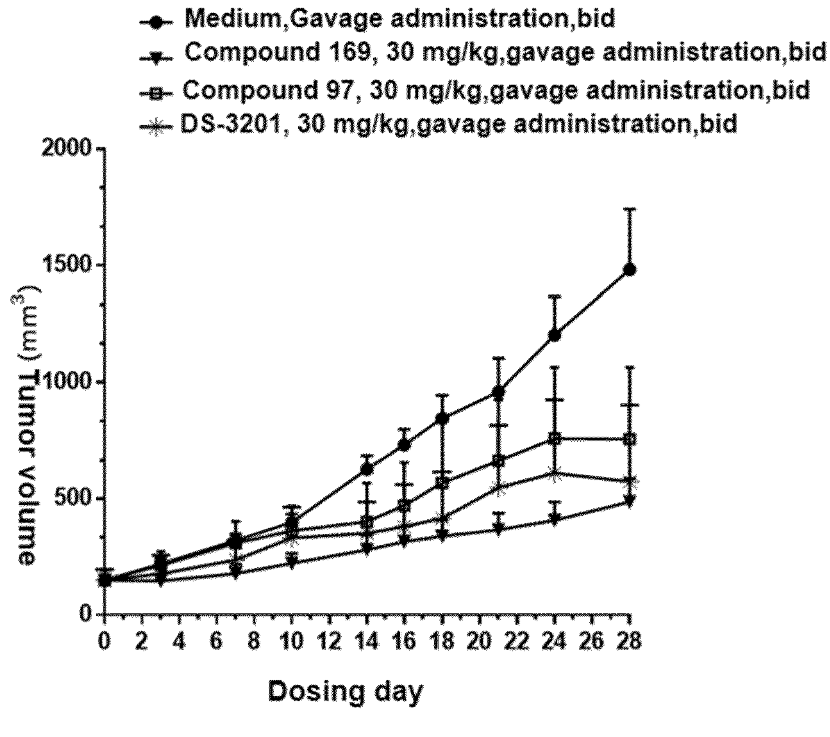


Figure 4

## INTERNATIONAL SEARCH REPORT

International application No.
**PCT/CN2022/139254**

**A. CLASSIFICATION OF SUBJECT MATTER**

C07D405/14(2006.01)i;C07D405/12(2006.01)i;A61K31/44(2006.01)i;A61P35/00(2006.01)i

According to International Patent Classification (IPC) or to both national classification and IPC

**B. FIELDS SEARCHED**

Minimum documentation searched (classification system followed by classification symbols)

C07D A61K A61P

Documentation searched other than minimum documentation to the extent that such documents are included in the fields searched

Electronic data base consulted during the international search (name of data base and, where practicable, search terms used)

CNABS, CNTXT, SIPOABS, DWPI, WOTXT, EPTXT, USTXT, CNKI, 万方, WANFANG, 读秀, DUXIU, PUBMED, ISI_Web of Science, Science Direct, STNext: 结构检索, EZH1/2, 癌症, 炎症, 感染, structure search, cancer, inflammation, infection

**C. DOCUMENTS CONSIDERED TO BE RELEVANT**

| Category* | Citation of document, with indication, where appropriate, of the relevant passages | Relevant to claim No. |
|---|---|---|
| A | CN 106132954 A (DAIICHI-SANKYO CO., LIMITED) 16 November 2016 (2016-11-16) abstract, embodiments 1-88, and claims 1-12 | 1-23 |
| A | CN 107847497 A (DAIICHI-SANKYO CO., LIMITED et al.) 27 March 2018 (2018-03-27) entire document | 1-23 |
| A | CN 110191722 A (DAIICHI-SANKYO CO., LIMITED et al.) 30 August 2019 (2019-08-30) entire document | 1-23 |
| A | CN 111989325 A (CONSTELLATION PHARMACEUTICALS INC.) 24 November 2020 (2020-11-24) entire document | 1-23 |
| A | WO 2015077194 A1 (BRISTOL-MYERS SQUIBB COMPANY) 28 May 2015 (2015-05-28) entire document | 1-23 |
| A | WO 2021180235 A1 (SICHUAN HAISCO PHARMACEUTICAL CO., LTD.) 16 September 2021 (2021-09-16) entire document | 1-23 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **07 February 2023** | **20 February 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| Facsimile No. **(86-10)62019451** | Telephone No. |

Form PCT/ISA/210 (second sheet) (July 2022)

**INTERNATIONAL SEARCH REPORT**
**Information on patent family members**

International application No.
**PCT/CN2022/139254**

| Patent document cited in search report | Publication date (day/month/year) | Patent family member(s) | Publication date (day/month/year) |
|---|---|---|---|
| CN 106132954 A | 16 November 2016 | SI 3121175 T1 | 31 July 2020 |
| | | WO 2015141616 A1 | 24 September 2015 |
| | | TW 201620900 A | 16 June 2016 |
| | | TWI 648275 B | 21 January 2019 |
| | | PL 3121175 T3 | 01 June 2020 |
| | | DK 3121175 T3 | 09 March 2020 |
| | | EP 3121175 A1 | 25 January 2017 |
| | | EP 3121175 A4 | 02 August 2017 |
| | | EP 3121175 B1 | 04 December 2019 |
| | | NZ 723312 A | 25 October 2019 |
| | | JP 5806438 B1 | 10 November 2015 |
| | | JPWO 2015141616 A1 | 06 April 2017 |
| | | CA 2942883 A1 | 24 September 2015 |
| | | CA 2942883 C | 04 December 2018 |
| | | PH 12016501813 B1 | |
| | | PH 12016501813 A1 | |
| | | HUE 049417 T2 | 28 September 2020 |
| | | RU 2016140420 A | 18 April 2018 |
| | | RU 2016140420 A3 | 08 August 2018 |
| | | RU 2679131 C2 | 06 February 2019 |
| | | RS 60022 B1 | 30 April 2020 |
| | | HRP 20200354 T1 | 12 June 2020 |
| | | PT 3121175 T | 05 March 2020 |
| | | BR 112016020621 A2 | 15 August 2017 |
| | | BR 112016020621 B1 | 07 June 2022 |
| | | US 2017073335 A1 | 16 March 2017 |
| | | US 10017500 B2 | 10 July 2018 |
| | | LT 3121175 T | 10 March 2020 |
| | | ZA 201605622 B | 27 September 2017 |
| | | KR 20160132391 A | 18 November 2016 |
| | | KR 102302830 B1 | 15 September 2021 |
| | | MY 192603 A | 29 August 2022 |
| | | US 2018282313 A1 | 04 October 2018 |
| | | US 10954219 B2 | 23 March 2021 |
| | | AU 2015232543 A1 | 15 September 2016 |
| | | AU 2015232543 A2 | 31 August 2017 |
| | | AU 2015232543 A9 | 26 September 2019 |
| | | AU 2015232543 B2 | |
| | | MX 2016011825 A | 16 December 2016 |
| | | MX 370676 B | 19 December 2019 |
| | | IL 247836 A0 | 30 November 2016 |
| | | IL 247836 B | 31 December 2019 |
| | | SG 11201607006 TA | 28 October 2016 |
| | | ES 2774900 T3 | 23 July 2020 |
| CN 107847497 A | 27 March 2018 | WO 2017018499 A1 | 02 February 2017 |
| | | HK 1253319 A1 | 14 June 2019 |
| | | AU 2016299614 A1 | 08 February 2018 |
| | | AU 2016299614 B2 | 07 May 2020 |
| | | KR 20180032596 A | 30 March 2018 |
| | | US 2018200238 A1 | 19 July 2018 |

Form PCT/ISA/210 (patent family annex) (July 2022)

**INTERNATIONAL SEARCH REPORT**
**Information on patent family members**

International application No.
**PCT/CN2022/139254**

| Patent document cited in search report | Publication date (day/month/year) | Patent family member(s) | Publication date (day/month/year) |
|---|---|---|---|
| | | US 10434091 B2 | 08 October 2019 |
| | | CA 2993605 A1 | 02 February 2017 |
| | | CA 2993605 C | 07 January 2020 |
| | | TW 201713335 A | 16 April 2017 |
| | | TWI 711451 B | 01 December 2020 |
| | | ES 2828961 T3 | 28 May 2021 |
| | | BR 112018001688 A2 | 18 September 2018 |
| | | EP 3329917 A1 | 06 June 2018 |
| | | EP 3329917 A4 | 13 March 2019 |
| | | EP 3329917 B1 | 02 September 2020 |
| CN 110191722 A | 30 August 2019 | KR 20190105602 A | 17 September 2019 |
| | | CA 3050221 A1 | 26 July 2018 |
| | | CA 3050221 C | 28 September 2021 |
| | | WO 2018135556 A1 | 26 July 2018 |
| | | EP 3572095 A1 | 27 November 2019 |
| | | EP 3572095 A4 | 18 November 2020 |
| | | BR 112019014924 A2 | 31 March 2020 |
| | | US 2019343816 A1 | 14 November 2019 |
| | | US 11311524 B2 | 26 April 2022 |
| | | AU 2018210099 A1 | 15 August 2019 |
| | | JPWO 2018135556 A1 | 14 November 2019 |
| | | JP 7125353 B2 | 24 August 2022 |
| | | TW 201831181 A | 01 September 2018 |
| CN 111989325 A | 24 November 2020 | PE 20201448 A1 | 10 December 2020 |
| | | MA 52288 A | 07 April 2021 |
| | | CO 2020014217 A2 | 08 March 2021 |
| | | TW 202003505 A | 16 January 2020 |
| | | PH 12020551578 A1 | 13 September 2021 |
| | | AR 114793 A1 | 14 October 2020 |
| | | EP 3781561 A1 | 24 February 2021 |
| | | CR 20200553 A | 08 April 2021 |
| | | CL 2020002698 A1 | 15 January 2021 |
| | | KR 20210003160 A | 11 January 2021 |
| | | WO 2019204490 A1 | 24 October 2019 |
| | | EA 202092490 A1 | 23 December 2020 |
| | | US 2019322651 A1 | 24 October 2019 |
| | | US 10689371 B2 | 23 June 2020 |
| | | IL 278013 A | 30 November 2020 |
| | | SG 11202009438 UA | 27 November 2020 |
| | | CA 3098428 A1 | 24 October 2019 |
| | | AU 2019255310 A1 | 15 October 2020 |
| | | AU 2019255310 B2 | 24 November 2022 |
| | | US 2020317651 A1 | 08 October 2020 |
| | | US 11274095 B2 | 15 March 2022 |
| | | BR 112020021194 A2 | 23 March 2021 |
| | | JP 2021522166 A | 30 August 2021 |
| WO 2015077194 A1 | 28 May 2015 | US 2016297805 A1 | 13 October 2016 |
| | | US 9822103 B2 | 21 November 2017 |
| WO 2021180235 A1 | 16 September 2021 | TW 202144341 A | 01 December 2021 |

Form PCT/ISA/210 (patent family annex) (July 2022)